# FASCICULE DE BREVET EUROPEEN

(11) **EP 1 124 779 B1**
(45) Date de publication et mention de la délivrance du brevet: **02.02.2005**
(21) Numéro de dépôt: 99950869.0
(22) Date de dépôt: 28.10.1999
(51) Int. Cl.: C07C 39/21, C07C 33/26, C07C 33/28, C07C 39/15, C07C 43/166, C07C 43/23, C07C 217/86, A61K 31/05, A61K 31/045, A61K 31/075, A61K 31/085, A61K 7/48

(54) **ANALOGUES DE LA VITAMINE D**
VITAMIN D-ANALOGA
VITAMIN D ANALOGUES

(30) Priorité: 02.11.1998 FR 9813747
(43) Date de publication de la demande: 22.08.2001
(73) Titulaire: Galderma Research & Development, S.N.C., 06560 Valbonne (FR)
(72) Inventeur: BERNARDON, Jean-Michel, F-06650 Le Rouret (FR)
(74) Mandataire: Andral, Christophe André Louis
(86) Numéro de dépôt international: PCT/FR1999/002637
(87) Numéro de publication internationale: WO 2000/026167

(56) Documents cités:
- EP-A- 0 776 881
- EP-A- 0 850 909

## Description

L'invention concerne, à titre de produits industriels nouveaux et utiles, des composés biaromatiques analogues de la vitamine D. Elle concerne également leur procédé de préparation et leur utilisation dans des compositions pharmaceutiques destinées à un usage en médecine humaine ou vétérinaire, ou bien encore dans des compositions cosmétiques.

Les composés selon l'invention ont une activité marquée dans les domaines de la prolifération et de la différenciation cellulaire et trouvent des applications plus particulièrement dans le traitement topique et systémique des affections dermatologiques (ou autres) liées à un désordre de la kératinisation, des affections à composante inflammatoire et/ou immunoallergique et de l'hyperprolifération des tissus d'origine ectodermique (peau, épithélium), qu'elle soit bénigne ou maligne. Ces composés peuvent en outre être utilisés pour lutter contre le vieillissement de la peau, qu'il soit photoinduit ou chronologique et traiter les troubles de la cicatrisation.

On peut également utiliser les composés selon l'invention dans des compositions cosmétiques pour l'hygiène corporelle et capillaire.

La vitamine D est une vitamine essentielle pour la prévention et le traitement des défauts de minéralisation du cartilage (rachitisme), et de l'os (ostéomalacie), et même de certaines formes d'ostéoporose chez le sujet âgé. Mais, il est maintenant admis que ses fonctions s'étendent bien au delà de la régulation du métabolisme osseux et de l'homéostasie calcique. Parmi celles-ci, peuvent être citées ses actions sur la prolifération et sur la différenciation cellulaire et le contrôle des défenses immunitaires. Leur découverte a ouvert la voie à de nouvelles approches thérapeutiques en dermatologie, cancérologie, ainsi que dans le domaine des maladies auto-immunes et celui des transplantations d'organes ou de tissus.

Un apport thérapeutique efficace s'est longtemps heurté à la toxicité de cette vitamine (hypercalcémie parfois mortelle).

Il est notamment connu de l'art antérieur des composés biaromatiques portant un groupe adamantyl en para (EP 0 776 881 A1 ) et des composés stilbéniques portant un groupe adamantyl (EP 0 850 909 A1) ayant une activité dans des domaines de la différenciation et de la prolifération cellulaire.

C'est un des buts de la présente invention de proposer de nouveaux composés analogues structuraux de la vitamine D qui montrent une activité sélective sur la prolifération et sur la différenciation cellulaire sans présenter de caractère hypercalcimiant.

Un autre but de la présente invention est de proposer de nouveaux composés analogues de la vitamine D qui soient plus facilement synthétisables et donc plus économiques par rapport à ce qui était connu antérieurement.

Ainsi, la présente invention concerne des composés qui peuvent être représentés par la formule générale (I) suivante : 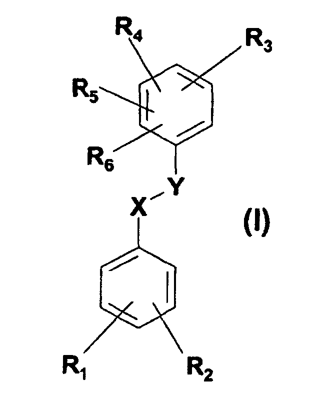 dans laquelle:
- R₁ représente un atome d' hydrogène, un radical méthyl ou un radical -(CH₂)ₙ-OR₇,
- R₂ représente un radical -(CH₂)ₙ-OR₈,
   n, R₇ et R₈ ayant les significations données ci-après,
- X-Y représente une liaison choisie parmi les liaisons de formules (a) et (b) suivantes pouvant être lues de gauche à droite ou inversement : 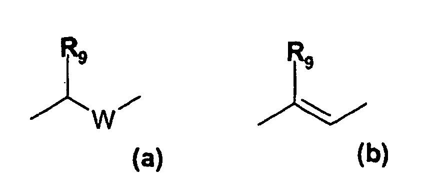 R₉ et W ayant les significations données ci-après,
- R₃ représente la chaîne de la vitamine D₂ ou de la Vitamine D₃, 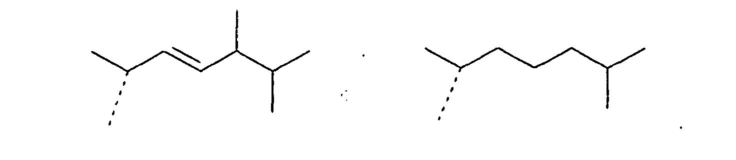 les traits en pointillés représentent la liaison reliant la chaîne au cycle benzénique représenté sur la figure (I),
   ou R₃ représente une chaîne ayant de 4 à 8 atomes de carbone substituée par un ou plusieurs groupements hydroxyles, les groupements hydroxyles pouvant être protégés sous forme d'acétoxy, de méthoxy ou d'éthoxy, de triméthylsilyloxy, de tertiobutyldiméthylsilyloxy, de tétrahydropyranyloxy et éventuellement en outre :
   - substituée par un ou plusieurs groupements alkyles linéaires ou ramifiés de 1 à 6 atomes de carbone ou cycloalkyles et/ou
   - substituée par un ou plusieurs atomes d'halogène et/ou
   - substituée par un ou plusieurs groupements CF₃ et/ou
   - dans laquelle un ou plusieurs atomes de carbone de la chaîne sont remplacés par un ou plusieurs atomes d'oxygène, de soufre ou d'azote, les atomes d'azote pouvant éventuellement être substitués par des radicaux alkyles inférieurs et/ou
   - dans laquelle un ou plusieurs atomes de carbone de la chaîne sont remplacés par un ou plusieurs atomes d'oxygène, de soufre ou d'azote, les atomes d'azote pouvant éventuellement être substitués par des radicaux alkyles linéaires ou ramifiés de 1 à 6 atomes de carbone et/ou
   - dans laquelle une ou plusieurs liaisons simples de la chaîne sont remplacées par une ou plusieurs liaisons doubles et/ou triples,
- R₃ étant positionné sur le cycle benzénique en *para* ou *méta* de la liaison X-Y,
- R₄, R₅ et R₆, identiques ou différents, représentent un atome d'hydrogène, un radical alkyle inférieur, un atome d'halogène, un radical -OR₁₀, un radical polyéther de 2 à 5 atomes de carbone interrompus par au moins 2 atomes d'oxygéne,
   R₁₀ ayant la signification donnée ci-après,
- n étant 0,1 ou 2,
- R₇ et R₈ identiques ou différents, représentent un atome d'hydrogène, un radical acétyle, un radical triméthylsilyle, un radical tertiobutyldiméthylsilyle, un radical tétrahydropyranyle,
- R₉ représente un atome d'hydrogène ou un radical alkyle linéaires ou ramifiés de 1 à 6 atomes de carbone,
- W représente un atome d'oxygène, de soufre, un radical -CH₂- ou un radical -NH- pouvant éventuellement être substitué par un radical alkyle linéaires ou ramifiés de 1 à 6 atomes de carbone,
- R₁₀ représente un atome d'hydrogène ou un radical alkyle linéaires ou ramifiés de 1 à 6 atomes de carbone,

L'invention vise également les isomères optiques et géométriques desdits composés de formule (I) ainsi que leurs sels dans le cas où X-Y représentent une liaison de formule (a) et W représente un radical -NH- éventuellement substitué par un radical alkyle linéaires ou ramifiés de 1 à 6 atomes de carbone.

Lorsque les composés selon l'invention se présentent sous forme de sels, par addition d'un acide, il s'agit de sels pharmaceutiquement ou cosmétiquement acceptables obtenus par addition d' un acide minéral ou organique, en particulier l'acide chlorhydrique, sulfurique, acétique, fumarique, hémisuccinique, maléique et mandélique.

Selon la présente invention on entend par radical alkyle inférieur, un radical linéaire ou ramifié ayant de 1 à 6 atomes de carbone, et de préférence, les radicaux méthyle, éthyle, isopropyle, tertiobutyle et hexyle.
Par radical cycloalkyle, on entend un radical alcane cyclique ou polycyclique contenant de 3 à 10 atomes de carbone. De préférence le radical cycloalkyle est choisi parmi un radical adamantyle ou un radical 1-méthylcyclohexyle.

Par atome d'halogène, on entend de préférence un atome de fluor, de chlore et de brome.

Par radical polyéther, on entend un radical ayant de 2 à 5 atomes de carbone interrompu par au moins deux atomes d'oxygène tels que les radicaux méthoxyméthoxy, méthoxyéthoxy et méthoxyéthoxyméthoxy.

Parmi les composés de formule (I) rentrant dans le cadre de la présente invention, on peut notamment citer les suivants :
3-hydroxyméthyl-5-{2-[3-(5-hydroxy-5-méthyl-hexyl)-phényl]-vinyl}-phenol,
3-hydroxyméthyl-5-{2-[3-(6-hydroxy-6-méthyl-heptyl)-phényl]-vinyl}-phenol,
3-[3-(5-hydroxy-1,5-diméthyl-hexyl)-phenoxyméthyl]-5-hydroxyméthyl-phenol,
6-[3-(3,4-bis-hydroxyméthyl-benzyloxy)-phényl]-2-méthyl-hepta-3,5-dien-2-ol,
6-[3-(3,4-bis-hydroxyméthyl-benzyloxy)-phényl]-2-méthyl-hexan-2-ol,
6-[3-(3,4-bis-hydroxyméthyl-phenoxyméthyl)-phényl]-2-méthyl-heptan-2-ol,
7-[3-(3,4-bis-hydroxyméthyl-phénoxyméthyl)-phényl]-3-éthyl-octan-3-ol,
5-{2-[4-(5-hydroxy-5-méthyl-hexyl)-phényl]-vinyl}-benzène-1,3-diol,
5-{2-[4-(5-hydroxy-5-méthyl-hexyl)-phényl]-éthyl}-benzène-1,3 diol,
5-{2-[4-(6-hydroxy-6-méthyl-heptyl)-phényl]vinyl}-benzene-1,3-diol,
5- {2-[3-(6-hydroxy-6-méthyl-hepty])-phényl]-vinyl}-benzene-1,3-diol,
5-{2-[4-(6-hydroxy-6-méthyl-heptyl)-phényl]-éthyl}-benzène-1,3-diol,
5- {2-[3-(6-hydroxy-6-méthyl-heptyl)-phényl]-éthyl}-benzène-1,3-diol,
2-hydroxyméthyl-4-{2-[3-(5-hydroxy-5-méthyl-hexyl)-phényl]-vinyl}-phenol,
2-hydroxyméthyl-4-{2-[4-(5-hydroxy-5-méthyl-hexyl)-phényl]-vinyl}-phénol,
2-hydroxyméthyl-4-{2-[3 -(6-hydroxy-6-méthyl-heptyl)-phényl]-vinyl}-phénol,
2-hydroxyméthyl-4-{2-[4-(6-hydroxy-6-méthyl-heptyl)-phényl]-vinyl}-phénol,
2-hydroxyméthyl-4-{2-[3-(5-hydroxy-5-méthyl-heptyl)-phényl]-éthyl}-phénol,
2-hydroxyméthyl-4-{2-[4-(5-hydroxy-5-méthyl-hexyl)-phényl]-éthyl}-phénol,
2-hydroxyméthyl-4-{2-[3-(6-hydroxy-6-méthyl-heptyl)-phényl]-éthyl}-phénol,
2-hydroxyméthyl-4-{2-[4-(6-hydroxy-6-méthyl-heptyl)-phényl]-éthyl}-phénol,
2-hydroxyméthyl-5-{2-[4-(5-hydroxy-5-méthyl-hexyl)-phényl]-vinyl}-phénol,
6-[3-(3,4-bis-hydroxyméthyl-benzyloxy)-phényl]-2-méthyl-heptan-2-ol,
4-[3-(5-hydroxy-1,5-diméthyl-hexyl)-phenoxyméthyl]-2-hydroxyméthyl-phenol,
6-{3-[2-(3,4-bis-hydroxyméthyl-phényl)-vinyl]-phényl}-2-méthyl-hexan-2-ol,
7-{4-[2-(3,4-bis-hydroxyméthyl-phényl)-vinyl]-phényl}-2-méthyl-heptan-2-ol,
6-{4-[2-(3,4-bis-hydroxyméthyl-phényl)-vinyl]-phényl}-2-méthyl-hexan-2-ol,
5-{2-[3-(6-hydroxy-6-méthyl-heptyl)-phényl]-1-méthyl-vinyl}-benzène-1,3-diol,
5-{2-[3-(5-hydroxy-5-méthyl-hexyl)-phényl]-vinyl}-benzène-1,3-diol,
5-[3-(6-hydroxy-6-méthyl-heptyl)-phénoxyméthyl]-benzène-1,3-diol,
5-{2-[3-(7-hydroxy-7-méthyl-oct-1-enyl)-phényl]-vinyl}-benzene-1,3-diol,
5-{2-[3-(7-hydroxy-7-méthyl-octyl)-phényl]-vinyl}-benzène-1,3-diol,
4-{2-[3-(6-hydroxy-6-méthyl-heptyl)-phényl]-vinyl}-benzène-1,2-diol,
3-{2-[3-(6-hydroxy-6-méthyl-heptyl)-phényl]-vinyl}-phénol,
6-{3-[2-(3,5-bis-hydroxyméthyl-phényl)-vinyl]-phényl}-2-méthyl-hexan-2-ol,
3-{2-[3-(7-hydroxy-7-méthyl-octyl)-phényl]-vinyl}-phénol,
7-{3-[2-(3,5-bis-hydroxyméthyl-phényl)-vinyl]-phényl}-2-méthyl-heptan-2-ol,
7-{3-[2-(3,4-bis-hydroxyméthyl-phényl)-vinyl]-phényl}-2-méthyl-heptan-2-ol,
7-{3-[2-(4-hydroxyméthyl-phényl)-vinyl]-phényl}-2-méthyl-heptan-2-ol,
4-{2-[3-(7-hydroxy-7-méthyl-oct-1-enyl)-phényl]-vinyl}-benzene-1,2-diol,
5-{2-[3-(6-hydroxy-6-méthyl-hept-1-enyl)-phényl]-vinyl}-benzene-1,3-diol,
5-{2-[3-(7-éthyl-7-hydroxy-non-1-enyl)-phényl]-vinyl}-benzène-1,3-diol,
5-{2-[3-(7-hydroxy-1-méthoxy-1,7-diméthyl-octyl)-phényl]-vinyl}-benzène-1,3-diol,
5-{2-[3-(6-hydroxy-1-méthoxy-1,6-diméthyl-heptyl)-phényl]-vinyl}-benzène-1,3-diol,
5-{2-[3-(5-hydroxy-pentyl)-phényl]-vinyl}-benzène-1,3-diol,
5-{2-[3-(5-hydroxy-6-méthyl-heptyl)-phényl]-vinyl}-benzène-1,3-diol,
5-{2-[3-(6-hydroxy-7-méthyl-octyl)-phényl]-vinyl}-benzène-1,3-diol,
5-{2-[3-(5-hydroxy-6-méthyl-hept-1-enyl)-phényl]-vinyl}-benzène-1,3-diol,
5-{2-[3-(6-hydroxy-7-méthyl-oct-1-enyl)-phényl]-vinyl}-benzène-1,3-diol,
5-{2-[3-(1,6-dihydroxy-1,6-diméthyl-heptyl)-phényl]-vinyl}-benzène-1,3-diol,
5-{2-[3-(6-hydroxy-1,6-diméthyl-hept-1-enyl)-phényl]-vinyl}-benzène-1,3-diol,
6-{[3-(3,4-Bis-hydroxyméthyl-benzyloxy)-phenyl]-méthyl-amino}-2-méthyl-hexan-2-ol,
5-{[3-(3,4-Bis-hydroxyméthyl-benzyloxy)-phenyl]-méthyl-amino}-2-méthyl-pentan-2-ol,
6-{[3-(3,4-Bis-hydroxyméthyl-benzyloxy)-phenyl]-méthyl-amino}-3-éthyl-hexan-3-ol,
7-{[3-(3,4-Bis-hydroxyméthyl-benzyloxy)-phenyl]-méthyl-amino}-3-éthyl-heptan-3-ol,
5-{[3-(3,4-Bis-hydroxyméthyl-benzyloxy)-phenyl]-éthyl-amino}-2-méthyl-pentan-2-ol,
6-{[3-(3,4-Bis-hydroxyméthyl-benzyloxy)-phenyl]-benzyl-amino}-3-éthyl-hexan-3-ol,
(4E,6E)-7-{3-[2-(3,4-Bis-hydroxyméthyl-phenyl)-éthyl]-phenyl}-3-éthyl-octa-4,6-dien-3-ol,
(3E,5E)-6-{3-[2-(3,4-Bis-hydroxyméthyl-phenyl)-éthyl]-phenyl}-2-méthyl-hepta-3,5-dien-2ol,
(4E,6E)-7-{3-[2-(3,4-Bis-hydroxyméthyl-phenyl)-vinyl]-phenyl}-3-éthyl-octa-4,6-dien-3-ol,
(3E,5E)-6-{3-[2-(3,4-Bis-hydroxyméthyl-phenyl)-vinyl]-phenyl}-2-méthyl-hepta-3,5-dien-2-ol,
7-[3-(3,4-Bis-hydroxyméthyl-benzyloxy)phényl]-3-éthyl-3-octanol,
(4E,6E)-7-[3-(3,4-Bis-hydroxyrnéthyl-benzyloxy)-phenyl]-3-éthyl-octa-4,6-dien-3-ol,
(4E,6Z)-7-[3-(3,4-Bis-hydroxyméthyl-benzyloxy)-phenyl]-3-éthyl-octa-4,6-dien-3-ol,
7-[4-(3,4-Bis-hydroxyméthyl-benzyloxy)phényl]-3-éthyl-3-octanol,
(4E,6E)-7-[3-(3,4-Bis-hydroxyméthyl-benzyloxy)-phenyl]-3-éthyl-nona-4,6-dien-3-ol,
(4E,6Z)-7-[3-(3,4-Bis-hydroxyméthyl-benzyloxy)-phenyl]-3-éthyl-nona-4,6-dien-3-ol,
(E)-6-[3-(3,4-Bis-hydroxyméthyl-benzyloxy)-phenyl]-2-méthyl-hept-3-en-2-ol,
(E)-7-[3-(3,4-Bis-hydroxyrnéthyl-benzyloxy)-phenyl]-3-éthyl-oct-4-en-3-ol,
(E)-7-[3-(3,4-Bis-hydroxyrnéthyl-benzyloxy)-phenyl]-3-éthyl-oct-6-en-3-el,
(Z)-7-[3-(3,4-Bis-hydroxyméthyl-benzyloxy)-phenyl]-3-éthyl-oct-6-en-3-ol,
(E)-8-[3-(3,4-Bis-hydroxyméthyl-benzyloxy)-phenyl]-2-méthyl-non-7-en-2-ol,
(Z)-8-[3-(3,4-Bis-hydroxyméthyl-benzyloxy)-phenyl]-2-méthyl-non-7-en-2-ol,
(E)-9-[3-(3,4-Bis-hydroxyrnéthyl-benzyloxy)-phenyl]-3-éthyl-dec-8-en-3-ol,
(Z)-9-[3-(3,4-Bis-hydroxyrnéthyl-benzyloxy)-phenyl]-3-éthyl-dec-8-en-3-ol,
8-[3-(3,4-Bis-hydroxyméthyl-benzyloxy)phényl]-2-méthyl-2-nonanol,
9-[3-(3,4-Bis-hydroxyméthyl-benzyloxy)-phenyl]-3-éthyl-decan-3-al,
(E)-7-[3-(3,4-Bis-hydroxyrnéthyl-benzyloxy)-phenyl]-3-éthyl-oct-6-en-4-yn-3-ol,
(3E,5E)-7-[3-(3,4-Bis-hydroxyméthyl-benzyloxy)-phenyl]-2,7-diméthyl-octa-3,5-dien-2-ol,
(4E,6E)-7-[3-(3,4-Bis-hydroxyméthyl-phenoxyméthyl)-phenyl]-3-éthyl-octa-4,6-dien-3-ol,
(3E,5E)-6-[3-(3,4-Bis-hydroxyméthyl-phenoxyméthyl)-phenyl]-2-méthyl-hepta-3,5-dien-2-ol,
(Z)-7-[3-(3,4-Bis-hydroxyméthyl-benzyloxy)-phenyl]-3-éthyl-non-6-en-3-ol,
(Z)-6-[3-(3,4-Bis-hydroxyméthyl-benzyloxy)-phenyl]-2-méthyl-oct-5-en-2-ol,
(Z)-7-[3-(3,4-Bis-hydroxyrnéthyl-benzyloxy)-phenyl]-3-éthyl-dec-6-en-3-ol,
(Z)-6-[3-(3,4-Bis-hydroxytnéthyl-benzyloxy)-phenyl]-2-méthyl-non-5-en-2-ol,
(Z)-8-[3-(3,4-Bis-hydroxyrnéthyl-benzyloxy)-phenyl]-3-éthyl-non-7-en-3-ol,
(E)-8-[3-(3,4-Bis-hydroxyméthyl-benzyloxy)-phenyl]-3-éthyl-non-7-en-3-ol,
8-[3-(3,4-Bis-hydroxyméthyl-benzyloxy)-phenyl]-3-éthyl-nonan-3-ol,
(4E,6E)-7-[5-(3,4-Bis-hydroxyrnéthyl-benzyloxy)-2-methoxy-phenyl]-3-éthyl-nona-4,6-dien-3-ol,
(4E,6E)-7-[5-(3,4-Bis-hydroxyméthyl-benzyloxy)-2-methyl-phenyl]-3-éthyl-nona-4,6-dien-3-ol,
(4E,6E)-7-[3-(3,4-Bis-hydroxyméthyl-benzyloxy)-5-methoxy-phenyl]-3-éthyl-nona-4,6-dien-3-ol,
(4E,6E)-7-[3-(3,4-Bis-hydroxyznéthyl-benzyloxy)-2-methoxy-phenyl]-3-éthyl-nona-4,6-dien-3-ol,
(4E,6E)-7-[3-(3,4-Bis-hydroxyméthyl-benzyloxy)-4-methyl-phenyl]-3-éthyl-nona-4,6-dien-3-ol,
1-[3-(3,4)-Bis-hydroxymethyl-benzyloxy)-phenyl]ethanone O -(2-hydroxy-2-methyl-propyl)-oxime,
1-{1-[3-(3,4-Bis-hydroxyméthyl-benzyloxy)-phenyl]-propoxy}-3-éthyl-pentan-3-ol,
(E)-7-[3-(3,4-Bis-hydroxyméthyl-phenoxyméthyl)-phenyl]-3-éthyl-non-6-en-3-ol,
(E)-7-[3-(3,4-Bis-hydroxyméthyl-benzylsulfanyl)-phenyl]-3-éthyl-oct-6-en-3-ol,
(E)-7-{3-[(3,4-Bis-hydroxyméthyl-benzyl)-methylamino]-phenyl}-3-éthyl-oct-6-en-3-ol,
(E)-6-[3-(3,4-Bis-hydroxyrnéthyl-benzyloxy)-phenyl]-3-ethyl-6-methyl-hept-4-en-3-ol,
7-[3-(3,4-Bis-hydroxyméthyl-benzyloxy)-phenyl]-3-ethyl-7-methyl-octan-3-ol,
7-[3-(3,4-Bis-hydroxymethyl-benzyloxy)-phenyl]-3,7-diethyl-nonan-3-ol,
(E)-6-[3-(3,4-Bis-hydroxymethyl-benzyloxy)-phenyl]-1,1,1-thfluoro-2-trifluoromethyl-oct-5-en-2-ol,
2-{4-[3-(3,4-Bis-hydroxymethyl-benzyloxy)-phenyl]-hexyl}-1,1,1,3,3,3-hexafluoro-propan-2-ol,
(E)-7-[3-(3,4-Bis-hydroxymethyl-benzyloxy)-phenyl]-3-ethyl-4,4-difluoro-non-6-en-3-ol,
7-[3-(3,4-Bis-hydroxymethyl-benzyloxy)-phenyl]-3-ethyl-4,4-difluoro-7-methyl-octan-3-ol,
(E)-6-[3-(3,4-Bis-hydroxymethyl-benzyloxy)-phenyl]-2-methyl-oct-5-en-3-ol,
(E)-6-[3-(3,4-Bis-hydroxymethyl-benzyloxy)-phenyl]-2-methyl-oct-5-en-3-ol,
(E)-4-[3-(3,4-Bis-hydroxymethyl-benzyloxy)-phenyl]-1-cyclopropyl-hex-3-en-1-ol,
(E)-7-[3-(3,4-Bis-hydroxymethyl-benzyloxy)-phenyl]-3-ethyl-4-methyl-non-6-en-3-ol,
(E)-7-[3-(3,4-Bis-hydroxymethyl-benzyloxy)-phenyl]-3-ethyl-4-methyl-dec-6-en-3-ol,
(4E,6E)-7-[3-(3,4-Bis-hydroxymethyl-phenoxymethyl)-phenyl]-3-ethyl-nona-4,6-dien-3-ol,
(4E,6E)-7-{3-[2-(3,4-Bis-hydroxymethyl-phenyl)-ethyl]-phenyl}-3-ethyl-nona-4,6-dien-3-ol,
(E)-7-{3-[2-(3,4-Bis-hydroxymethyl-phenyl)-ethyl]-phenyl}-3-ethyl-non-6-en-3-ol,
(E)-3-Ethyl-7-[3-(3-hydroxymethyl-phenoxymethyl)-phenyl]-non-6-en-3-ol,
(E)-3-Ethyl-7-[3-(4-hydroxymethyl-phenoxymethyl)-phenyl]-non-6-en-3-ol,
(E)-3-Ethyl-7-[(E)-3-(3-hydroxymethyl-4-methyl-phenoxymethyl)-phenyl]-non-6-en-3-ol,
(E)-3-Ethyl-7-[(E)-3-(4-hydroxymethyl-3-methyl-phenoxymethyl)-phenyl]-non-6-en-3-ol,
2-{4-[3-(3,4-Bis-hydroxymethyl-benzyloxy)-phenyl]-4-methyl-pentyl}-1,1,1,3,3,3-hexafluoro-propan-2-ol,
7-[3-(3,4-Bis-hydroxymethyl-benzyloxy)-phenyl]-3-ethyl-4,4-difluoro-7-methyl-octan-3-ol,
7-{3-[2-(3,4-Bis-hydroxymethyl-phenyl)-ethyl]-phenyl}-3,7-diethyl-nonan-3-ol
7-[3-(3,4-Bis-hydroxymethyl-benzyloxy)-phenyl]-3-ethyl-7-methyl-octane-3,4-diol
7-{3-[2-(3,4-Bis-hydroxymethyl-phenyl)-ethyl]-phenyl}-3,7-diethyl-nonane-3,4-diol
7-{3-[2-(3,4-Bis-hydroxymethyl-phenyl)-ethyl]-phenyl}-3-ethyl-7-methyl-octane-3,4-diol
(E)-4-{3-[2-(3,4-Bis-hydroxymethyl-phenyl)-ethyl]-phenyl}-1-cyclopropyl-hex-3-en-1-ol
(4E,6E)-7-{3-[2-(3,4-Bis-hydroxymethyl-phenyl)-ethyl]-phenyl}-3-ethyl-4-methyl-nona-4,6-dien-3-ol
(4E,6E)-7-[3-(3,4-Bis-hydroxymethyl-benzyloxy)-phenyl]-3-ethyl-4-methyl-nona-4,6-dien-3-ol
(E)-3-Ethyl-7-[3-(4-hydroxymethyl-3-methyl-phenoxymethyl)-phenyl]-non-6-en-3-ol ,
(E)-3-Ethyl-7-[3-(3-hydroxyrnethyl-4-methyl-phenoxymethyl)-phenyl]-non-6-en-3-ot,
(E)-7-[3-(3,4-Bis-hydroxytnethyl-phenoxymethyl)-phenyl]-3-ethyl-oct-6-en-3-ol,
(E)-3-Ethyl-7-[3-(3-hydroxymethyl-phenoxymethyl)-phenyl]-non-6-en-3-ol,
(E)-3-Ethyl-7-[3-(4-hydroxymethyl-phenoxyrnethyl)-phenyl]-non-6-en-3-ol.

Selon la présente invention, les composés de formule (1) plus particulièrement préférés sont ceux pour lesquels l'une au moins, et de préférence toutes les conditions ci-dessous sont respectées :
- R₁ représente le radical -(CH₂)ₙOH,
- R₂ représente le radical -(CH₂)ₙOH,
- X-Y représente une liaison de formule (a) ou (b),
- R₃ représente une chaîne de 4 à 8 atomes de carbone substituée par au moins un radical hydroxyle et/ou un radical alkyle inférieur.

Selon la présente invention, les composés de formule (I) plus particulièrement préférés sont ceux pour lesquels l'une au moins, et de préférence toutes les conditions ci-dessous sont respectées :
- R₁ représente le radical -(CH₂)ₙOH,
- R₂ représente le radical -(CH₂)ₙOH,
- X-Y représente une liaison de formule (a) ou (b),
- R₃ représente une chaîne de 4 à 8 atomes de carbone substituée par au moins un radical hydroxyle et/ou un radical alkyle inférieur.

La présente invention a également pour objet les procédés de préparation des composés de formule (I).

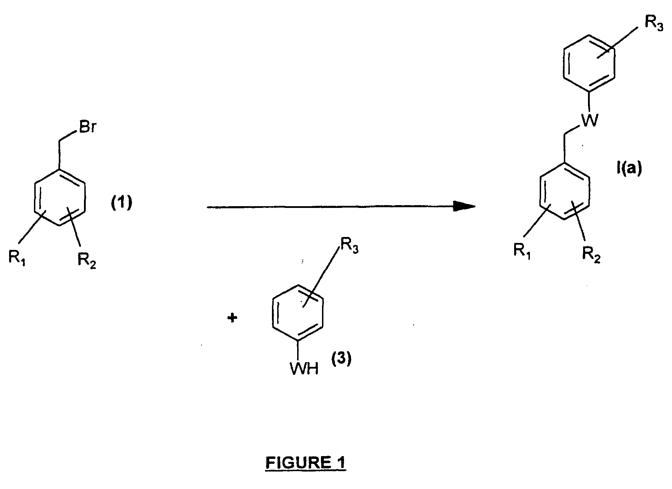

Les figures 1 à 4 représentent des schémas réactionnels qui peuvent être mis en oeuvre pour la préparation des composés selon l'invention.

Ainsi, les composés de formule **I(a)** peuvent être obtenus **(Figure 1)** par réaction d'un composé halogéné de préférence bromé **(1)** avec un dérivé **(3)** phénolique (W=OH), thiophénolique (W=SH), d'aniline (W=NH-COO-tertiobutyl) en présence d'une base telle K₂CO₃ dans un solvant tel l'acétone ou la méthyléthylcétone.

Les composés de composés de formule **I(a)** peuvent être également obtenus **(Figure 1)** par réaction d'un composé halogéné de préférence bromé **(1)** avec le sel de sodium ou de potassium d'un dérivé **(3)** phénolique (W=OH), thiophénolique (W=SH), d'aniline (W=NH-COO-tertiobutyl) dans un solvant tel le diméthylformamide (DMF).

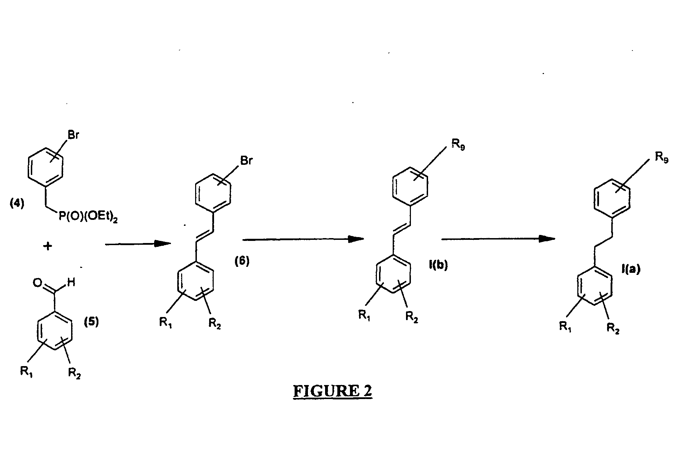

Les composés de formule **I(b)** peuvent être obtenus (**Figure 2**) par une réaction de type Homer-Emmons entre le dérivé phosphonate (4) (obtenu à partir du bromure de benzyle correspondant par une réaction de type Arbuzov) et le benzaldéhyde (5).

Les composés de formule **I(a)** peuvent être obtenus à partir des composés **I(b)** par hydrogénation en présence de palladium sur charbon.

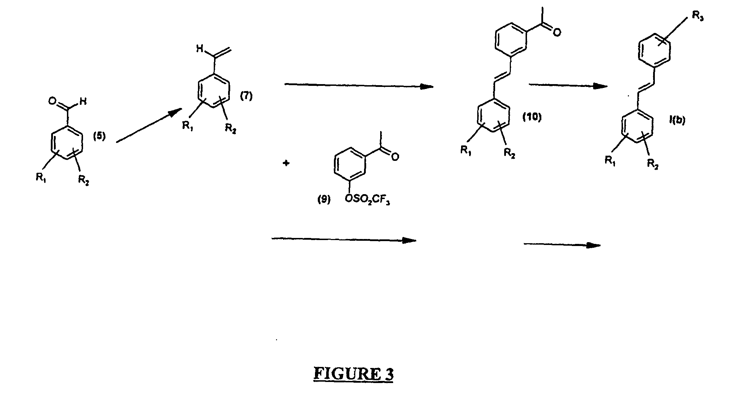

Les composés de formule **I(b)** peuvent être ausi obtenus (**Figure 3**) par une réaction de type Heck entre un dérivé éthylénique **(7)** (obtenu par réaction du benzaldéhyde **(5)** avec le bromure de méthyltriphénylphosphine) et le dérivé triflate **(9)** en présence d'un catalyseur de métaux de transition tel Pd(Cl)₂(PPh₃)₂ dans un solvant tel la triéthylamine.

La chaîne R₃ peut être introduite en utilisant par exemple les méthodes décrites dans Medicinal Research Reviews, Vol 7, N° 2, 147-171 (1987) T. KAMETANI et H. FURUYAMA, Chem. Rev. Vol 78, N° 3, 199-241 (1978) D. M. PIATAK et J. WICHA, ou dans Chem. Rev. Vol 95, N° 6,1877-1952 (1995) G. ZHU et W. H. OKAMURA.

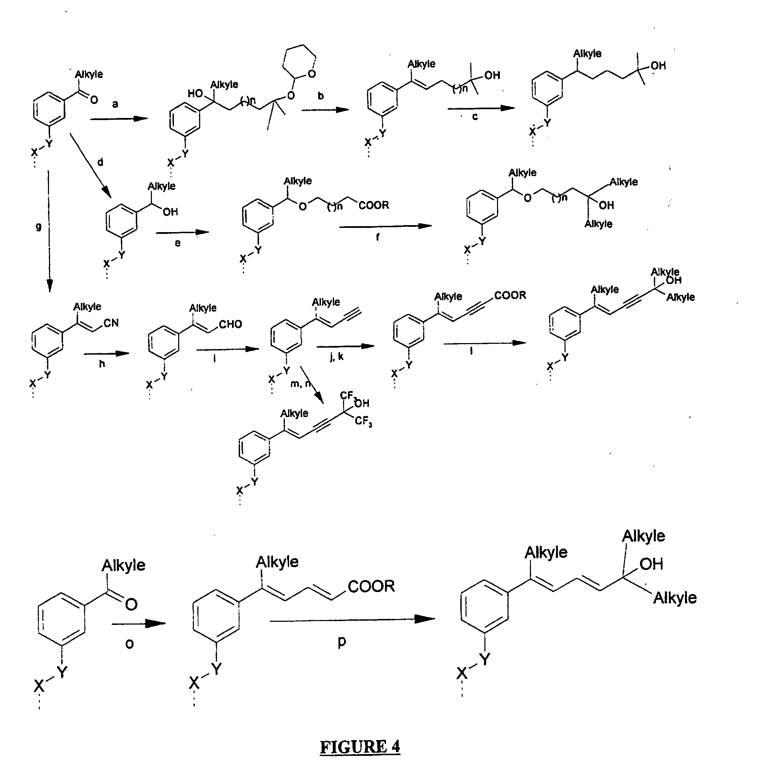

Ainsi, à titre d'exemples, quelques méthodes résumées sont données dans la figure 4 dans laquelle, (a) représente une réaction avec MgBr-CH₂-(CH₂)ₙ-C(CH₃)₂-O-tétrahydmpyrane dans un solvant tel que le tétrahydrofuranne, (b) représente une réaction en présence d'acide paratoluènesulfonique ou d'acide sulfurique, (c) représente une réaction d'hydrogénation en présence d'un catalyseur de palladium sur charbon, (d) représente une réaction de réduction avec du borohydrure de sodium dans un solvant Méthanol-tétrahydrofuranne, (e) représente une réaction avec du Br-CH₂-(CH₂)ₙ-CH₂-COOR en présence d'hydrure de potassium dans un solvant diméthylformamide, (f) représente une réaction avec MgXAlkyle, X représentant un atome d'halogène, dans un solvant tétrahydrofuranne, (g) représente une réaction avec NC-CH₂-P(O)(OC₂H₅)₂ en présence d'hydrure de sodium dans un solvant tétrahydrofuranne, (h) représente une réaction avec de l'hydrure de diisobutylaluminium dans un solvant tétrahydrofuranne, (i) représente une réaction avec du tétrabromure de carbone en présence de triphénylphosphine dans un mélange de tétrahydrofuranne suivi d'une réaction avec du n-butyllithium, (j) représente une réaction avec du n-butyllithium dans du tétrahydrofuranne, (k) représente une réaction avec le chloroforrniate d'alkyle CI-COOR, (1) représente une réaction avec MgXAlkyle, X représentant un atome d'halogène, dans un solvant tétrahydrofuranne, (m) représente une réaction avec du n-butyllithium dans du tétrahydrofuranne, (n) représente une réaction avec CF₃-CO-CF₃, (o) représente une réaction avec ROOC-CH=CH-CH₂-P(O)(OC₂H₅)₂ en présence de diisopropylamidure de lithium dans du tétrahydrofuranne, (p) représente une réaction avec MgXAlkyle, X représentant un atome d'halogène, dans un solvant tétrahydrofuranne.

Les composés de formule générale (I) présentent des propriétés biologiques analogues à celles de la vitamine D, notamment les propriétés de transactivation des éléments de réponse à la vitamine D (VDRE), telles qu'une activité agoniste ou antagoniste vis-à-vis de récepteurs de la vitamine D ou de ses dérivés. Par vitamines D ou leurs dérivés on entend par exemple les dérivés de la vitamine D₂ ou D₃ et en particulier la 1,25-dihydroxyvitamine D₃ (calcitriol).

Cette activité agoniste vis-à-vis de récepteurs de la vitamine D ou de ses dérivés peut être mise en évidence "in vitro" par des méthodes reconnues dans le domaine de l'étude de la transcription génique (Hansen et al., The Society For Investigative Dermatologie, vol.1, N°1, April 1996).

A titre d'exemple, l'activité agoniste VDR peut être testée sur la lignée cellulaire HeLa, par cotransfection d'un vecteur d'expression du récepteur VDR humain et du plasmide rapporteur p240Hase-CAT qui contient la région - 1399 à +76 du promoteur de la 24-hydroxylase de rat, clonée en amont de la phase codante du gène de la chloramphénicol-acétyl-transférase (CAT). 18 heures après cotransfection, le produit test est ajouté dans le milieu. Après 18 heures de traitement, le dosage de l'activité CAT des lysats cellulaires est effectuée par un test Elisa. Les résultats sont exprimés en pourcentage de l'effet normalement observé avec 10⁻⁷ M de calcitriol.

L'activité agoniste peut aussi être caractérisée dans ce système de cotransfection, par la détermination de la dose nécessaire pour atteindre 50 % de l'activité maximale du produit (AC50).

Les propriétés biologiques analogues à la vitamine D peuvent être également mesurées par la capacité du produit à inhiber la prolifération des kératinocytes humaines normaux (KHN en culture). Le produit est ajouté à des KHN cultivés dans des conditions favorisant l'état prolifératif. Le produit est laissé au contact des cellules pendant cinq jours. Le nombre de cellules prolifératives est mesuré par incorporation de Bromodeoxyuridine (BRdU) dans l'ADN.

L'activité agoniste aux récepteurs de la vitamine D des composés de l'invention peut être également évaluée "in vivo" par induction de la 24-Hydroxylase chez la souris SKH. (Voorhees and al. 1997.108 : 513-518). Le protocole de test utilisé est décrit dans l'exemple 108 de la présente demande.

La présente invention a également pour objet à titre de médicament les composés de formule (I) tels que décrits ci-dessus.

Les composés selon l'invention conviennent particulièrement à une utilisation pour la fabrication d'un médicament destiné au traitement suivants :
1) Pour traiter les affections dermatologiques liées à un désordre de la différenciation ou de la prolifération des kératinocytes ou des sébocytes, pour traiter les acnés vulgaires, comédoniennes, polymorphes, rosacées, les acnés nodulokystiques, conglobata, les acnés séniles, les acnés secondaires, l'acné solaire, médicamenteuse ou professionnelle.
2) Pour traiter les ichtyoses, les états ichtyosiformes, la maladie de Darier, les kératodermies palmoplantaires, les leucoplasies et les états leucoplasiformes, le lichen cutané ou muqueux (buccal).
3) Pour traiter des affections dermatologiques liées à un trouble de la kératinisation avec une composante inflammatoire et/ou immuno-allergique, toutes les formes de psoriasis qu'il soit cutané, muqueux ou unguéal, le rhumatisme psoriatique, ou encore l'atopie cutanée, l'eczéma ou l'atopie respiratoire, l'hypertrophie gingivale; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient ou non d'origine virale, les verrues vulgaires, les verrues planes et l'épidermodysplasie verruciforme, les papillomatoses orales ou florides et les proliférations pouvant être induites par les ultra-violets, des épithélioma baso et spinocellulaires.
5) Pour traiter d'autres désordres dermatologiques, les dermatoses bulleuses et les maladies du collagène.
6) Pour réparer ou lutter contre le vieillissement de la peau, qu'il soit photo-induit ou chronologique ou pour réduire les pigmentations et les kératoses actiniques, toutes pathologies associées au vieillissement chronologique ou actinique.
7) Pour prévenir ou traiter les troubles de la cicatrisation ou pour prévenir ou réparer les vergetures.
8) Pour lutter contre les troubles de la fonction sébacée, l'hyperséborrhée de l'acné, la séborrhée simple, l'eczéma séborrhéique.
9) Pour traiter certains troubles ophtalmologiques, les coméopathies.
10) Dans le traitement ou la prévention des états cancéreux ou précancéreux de cancers présentant ou pouvant être induits pour présenter des récepteurs de vitamine D, le cancer du sein, la leucémie, les syndromes myélodysplasiques et les lymphomes, les carcinomes des cellules de l'épithélium malpighien et les cancers gastro-intestinaux, les mélanomes, et l'ostéosarcome.
11) Dans le traitement d'affections inflammatoires, l'arthrite ou la polyarthrite rhumatoïde.
12) Dans le traitement de toute affection d'origine virale au niveau cutané ou général.
13) Dans la prévention ou le traitement de l'alopécie de différentes origines, l'alopécie due à la chimiothérapie ou aux rayonnements;.
14) Dans le traitement d'affections dermatologiques ou générales à composante immunblogique.
15) Dans le traitement d'affections immunitaires, les maladies auto-immunes (le diabète sucré de type 1, la sclérose en plaques, le lupus et les affections de type lupus, l'asthme, la glomérulonéphrite, etc.), des dysfonctionnements sélectifs du système immunitaire (par exemple le SIDA) et la prévention du rejet immun [comme les rejets de greffons (par exemple le rein, le coeur, la moelle osseuse, le foie, des îlots pancréatiques ou tout le pancréas, la peau, etc.) ou la prévention de la maladie du greffon contre l'hôte].
16) Dans le traitement d'affections endocriniennes étant donné que les analogues de la vitamine D peuvent moduler la sécrétion hormonale telle que l'augmentation de la sécrétion d'insuline ou la suppression sélective de la sécrétion de l'hormone parathyroïdienne (par exemple dans l'insuffisance rénale chronique et l'hyperparathyroïdie secondaire).
17) Dans le traitement d'affections caractérisées par une gestion anormale du calcium intracellulaire.
18) Dans le traitement et ou la prévention des carences en vitamine D et d'autres affections de l'homéostasie des minéraux dans le plasma et les os, le rachitisme, l'ostéomalacie, l'ostéoporose, l'ostéodystrophie rénale, les troubles de la fonction parathyroïdienne.

Dans les domaines thérapeutiques mentionnés ci-dessus, les composés selon l'invention peuvent être avantageusement employés en combinaison avec des rétinoïdes, avec des corticostéroïdes ou des oestrogènes, en association avec des anti-oxydants, avec des α-hydroxy ou α-céto acides ou leurs dérivés, avec des bloqueurs de canaux potassiques, ou encore en association avec d'autres médicaments connus pour interférer avec le système immunitaire (par exemple la cyclosporine, le FK 506, les glucocorticoïdes, les anticorps monoclonaux, les cytokines ou les facteurs de croissance ...).

Par rétinoides on entend des ligands des récepteurs RAR ou RXR, soit naturel ou synthétique.

Par antiradicaux libres on entend par exemple l'α-tocophérol, la Super Oxyde Dismutase, l'Ubiquinol ou certains chélatants de métaux.

Par α-hydroxy ou α-céto acides ou leurs dérivés, on entend par exemple les acides lactique, malique, citrique, glycolique, mandélique, tartrique, glycérique, ascorbique, les dérivés d'acide salicylique, ainsi que leurs sels, amides ou esters.

Par bloqueurs de canaux potassiques, on entend par exemple le Minoxidil (2,4-diamino-6-pipéridino-pyrimidine-3-oxyde) et ses dérivés.

La présente invention a également pour objet une composition pharmaceutique comprenant au moins un composé de formule (I) tel que défini ci-dessus.

La présente invention a donc aussi pour objet une telle composition pharmaceutique destinée notamment au traitement des affections susmentionées.

L'administration des composés selon l'invention peut être effectuée par voie entérale, parentérale, topique ou oculaire.

Par voie entérale, les compositions pharmaceutiques peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection. Les composés selon l'invention sont généralement administrés à une dose journalière d'environ 0,001 µg/kg à 1000 µg/kg et de préférence d'environ 0,01 µg/kg à 100 µg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé de formule (I) tel que défini ci-dessus à une concentration de préférence comprise entre 0,0001 et 5 % et de préférence entre 0,001 à 1% par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec les rétinoïdes, avec des corticostéroïdes, en association avec des anti-radicaux libres, avec des α-hydroxy ou α-céto acides ou leurs dérivés, ou encore avec des bloqueurs de canaux ioniques.

Les différents produits pris en association avec les composés de la présente invention étant tels que définis ci-dessus.

La présente invention vise donc également une composition cosmétique contenant, dans un support cosmétiquement acceptable, au moins un composé de formule I tel que défini ci-dessus. Cette composition cosmétique peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé de formule I dans les compositions cosmétiques peut être comprise entre 0,001 et 3 % en poids par rapport au poids total de la composition.

Les compositions pharmaceutiques et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione) ; des agents anti-inflammatoires non stéroïdiens; des caroténoïdes et, notamment le β-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des anti-oxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés ainsi qu'un exemple de test d'évaluation de l'activité biologique de composés de formule (I) selon l'invention.

### EXEMPLE 1

### 3-hydroxyméthyl-5-{2-[3-(5-hydroxy-5-méthyl-hexyl)-phényl]-vinyl}-phenol.

### a) 5-hydroxy-isophthalate de méthyle.

30 ml d'acide sulfurique concentré sont ajoutés, goutte à goutte, à une solution de 54,6 g (0,3 mol) de l'acide 5-hydroxisophtalique dans 500 ml de méthanol. Le milieu réactionnel est porté à reflux pendant 24 heures. Après évaporation, le résidu est repris par de l'acétate d'éthyle, extrait à l'eau. La phase organique est séchée sur sulfate de magnésium anhydre puis concentrée. Le résidu est trituré dans de l'heptane.
Solide blanc. m= 59,8 g. R= 95%. Tf= 162-4°C.
**RMN 1H** (CDCl3): 3,91 (6H, s), 7,71 (2H, d), 8,15-8,16 (1H, t).

### b) Acide 5-hydroxy-isophtalique monométhyl ester.

119 g (2,8 mol) d'hydroxyde de Lithium mono-hydraté sont ajoutés à une solution de 59,7 g (0,28 mol) de 5-hydroxy-isophthalate de méthyle dans 500 ml de THF. Le mélange est porté à reflux pendant 24 heures. A température ambiante, il est versé doucement dans de l'acide chlorhydrique concentré, extrait avec de l'acétate d'éthyle et lavé à l'eau. Après décantation, la phase organique est séchée sur sulfate de magnésium et concentrée. Le résidu est purifié sur colonne de silice (Dichlorométhane 93 - Heptane 7)
Cristaux blancs. m= 40 g. R= 78%. Tf= 238-40°C.
**RMN 1H** (DMSO): 3,87 (3H, s), 7,54-7,58 (2H, m), 7,95-7,96 (1H, t).

### c) 3-hydroxy-5-hydroxyméthyl-benzoate de méthyle.

375 ml de borane 1M/THF sont ajoutés, goutte à goutte, à 0°C, à une solution de 37 g (0,19 mol) de l'acide 5-hydroxy-isophtalique monométhyl ester dans 200 ml de THF. A la fin de l'addition, le milieu est chauffé à 40°C pendant 12 heures. 200 ml d'une solution (1/1) THF/eau sont ajoutés très lentement. Après avoir évaporé le THF, la phase aqueuse restante est extraite par du dichlorométhane, puis du carbonate de potassium est ajouté. Après décantation, la phase organique est lavée à l'eau puis séchée sur sulfate de magnésium et concentrée. Le résidu est filtré sur silice avec de l'acétate d'éthyle.
Cristaux blancs. m= 27 g. R= 78%.
**RMN 1H** (CDCl3): 3,87 (3H, s), 4,62-4,64 (2H, d), 7,09 (1H, s), 7,39-7,41 (1H, t), 7,51 (1H, s), 8,96 (1H, s).

### d) 3-éthoxyméthoxy-5-éthoxyméthoxyméthyl-benzoate de méthyle.

26 g (143 mmol) de 3-hydroxy-5-hydroxyméthyl-benzoate de méthyle dans 200 ml de diméthylformamide sont ajoutés, goutte à goutte, à une solution de 11 g (344 mmol) d'hydrure de sodium (75%) dans 100 ml de DMF. Le milieu est agité pendant 1 heure, puis 29,1 ml (314 mmol) de chlorure de méthoxyméthyle sont additionnés lentement. L'agitation se poursuit toute la nuit. Le milieu est alors versé dans de l'eau glacée et extrait à l'acétate d'éthyle. La phase organique est séchée sur sulfate de magnésium puis concentrée. Le résidu est purifié sur colonne de silice (Dichlorométhane 90 - Heptane 10).
Huile jaune. m= 26,5 g. R= 63%.
**RMN 1H** (CDCl3): 1,19-1,26 (6H, m), 3,61-3,77 (4H, m), 3,91 (3H, s), 4,61 (2H, s), 4,77 (2H, s), 5,26 (2H, s), 7,23 (1H, s), 7,61-762 (1H, c), 7,67 (1H, s).

### e) (3-éthoxyméthoxy-5-éthoxyméthoxyméthyl-phényl)-méthanol.

203 ml d'hydrure de diisobutylaluminium 1M/Toluène sont ajoutés, à -78°C, goutte à goutte, à une solution de 20 g (68 mmol) de 3-éthoxyméthoxy-5-éthoxyméthoxyméthylbenzoate de méthyle dans 150 ml de toluène. La solution est agitée 2 heures à -78°C. Une solution de 32 g de tartrate de sodium dans 300 ml d'eau est, alors, ajoutée. Le solide blanc formé est filtré et le filtrat est évaporé. Le résidu est repris par de l'acétate d'éthyle et versé dans de la glace. La phase organique est lavée plusieurs fois à l'eau, puis séchée sur sulfate de magnésium et concentrée. Le résidu est purifié sur colonne de silice (Acétate d'éthyle 30 - Heptane 70).
Huile jaune. m= 14,5 g. R= 79%.
**RMN 1H** (CDCl3): 1,19-1,26 (6H, m), 1,94-1;98 (1H, OH, t), 3,59-3,76 (4H, m), 4,56 (2H, s), 4,64-4,66 (2H, d), 4,75 (2H, s), 5,22 (2H, s), 6,96-6,99 (3H, m).

### f) 3-éthoxyméthoxy-5-éthoxyméthoxyméthyl-benzaldehyde.

4,9 ml d'acide acétique sont ajoutés, goutte à goutte, à 10°C, à une solution de 13,5 g (0,05 mol) de (3-éthoxyméthoxy-5-éthoxyméthoxyméthyl-phényl)-méthanol, de 28,2 g (0,075 mol) de pyridinium dichromate et 30 g de tamis moléculaire broyé dans 250 ml de dichlorométhane anhydre. Après 15 minutes d'agitation à température ambiante, 300 ml d'éther sont ajoutés. Le précipité formé est filtré sur silice, rincé à l'éther, puis le filtrat est évaporé. Le résidu est purifié sur colonne de silice (Dichlorométhane 20 - Heptane 80).
Huile jaune. m= 10 g. R= 75%.
**RMN 1H** (CDCl3): 1,19-1,27 (6H, m), 3,62-3,78 (4H, m), 4,65 (2H, s), 4,79 (2H, s), 5,28 (2H, s), 7,29 (1H, s), 7,47 (1H, s), 7,52 (1H, s), 9,97 (1H, s).

### g) 3-bromo-[2-(3-éthoxyméthoxy-5-éthoxyméthoxyméthyl-phényl)-vinyl]-phényl.

290 mg (8,9 mmol) d'hydrure de sodium 75% sont ajoutés, par petites quantités, à une solution de 2 g (7,4 mmol) de 3-éthoxyméthoxy-5-éthoxyméthoxyméthyl-benzaldehyde et de 2,7 g (8,8 mmol) de (3-bromo-benzyl)-phosphonate d'éthyle dans 60 ml de THF avec une goutte de 15-Crown-5. Le mélange est agité toute la nuit à température ambiante. Il est ensuite concentré, repris par de l'éther éthylique et lavé plusieurs fois à l'eau. Après décantation, la phase organique est séchée sur sulfate de magnésium et concentrée. Le résidu est purifié sur colonne de silice (Acétate d'éthyle 10 - Heptane 90).
Huile jaune. m= 2.8 g. R= 89%.
**RMN 1H** (CDCl3): 1,18-1,28 (6H, m), 3,63-3,79 (4H, m), 4,60 (2H, s), 4,79 (2H, s), 5,26 (2H, s), 6,96 (1H, s), 7,03-7,04 (2H, d), 7,11-7,16 (1H, d, J= 12,1 Hz), 7,22-7,25 (1H, d, J=7,8 Hz), 7,36-7,42 (2H, m), 7,65-7,66 (1H, t).

### h) 5-{3-[2-(3-éthoxyméthoxy-5-éthoxyméthoxyméthyl-phényl)-vinyl]-phényl}- pentanoate de méthyle.

Une solution de 1,4 g (5,7 mmol) de 9-borabicyclo[3,3,1]nonane dans 15 ml de THF est ajoutée, goutte à goutte, à 0°C, à 420 mg (3,7 mmol) de pent-4-enoate de méthyle. Elle est agitée 5 heures à température ambiante. On ajoute alors 1,25 g (3 mmol) de 3-bromo-[2-(3-éthoxyméthoxy-5-éthoxyméthoxyméthyl-phényl)-vinyl]-phényl dans 10 ml de diméthylformamide, 850 mg (6 mmol) de carbonate de potassium et 150 mg (0,18 mmol) de [1,1'-bis(diphénylphosphino)ferrocene]dichloropalladium. Le mélange est agité à 50°C toute la nuit. Du dichlorométhane est ajouté, la phase organique est alors lavée plusieurs fois à l'eau. Après décantation, elle est séchée sur sulfate de magnésium et concentrée. Le résidu est purifié sur colonne de silice (Acétate d'éthyle 8 - Heptane 92).
Huile jaune. m= 830 mg. R= 61%.
**RMN 1H** ( CDCl3 ): 1,21-1,28 (6H, m), 1,69 (4H, m), 2,35 (2H, m), 2,65 (2H, t), 3,67 (3H, s), 3,63-3,79 (4H, m), 4,60 (2H, s), 4,79 (2H, s), 5,26 (2H, s), 7,07-7,16 (5H, m), 7,25-7,32 (3H, m).

### i) 6-{3-[2-(3-éthoxyméthoxy-5-éthoxyméthoxyméthyl-phényl)-vinyl]-phényl}-2-méthylhexan-2-ol.

3,6 ml (10,8 mmol) de bromure de méthylmagnésium 3M/Éther sont ajoutés, goutte à goutte, à 820 mg (1,8 mmol) de 5-{3-[2-(3-éthoxyméthoxy-5-éthoxyméthoxyméthyl-phényl)-vinyl]-phényl}-pentanoate de méthyle dans 20 ml d'éther éthylique. Le mélange est agité 1 heure à température ambiante. Une solution aqueuse saturée en chlorure d'ammonium est alors très lentement additionnée ainsi que de l'éther. La phase organique est lavée plusieurs fois à l'eau et, après décantation, elle est séchée sur sulfate de magnésium et concentrée. Le résidu est purifié sur colonne de silice (Acétate d'éthyle 25 - Heptane 75).
Huile jaune. m= 600 mg. R= 73%.
**RMN 1H** ( CDCl3 ): 1,22 (6H,s), 1,18-1,25 (6H,m), 1,50-1,60 (6H, m), 2,58-2,66 (2H, m), 3,57-3,86 (4H, m), 4,60 (2H, s), 4,79 (2H, s), 5,27 (2H, s), 6,94 (1H, c), 7,08-7,17 (6H, m), 7,33 (2H, c).

### j) 3-hydroxyméthyl-5-{2-[3-(5-hydroxy-5-méthyl-hexyl)-phényl]-vinyl}-phenol.

Une solution de 0,3 ml d'acide sulfurique concentré dans 5 ml de méthanol est ajoutée à 600 mg (1,3 mmol) de 6-{3-[2-(3-éthoxyméthoxy-5-éthoxyméthoxyméthyl-phényl)-vinyl]-phényl}-2-méthyl-hexan-2-ol dans 5 ml de méthanol et 5 ml de THF. Après 4 heures à température ambiante, de l'eau et de l'acétate d'éthyle sont ajoutés. La phase organique est lavée plusieurs fois à l'eau, puis séchée sur sulfate de magnésium et concentrée. Le résidu est purifié sur colonne de silice (Acétate d'éthyle 50 - Heptane 50).
Cristaux blancs. m= 300 mg. R= 67%. Tf= 108-10°C.
**RMN 1H** (CDCl3): 1,20 (6H, s), 1,38-1,54 (4H, m), 1,59-1,71 (2H, m), 2,61-2,67 (2H, t), 4,61 (2H, s), 6,80 (1H, s), 6,90 (1H, s), 7,04-7,08 (4H, m), 7,21-7,29 (3H, m), 8,74 (1H, OH, s).

### EXEMPLE 2

### 3-hydroxyméthyl-5-{2-[3-(6-hydroxy-6-méthyl-heptyl)-phényl]-vinyl}-phenol.

### a) 6-{3-[2-(3-éthoxyméthoxy-5-éthoxyméthoxyméthyl-phényl)-vinyl]-phényl}-hexanoate de méthyle.

De manière analogue à l'exemple 1(h), par réaction de 1,3 g (3 mmol) de 3-bromo-[2-(3-éthoxyméthoxy-5-éthoxyméthoxyméthyl-phényl)-vinyl]-phényl dans 10 ml de diméthylformamide, avec la solution à 0°C, de 1,4 g (5,7 mmol) de 9-borabicyclo[3,3,1]nonane et 475 mg (3,7 mmol) de hex-5-enoate de méthyle dans 15 ml de THF, une huile jaune (m= 1,08 g ; R= 74%) est obtenue, après purification sur colonne de silice (Acétate d'éthyle 8 - Heptane 92).
**RMN 1H** ( CDCl3 ): 1,21-1,28 (6H, m), 1,64-1,67 (2H, m), 1,85 (4H, m), 2,29-2,35 (2H, t), 2,60-2,66 (2H, t), 3,66 (3H, s), 3,63-3,79 (4H, m), 4,60 (2H, s), 4,79 (2H, s), 5,26 (2H, s), 6,94 (1H, s), 7,08-7,16 (5H, m), 7,23-7,32 (3H, m).

### b) 7-{3-[2-(3-éthoxyméthoxy-5-éthoxyméthoxyméthyl-phényl)-vinyl]-phényl}-2-méthyl-heptan-2-ol.

De manière analogue à l'exemple 1(i), par réaction de 3,8 ml (11,4 mmol) de bromure de méthylmagnésium 3M/Éther avec 1,08 g (2,3 mmol) de 6-{3-[2-(3-éthoxyméthoxy-5-éthoxyméthoxyméthyl-phényl)-vinyl]-phényl}-hexanoate de méthyle dans 20 ml d'éther éthylique, une huile jaune (m= 480 mg ; R= 44%) est obtenue, après purification sur colonne de silice (acétate d'éthyle 30 - heptane 70).
**RMN 1H** ( CDCl3 ): 1,21 (6H, s), 1,22-1,28 (6H, m), 1,40-1,66 (8H, m), 2,60-2,66 (2H, t), 3,63-3,79 (4H, m), 4,60 (2H, s), 4,79 (2H, s), 5,26 (2H, s), 6,93 (1H, s), 7,07-7,16 (5H, m), 7,29-7,32 (3H, m).

### c) 3-hydroxyméthyl-5-{2-[3-(6-hydroxy-6-méthyl-heptyl)-phényl]-vinyl}-phenol.

De manière analogue à l'exemple 1 (j), par réaction de 0,3 ml d'acide sulfurique concentré dans 5 ml de méthanol avec 470 mg (1,0 mmol) de 7-{3-[2-(3-éthoxyméthoxy-5-éthoxyméthoxyméthyI-phényl)-vinyl]-phényl}-2-méthyl-heptan-2-ol dans 5 ml de méthanol et 5 ml de THF, une huile incolore (m= 109 mg ; R= 31%) est obtenue, après purification sur colonne de silice (Acétate d'éthyle 40 - Heptane 60).
**RMN 1H** ( CDCl3 ): 1,21 (6H, s), 1,39-1,66 (8H, m), 2,59-2,65 (2H, t), 4,66 (2H, s), 6,76 (1H, s), 6,90 (1H, s), 7,03-7,09 (4H, m), 7,26-7,30 (3H, m).

### EXEMPLE 3

### 3-[3-(5-hydroxy-1,5-diméthyl-hexyl)-phenoxyméthyl]-5-hydroxyméthyl-phenol.

### a) (3-éthoxyméthoxy-5-éthoxyméthoxyméthyl-phényl)-méthanol.

135 mg (3,55 mmol) de borohydrure de sodium sont ajoutés, par petites quantités, à une solution de 1,9 g (7,1 mmol) de 3-éthoxyméthoxy-5-éthoxyméthoxyméthyl-benzaldehyde dans 30 ml de méthanol et 20 ml de THF. Après avoir été agité 15 minutes à température ambiante, le milieu est versé dans de l'eau et extrait à l'acétate d'éthyle. Après décantation, la phase organique est séchée sur sulfate de magnésium et concentrée.
Huile jaune. m= 1,9 g. R= 100%.
**RMN 1H** ( CDCl3 ): 1,19-1,26 (6H, m), 3,61-3,77 (4H, m), 4,57 (2H, s), 4,66 (2H, s), 4,85 (2H, s), 5,23 (2H, s), 6,96-7,00 (3H, m).

### b) 1-bromométhyl-3-éthoxyméthoxy-5-éthoxyméthoxyméthyl-benzene.

A 0°C, 6,9 ml (15,4 mmol) de trioctylphosphine sont additionnés à une solution de 1,9 g (7 mmol) de (3-éthoxyméthoxy-5-éthoxyméthoxyméthyl-phényl)-méthanol et de 5,1 g (15,4 mmol) de tetrabromure de carbone dans 50 ml d'éther éthylique. Après 15 minutes à 0°C, de l'éther est ajouté et le milieu est lavé plusieurs fois à l'eau. Après décantation, la phase organique est séchée sur sulfate de magnésium et concentrée. Le résidu est purifié sur colonne de silice (AcOEt 8 - Heptane 92).
Huile jaune. m= 1,5 g. R= 64%.
**RMN 1H** (CDCl3): 1,19-1,26 (6H, s), 3,61-3,77 (4H, m), 4,45 (2H, s), 4,54-4,57 (2H, m), 4,76 (2H, s), 5,22 (2H, s), 6,97-7,02 (3H, m).

### c) 1-[3-(tert-butyl-diméthyl-silanyloxy)-phényl]éthanone.

45,2 g (0,3 mol) de chlorure de *tert*-butyldiméthylsilane dans 250 ml de diméthylformamide sont ajoutés, goutte à goutte, à une solution de 34 g (0,25 mol) de 3-hydroxyacétophenone dans 200 ml de DMF avec 38,2 ml (0,27 mol) de triéthylamine et 1,2 g (9,8 mmol) de diméthylaminopyridine. Le mélange est agité 2 heures 30 à température ambiante. Il est ensuite versé dans de l'eau glacée et extrait à l'éther. La phase organique est séchée sur sulfate de magnésium et concentrée. Le résidu est purifié sur colonne de silice (Dichlorométhane 40 - Heptane 60).
Huile orangée. M= 57 g. R= 91%.
**RMN 1H** ( CDCl3 ): 0,01 (6H, s), 0,79 (9H, s), 2,38 (3H, s), 6,82-6,85 (1H, dd), 7,06-7,15 (1H, m), 7,20-7,22 (1H, t), 7,33-7,36 (1H, d, J=7,6 Hz).

### d) 3-[3-(tert-butyl-diméthyl-silanyloxy)-phényl]-but-2-enenitrile.

62 ml (0,38 mol) de diéthylcyanométhylphosphonate dans 200 ml de THF sont ajoutés, goutte à goutte, à 0°C; à une solution de 12,2 g (0,38 mol) d'hydrure de sodium dans 50 ml de THF. Le mélange est agité 2 heures à température ambiante. On ajoute, ensuite, à 0°C, 57 g (0,23 mol) de 1-[3-(*tert*-butyl-diméthyl-silanyloxy)-phényl]-éthanone dans 200 ml de THF. On laisse remonter à température ambiante et agiter pendant 4 heures. Après évaporation du THF, le produit est repris par de l'éther éthylique. La phase éthérée est lavée plusieurs fois à l'eau.

Après décantation, la phase organique est séchée sur sulfate de magnésium et concentrée. Le résidu est purifié sur colonne de silice (heptane).
Huile jaune. m= 58,3 g. R= 94%.
**RMN 1H**(CDCl3): 0,01 (6H, s), 0,79 (9H, s), 2,24 (3H, d, J= 1Hz), 5,38-5,39 (1H, d, J= 1Hz), 6,67-6,70 (2H, m), 6,83-6,87 (1H, dd), 7,02-7,08 (1H, m).

### e) 3-[3-(tert-butyl-diméthyl-silanyloxy)-phényl]-but-2-enal.

De manière analogue à l'exemple 1(e), par réaction de 280 ml d'hydrure de diisobutylaluminium 1M/Toluène, à -78°C, avec 58,2 g (0,21 mol) de 3-[3-(*tert*-butyl-diméthyl-silanyloxy)-phényl]-but-2-enenitrile, une huile orangée (m= 28,5 g ; R= 48%) est obtenue, après purification sur colonne de silice (dichlorométhane 50 - Heptane 50).
**RMN 1H** (CDCl3): 0,01 (6H, s), 0,79 (9H, s), 2,34 (3H, d, J= 1,1 Hz), 6,14-6,18 (1H, d, J= 6,7 Hz), 6,67-6,71 (1H, dd), 6,79-6,80 (1H, t), 6,92-6,95 (1H, d), 7,03-7,10 (1H, m), 9,96-9,99 (1H, d, J= 7,9 Hz).

### f) 5-[3-(tert-butyl-diméthyl-silanyloxy)-phényl]-hexa-2,4-dienoate d'éthyle.

62 ml (154 mmol) de n-Butyllithium 2,5 M/Hexane sont ajoutés, goutte à goutte, à 0°C, à 150 ml de THF et 150 ml de HMPA. Rapidement, à -30°C, 21,5 ml (154 mmol) de diisopropylamine sont ajoutés, suivis, à -60°C et goutte à goutte, de 28,5 ml (144 mmol) de triéthylphosphonoacétate. Le mélange est agité 1 heure à -60°C puis, une solution de 28,4 g (103 mmol) de 3-[3-(*tert*-butyl-diméthyl-silanyloxy)-phényl]-but-2-enal dans 60 ml de THF est additionnée goutte à goutte. On laisse remonter à température ambiante. Les solvants sont évaporés et le résidu est repris par de l'éther éthylique. La phase organique est extraite avec une solution aqueuse saturée en chlorure d'ammonium puis lavée plusieurs fois à l'eau. Après décantation, elle est séchée sur sulfate de magnésium et concentrée. Le résidu est purifié sur colonne de silice (heptane).
Huile orangée. m= 24 g. R= 67%.
**RMN 1H** ( CDCl3 ): 0,21 (6H, s), 0,99 (9H, s), 1,29-1,34 (3H, t), 2,26-2,27 (3H, d), 4,19-4,27 (2H, q), 5,95.6,01 (1H, d, J= 15 Hz), 6,79-6,81 (1H, m), 6,93-6,94 (1H, t), 7,06-7,10 (1H, dd), 7,18-7,21 (1H, d), 7,68-7,79 (1H, q).

### g) 5-[3-(tert-butyl-diméthyl-silanyloxy)-phényl]-hexanoate d'éthyle.

Dans un réacteur, 15 g (43,3 mmol) de 5-[3-(*tert*-butyl-diméthyl-silanyloxy)-phényl]-hexa-2,4-dienoate d'éthyle sont dissous dans 200 ml d'acétate d'éthyle et 800 mg de Palladium/charbon 5% sont ajoutés. La solution est agitée à température ambiante et sous pression de 4 bars d'hydrogène. 2 heures après, elle est filtrée sur célite et évaporée. Le résidu est purifié sur colonne de silice (acétate d'éthyle 3 - Heptane 97).
Huile jaune. m= 11,7 g. R= 77%.
**RMN 1H** ( CDCl3 ): 0,08 (6H, s), 0,79 (9H, s), 1,01-1,07 (6H, t), 1,32-1,39 (4H, m), 2,03-2,08 (2H, m), 240-2,45 (1H, m), 3,86-3,95 (2H, q), 6,45-6,48 (2H, m), 6,56-6,59 (1H, d, J=7,6 Hz), 6,90-6,97 (1H, t).

### h) 6-[3-(tert-butyl-diméthyl-silanyloxy)-phényl]-2-méthyl-heptan-2-ol.

De manière analogue à l'exemple 1(i), par réaction de 55 ml (165 mmol) de bromure de méthylmagnésium 3M/éther avec 11,6 g (33 mmol) de 5-[3-(*tert*-butyl-diméthyl-silanyloxy)-phényl]-hexanoate d'éthyle dans 100 ml d'éther, une huile jaune (m= 10,65 g ; R= 96%) est obtenue.
**RMN 1H** ( CDCl3 ): 0,00 (6H, s), 0,79 (9H, s), 1,01 (3H, s), 1,03-1,08 (3H, d), 1,20-1,39 (6H, m), 2,40-2,48 (1H, m), 6,45-6,47 (2H, m), 6,56-6,59 (1H, d, J=7,6 Hz), 6,90-6,97 (1H, t).

### i) 3-(5-hydroxy-1,5-diméthyl-hexyl)-phenol.

38 ml de fluorure de tétrabutylammonium 1M/THF sont ajoutés à 10,6 g (31,5 mmol) de 6-[3-(*tert*-butyl-diméthyl-silanyloxy)-phényl]-2-méthyl-heptan-2-ol dans 100 ml de THF. La solution est agitée à température ambiante. 2 heures après, le solvant est évaporé. Le résidu est repris par de l'éther éthylique, lavé à l'eau. La phase organique est séchée sur sulfate de magnésium et concentrée. Le produit est purifié sur colonne de silice (acétate d'éthyle 25 - Heptane 75).
Huile jaune. m= 6g. R= 85%.
**RMN 1H** (CDCl3): 1,17 (6H, s), 1,20-1,23 (3H, d), 1,40-1,66 (6H, m), 2,60-2,69 (1H, m), 5,19 (1H, OH, s), 6,62-6,66 (2H, m), 6,73-6,76 (1H, d, J=7,7 Hz), 7,11-7,17 (1H, t).

### j) 6-[3-(3-éthoxyméthoxy-5-éthoxyméthoxyméthyl-benzyloxy)-phényl]-2-méthyl-heptan-2-ol.

Une solution de 514 mg (2,3 mmol) de 3-(5-hydroxy-1,5-diméthyl-hexyl)-phenol, de 81 mg (2,5 mmol) d'hydrure de sodium 75% dans 10 ml de diméthylformamide est agitée 30 minutes à température ambiante. 700 mg (2,1 mmol) de l-bromométhyl-3-éthoxyméthoxy-5-éthoxyméthoxyméthyl-benzene dans 5 ml de diméthylformamide sont, ensuite, ajoutés. Le milieu est agité une nuit à température ambiante. Il est, alors, versé dans l'eau et extrait à l'éther. La phase organique est séchée sur sulfate de magnésium et concentrée. Le produit est purifié sur colonne de silice (Acétate d'éthyle 25 - heptane 75).
Huile jaune. m= 871 mg. R= 87%.
**RMN 1H** (CDCl3): 1,15 (6H,s), 1,19-1,24 (6H, m), 1,39-1,55 (4H, m), 2,62-2,71 (2H, m), 3,61-3,77 (4H, m), 4,59 (2H, s), 4,76 (2H, s), 5,00 (2H, s), 5,23 (2H, s), 6,77-6,80 (3H, m), 6,99 (1H, s), 7,05-7,08 (2H, d), 7,17-7,23 (1H, t).

### k) 3-[3-(5-hydroxy-1,5-diméthyl-hexyl)-phenoxyméthyl]-5-hydroxyméthyl-phenol.

De manière analogue à l'exemple 1(j), par réaction de 0,4 ml d'acide sulfurique concentré dans 5 ml de méthanol avec 860 mg (1,8 mmol) de 6-[3-(3-éthoxyméthoxy-5-éthoxyméthoxyméthyl-benzyloxy)-phényl]-2-méthyl-heptan-2-ol dans 5 ml de méthanol et 5 ml de THF, après purification sur colonne de silice (acétate d'éthyle 60 - heptane 40), une huile incolore (m= 510 mg ; R= 79%) est obtenue.
**RMN 1H** (CDCl3): 1,14 (6H, s), 1,20-1,23 (3H, d), 1,34-1,57 (6H, m), 2,30 (1H, OH, s), 2,61-2,69 (1H, m), 4,60 (2H, s), 5,02 (2H, s), 6,73-6,78 (5H, m), 6,91 (1H, s), 7,14-7,20 (1H, t).

### EXEMPLE 4

### 6-[3-(3,4-bis-hydroxyméthyl-benzyloxy)-phényl]-2-méthyl-hepta-3,5-dien-2-ol.

### a) (2-hydroxyméthyl-5-iodo-phényl)-méthanol.

Une solution de 2,55 g (37 mmol) de nitrite de sodium dans 10 ml d'eau est ajoutée, goutte à goutte, à 0°C, à 5g (27,6 mmol) d' acide 4-amino phtalique dans 30 ml d'acide sulfurique 20%. Cette solution est additionnée à 7,26 g (43,7 mmol) de iodure de potassium, de 7,35 g (38,6 mmol) de iodure de cuivre dans 30 ml d'acide sulfurique 20%. L'agitation se poursuit pendant 2 heures à température ambiante puis 3 heures à 50°C. Le milieu est ensuite versé dans de l'acétate d'éthyle. Il est extrait avec une solution aqueuse saturée en thiosulfate de sodium puis avec une solution aqueuse saturée en hydrogénocarbonate de sodium. La phase aqueuse est ensuite acidifiée avec de l'acide chlorhydrique jusqu'à pH 1 et extraite avec de l'acétate d'éthyle. La phase organique est séchée sur sulfate de magnésium et concentrée.
Le produit obtenu est dissous dans du THF anhydre et refroidi à 0°C. 110 ml de borane 1M/THF sont ajoutés, goutte à goutte. 3 heures après, on ajoute très lentement une solution de 250 ml d'un mélange THF/eau (1/1). Après décantation, la phase aqueuse est extraite avec de l'éther. La phase organique est lavée plusieurs fois à l'eau, puis séchée sur sulfate de magnésium et concentrée. Le résidu est purifié sur colonne de silice (acétate d'éthyle 80 - heptane 20).
Huile incolore. m= 4,9 g. R= 69%.
**RMN 1H** (DMSO): 4,56-4,61 (4H, m), 5,25-5,35 (2H, OH, m), 7,28-7,31 (1H, d, J= 8 Hz), 7,68-7,72 (1H, dd, J= 6,5 Hz, J'=1,5 Hz), 7,84 (1H, s).

### b) 4-iodo-1-(tert-butyl-diméthyl-silanyloxy)-3-(tert-butyl-diméthyl-silanyloxyméthyl)-phényl.

De manière analogue à l'exemple 3(c), par réaction de 3,86 g (14,6 mmol) de (2-hydroxyméthyl-5-iodo-phényl)-méthanol avec 5,08 ml de triéthylamine, 4,63 g (30,7 mmol) de chlorure de *tert*-butyldiméthylsilane et 95 mg (0,78 mmol) de diméthylaminopyridine dans 30 ml de diméthylformamide, une huile incolore (m= 7 g ; R= 98%) est obtenue.

### c) 4-(tert-butyl-diméthyl-silanyloxy)-3-(tert-butyl-diméthyl-silanyloxyméthyl)-benzaldehyde.

6,3 ml (15,75 mmol) de n-Butyllithium 2,5 M/Hexane sont ajoutés, goutte à goutte, à -78°C, à 7 g (14,2 mmol) de 4-iodo-1-(*tert*-butyl-diméthyl-silanyloxy)-3-(*tert*-butyl-diméthyl-silanyloxyméthyl)-phényl dans 50 ml de THF. 10 minutes après, on additionne 1,2 ml (15,6 mmol) de diméthylformamide anhydre. On laisse remonter à température ambiante, pendant 1 heure. On ajoute, alors, de l'eau et de l'éther. Après décantation, la phase éthérée est extraite avec une solution aqueuse saturée en chlorure d'ammonium puis avec de l'eau. La phase organique est séchée sur sulfate de magnésium et concentrée. Le résidu est purifié sur colonne de silice (Dichlorométhane 50 - heptane 50).
Solide jaunâtre. m= 3,07 g. R= 55%.
**RMN 1H** (CDCl3): 0,00 (12H, s), 0,83 (18H, s), 4,64 (2H, s), 4,69 (2H, s), 7,52-7,56 (1H, d, J=7,8 Hz), 7,66-7,70 (1H, dd, J=7,8 Hz, J'=1,4 Hz), 7,80 (1H, s), 9,89 (1H, s).

### d) [4-(tert-butyl-diméthyl-silanyloxy)-3-(tert-butyl-diméthyl-silanyloxyméthyl)-phényl]-méthanol.

De manière analogue à l'exemple 3(a), par réaction de 290 mg (7,6 mmol) de borohydrure de sodium avec 3 g (7,6 mmol) de 4-(*tert*-butyl-diméthyl-silanyloxy)-3-(*tert*-butyl-diméthyl-silanyloxyméthyl)-benzaldehyde dans 30 ml de méthanol et 20 ml de THF, après purification sur colonne de silice (acétate d'éthyle 20 - heptane 80), une huile jaune (m= 2,6 g ; R= 87%) est obtenue.
**RMN 1H** (CDCl3): 0,00 (6H, s), 0,01 (6H, s), 0,843 (9H, s), 0,849 (9H, s), 4,58-4,60 (2H, d, J=5,8 Hz), 4,651 (2H, s), 4,659 (2H, s), 7,15-7,18 (1H, m), 7,31-7,33 (2H, m).

### e) 4-bromométhyl-1-(tert-butyl-diméthyl-silanyloxy)-2-(tert-butyl-diméthyl-silanyloxyméthyl)-benzene.

De manière analogue à l'exemple 3(b), par réaction de 4,2 ml (9,4 mmol) de trioctylphosphine, de 3,1 g (9,4 mmol) de tetrabromure de carbone avec 1,7 g (4,3 mmol) de [4-(*tert*-butyl-diméthyl-silanyloxy)-3-(*tert*-butyl-diméthyl-silanyloxyméthyl)-phényl]-méthanol dans 30 ml d'éther éthylique, après purification sur colonne de silice (AcOEt 10 - Heptane 90), une huile jaune (m= 1,7 g ; R= 86%) est obtenue.

### f) 6-[3-(tert-butyl-diméthyl-silanyloxy)-phényl]-2-méthyl-hepta-3,5-dien-2-ol.

De manière analogue à l'exemple 1(i), par réaction de 31 ml (93 mmol) de bromure de méthylmagnésium 3M/éther avec 8 g (23 mmol) de 5-[3-*tert*-butyl-diméthyl-silanyloxy)-phényl]-hexa-2,4-dienoate d'éthyle dans 100 ml d'éther, après purification sur colonne de silice (dichlorométhane 70 - heptane 30), une huile jaune (m= 4,6 g ; R= 60%) est obtenue.
**RMN 1H** ( CDCl3 ): 0,20 (6H, s), 0,99 (9H, s), 1,39 (6H, s), 2,15 (3H, s), 5,92-5,98 (1H, d, J= 15,1 Hz), 6,38-6,42 (1H, d, J= 10,9 Hz), 6,58-6,68 (1H, q), 6,73-6,74 (1H, dd), 6,89-6,90 (1H, t), 7,01-7,04 (1H, dd), 7,14-7,20 (1H, t).

### g) 3-(5-hydroxy-1,5-diméthyl-hexa-1,3-diènyl)-phénol.

De manière analogue à l'exemple 3(i), par réaction de 16 ml de fluorure de tétrabutylammonium 1M/THF avec 4,5 g (13,5 mmol) de 6-[3-(*tert*-butyl-diméthyl-silanyloxy)-phényl]-2-méthyl-hepta-3,5-dien-2-ol dans 50 ml de THF, après purification sur colonne de silice (acétate d'éthyle 30 - Heptane 70), une huile jaune (m= 2,2 g ; R= 74%) est obtenue.
**RMN 1H** (CDCl3): 1,38 (6H, s), 2,32 (3H, s), 5,90-5,96 (1H,d, J=15 Hz), 6,39-6,44 (1H, d, J=11,5 Hz), 6,57-6,68 (1H, q), 6,73-6,76 (1H, dd), 6,91-6,94 (2H, m), 7,11-7,17 (1H, t), 8,36 (1H, s).

### h) 6-{3-[3,4-bis-(tert-butyl-diméthyl-silanyloxyméthyl)-benzyloxy]-phényl}-2-méthyl-hepta-3,5-dien-2-ol.

De manière analogue à l'exemple 3(j), par réaction de 218 mg (1 mmol) de 3-(5-hydroxy-1,5-diméthyl-hexa-1,3-diènyl)-phénol, de 39 mg (1,2 mmol) d'hydrure de sodium 75% dans 5 ml de diméthylformamide avec 460 mg (1 mmol) de 4-bromométhyl-1-(*tert*-butyl-diméthyl-silanyloxy)-2-(*tert*-butyl-diméthyl-silanyloxyméthyl)-benzene dans 5 ml de diméthylformamide, après purification sur colonne de silice (Acétate d'éthyle 20 - heptane 70), une huile jaune (m= 440 mg ; R= 74%) est obtenue.
**RMN 1H** (CDCl3): -0,007 (6H, s), 0,000 (6H, s), 0,84 (18H, s), 1,29 (3H, s), 4,65-4,66 (4H, d), 4,98 (2H, s), 5,82-5,88 (1H, d), 6,31-6,35 (1H, d), 6,49-6,59 (1H, m), 6,93-6,96 (2H, m), 7,10-7,13 (1H, d), 7,21-7,24 (1H, d), 7,32-7,36 (1H, d), 7,41 (1H, s).

### i) 6-[3-(3,4-bis-hydroxyméthyl-benzyloxy)-phényl]-2-méthyl-hepta-3,5-dien-2-ol.

De manière analogue à l'exemple 3(i), par réaction de 1,6 ml de fluorure de tétrabutylammonium 1M/THF avec 424 mg (0,71 mmol) de 6-{3-[3,4-bis-(*tert*-butyl-diméthyl-silanyloxyméthyl)-benzyloxy]-phényl}-2-méthyl-hepta-3,5-dien-2-ol dans 15 ml de THF, après purification sur colonne de silice (acétate d'éthyle 90 - Heptane 10), une huile jaune (m= 206 mg ; R= 79%) est obtenue.
**RMN 1H** (CDCl3): 1,38 (6H, s), 2,15 (3H, s), 4,33-4,35 (2H, m), 4,70-4,74 (2H, m), 5,07 (2H, s), 5,92-5,98 (1H, d, J= 15 Hz), 6,40-6,44 (1H, d, J= 10,9 Hz), 6,58-6,68 (1H, q), 6,82-6,85 (1h, d, J= 6,2 Hz), 7,03-7,05 (2H, m), 7,19-7,26 (1H, m), 7,37 (2H, s), 7,45 (1H, s).

### EXEMPLE 5

### 6-[3-(3,4-bis-hydroxyméthyl-benzyloxy)-phényl]-2-méthyl-hexan-2-ol.

### a) 3-bromo-[3,4-bis-(tert-butyl-diméthyl-silanyloxyméthyl)-benzyloxy]-phényl.

De manière analogue à l'exemple 3(j), par réaction de 260 mg (1,5 mmol) de 3-bromophénol, de 58 mg (1,8 mmol) d'hydrure de sodium 75% dans 5 ml de diméthylformamide avec 690 mg (1,5 mmol) de 4-bromométhyl-1-(*tert*-butyl-diméthyl-silanyloxy)-2-(*tert*-butyl-diméthyl-silanyloxyméthyl)-benzène dans 10 ml de diméthylformamide, après purification sur colonne de silice (Acétate d'éthyle 7 - heptane 93), une huile jaune (m= 790 mg ; R= 95%) est obtenue.
**RMN 1H** (CDCl3): 0,00 (12H, s), 0,84 (18H, s), 4,65-4,66 (4H, d), 4,95 (2H, s), 6,78-6,81 (1H, dd), 6,97-7,07 (3H, m), 7,16-7,18 (1H, d), 7,33-7,39 (2H, m).

### b) 5-{3-[3,4-bis-(tert-butyl-diméthyl-silanyloxyméthyl)-benzyloxy]-phényl}-pentanoate de méthyle.

De manière analogue à l'exemple 1(h), par réaction de 780 mg (1,4 mmol) de 3-bromo-[3,4-bis-(*tert*-butyl-diméthyl-silanyloxyméthyl)-benzyloxy]-phényl dans 10 ml de diméthylformamide avec la solution à 0°C, de 750 mg (3 mmol) de 9-borabicyclo[3,3,1]nonane et 230 mg (2 mmol) de pent-4-enoate de méthyle dans 20 ml de THF, après purification sur colonne de silice (acétate d'éthyle 5 - heptane 95), une huile jaune (m= 515 mg ; R= 62%) est obtenue.
**RMN 1H** (CDCL3): 0,000 (s, 6H), 0,006 (s, 6H), 0,84 (s, 18H), 1,55-1,58 (m, 4H), 2,21-2,24 (t, 2H), 2,50-2,53 (t, 2H), 3,57 (s, 3H), 4,65-4,66 (d,4H), 4,95 (s, 2H), 6,66-6,71 (m, 3H), 7,06-7,12 (t, 1H), 7,21-7,24 (d, 1H),7,31-7,36 (d, 1H), 7,40 (s, 1H).

### c) 6-{3-[3,4-bis-(tert-butyl-diméthyl-silanyloxyméthyl)-benzyloxy]-phényl}-2-méthyl-hexan-2-ol.

De manière analogue à l'exemple 1(i), par réaction de 1,4 ml (4,2 mmol) de bromure de méthylmagnésium 3M/éther avec 509 mg (0,87 mmol) de 5-{3-[3,4-bis-(*tert*-butyl-diméthyl-silanyloxyméthyl)-benzyloxy]-phényl}-pentanoate de méthyle dans 15 ml d'éther, une huile jaune (m= 503 mg ; R= 99%) est obtenue.
**RMN 1H** 1H (CDCl3 ): 0,000 (s, 6H); 0,005 (s, 6H); 0,847 (s, 18H); 1,115 (s, 6H); 1,29-1,56 (m, 6H); 2,47-2,54 (t, 2H); 4,65 (s, 2H); 4,66 (s, 2H); 4,95 (s, 2H); 6,67-6,72 (m, 3H); 7,06-7,12 (t, 1H); 7,21-7,24 (d, 1H); 7,32-7,40 (m, 2H).

### d) 6-[3-(3,4-bis-hydroxyméthyl-benzyloxy)-phényl]-2-méthyl-hexan-2-ol.

De manière analogue à l'exemple 3(i), par réaction de 2 ml de fluorure de tétrabutylammonium 1M/THF avec 490 mg (0,83 mmol) de 6-{3-[3,4-bis-(*tert*-butyl-diméthyl-silanyloxyméthyl)-benzyloxy]-phényl}-2-méthyl-hexan-2-ol dans 15 ml de THF, après purification sur colonne de silice (acétate d'éthyle 90 - Heptane 10), une huile jaune (m= 196 mg ; R = 66%) est obtenue.
**RMN 1H** 1H ( CDCl3 ):1,17 (s, 6H); 1,31-1,64 (m, 4H); 2,56-2,62 (t,2H); 3,16-3,18 (m, 2H); 4,71 (s, 2H); 4,72 (s, 2H); 5,05 (s, 2H);6,77-6,79 (m, 3H); 7,15-7,21 (m, 1H); 7,35-7,41 (m, 3H).

### EXEMPLE 6

### 6-[3-(3,4-bis-hydroxyméthyl-phenoxyméthyl)-phényl]-2-méthyl-heptan-2-ol.

### a) Acide 4-amino-phtalique.

1 g (4,73 mmol) d'acide 4 nitrophtalique est dissous dans 10 ml d'éthanol anhydre. La solution est agitée à température ambiante et dégazée sous argon. 50 mg de Palladium/Charbon 5% sont ajoutés en une seule fois et on fait buller de l'hydrogène dans la solution. Après 3 heures, la solution est filtrée sur Célite puis évaporée.
Huile orangée. m= 820 mg. R= 96%.
**RMN 1H** (DMSO): 3,32 (1H, s), 5,95 (1H, s), 6,49-6,53 (2H, m), 7,46-7,50 (1H, d, J= 8,8 Hz), 12,33 (2H, COOH, s).

### b) 4-hydroxy-phthalate de diméthyle.

Une solution de 5 g (27,6 mmol) d'acide 4-amino-phtalique dans 50 ml d'acide sulfurique 1 M est refroidie à 0°C, on ajoute, alors, lentement une solution de 2,27 g de nitrite de sodium dans 6 ml d'eau. Après 15 minutes à 0°C, 15 ml d'acide sulfurique concentré sont ajoutés et le mélange est porté à 100°C, sous forte agitation, et pendant 1 heure. A température ambiante, le milieu réactionnel est extrait avec de l'acétate d'éthyle et lavé avec de l'eau. Après décantation, la phase organique est séchée sur sulfate de magnésium et concentrée. Le résidu est purifié sur colonne de silice (dichlorométhane 80 - méthanol 20). Il est ensuite dissous dans 100 ml de méthanol et mis à reflux avec 2 ml d'acide sulfurique. Après disparition du diacide, le méthanol est évaporé et le produit est repris par de l'acétate d'éthyle et lavé à l'eau.
M= 5,2 g. R= 90%.
**RMN 1H** (DMSO): 3,64 (3H, s), 3,67 (3H, s), 6,79-6,86 (2H, m), 7,56-7,60 (1H, d, J= 8,4 Hz), 10,51 (1H, OH, s).

### c) 1-(3-chlorométhyl-phényl)-éthanone.

30 ml de méthyllithium sont ajoutés, goutte à goutte, à 0°C, à 4 g (23,4 mmol) de l'acide 3-chlorométhyl-benzoïque dans 250 ml d'éther éthylique anhydre. A la fin de l'addition, la solution est encore agitée à 0°C pendant 30 minutes. On ajoute, alors, lentement de l'eau puis on acidifie avec de l'acide chlorhydrique. La phase organique est lavée plusieurs fois à l'eau, séchée sur sulfate de magnésium puis concentrée. Le résidu est purifié sur colonne de silice (acétate d'éthyle 5 - heptane 95).
M= 2,7 g. R= 69%.
**RMN 1H** (CDCl3): 2,62 (3H, s), 4,63 (2H, s), 7,44-7,50 (1H, t), 7,59-7,62 (1H, d, J= 7,6 Hz), 7,89-7,93 (1H, d, J= 6,5 Hz), 7,97 (1H, s).

### d) 2-(3-chlorométhyl-phényl)-2-méthyl-[1,3]dioxolane.

Dans un tricol équipé d'un système Dean-Stark, 2,5 g (14,8 mmol) de 1-(3-chlorométhyl-phényl)-éthanone sont dissous dans 20 ml de toluène. On ajoute 4 ml (74 mmol) d'éthylène glycol et 250 mg (1,48 mmol) d'acide *para*-toluène sulfonique. On chauffe au reflux une nuit. 200 mg de carbonate de potassium sont additionnés et le milieu est versé dans l'eau et extrait avec du dichlorométhane. La phase organique est séchée sur sulfate de magnésium et concentrée. Le produit est purifié sur colonne de silice (acétate d'éthyle 10 - heptane 90).
Huile. m= 1,9 g. R= 59%.
**RMN 1H** (CDCl3): 1,65 (3H, s), 3,71-3,84 (2H, m), 3,97-4,11 (2H, m), 4,59 (2H, s), 7,32-5 7,37 (2H, m), 7,42-7,46 (1H, m), 7,50 (1H, s).

### e) 4-[3-(2-méthyl-[1,3]dioxolan-2-yl)-benzyloxy)-phthalate de diméthyle.

Une solution de 1,83 g (8,6 mmol) de 2-(3-chlorométhyl-phényl)-2-méthyl-[1,3]dioxolane, de 1,9 g (9,03 mmol) de 4-hydroxy-phthalate de diméthyle, de 1,25 g (9,03 mmol) de carbonate de potassium et de 200 mg de iodure de potassium dans 75 ml de 2-butanone est chauffée au reflux. Après 3 heures et à température ambiante, le mélange est filtré, concentré et purifié sur colonne de silice (acétate d'éthyle 20 - heptane 80)
Huile incolore. m= 3,2 g. R= 97%.
**RMN 1H** (CDCl3): 1,66 (3H, s), 3,70-3,84 (2H, m), 3,87 (3H, s), 3,91 (3H, s), 4,01-4,11 (2H, m), 5,12 (2H, s), 7,04-7,08 (1H, dd, J= 6Hz, J'= 2,6 Hz), 7,16-7,17 (1H, d, J= 2,5 Hz), 7,35-7,41 (2H, m), 7,45-7,49 (1H, m), 7,53 (1H, s), 7,79-7,82 (1H, d, J= 8,6 Hz).

### f) {2-hydroxyméthyl-5-[3-(2-méthyl-[1,3]dioxolan-2-yl)-benzyloxy]phényl}-méthanol.

3,2 g (8,3 mmol) de 4-[3-(2-méthyl-[1,3]dioxolan-2-yl)-benzyloxy)-phthalate de diméthyle sont dissous dans 10 ml de THF et 3 ml de toluène. On ajoute, alors, 460 mg (21 mmol) de borohydrure de lithium et le mélange est chauffé à reflux pendant 1 heure 30. Le milieu réactionnel est évaporé et repris dans de l'eau, acidifié avec de l'acide chlorhydrique 1N puis extrait avec de l'acétate d'éthyle. La phase organique est séchée sur sulfate de magnésium et concentrée.
Huile incolore. m= 2,7 g. R= 99%.
**RMN 1H** (CDCl3): 1,66 (3H, s), 3,74-3,80 (2H, m), 4,01-4,10 (2H, m), 5,07 (2H, s), 6,90 (1H, m), 6,99 (1H, m), 7,22 (1H, m), 7,35-7,37 (2H, m), 7,45 (1H, m), 7,54 (1H, s).

### g) 1-[3-(3,4-bis-hydroxyméthyl-phénoxyméthyl)-phényl]-éthanone.

2,7 g (8,3 mmol) de {2-hydroxyméthyl-5-[3-(2-méthyl-[1,3]dioxolan-2-yl)-benzyloxy]phényl}-méthanol et 200 mg (0,8 mmol) de pyridinium-*para*-toluène sulfonique dans 5 ml d'eau et 20 ml d'acétone sont chauffés à reflux pendant 6 heures. A température ambiante, le mélange est repris par de l'acétate d'éthyle et lavé avec une solution aqueuse saturée en bicarbonate de sodium. La phase organique est séchée sur sulfate de magnésium et concentrée.

### h) 1-{3-[3,4-bis-(tert-butyl-diméthyl-silanyloxyméthyl)-phénoxyméthyl]-phényl}-éthanone.

De manière analogue à l'exemple 3(c), par réaction de 3 g (20 mmol) de chlorure de *tert*-butyldiméthylsilane avec 2,3 g (8 mmol) de 1-[3-(3,4-bis-hydroxyméthyl-phénoxyméthyl)-phényl]-éthanone, 50 mg de diméthylaminopyridine dans 3,5 ml (24 mmol) de triéthylamine et 50 ml de diméthylformamide, on obtient 3 g d'une huile incolore.
**RMN 1H** (CDCl3): 0,00 (6H, s), 0,01 (6H, s), 0,84 (9H, s), 0,86 (9H, s), 2,54 (3H, s), 4,57 (2H, s), 4,68 (2H, s), 5,05 (2H, s), 6,72-6,77 (1H, dd, J= 5,7 Hz, J'= 2,6 Hz), 7,06-7,07 (1H, d, J= 2,6 Hz), 7,17-7,20 (1H, m), 7,37-7,43 (1H, t), 7,56-7,59 (1H, d, J= 7,6 Hz), 7,82-7,85 (1H, d, J= 7,8 Hz), 7,94 (1H, s).

### i) 5-{3-[3,4-bis-(tert-butyl-diméthyl-silanyloxyméthyl)-phénoxyméthyl]-phényl}-hexa-2,4-dienoate d'éthyle.

2,48 ml (11 mmol) de 4-(diéthoxy-phosphoryl)-but-2-enoate d'éthyle dans 5 ml de THF sont ajoutés à 0°C à une solution de 360 mg (11,25 mmol) d'hydrure de sodium dans 5 ml de THF et 10 ml de 1,3-diméthyl-3,4,5,6-tetrahydro-2(1H)-pyrimidinone. Le mélange est agité à 0°C pendant 1 heure puis on ajoute, goutte à goutte, 2,89 g (5,6 mmol) de 1-{3-[3,4-bis-(*tert*-butyl-diméthyl-silanyloxyméthyl)-phénoxyméthyl]-phényl}-éthanone dans 20 ml de THF. On laisse agiter à température ambiante pendant 24 heures. On ajoute, alors, de l'eau et on acidifie avec de l'acide chlorhydrique 1N et on extrait avec du dichlorométhane. La phase organique est lavée à l'eau, séchée sur sulfate de sodium et concentrée. Le résidu est purifié sur colonne de silice (acétate d'éthyle 5 - heptane 95).
Huile. m= 1,2 g. R= 36%.
**RMN 1H** (CDCl3): 0,00 (6H, s), 0,01 (6H, s), 0,84 (9H, s), 0,86 (9H, s), 1,19 (3H, s), 1,21-1,27 (3H, t), 4,57 (2H, s), 4,68 (2H, s), 5,01 (2H, s), 5,88-5,94 (1H, d, J= 15 Hz), 6,48-6,53 (1H, d, J= 11,6 Hz), 6,74-6,77 (1H, dd, J= 8,3 Hz), 7,08 (1H, d), 7,17-7,20 (2H, m), 7,31 (2H, m), 7,47 (1H, s), 7,62-7,72 (1H, q).

### j) 5-{3-[3,4-bis-(tert-butyl-diméthyl-silanyloxyméthyl)-phénoxyméthyl]-phényl}-hexanoate d'éthyle.

350 mg de Rhodium/Alumine 5% sont ajoutés à 750 mg (1,22 mmol) de 5-{3-[3,4-bis-(*tert*-butyl-diméthyl-silanyloxyméthyl)-phénoxyméthyl]-phényl}-hexa-2,4-dienoate d'éthyle dans 50 ml d'acétate d'éthyle. On fait buller de l'hydrogène dans le milieu pendant 1 heure 30. Le mélange est ensuite filtré sur célite et concentré.
Huile incolore. m= 665 mg. R= 88%.

### k) 6-{3-[3,4-bis-(tert-butyl-diméthyl-silanyloxyméthyl)-phénoxyméthyl]-phényl}-2-méthyl-heptan-2-ol.

De manière analogue à l'exemple 1(i), par réaction de 0,53 ml (1,6 mmol) de bromure de méthylmagnésium avec 320 mg (0,52 mmol) de 5-{3-[3,4-bis-(*tert*-butyt-diméthyl-silanyloxyméthyl)-phénoxyméthyl]-phényl}-hexanoate d'éthyle dans 10 ml de THF, après purification sur colonne de silice (acétate d'éthyle 10 - heptane 90), une huile incolore. (m= 265 mg ; R= 85%) est obtenue.
**RMN 1H** (CDCl3): 0,00 (6H, s), 0,01 (6H, s), 0,85 (9H, s), 0,87 (9H, s), 1,08 (6H, s), 1,15-1,18 (3H, d), 1,15-1,48 (6H, m), 2,63-2,66 (1H, m), 4,58 (2H, s), 4,68 (2H, s), 4,96 (2H, s), 6,73-6,77 (1H, dd, J= 5,6 Hz, J'= 2,6 Hz), 7,05-7,07 (2H, m), 7,16-7,23 (4H, m).

### l) 6-[3-(3,4-bis-hydroxyméthyl-phénoxyméthyl)-phényl]-2-méthyl-heptan-2-ol.

De manière analogue à l'exemple 3(i), par réaction de 1,25 ml de fluorure de tétrabutylammonium avec 250 mg (0,41 mmol) de 6-{3-[3,4-bis-(*tert*-butyl-diméthyl-silanyloxyméthyl)-phénoxyméthyl]-phényl}-2-méthyl-heptan-2-ol dans 7 ml de THF, après purification sur colonne de silice (acétate d'éthyle 30 - heptane 70), une huile incolore (m= 130 mg ; R= 85%) est obtenue.
**RMN 1H** (CDCl3): 1,11 (6H, s), 1,22-1,25 (3H, d), 1,16-1,42 (4H, m), 1,51-1,60 (2H, q), 2,66-2,74 (1H, m), 4,63 (2H, s), 4,65 (2H, s), 5,07 (2H, s), 6,83-6,88 (1H, dd, J= 5,6 Hz, J'= 2,6 Hz), 6,97-6,98 (1H, d, J= 2,5 Hz), 7,11-7,14 (1H, d, J= 7,26 Hz), 7,20-7,32 (4H, m).

### EXEMPLE 7

### 7-[3-(3,4-bis-hydroxyméthyl-phénoxyméthyl)-phényl]-3-éthyl-octan-3-ol.

### a) 7-{3-[3,4-bis-(tert-butyl-diméthyl-silanyloxyméthyl)-phénoxyméthyl]-phényl}-3-éthyl-octan-3-ol.

De manière analogue à l'exemple 1(i), par réaction de 370 mg (0,6 mmol) de 5-{3-[3,4-bis-(*tert*-butyl-diméthyl-silanyloxyméthyl)-phénoxyméthyl]-phényl}-hexanoate d'éthyle avec 1,8 ml (1,8 mmol) de bromure d'éthylmagnésium, une huile incolore (m= 265 mg ; R= 70%) est obtenue.
**RMN 1H** (CDCl3): 0,00 (6H, s), 0,01 (6H, s), 0,70-0,76 (6H, t), 0,84 (9H, s), 0,87 (9H, s), 1,15-1,18 (3H, d), 1,15-1,53 (10H, m), 2,63-2,66 (1H, m), 4,58 (2H, s), 4,69 (2H, s), 4,96 (2H, s), 6,74-6,77 (1H, dd, J= 5,6 Hz, J'= 2,6 Hz), 7,05-7,07 (2H, m), 7,16-7,22 (4H, m).

### b) 7-[3-(3,4-bis-hydroxyméthyl-phénoxyméthyl)-phényl]-3-éthyl-octan-3-ol.

De manière analogue à l'exemple 3(i), par réaction de 230 mg (0,36 mmol) de 7-{3-[3,4-bis-(*tert*-butyl-diméthyl-silanyloxyméthyl)-phénoxyméthyl]-phényl}-3-éthyl-octan-3-ol avec 1,2 ml de fluorure de tetrabutylammonium, une huile incolore (m= 110 mg ; R= 75%) est obtenue.
**RMN 1H** (CDCl3): 0,75-0,81 (6H, t), 1,22-1,25 (3H, d), 1,22-1,69 (10H, m), 2,66-2,75 (1H, m), 4,64 (2H, s), 4,66 (2H, s), 5,07 (2H, s), 6,84-6,89 (1H, dd, J= 5,6 Hz, J'= 2,6 Hz), 6,98-6,99 (1H, d, J= 2,5 Hz), 7,11-7,14 (1H, d, J= 7,3 Hz), 7,21-7,32 (4H, m).

### EXEMPLE 8

### 5-{2-[4-(5-hydroxy-5-méthyl-hexyl)-phényl]-vinyl}-benzène-1,3-diol.

### a) 6-bromo-2-méthyl-hexan-2-ol.

340 ml (476 mmol) de bromure de méthylmagnésium 1,4 M/THF sont ajoutés, goutte à goutte, à-78°C, à une solution de 25 g (120 mmol) d'éthyl-5-bromovalérate dans 150 ml de THF. Le mélange est agité pendant 3 heures à une température comprise entre -78 et -30°C. A -30°C, on ajoute lentement une solution aqueuse saturée en chlorure d'ammonium. A température ambiante, le milieu est extrait avec de l'éther. La phase éthérée est lavée à l'eau, séchée sur sulfate de magnésium et concentrée. Le résidu est purifié sur colonne de silice (dichlorométhane).
Huile jaune. m= 21,5 g. R= 92%.
**RMN 1H** (CDCl3): 1,23 (6H, s), 1,45-1,59 (4H, m), 1,82-1,93 (2H, m), 3,40-3,45 (2H, t).

### b) 2-(5-bromo-1,1-diméthyl-pentyloxy)-tétrahydro-pyranne.

Une solution de 21,5 g (0,11 mol) de 6-bromo-2-méthyl-hexan-2-ol dans 150 ml d'éther éthylique avec 15 ml (0,16 mol) de dihydropyranne et 100 mg d'acide *para*-toluène sulfonique est agitée une nuit à température ambiante. On ajoute, alors, une solution aqueuse saturée en bicarbonate de sodium et on extrait à l'éther. La phase organique est lavée à l'eau, séchée sur sulfate de sodium et concentrée. Le résidu est purifié sur colonne de silice (dichlorométhane 70 - heptane 30).
Huile jaune. m= 29,2 g. R= 95%.

### c) 7-bromo-2-méthyl-heptan-2-ol.

De manière analogue à l'exemple 8(a), par réaction de 160 ml (0,48 mol) de bromure de méthylmagnésium avec 26,8 g (0,12 mol) d'éthyl-6-bromohexanoate, une huile jaunâtre (m= 25,2 g ; R= 100%) est obtenue.
**RMN 1H** (CDCl3): 1,21 (6H, s), 1,37-1,50 (6H, m), 1,82-1,93 (2H, m), 3,39-3,44 (2H, t).

### d) 2-(6-bromo-1,1-diméthyl-hexyloxy)-tétrahydro-pyranne

De manière analogue à l'exemple 8(b), par réaction de 14,11 g (0,168 mol) de dihydropyranne avec 25,2 g (0,12 mol) de 7-bromo-2-méthyl-heptan-2-ol , une huile jaunâtre (m= 34,8 g ; R= 90%) est obtenue.
**RMN 1H** (CDCl3): 1,18-1,20 (6H, d), 1,39-1,90 (14H, m), 3,38-3,44 (2H, t), 3,38-3,48 (1H, m), 3,92-3,96 (1H, m), 4,69-4,71 (1H, d).

### e) (4-bromo-benzyl)-phosphonate d'éthyle.

Une solution de 25 g (0,1 mol) de bromure de 4-bromobenzyle et 19 ml (0,11 mol) de triéthylphosphite est chauffée à 100°C pendant 24 heures. A température ambiante, le résidu est purifié sur colonne de silice (dichlorométhane puis acétate d'éthyle).
Huile jaune. m= 31 g. R= 100%.
**RMN 1H** (CDCl3): 1,22-1,28 (6H, t), 3,05-3,13 (2H, d), 3,96-4,08 (4H, q), 7,15-7,19 (2H, dd, J= 6 Hz, J'= 2,4 Hz), 7,42-7,45 (2H, d, J= 8 Hz).

### f) 3-(tétrahydro-pyran-2-yl)-5-(tétrahydro-pyran-2-yloxy)-benzaldehyde.

30,25 ml (0,33 mol) de dihydropyranne sont ajoutés, goutte à goutte, à une solution de 11,5 g (0,083 mol) de 3,5-dihydroxybenzaldehyde et 1,05 g (4,18 mmol) de pyridinium *para*-toluène sulfonate dans 250 ml de dichlorométhane. Le mélange est agité pendant 2 heures à température ambiante. Le milieu réactionnel est versé dans une solution aqueuse saturée en hydrogénocarbonate de sodium. La phase organique est lavée à l'eau, séchée sur sulfate de magnésium et concentrée. Le résidu est purifié sur colonne de silice (dichlorométhane).
Huile jaune. m= 22,8 g. R= 90%.
**RMN 1H** (CDCl3): 1,57-1,71 (6H, m), 1,83-2,06 (6H, m), 3,60-3,65 (2H, m), 3,83-3,93 (2H, m), 5,46-5,49 (2H, m), 7,02 (1H, s), 7,20-7,21 (2H, c), 9,90 (1H, s).

### g) (3-bromo-benzyl)-phosphonate d'éthyle.

De manière analogue à l'exemple 8(e), par réaction de 25,2 g (0,1 mol) de bromure 3-bromobenzyle avec 19 ml (0,11 mol) de triéthylphosphite, une huile jaunâtre (m= 32,6 g ; R= 100%) est obtenue.
**RMN 1H** (CDCl3): 1,26-1,31 (6H, t), 3,06-3,15 (2H, d), 3,97-4,09 (4H, q), 7,14-7,23 (2H, m), 7,36-7,44 (2H, m).

### h) 4-bromo-{2-[3-(tétrahydro-pyran-2-yl)-5-(tétrahydro-pyran-2-yloxy)-phényl]-vinyl}-phényl.

De manière analogue à l'exemple 1(g), par réaction de 157 mg (5,2 mmol) d'hydrure de sodium avec une solution de 1,32 g (4,3 mmol) de 3-(tétrahydro-pyran-2-yl)-5-(tétrahydro-pyran-2-yloxy)-benzaldehyde et 1,59 g (5,2 mmol) de (4-bromo-benzyl)-phosphonate d'éthyle dans 30 ml de THF, après purification sur colonne de silice (dichlorométhane 60 - heptane 40), un solide jaunâtre (m=1,78 g ; R= 90%) est obtenu. Tf= 77-9°C.
**RMN 1H** (CDCl3): 1,57-1,76 (6H, m), 1,83-2,07 (6H, m), 3,60-3,65 (2H, m), 3,88-3,97 (2H, m), 5,44-5,45 (2H, d), 6,72 (1H, s), 6,86 (2H, s), 6,99 (2H, s), 7,32-7,35 (2H, d, J= 8,5 Hz), 7,44-7,47 (2H, d, J= 8,4 Hz).

### i) 3-bromo-{2-[3-(tétrahydro-pyran-2-yl)-5-(tétrahydro-pyran-2-yloxy)-phényl]-vinyl}-phényl.

De manière analogue à l'exemple 1(g), par réaction de 3,08 g (0,01 mol) de 3-(tétrahydro-pyran-2-yl)-5-(tétrahydro-pyran-2-yloxy)-benzaldehyde et de 3,68 g (0,12 mol) de (3-bromo-benzyl)-phosphonate d'éthyle avec 362 mg (0,012 mol) d'hydrure de sodium, une huile jaunâtre (m= 4,43 g ; R= 96%) est obtenue.
**RMN 1H** (CDCl3): 1,58-1,77 (6H, m), 1,83-2,07 (6H, m), 3,61-3,65 (2H, m), 3,88-3,97 (2H, m), 5,44-5,45 (2H, m), 6,72 (1H, s), 6,86 (2H, s), 6,92-7,06 (2H, q), 7,17-7,23 (1H, m), 7,34-7,40 (2H, m), 7,63 (1H, s).

### j) 2-[5-(4-{2-[3-(tétrahydro-pyran-2-yl)-5-(tétrahydro-pyran-2-yloxy)-phényl]-vinyl}-phényl)-1,1-diméthyl-pentyloxy]-tétrahydro-pyranne.

2,17 g (7,8 mmol) de 2-(5-bromo-1,1-diméthyl-pentyloxy)-tetxahydro-pyranne sont ajoutés, à 35°C, à une solution de 208 mg (8,6 mmol) de magnésium dans 8 ml de THF anhydre avec un cristal d'iode. Le magnésien est chauffé à 35°C pendant 4 heures. A température ambiante, on ajoute 1,43 g (3,11 mmol) de 4-bromo-{2-[3-(tétrahydro-pyran-2-yl)-5-(tétrahydro-pyran-2-yloxy)-phényl]-vinyl}-phényl, 63 mg (0,12 mmol) de [1,2-bis(diphénylphosphino)éthane]dichloronickel et 6 ml d'éther anhydre et on rechauffe à 35°C toute la nuit. Le milieu réactionnel est versé dans une solution aqueuse saturée en chlorure d'ammonium et extrait à l'éther. La phase organique est lavée à l'eau, séchée sur sulfate de magnésium et concentrée. Le produit est purifié sur colonne de silice (acétate d'éthyle 10 - heptane 90).
Huile jaunâtre. m= 1,75 g. R= 97%.
**RMN 1H** (CDCl3): 1,18-1,20 (6H, d), 1,26 (6H, s), 1,37-2,05 (24H, m), 2,58-2,64 (2H, t), 3,40-3,45 (1H, m), 3,60-3,65 (2H, m), 3,90-3,97 (3H, m), 4,68 (1H, m), 5,44-5,46 (2H, d), 6,69 (1H, s), 6,86 (2H, s), 6,93-7,09 (2H, q), 7,14-7,17 (2H, d, J= 8 Hz), 7,38-7,41 (2H, d, J= 8 Hz).

### k) 5-{2-[4-(5-hydroxy-5-méthyl-hexyl)-phényl]-vinyl}-benzène-1,3-diol.

Une solution de 1,74 g (3 mmol) de 2-[5-(4-{2-[3-(tétrahydro-pyran-2-y!)-5-(tétrahydro-pyran-2-yloxy)-phényl]-vinyl}-phényl)-1,1-diméthyl-pentyloxy]-tétrahydro-pyranne dans 50 ml d'acide acétique, 25 ml de THF et 12 ml d'eau est agitée une nuit à température ambiante. Elle est ensuite évaporée à sec et le résidu ainsi obtenu est trituré dans un mélange d'éther éthylique et hexane. Après filtration, le produit est séché.
Cristaux blancs. m= 423 mg. R= 55%. Tf= 177-8°C.
**RMN 1H** (DMSO): 1,23 (6H, s), 1,53 (4H, m), 1,72 (2H, m), 2,72-2,75 (2H, t), 4,24 (1H, OH, s), 6,33 (1H, s), 6,61 (2H, s), 7,17 (2H, s), 7,34-7,37 (2H, d, J= 7,6 Hz), 7,64-7,67 (2H, d, J= 7,7 Hz), 9,42 (2H, OH, s).

### EXEMPLE 9

### 5-{2-[4-(5-hydroxy-5-méthyl-hexyl)-phényl]-éthyl}-benzène-1,3 diol.

Dans un réacteur, on place 414 mg (1,27 mmol) de 5-{2-[4-(5-hydroxy-5-méthyl-hexyl)-phényl]-vinyl}-benzène-1,3-diol dans 40 ml de dioxanne et 42 mg de Palladium/charbon 10%. On exerce une pression d'hydrogène de 7 bars pendant 4 heures à température ambiante. Le milieu réactionnel est filtré sur célite et le filtrat est évaporé. Le produit est cristallisé dans un mélange acétate d'éthyle/hexane.
Poudre blanchâtre. m= 227 mg. R= 69%. Tf= 144-5°C.
**RMN 1H** (CDCl3): 1,19 (6H, s), 1,28-1,66 (6H, m), 2,55-2,61 (2H, t), 2,72-2,83 (4H, m), 6,20-6,24 (3H, m), 7,07 (4H, s), 8,12 (2H, OH, s).

### EXEMPLE 10

### 5-{2-[4-(6-hydroxy-6-méthyl-heptyl)-phényl]vinyl}-benzene-1,3-diol

### a) 2-[6-(4-{2-[3-(tétrahydro-pyran-2-yl)-5-(tétrahydro-pyran-2-yloxy)-phényl]-vinyl}-phényl)-1,1-diméthyl-hexyloxy]-tétrahydro-pyranne.

De manière analogue à l'exemple 8(j), par réaction de 4,33 g (9,42 mmol) de 4-bromo-{2-[3-(tétrahydro-pyran-2-yl)-5-(tétrahydro-pyran-2-yloxy)-phényl]-vinyl}-phényl dans 12 ml d'éther avec la solution de 630 mg (26 mmol) de magnésium, 7 g (23,8 mmol) de 2-(6-bromo-1,1-diméthyl-hexyloxy)-tetrahydro-pyranne dans 20 ml de THF et catalysée par 193 mg (0,36 mmol) de [1,2-bis-(diphénylphosphino)éthane]dichloronickel, une huile jaunâtre (m= 5 g ; R= 90%) est obtenue.
**RMN 1H** (CDCl3): 1,18-1,20 (6H, d), 1,26-1,63 (20H, m), 1,84-1,87 (4H, m), 1,97-2,01 (2H, m), 2,57-2,63 (2H, t), 3,41-3,45 (1H, m), 3,60-3,65 (2H, m), 3,90-3,97 (3H, m), 4,69 (1H, m), 5,45-5,46 (2H, m), 6,69 (1H, s), 6,86 (2H, s), 6,93-7,09 (2H, q), 7,13-7,17 (2H, d, J= 8 Hz), 7,38-7,41 (2H, d, J= 8 Hz).

### b) 5-{2-[4-(6-hydroxy-6-méthyl-heptyl)-phényl]vinyl}-benzène-1,3-diol.

De manière analogue à l'exemple 8(k), par réaction de 4,98 g (8,4 mmol) de 2-[6-(4-{2-[3-(tétrahydro-pyran-2-yl)-5-(tétrahydro-pyran-2-yloxy)-phényl]-vinyl}-phényl)-1,1-diméthyl-hexyloxy]-tétrahydro-pyranne avec la solution de 100 ml d'acide acétique, 50 ml de THF et 25 ml d'eau, des cristaux blanchâtres (m= 1,85 g ; R= 65%) sont obtenus. Tf= 158-60°C.
**RMN 1H** (CDCl3): 1,19 (6H, s), 1,35-1,43 (6H, m), 1,63 (2H, m), 2,08 (1H, OH, s), 2,56-2,62 (2H, t), 6,33 (1H, s), 6,52-6,53 (2H, d), 6,86-7,03 (2H, q), 7,12-7,15 (2H, d, J= 8 Hz), 7,36-7,39 (2H, d, J= 8 Hz), 8,40 (2H, OH, s).

### EXEMPLE 11

### 5-{2-[3-(6-hydroxy-6-méthyl-heptyl)-phényl]-vinyl}-benzene-1,3-diol.

### a) 2-[6-(3-{2-[3-(tétrahydro-pyran-2-yl)-5-(tétrahydro-pyran-2-yloxy)-phényl]-vinyl}-phényl)-1,1-diméthyl-hexyloxy]-tétrahydro-pyranne.

De manière analogue à l'exemple 8(j), par réaction de 4,39 g (9,56 mmol) de 3-bromo-{2-[3-(tétrahydro-pyran-2-yl)-5-(tétrahydro-pyran-2-yloxy)-phényl]-vinyl}-phényl dans 12 ml d'éther avec la solution de 630 mg (26 mmol) de magnésium, de 7 g (23,8 mmol) de 2-(6-bromo-1,1-diméthyl-hexyloxy)-tétrahydro-pyranne dans 20 ml de THF et catalysée par 193 mg (0,36 mmol) de [1,2-bis-(diphénylphosphino)éthane]dichloronickel, une huile jaunâtre (m= 4,5 g ; R= 79%) est obtenue.
**RMN 1H** (CDCl3): 1,18-1,20 (6H, d), 1,26-1,67 (20H, m), 1,84-1,87 (4H, m), 2,01 (2H, m), 2,58-2,64 (2H, t), 3,41-3,45 (1H, m), 3,61-3,65 (2H, m), 3,90-3,97 (3H, m), 4,69 (1H, m), 5,45-5,46 (2H, m), 6,70 (1H, s), 6,87 (2H, s), 6,96-7,08 (3H, m), 7,21-7,30 (3H, m).

### b) 5-{2-[3-(6-hydroxy-6-méthyl-heptyl)-phényl]-vinyl}-benzène-1,3-diol.

De manière analogue à l'exemple 8(k), par réaction de 4,50 g (7,6 mmol) de 2-[6-(3-{2-[3-(tétrahydro-pyran-2-yl)-5-(tétrahydro-pyran-2-yloxy)-phényl]-vinyl}-phényl)-1,1-diméthyl-hexyloxy]-tétrahydro-pyranne avec la solution de 100 ml d'acide acétique, 50 ml de THF et 25 ml d'eau, et après purification sur colonne de silice (acétate d'éthyle 50 - heptane 50), des cristaux beiges (m= 1,23 g ; R= 48%) sont obtenus. Tf= 98-100°C.
RMN 1H (CDCl3): 1,20 (6H, s), 1,35-1,44 (6H, m), 1,64-1,67 (2H, m), 2,57-2,63 (2H, t), 6,35 (1H, s), 6,55 (2H, s), 6,96-6,97 (2H, d), 7,03-7,06 (1H, d, J= 6,6 Hz), 7,19-7,29 (3H, m), 8,22 (2H, OH, s).

### EXEMPLE 12

### 5-{2-[4-(6-hydroxy-6-méthyl-heptyl)-phényl]-éthyl}-benzène-1,3-diol.

De manière analogue à l'exemple 9(a), par réaction de 1 g (2,9 mmol) de 5-{2-[4-(6-hydroxy-6-méthyl-heptyl)-phényl]vinyl}-benzène-1,3-diol avec 100 mg de Palladium/charbon 10%, des cristaux blanchâtres (m= 728 mg ; R= 91%) sont obtenus. Tf= 128-30°C.
**RMN 1H** (CDCl3): 1,19 (6H, s), 1,33-1,42 (6H, m), 1,61-1,63 (2H, m), 1,99 (1H, OH, s), 2,53-2,59 (2H, t), 2,73-2,86 (4H, m), 6,24 (3H, s), 7,05-7,12 (4H, dd, J= 8,8 Hz), 8,08 2H, OH, s).

### EXEMPLE 13

### 5-{2-[3-(6-hydroxy-6-méthyl-heptyl)-phényl]-éthyl}-benzène-1,3-diol.

De manière analogue à l'exemple 9(a), par réaction de 600 mg (1,76 mmol) de 5-{2-[3-(6-hydroxy-6-méthyl-heptyl)-phényl]-vinyl}-benzène-1,3-diol avec 60 mg de Palladium/charbon 10%, des cristaux rosâtres (m= 368 mg ; R= 61%) sont obtenus. Tf= 102-3°C.
**RMN 1H** (CDCl3): 1,20 (6H, s), 1,23-1,56 (8H, m), 1,99 (1H, OH, s), 2,46-2,52 (2H, t), 2,74-2,82 (4H, m), 6,14-6,18 (3H, m), 6,47 (2H, OH, s), 6,79 (1H, s), 6,92-6,97 (2H, m), 7,11-7,17 (1H, t).

### EXEMPLE 14

### 2-hydroxyméthyl-4-{2-[3-(5-hydroxy-5-méthyl-hexyl)-phényl]-vinyl}-phenol.

### a) 4-bromo-2-hydroxyméthyl-phénol .

De manière analogue à l'exemple 3(a), par réaction de 190 mg (5 mmol) de borohydrure de sodium avec 1 g (5 mmol) de 5-bromosalicylilaldehyde dans 10 ml de méthanol et 15 ml de THF, des cristaux beiges (m= 900 mg ; R= 89%) sont obtenus.
**RMN 1H** (CDCl3 + DMSO): 4,47 (1H, OH, s), 4,71 (2H, s), 6,73-6,76 (1H, d, J= 9,2 Hz), 7,19-7,22 (2H, m), 8,82 (1H, OH, s).

### b) 6-bromo-2,2-diméthyl-4H-benzo[1,3]dioxine.

59 ml (0,48 mol) de diméthoxypropane et 2 g (0,01 mol) d'acide para-toluène sulfonique sont ajoutés à 42,5 g (0,2 mol) de 4-bromo-2-hydroxyméthyl-phénol dans 400 ml de diméthylformamide. Le milieu réactionnel est agité toute une nuit à température ambiante. Il est versé dans de l'eau glacée et extrait à l'éther. La phase organique est séchée sur sulfate de magnésium et concentrée. Le produit est purifié sur colonne de silice (dichlorométhane).
Huile jaune. m= 47,2 g. R= 93%.
**RMN 1H** (CDCl3): 1,52 (6H, s), 4,80 (2H, s), 6,68-6,72 (1H, d, J= 8,7 Hz), 7,09-7,10 (1H, d, J= 2,2 Hz), 7,22-7,27 (1H, m).

### c) 2,2-diméthyl-4H-benzo[1,3]dioxine-6-carbaldéhyde.

De manière analogue à l'exemple 4(c), par réaction de 49 ml de n-Butyllithium 2,5 M/Hexane, à -78°C, avec 27 g 111 mmol) de 6-bromo-2,2-diméthyl-4H-benzo[1,3]dioxine dans 200 ml de THF et 30 minutes après, avec 8,5 ml (111 mmol) de diméthylformamide, après purification sur colonne de silice (dichlorométhane 70 - heptane 30), des cristaux blancs (m= 7,7 g ; R= 36%) sont obtenus. (rendement faible, le produit se dégrade très rapidement).Tf= 49-51°C.
**RMN 1H** (CDCl3): 1,57 (6H, s), 4,90 (2H, s), 6,91-6,94 (1H, d, J= 8,4 Hz), 7,55 (1H, t), 7,68-7,72 (1H, dd, J= 6,5 Hz, J'= 1,9 Hz).

### d) 6-[2-(3-bromo-phényl)-vinyl]-2,2-diméthyl-4H-benzo[1,3]dioxine.

De manière analogue à l'exemple 1(g), par réaction de 78 mg (2,59 mmol) d'hydrure de sodium avec 410 mg (2,14 mmol) de 2,2-diméthyl-4H-benzo[1,3]dioxine-6-carbaldéhyde et 775 mg (2,52 mmol) de (3-bromo-benzyl)-phosphonate d'éthyle dans 20 ml de THF, des cristaux jaunâtres (m= 596 mg ; R= 81%) sont obtenus. Tf= 128-9°C.
**RMN 1H** (CDCl3): 1,56 (6H, s), 4,87 (2H, s), 6;80-6,89 (2H, m), 6,98-7,04(1H, d, J= 16,3 Hz), 7,11 (1H, s), 7,16-7,22 (1H, m), 7,31-7,3 8 (3H, m), 7,62 (1H, s).

### e) 2,2-diméthyl-6-(2-{3-[5-méthyl-5-(tétrahydro-pyran-2-yloxy)-hexyl]-phényl}-vinyl)-4H-benzo[1,3]dioxine.

De manière analogue à l'exemple 8(j), par réaction de 500 mg (1,45 mmol) de 6-[2-(3-bromo-phényl)-vinyl]-2,2-diméthyl-4H-benzo[1,3]dioxine dans 2 ml d'éther avec la solution de 97 mg (4 mmol) de magnésium, 2,02 g (7,24 mmol) de 2-(5-bromo-1,1-diméthyl-pentyloxy)-tétrahydro-pyranne dans 7 ml de THF et catalysée par 31 mg (0,058 mmol) de [1,2-bis-(diphénylphosphino)éthane]dichloronickel, une huile jaune (m= 323 mg ; R= 48%) est obtenue.
**RMN 1H** (CDCl3): 1,19-1,21 (6H, d), 1,56 (6H, s), 1,48-1,82 (12H, m), 2,60-2,66 (2H, t), 3,43-3,45 (1H, m), 3,91 (1H, m), 4,69 (1H, m), 4,87 (2H, s), 6,79-6,83 (1H, d, J= 8,4 Hz), 6,96-6,99 (1H, d, J= 7,2 Hz), 7,05-7,07 (1H, d, J= 4,95 Hz), 7,12 (1H, s), 7,21-7,35 (4H, m).

### f) 2-hydroxyméthyl-4-{2-[3-(5-hydroxy-5-méthyl-hexyl)-phényl]-vinyl}-phénol.

De manière analogue à l'exemple 8(k), par réaction de 313 mg (0,67 mmol) de 2,2-diméthyl-6-(2-{3-[5-méthyl-5-(tétrahydro-pyran-2-yloxy)-hexyl]-phényl}-vinyl)-4H-benzo[1,3]dioxine dans 10 ml d'acide acétique, 5 ml de THF et 2,5 ml d'eau, et après purification sur colonne de silice (acétate d'éthyle 40 - heptane 60), des cristaux blancs (m= 91 mg ; R= 40 %) sont obtenus. Tf= 130-1°C.
**RMN 1H** (CDCl3): 1,01 (6H, s), 1,32 (4H, m), 1,51 (2H, m), 2,50-2,56 (2H, t), 4,04 (1H, OH s), 4,44-4,46 (2H, d, J= 4,4 Hz), 4,98 (1H? OH, m), 6,70-6,74 (1H, d, J= 8,3 Hz), 6,88-7,34 (7H, m), 7,50 (1H, s), 9,51 (1H, OH, s).

### EXEMPLE 15

### 2-hydroxyméthyl-4-{2-[4-(5-hydroxy-5-méthyl-hexyl)-phényl]-vinyl}-phénol.

### a) 6-[2-(4-bromo-phényl)-vinyl]-2,2-diméthyl-4H-benzo[1,3]dioxine.

De manière analogue à l'exemple 1(g), par réaction de 188 mg (6,24 mmol) d'hydrure de sodium avec 1 g (5,2 mmol) de 2,2-diméthyl-4H-benzo[1,3]dioxine-6-carbaldéhyde et 1,52 g (5,2 mmol) de (4-bromo-benzyl)-phosphonate d'éthyle dans 10 ml de THF, un solide blanc (m= 1,4 g; R= 78%) est obtenu.
**RMN 1H** (CDCl3): 1,55 (6H, s), 4,86 (2H, s), 6,79-6,96 (3H, m), 7,03 (1H, s), 7,30-7,33 (3H, m), 7,43-7,46 (2H, d, J= 8,5 Hz).

### b) 2,2-diméthyl-6-(2-{4-[5-méthyl-5-(tétrahydro-pyran-2-yloxy)-hexyl]-phényl]}-vinyl)-4H-benzo[1,3]dioxine.

De manière analogue à l'exemple 8(j), par réaction de 500 mg (1,45 mmol) de 6-[2-(4-bromo-phényl)-vinyl]-2,2-diméthyl-4H-benzo[1,3]dioxine dans 2 ml d'éther avec la solution de 97 mg (4 mmol) de magnésium, 2,02 g (7,24 mmol) de 2-(5-bromo-1,1-diméthyl-pentyloxy)-tétrahydro-pyranne dans 7 ml de THF et catalysée par 31 mg (0,058 mmol) de [1,2-bis-(diphénylphosphino)éthane]dichloronickel, un solide jaunâtre (m= 654 mg ; R= 97%) est obtenu. Tf= 86-7°C.
RMN 1H (CDCl3): 1,18-1,20 (6H, d), 1,50-1,83 (18H, m), 2,58-2,61 (2H, t), 3,45 (1H, m), 3,93 (1H, m), 4,71 (1H, m), 4,87 (2H, m), 6,79-6,82 (1H, m), 6,96 (2H, m), 7,12-7,17 (3H, m), 7,31-7,40 (3H, m).

### c) 2-hydroxyméthyl-4-{2-[4-(5-hydroxy-5-méthyl-hexyl)-phényl]-vinyl}-phénol.

De manière analogue à l'exemple 8(k), par réaction de 640 mg (1,38 mmol) de 2,2-diméthyl-6-(2-{4-[5-méthyl-5-(tétrahydro-pyran-2-yloxy)-hexyl]-phényl)}-vinyl)-4H-benzo[1,3]dioxine dans 24 ml de la solution (20 ml d'acide acétique + 10 ml de THF + 5 ml d'eau), une poudre blanche (m= 140 mg ; R= 50%) est obtenue. Tf= 139-40°C.
**RMN 1H** (DMSO): 1,11 (6H, s), 1,42 (4H, m), 1,60 (2H, m), 2,57-2,63 (2H, t), 4,14 (1H, s), 4,55-4,57 (2H, d, J= 4,6 Hz), 5,09 (1H, OH, m), 6,81-6,84 (1H, d, J= 8,3 Hz), 6,98-7,14 (2H, m), 7,21-7,24 (2H, d, J= 8,1 Hz), 7,32-7,35 (1H, d, J= 8,9 Hz), 7,50-7,53 (2H, d, J= 7,8 Hz), 7,60 (1H, s), 9,61 (1H, s).

### EXEMPLE 16

### 2-hydroxyméthyl-4-{2-[3-(6-hydroxy-6-méthyl-heptyl)-phényl]-vinyl}-phénol.

### a) 2,2-diméthyl-6-(2-{3-[6-méthyl-6-(tétrahydro-pyran-2-yloxy)-heptyl]-phényl}-vinyl)-4H-benzo[1,3]dioxine.

De manière analogue à l'exemple 8(j), par réaction de 467 mg (1,35 mmol) de 6-[2-(3-bromo-phényl)-vinyl]-2,2-diméthyl-4H-benzo[1,3]dioxine dans 2 ml d'éther avec la solution de 90 mg (3,71 mmol) de magnésium, 990 mg (3,37 mmol) de 2-(6-bromo-1,1-diméthyl-hexyloxy)-tétrahydro-pyranne dans 3,5 ml de THF et catalysée par 29 mg (0,05 mmol) de [1,2-bis-(diphénylphosphino)éthane]dichloronickel, une huile jaunâtre (m= 363 mg ; R= 56%) est obtenue.
**RMN 1H** (CDCl3): 1,18-1,20 (6H, d), 1,56 (6H, s), 1,35-1,83 (14H, m), 2,58-2,64 (2H, t), 3,41-3,45 (1H, m), 3,92-3,96 (1H, m), 4,69 (1H, m), 4,87 (2H, s), 6,79-6,83 (1H, d, J= 8,5 Hz), 6,90-7,04 (2H, dd, J= 16,3 Hz, J'= 7,4 Hz), 7,04-7,07 (1H, m), 7,13 (1H, s), 7,21-7,35 (4H, m).

### b) 2-hydroxyméthyl-4-{2-[3-(6-hydroxy-6-méthyl-heptyl)-phényl]-vinyl}-phénol.

De manière analogue à l'exemple 8(k), par réaction de 350 mg (0,73 mmol) de 2,2-diméthyl-6-(2-{3-[6-méthyl-6-(tétrahydro-pyran-2-yloxy)-heptyl]-phényl}-vinyl)-4H-benzo[1,3]dioxine dans 15 ml de la solution (20 ml d'acide acétique + 10 ml de THF + 5 ml d'eau), une poudre blanchâtre (m= 165 mg ; R= 70%) est obtenue. Tf= 120-22°C.
**RMN 1H** (DMSO): 0,85 (6H, s), 1,12 (6H, m), 1,40 (2H, m), 2,35-2,41 (2H, t), 3,87 (1H, OH, s), 4,29-4,31 (2H, d, J= 4,8 Hz), 4,83 (1H, t), 6,55-6,59 (1H, d, J= 8,2 Hz), 6,73-7,18 (7H, m), 7,35 (1H, s), 9,36 (1H, s).

### EXEMPLE 17

### 2-hydroxyméthyl-4-{2-[4-(6-hydroxy-6-méthyl-heptyl)-phényl]-vinyl}-phénol.

### a) 2,2-diméthyl-6-(2-{4-[6-méthyl-6-(tétrahydro-pyran-2-yloxy)-heptyl]-phényl}-vinyl)-4H-benzo[1,3]dioxine.

De manière analogue à l'exemple 8(j), par réaction de 467 mg (1,35 mmol) de 6-[2-(4-bromo-phényl)-vinyl]-2,2-diméthyl-4H-benzo[1,3]dioxine dans 2 ml d'éther avec la solution de 90 mg (3,71 mmol) de magnésium, 990 mg (3,37 mmol) de 2-(6-bromo-1,1-diméthyl-hexyloxy)-tétrahydro-pyranne dans 3,5 ml de THF et catalysée par 29 mg (0,05 mmol) de [1,2-bis-(diphénylphosphino)éthane]dichloronickel, des cristaux blanchâtres (m= 563 mg ; R= 87%) sont obtenus. Tf= 72°C.
**RMN 1H** (CDCl3): 1,18-1,20 (6H, d), 1,55 (6H, s), 1,25-1,83 (14H, m), 2,56-2,63 (2H, t), 3,41-3,45 (1H, m), 3,92-3,96 (1H, m), 4,69-4,71 (1H, m), 4,87 (2H, s), 6,79-6,82 (1H, d, J= 8,5 Hz), 6,88-7,02 (2H, dd, J= 16,4 Hz, J'= 2,3 Hz), 7,11-7,16 (3H, m), 7,30-7,33 (1H, d, J= 8,5 Hz), 7,37-7,40 (2H, d, J= 8,1 Hz).

### b) 2-hydroxyméthyl-4-{2-[4-(6-hydroxy-6-méthyl-heptyl)-phényl]-vinyl}-phénol.

De manière analogue à l'exemple 8(k), par réaction de 548 mg (1,14 mmol) de 2,2-diméthyl-6-(2-{4-[6-méthyl-6-(tétrahydro-pyran-2-yloxy)-heptyl]-phényl}-vinyl)-4H-benzo[1,3]dioxine dans 20 ml de la solution (20 ml d'acide acétique + 10 ml de THF + 5 ml d'eau), une poudre blanche (m= 290 mg ; R= 72%) est obtenue. Tf= 145-6°C.
**RMN 1H** (DMSO): 0,87 (6H, s), 1,13 (6H; m), 1,39 (2H, m), 2,37-2,40 (2H, t), 3,88 (1H, OH, s), 4,30-4,32 (2H, d, J= 5,1 Hz), 4,83-4,85 (1H, t), 6,57-6,6 (1H, d, J= 8,25 Hz), 6,74-6,89 (2H, m), 6,96-6,99 (2H, d, J= 8,2 Hz), 7,07-7,11 (1H, d, J= 8,3 Hz), 7,26-7,29 (2H, d, J= 8 Hz), 7,35 (1H, s), 9,36 (1H, s).

### EXEMPLE 18

### 2-hydroxyméthyl-4-{2-[3-(5-hydroxy-5-méthyl-heptyl)-phényl]-éthyl}-phénol.

De manière analogue à l'exemple 9(a), par réaction de 50 mg (0,147 mmol) de 2-hydroxyméthyl-4-{2-[3-(5-hydroxy-5-méthyl-hexyl)-phényl]-vinyl}-phénol avec 10 mg de Palladium/charbon 10%, des cristaux blanchâtres (m= 26 mg ; R= 52%) sont obtenus. Tf= 101-4°C.
**RMN 1H** (DMSO): 0,93 (6H, s), 1,23 (4H, m), 1,40 (2H, m), 2,39-2,44 (6H, m), 3,96 (1H, OH, s), 4,32-4,34 (2H, d), 4,78-4,83 (1H, t), 6,51-6,55 (1H, d, J= 8,1 Hz), 6,74-7,07 (6H, m), 8,97 (1H, OH, s).

### EXEMPLE 19

### 2-hydroxyméthyl-4-{2-[4-(5-hydroxy-5-méthyl-hexyl)-phényl]-éthyl}-phénol.

De manière analogue à l'exemple 9(a), par réaction de 110 mg (0,32 mmol) de 2-hydroxyméthyl-4-{2-[4-(5-hydroxy-5-méthyl-hexyl)-phényl]-vinyl}-phénol avec 30 mg de Palladium/charbon 10%, des cristaux (m= 81 mg ; R= 74%) sont obtenus. Tf= 124-6°C.
**RMN 1H** (DMSO): 1,12 (6H, s), 1,42 (4H, m), 1,59 (2H, m), 2,58 (6H, m), 4,52-4,54 (2H, d), 4,98-5,03 (1H, OH, t), 5,84 (1H, s), 6,71-6,74 (1H, d, J= 8,1 Hz), 6,94-6,97 (1H, d, J= 8,3 Hz), 7,14-7,24 (5H, m), 9,17 (1H, OH s).

### EXEMPLE 20

### 2-hydroxyméthyl-4-{2-[3-(6-hydroxy-6-méthyl-heptyl)-phényl]-éthyl}-phénol.

De manière analogue à l'exemple 9(a), par réaction de 97 mg (0,27 mmol) de 2-hydroxyméthyl-4-{2-[3-(6-hydroxy-6-méthyl-heptyl)-phényl]-vinyl}-phénol avec 27 mg de Palladium/charbon 10%, des cristaux jaunâtres (m= 70 mg ; R= 73%) sont obtenus. Tf= 73-5°C.
**RMN 1H** (CDCl3): 1,21 (6H, s), 1,23-1,61 (8H, m), 2,51-2,57 (3H, t), 2,82 (4H, s), 4,80 (2H, s), 6,75-7,00 (6H, m), 7,15-7,21 (1H, t), 7,36 (1H, OH, s).

### EXEMPLE 21

### 2-hydroxyméthyl-4-{2-[4-(6-hydroxy-6-méthyl-heptyl)-phényl]-éthyl}-phénol.

De manière analogue à l'exemple 9(a), par réaction de 150 mg (0,42 mmol) de 2-hydroxyméthyl-4-{2-[4-(6-hydroxy-6-méthyl-heptyl)-phényl]-vinyl}-phénol avec 42 mg de Palladium/charbon 10%, des cristaux (m= 65 mg ; R= 43%) sont obtenus. Tf= 110-1°C.
**RMN 1H** (DMSO): 0,85 (6H, s), 1,10 (6H, m), 1,34 (2H, m), 2,29-2,35 (6H, m), 3,86 (1H, s), 4,24-4,26 (2H, d), 4,70-4,74 (1H, t), 6,43-6,46 (1H, d, J= 8,09 Hz), 6,66-6,70 (1H, d, J= 8,1 Hz), 6,86-6,96 (5H, m), 8,89 (1H, s).

### EXEMPLE 22

### 2-hydroxyméthyl-5-{2-[4-(5-hydroxy-5-méthyl-hexyl)-phényl]-vinyl}-phénol.

### a) Acide 2-hydroxy-4-iodo-benzoïque.

40 g (1 mol) de soude sont ajoutés à 55,6 g (0,2 mol) de méthyl-4-iodosalicylate dans 600 ml de THF, 10 ml de méthanol et 10 ml d'eau. La solution est chauffée à 40°C pendant la nuit. Elle est, alors, évaporée. Le résidu est repris par de l'eau. On acidifie à pH 1 avec de l'acide chlorhydrique concentré et on extrait avec de l'acétate d'éthyle. La phase organique est lavée plusieurs fois à l'eau, séchée sur sulfate de magnésium et concentrée. Le produit est trituré dans un mélange dichlorométhane - hexane puis est filtré.
Poudre orange. m= 48,53 g. R= 92%.
**RMN 1H** (CDCl3): 7,20-7,24 (1H, dd, J= 6,8 Hz, J'= 1,5 Hz), 7,36-7,37 (1H, d, J= 1,5 Hz), 7,52-7,56 (1H, d, J= 8,3 Hz), 11,29 (1H, s).

### b) 5-hydroxyméthyl-5-iodo-phénol.

De manière analogue à l'exemple 1(c), par réaction de 300 ml de borane 1M/THF avec 48,45 g (0,183 mol) de l'acide 2-hydroxy-4-iodo-benzoïque dans un litre de THF, après purification sur colonne de silice (dichlorométhane puis acétate d'éthyle), des cristaux beiges (m= 26,82 g ; R= 59%) sont obtenus.
**RMN 1H** (CDCl3): 4,69 (2H, s), 6,80-6,83 (1H, d, J= 7,9 Hz), 7,12-7,16 (1H, dd, J= 6,3 Hz, J'= 1,6 Hz), 7,21-7,22 (1H, d, J= 1,6 Hz), 8,94 (1H, s).

### c) 7-iodo-2,2-diméthyl-4H-benzo[1,3]dioxine.

De manière analogue à l'exemple 14(b), par réaction de 22,7 g (0,218 mol) de 2,2-diméthoxypropane, 554 mg (2,9 mmol) d'acide *para*-toluène sulfonique avec 24,25 g (0,097 mol) de 5-hydroxyméthyl-5-iodo-phénol dans 500 ml de DMF, après purification sur colonne de silice (dichlorométhane 50 - heptane 50), des cristaux blancs (m= 21,88 g ; R= 78%) sont obtenus. Tf= 50-2°C.
**RMN 1H** (CDCl3): 1,52 (6H, s), 4,77 (2H, s), 6,70 (1H, m), 7,19-7,22 (2H, m).

### d) 2,2-diméthyl-4H-benzo[1,3]dioxine-7-carbaldéhyde.

De manière analogue à l'exemple 4(c), par réaction de 31 ml de n-Butyllithium 2,5 M/Hexane, à -78°C, avec 20,4 g (70 mmol) de 7-iodo-2,2-diméthyl-4H-benzo[1,3]dioxine dans 300 ml de THF et 30 minutes après, avec 6 ml (77 mmol) de diméthylformamide, après purification sur colonne de silice (dichlorométhane 60 - heptane 40), des cristaux jaunâtres (m= 10,38 g ; R= 85%) sont obtenus.Tf= 53-4°C.
**RMN 1H** (CDCl3): 1,56 (6H, s), 4,90 (2H, s), 7,11-7,14 (1H, d, J= 7,8 Hz), 7,31-7,32 (1H, d, J= 1,26 Hz), 7,40-7,44 (1H, dd, J= 6,4 Hz, J'= 1,4 Hz), 9,91 (1H, s).

### e) 7-[2-(4-bromo-phényl)- vinyl]-2,2-diméthyl-4H-benzo[1,3]dioxine.

De manière analogue à l'exemple 1(g), par réaction de 724 mg (24 mmol) d'hydrure de sodium avec 3,84 g (20 mmol) de 2,2-diméthyl-4H-benzo[1,3]dioxine-7-carbaldéhyde et 7,37 g (24 mmol) de (4-bromo-benzyl)-phosphonate d'éthyle dans 110 ml de THF, un solide blanc (m= 6,5 g ; R= 94%) est obtenu. Tf= 137-9°C.
**RMN 1H** (CDCl3): 1,55 (6H, s), 4,85 (2H, s), 6,93-7,05 (5H, m), 7,32-7,35 (2H, d, J= 8,5 Hz), 7,44-7,47 (2H, d, J= 8,5 hz).

### f) 2,2-diméthyl-7-(2-{4-[5-méthyl-5-(tétrahydro-pyran-2-yloxy)-hexyl]-phényl}-vinyl)-4H-benzo[1,3]dioxine.

De manière analogue à l'exemple 8(j), par réaction de 1,7 g (4,9 mmol) de 7-[2-(4-bromo-phényl)-vinyl]-2,2-diméthyl-4H-benzo[1,3]dioxine dans 6 ml d'éther avec la solution de 330 mg (13,5 mmol) de magnésium, 3,43 g (12,3 mmol) de 2-(5-bromo-1,1-diméthyl-pentyloxy)-tétrahydro-pyranne dans 12 ml de THF et catalysée par 105 mg (0,199 mmol) de [1,2-bis-(diphénylphosphino)éthane]dichloronickel, après purification sur colonne de silice (acétate d'éthyle 10 - heptane 90), des cristaux blancs (m= 2,02 g ; R= 89%) sont obtenus. Tf= 70-4°C. **RMN 1H** (CDCl3): 1,11-1,14 (6H, d), 1,49 (6H, s), 1,19-1,76 (12 H, m), 2,52-2,58 (2H, t), 3,34-3,38 (1H, m), 3,85-3,89 (1H, m), 4,62-4,64 (1H, m), 4,78 (2H, s), 6,85-7,02 (6H, m), 7,08-7,11 (2H, d, J= 8 Hz), 7,32-7,35 (2H, d, J= 8 Hz).

### g) 2-hydroxyméthyl-5- {2-[4-(5-hydroxy-5-méthyl-hexyl)-phényl]-vinyl}-phénol.

De manière analogue à l'exemple 8(k), par réaction de 2 g (4,3 mmol) de 2,2-diméthyl-7-(2-{4-[5-méthyl-5-(tétrahydro-pyran-2-yloxy)-hexyl]-phényl}-vinyl)-4H-benzo[1,3]dioxine dans une solution de 50 ml d'acide acétique + 25 ml de THF + 12,5 ml d'eau, après purification sur colonne de silice (acétate d'éthyle 50 - heptane 50), une poudre blanche (m= 44 mg ; R= 3%) est obtenue. Tf=163-5°C.
**RMN 1H** (DMSO): 0,87 (6H, s), 1,18 (4H, m), 1,36 (2H, m), 2,40 (2H, t), 3,89 (1H, s), 4,30 (2H, s), 6,77 (1H, s), 6,88 (3H, m), 6,99-7,02 (2H, d, J= 7,6 Hz), 7,08-7,11 (1H, d, J= 7,9 Hz), 7,30-7,33 (2H, d, J= 7,8 Hz), 9,24 (1H, s).

### EXEMPLE 23

### 6-[3-(3,4-bis-hydroxyméthyl-benzyloxy)-phényl]-2-méthyl-heptan-2-ol.

### a) 6-{3-[3-(tert-butyl-diméthyl-silanyloxyméthyl)-4-(1,1,2,2-tétraméthyl-propoxyméthyl)-benzyloxy]-phényl}-2-méthyl-heptan-2-ol.

De manière analogue à l'exemple 3(j), par réaction de 24 mg (0,1 mmol) de 3-(5-hydroxy-1,5-diméthyl-hexyl)-phénol, de 3,4 mg (0,11 mmol) d'hydrure de sodium 80% dans 2 ml de diméthylformamide avec 50 mg (0,1 mmol) de 4-bromométhyl-1-(*tert*-butyl-diméthyl-silanyloxy)-2-(*tert*-butyl-diméthyl-silanyloxy méthyl)-benzène dans 4 ml de diméthylformamide, après purification sur colonne de silice (Acétate d'éthyle 20 - heptane 80), une huile jaune (m= 50 mg ; R= 77%) est obtenue.
**RMN 1H** (CDCl3): 0,00 (12H, s), 0,84 (18H, s), 1,06 (6H, s), 1,11-1,14 (3H, d), 1,19-1,48 (6H, m), 2,56-2,58 (1H, m), 4,65 (2H, s), 4,66 (2H, s), 4,95 (2H, s), 6,67-6,71 (3H, m), 7,07-7,13 (1H, m), 7,21-7,25 (1H, d, J= 8,4 Hz), 7,32-7,35 (1H, d, J= 7,8 Hz), 7,41 (1H, s).

### b) 6-[3-(3,4-bis-hydroxyméthyl-benzyloxy)-phényl]-2-méthyl-heptan-2-ol.

De manière analogue à l'exemple 3(i), par réaction de 50 mg (0,08 mmol) de 6-{3-[3-(*tert*-butyl-diméthyl-silanyloxyméthyl)-4-(1,1,2,2-tétrainéthyl-propoxyméthyl)-benzyloxy]-phényl}-2-méthyl-heptan-2-ol avec 0,17 ml de fluorure de tetrabutylammonium 1M/THF, une huile incolore (m= 29 mg ; R= 94%) est obtenue.
**RMN 1H** (CDCl3): 1,11 (6H, s), 1,20-1,23 (3H, d), 1,33-1,56 (6H, m), 2,60-2,69 (1H, m), 3,64 (2H, OH, s), 4,66 (4H, s), 5,04 (2H, s), 6,76-6,78 (3H, m), 7,15-7,38 (4H, m).

### EXEMPLE 24

### 4-[3-(5-hydroxy-1,5-diméthyl-hexyl)-phenoxyméthyl]-2-hydroxyméthyl-phenol.

### a) (2,2-diméthyl-4H-benzo[1,3]dioxin-6-yl)méthanol.

De manière analogue à l'exemple 3(a), par réaction de 115 mg (3 mmol) de borohydrure de sodium avec 580 mg (3 mmol) de 2,2-diméthyl-4H-benzo[1,3]dioxine-6-carbaldéhyde dans 15 ml de méthanol et 10 ml de THF, après purification sur colonne de silice (acétate d'éthyle 30 - heptane 70), un solide blanc (m= 500 mg ; R= 85%) est obtenu. Tf= 42-4°C.

### b) 6-bromométhyl-2,2-diméthyl-4H-benzo[1,3]dioxine.

De manière analogue à l'exemple 3(b), par réaction de 2,5 ml (5,6 mmol) de trioctylphosphine, de 1,9 g (5,6 mmol) de tétrabromure de carbone avec 500 mg (2,6 mmol) de (2,2-diméthyl-4H-benzo[1,3]dioxin-6-yl)méthanol dans 20 ml d'éther éthylique, après purification sur colonne de silice (AcOEt 8 - Heptane 92), une huile incolore (m= 445 mg ; R= 67%) est obtenue.

### c) 6-[3-(2,2-diméthyl-4H-benzo[1,3]dioxin-6-ylméthoxy)-phényl]-2-méthyl-heptan-2-ol.

De manière analogue à l'exemple 3(j), par réaction de 44 mg (0,2 mmol) de 3-(5-hydroxy-1,5-diméthyl-hexyl)-phénol, de 6,2 mg (0,22 mmol) d'hydrure de sodium 80% dans 5 ml de diméthylformamide avec 52 mg (0,2 mmol) de 6-bromométhyl-2,2-diméthyl-4H-benzo[1,3]dioxine dans 5 ml de diméthylformamide, après purification sur colonne de silice (Acétate d'éthyle 20 - heptane 80), une huile incolore (m= 43 mg ; R= 55%) est obtenue.
**RMN 1H** (CDCl3): 1,15 (6H, s), 1,21-1,23 (3H, d), 1,23-1,54 (6H, m), 2,65 (1H, m), 4,86 (2H, s), 4,93 (2H, s), 6,78-6,85 (4H, m), 7,07 (1H, s), 7,20-7,26 (2H, m).

### d) 4-[3-(5-hydroxy-1,5-diméthyl-hexyl)-phénoxyméthyl]-2-hydroxyméthyl-phénol.

De manière analogue à l'exemple 8(k), par réaction de 43 mg (0,1 mmol) de 6-[3-(2,2-diméthyl-4H-benzo[1,3]dioxin-6-ylméthoxy)-phényl]-2-méthyl-heptan-2-ol dans 2 ml de la solution (5 ml d'acide acétique + 2,5 ml de THF + 1,25 ml d'eau), une huile incolore( m= 12 mg ; R= 31 %) est obtenue.
**RMN 1H** (CDCl3): 1,12 (6H, s), 1,19-1,22 (3H, d), 1,32-1,56 (6H, m), 2,59-2,68 (1H, m), 4,81 (2H, s), 4,96 (2H, s), 6,74-6,79 (3H, m), 6,85-6,89 (1H, d, J= 8,2 Hz), 7,07 (1H, s), 7,15-7,22 (2H, m), 7,69 (1H, OH, s).

### EXEMPLE 25

### 6-{3-[2-(3,4-bis-hydroxyméthyl-phényl)-vinyl]-phényl}-2-méthyl-hexan-2-ol.

### a) 3-bromo-[2-{3,4-bis-(tert-butyl-diméthyl-silanyloxyméthyl)-phényl}-vinyl]-phényl.

De manière analogue à l'exemple 1(g), par réaction de 53 mg (1,8 mmol) d'hydrure de sodium avec 600 mg (1,5 mmol) de 4-(*tert*-butyl-diméthyl-silanyloxy)-3-(*tert*-butyl-diméthyl-silanyloxyméthyl)-benzaldéhyde et 560 mg (1,8 mmol) de (3-bromo-benzyl)-phosphonate d'éthyle dans 30 ml de THF, une huile incolore (m= 830 mg ; R= 100%) est obtenue.
**RMN 1H** (CDCl3): 0,00 (6H, s), 0,02 (6H, s), 0,84 (9H, s), 086 (9H, s), 4,64 (2H, s), 4,66 (2H, s), 6,86-7,04 (2H, dd, J= 16,3 Hz, J'= 11,6 Hz), 7,08-7,14 (1H, m), 7,24-7,32 (4H, m), 7,48 (1H, s), 7,55 (1H, s).

### b) 2-[5-(3-{3,4-bis-(tert-butyl-diméthyl-silanyloxyméthyl)-phényl]-vinyl}-phényl)-1,1-diméthyl-pentyloxy]-tétrahydro-pyranne.

De manière analogue à l'exemple 8(j), par réaction de 236 mg (0,43 mmol) de 3-bromo-[2-{3,4-bis-(*tert*-butyl-diméthyl-silanyloxyméthyl)-phényl}-vinyl]-phényl dans 1.ml d'éther avec la solution de 29 mg (1,18 mmol) de magnésium, 300 mg (1,07 mmol) de 2-(5-bromo-1,1-diméthyl-pentyloxy)-tétrahydro-pyranne dans 2 ml de THF et catalysée par 10 mg (0,019 mmol) de [1,2-bis-(diphénylphosphino)éthane]dichloronickel, après purification sur colonne de silice (acétate d'éthyle 3 - heptane 97), une huile jaunâtre (m= 34 mg ; R= 12%) est obtenue.
**RMN 1H** (CDCl3): -0,02 (6H, s), 0,00 (6H, s), 0,82 (9H, s), 0,84 (9H, s), 1,06-1,08 (6H, d), 1,13-1,70 (12H, m), 2,48-2,54 (2H, t), 3,27-3,36 (2H, m), 3,79 (1H, m), 4,62 (2H, s), 4,64 (2H, s), 6,93-6,97 (3H, m), 7,10-7,19 (3H, m), 7,27 (2H, s), 7,46 (1H, s).

### c) [2-hydroxyméthyl-5-(2-{3-[5-méthyl-5-(tétrahydro-pyran-2-yloxy)-hexyl]-phényl}-vinyl)-phényl]-méthanol.

De manière analogue à l'exemple 3(i), par réaction de 67 mg (0,1 mmol) de 2-[5-(3-{3,4-bis-(*tert*-butyl-diméthyl-silanyloxyméthyl)-phényl]-vinyl}-phényl)-1,1-diméthyl-pentyloxy]-tétrahydro-pyranne avec 0,2 ml de fluorure de tetrabutylammonium 1M/THF, une huile jaunâtre (m= 44 mg ; R= 100%) est obtenue.
**RMN 1H** (CDCl3): 1,19-1,20 (6H, d), 1,43-1,82 (12H, m), 2,61-2,67 (2H, t), 3,42 (1H, m), 3,91 (1H, m), 4,69 (1H, m), 4,75 (2H, s), 4,78 (2H, s), 7,09 (3H, m), 7,26 (2H, m), 7,29-7,35 (2H, m), 7,42-7,45 (1H, m), 7,51 (1H, s).

### d) 6-{3-[2-(3,4-bis-hydroxyméthyl-phényl)-vinyl]-phényl}-2-méthyl-hexan-2-ol.

0,09 ml d'acide sulfurique concentré sont ajoutés à 44 mg (0,1 mmol) de [2-hydroxyméthyl-5-(2-{3-[5-méthyl-5-(tétrahydro-pyran-2-yloxy)-hexyl]-phényl}-vinyl)-phényl]-méthanol dans 1 ml de THF et 1 ml d'eau. La solution est chauffée à 40°C pendant 48 heures. On rajoute de l'eau et on extrait à l'éther. La phase organique est séchée sur sulfate de magnésium et concentrée. Le produit est trituré dans un mélange éther - hexane puis est filtré et séché.
Cristaux blanchâtres. m= 15 mg. R= 42%. Tf= 115-7°C.
**RMN 1H** (CDCl3): 1,21 (6H, s), 1,49-1,66 (6H, m), 2,62-2,65 (2H, t), 4,74 (2H, s), 4,77 (2H, s), 7,10-7,51 (9H, m).

### EXEMPLE 26

### 7-{4-[2-(3,4-bis-hydroxyméthyl-phényl)-vinyl]-phényl}-2-méthyl-heptan-2-ol.

### a) 4-bromo-[2-{3,4-bis-(tert-butyl-diméthyl-silanyloxyméthyl)-phényl}-vinyl]-phényl.

De manière analogue à l'exemple 1(g), par réaction de 61 mg (2,03 mmol) d'hydrure de sodium avec 667 mg (1,69 mmol) de 4-(*tert*-butyl-diméthyl-silanyloxy)-3-(*tert*-butyl-diméthyl-silanyloxyméthyl)-benzaldéhyde et 623 mg (2,03 mmol) de (4-bromo-benzyl)-phosphonate d'éthyle dans 15 ml de THF, après purification sur colonne de silice (dichlorométhane 10 - heptane 90), des cristaux blancs (m= 790 mg ; R= 85%) sont obtenus. Tf= 80-1°C.
**RMN 1H** (CDCl3): 0,00 (6H, s), 0,02 (6H, s), 0,84 (9H, s), 0,86 (9H, s), 4,64 (2H, s), 4,66 (2H, s), 6,88-7,04 (2H, q), 7,25-7,30 (4H, m), 7,35-7,38 (2H, d), 7,48 (1H, s).

### b) 2-[6-(4-{2-[3,4-bis-(tert-butyl-diméthyl-silanyloxyméthyl)-phényl]-vinyl}-phényl)-1,1-diméthyl-hexyloxy]-tétrahydro-pyranne.

De manière analogue à l'exemple 8(j), par réaction de 390 mg (0,71 mmol) de 4-bromo-[2-{3,4-bis-(*tert*-butyl-diméthyl-silanyloxyméthyl)-phényl}-vinyl]-phényl dans 1 ml d'éther avec la solution de 48 mg (1,96 mmol) de magnésium, 520 mg (1,77 mmol) de 2-(6-bromo-1,1-diméthyl-hexyloxy)-tétrahydro-pyranne dans 2 ml de THF et catalysée par 15 mg (0,028 mmol) de [1,2-bis-(diphénylphosphino)éthane]dichloronickel, après purification sur colonne de silice (acétate d'éthyle 3 - heptane 97), une huile jaunâtre (m= 347 mg ; R= 72%) est obtenue.
**RMN 1H** (CDCl3): 0,00 (6H, s), 0,02 (6H, s), 0,84 (9H, s), 0,87 (9H, s), 1,08-1,10 (6H, d), 1,13-1,74 (14H, m), 2,48-2,54 (2H, t), 3,31-3,39 (1H, m), 3,82 (1H, m), 4,59 (1H, m), 4,64 (2H, s), 4,67 (2H, s), 6,97 (2H, s), 7,04-7,08 (2H, d, J= 8 Hz), 7,29-7,34 (4H, m), 7,49 (1H, s).

### c) 7-{4-[2-(3,4-bis-hydroxyméthyl-phényl)-vinyl]-phényl}-2-méthyl-heptan-2-ol.

De manière analogue à l'exemple 25(d), par réaction de 5 gouttes d'acide sulfurique concentré avec 335 mg (0,49 mmol) de 2-[6-(4-{2-[3,4-bis-(*tert*-butyl-diméthyl-silanyloxyméthyl)-phényl]-vinyl}-phényl)-1,1-diméthyl-hexyloxy]-tétrahydro-pyranne dans 5 ml de THF et 5 ml d'eau, des cristaux blancs (m= 94 mg ; R= 52%) sont obtenus. Tf 112-4°C.
**RMN 1H** (CDCl3): 1,20 (6H, s), 1,38 (6H, m), 1,64 (2H, m), 2,61 (2H, t), 4,72 (2H, s), 4,75 (2H, s), 7,06-7,25 (4H, m), 7,347,49 (5H, m).

### EXEMPLE 27

### 6-{4-[2-(3,4-bis-hydroxyméthyl-phényl)-vinyl]-phényl}-2-méthyl-hexan-2-ol.

### a) 2-[5-(4-{2-[3,4-bis-(tert-butyl-diméthyl-silanyloxyméthyl)-phényl]-vinyl}-phényl)-1,1-diméthyl-pentyloxy}-tétrahydro-pyranne.

De manière analogue à l'exemple 8(j), par réaction de 375 mg (0,68 mmol) de 4-bromo-[2-{3,4-bis-(*tert*-butyl-diméthyl-silanyloxyméthyl)-phényl}-vinyl]-phényl dans 1 ml d'éther avec la solution de 46 mg (1,88 mmol) de magnésium, 478 mg (1,71 mmol) de 2-(5-bromo-1,1-diméthyl-pentyloxy)-tétrahydro-pyranne dans 2 ml de THF et catalysée par 15 mg (0,028 mmol) de [1,2-bis-(diphénylphosphino)éthane]dichloronickel, après purification sur colonne de silice (acétate d'éthyle 3 - heptane 97), une huile jaunâtre (m= 278 mg ; R= 61%) est obtenue.
**RMN 1H** (CDCl3): 0,00 (6H, s), 0,02 (6H, s), 0,84 (9H, s), 0,86 (9H, s), 1,08-1,10 (6H, d), 1,18-1,73 (12H, m), 2,49-2,55 (2H, t), 3,36-3,39 (1H, m), 3,81 (1H, m), 4,59 (1H, m), 4,64 (2H, s), 4,67 (2H, s), 6,97 (2H, s), 7,05-7,08 (2H, d, J= 8 Hz), 7,29-7,34 (4H, m), 7,48 (1H, s).

### b) 6-{4-[2-(3,4-bis-hydroxyméthyl-phényl)-vinyl]-phényl}-2-méthyl-hexan-2-ol.

De manière analogue à l'exemple 25(d), par réaction de 10 gouttes d'acide sulfurique concentré avec 265 mg (0,4 mmol) de 2-[5-(4-{2-[3,4-bis-(*tert*-butyl-diméthyl-silanyloxyméthyl)-phényl]-vinyl}-phényl)-1,1-diméthyl-pentyloxy]-tétrahydro-pyranne dans 5 ml de THF et 5 ml d'eau, des cristaux blancs (m= 73 mg ; R =51%) sont obtenus. Tf= 102-4°C.
**RMN 1H** (CDCl3): 1,20 (6H, s), 1,47 (4H, m), 1,63 (2H, m), 2,63 (2H, t), 4,72 (2H, s), 4,74 (2H, s), 6,99-7,15 (2H, dd, J= 16,5 Hz, J'= 5 Hz), 7,15-7,18 (2H, d, J= 8 Hz), 7,31-7,34 (1H, m), 7,40-7,44 (3H, d, J= 8 Hz), 7,50 (1H, s).

### EXEMPLE 28

### 5-{2-[3-(6-hydroxy-6-méthyl-heptyl)-phényl]-1-méthyl-vinyl}-benzène-1,3-diol.

### a) 1-[3,5-bis-(tert-butyl-diméthyl-silanyloxy)-phényl]-éthanone.

De manière analogue à l'exemple 3(c), par réaction de 34,7 g (0,22 mmol) de chlorure de *tert*-butyldiméthylsilane avec 15,68 g (0,1 mmol) de 3,5-dihydroxy-acétophénone et 611 mg de diméthylaminopyridine dans 30,7 ml (0,22 mmol) de triéthylamine et 160 ml de diméthylformamide, après purification sur colonne de silice (acétate d'éthyle 10 -heptane 90), une huile orange (m= 33 22 g ; R= 87%) est obtenue.
**RMN 1H** (CDCl3): 0,01 (12H, s), 0,78 (18H, s), 2,33 (3H, s), 6,32-6,33 (1H, t), 6,82 (1H, s), 6,83 (1H, s).

### b) 3-bromo-{2-[3,5-bis-(tert-butyl-diméthyl-silanyloxy)-phényl]-propenyl}-phényl.

De manière analogue à l'exemple 1(g), par réaction de 362 mg (12 mmol) d'hydrure de sodium avec 3,8 g (10 mmol) de 1-[3,5-bis-(*tert*-butyl-diméthyl-silanyloxy)-phényl]-éthanone et 3,68 g (12 mmol) de (3-bromo-benzyl)-phosphonate d'éthyle dans 75 ml de THF, après purification sur colonne de silice (acétate d'éthyle 10 - heptane 90), des cristaux sont obtenus (m= 2,12 g ; R= 52%).
**RMN 1H** (CDCl3): 0,00 (12H, s), 0,84 (18H, s), 2,03 (3H, d), 6,14 (3H, s), 6,22 (1H, s), 6,77-6,89 (2H, m), 7,00-7,08 (2H, m).

### c) 2-[6-(3-{2-[3,5-bis-(tert-butyl-diméthyl-silanyloxy)-phényl]-propenyl}-phényl)-1,1-diméthyl-hexyloxy]-tétrahydro-pyranne.

De manière analogue à l'exemple 8(j), par réaction de 1,04 g (2 mmol) de 3-bromo-{2-[3,5-bis-(*tert*-butyl-diméthyl-silanyloxy)-phényl]-propenyl}-phényl dans 2,5 ml d'éther avec la solution de 134 mg (5,5 mmol) de magnésium, 1,47 g (5 mmol) de 2-(6-bromo-1,1-diméthyl-hexyloxy)-tétrahydro-pyranne dans 5 ml de THF et catalysée par 43 mg (0,08 mmol) de [1,2-bis-(diphénylphosphino)éthane]dichloronickel, après purification sur colonne de silice (acétate d'éthyle 3 - heptane 97), une huile jaunâtre(m= 1,08 g ; R= 66 %) est obtenue.
**RMN 1H** (CDCl3): 0,00 (12H, s), 0,85 (18H, s), 1,10-1,76 (20H, m), 2,04 (3H, d), 2,31-2,37 (2H, t), 3,36 (1H, m), 3,84 (1H, m), 4,62 (1H, m), 6,14-6,31 (4H, m),6,67-6,93 (4H, m).

### d) 7-(3-{2-[3,5-bis-(tert-butyl-diméthyl-silanyloxy)-phényl]-propenyl}-phényl)-2-méthyl-heptan-2-ol.

De manière analogue à l'exemple 25(d), par réaction de 0,28 ml d'acide sulfurique concentré avec 1,06 g (1,1 mmol) de 2-[6-(3-{2-[3,5-bis-(*tert*-butyl-diméthyl-silanyloxy)-phényl]-propenyl}-phényl)-1,1-diméthyl-hexyloxy]-tétrahydro-pyranne dans 25 ml de THF et 25 ml d'eau, après purification sur colonne de silice (acétate d'éthyle 10 - heptane 90), une huile jaune (m= 420 mg ; R= 43%) est obtenue.
**RMN 1H** (CDCl3): 0,00 (12H, s), 0,85 (18H, s), 1,12 (6H, s), 1,19-1,51 (8H, m), 2,04 (3H, d), 2,31-2,38 (2H, t), 6,14-6,19 (3H, m), 6,31 (1H, s), 6,68-6,79 (3H, m), 6,88-6,94 (1H, t).

### e) 5-{2-[3-(6-hydroxy-6-méthyl-heptyl)-phényl]-1-méthyl-vinyl}-benzène-1,3-diol.

De manière analogue à l'exemple 3(i), par réaction de 402 mg (0,69 mmol) de 7-(3-{2-[3,5-bis-(*tert*-butyl-diméthyl-silanyloxy)-phényl]-propenyl}-phényl)-2-méthyl-heptan-2-ol avec 1,4 ml de fluorure de tetrabutylammonium 1M/THF, après purification sur colonne de silice (acétate d'éthyle 40 - heptane 60), des cristaux blancs (m= 176 mg ; R= 72%) sont obtenus. Tf= 113-4°C.
**RMN 1H** (CDCl3):1,20 (6H, s), 1,20-1,44 (8H,m), 2,12-2,13 (3H, d), 2,33-2,39 (2H, t), 2,89 (1H, OH, s), 6,19-6,20 (2H, d, J= 2 Hz), 6,30-6,35 (2H, m), 6,80-6,90 (3H, m), 7,01-7,07 (1H, t), 7,98 (2H, OH, s).

### EXEMPLE 29

### 5-{2-[3-(5-hydroxy-5-méthyl-hexyl)-phényl]-vinyl}-benzène-1,3-diol

### a) 2-[5-(3-{3,5-bis-(tétrahydropyran-2-yloxy)-phényl]-vinyl}-phényl)-1,1-diméthyl-pentyloxy]-tétrahydro-pyranne.

De manière analogue à l'exemple 8(j), par réaction de 2,59 g (5,64 mmol) de 3-bromo-{2-[3-(tétrahydro-pyran-2-yl)-5-(tétrahydro-pyran-2-yloxy)-phényl]-vinyl}-phényl dans 7 ml d'éther avec la solution de 377 mg (15,5 mmol) de magnésium, de 3,94 g (14,1 mmol) de 2-(5-bromo-1,1-diméthyl-pentyloxy)-tétrahydro-pyranne dans 14 ml de THF et catalysée par 121 mg (0,23 mmol) de [1,2-bis-(diphénylphosphino)éthane]dichloronickel, après purification sur colonne de silice (acétate d'éthyle 10 - heptane 90), une huile jaunâtre (m= 2,65 g ; R= 81%) est obtenue.
**RMN 1H** (CDCl3): 1,19-1,21 (6H, d), 1,48-1,63 (18H, m), 1,87-2,01 (6H, m), 2,60-2,66 (2H, t), 3,41-3,45 (1H, m), 3,61-3,65 (2H, m), 3,90-3,97 (3H, m), 4,69 (1H, m), 5,45-5,46 (2H, d), 6,70 (1H, s), 6,87 (2H, s), 7,03-7,08 (3H, m), 7,21-7,30 (3H, m).

### b) 5-{2-[3-(5-hydroxy-5-méthyl-hexyl)-phényl]-vinyl}-benzène-1,3-diol.

De manière analogue à l'exemple 25(d), par réaction de 0,75 ml d'acide sulfurique concentré avec 2,58 g (4,46 mmol) de 2-[5-(3-{3,5-bis-(tétrahydropyran-2-yloxy)-phényl]-vinyl}-phényl)-1,1-diméthyl-pentyloxy]-tétrahydro-pyranne dans 15 ml de THF et 15 ml d'eau, après purification sur colonne de silice (acétate d'éthyle 40 - heptane 60), des cristaux blancs (m= 680 mg ; R= 47%) sont obtenus. Tf= 145-7°C.
**RMN 1H** (CDCl3): 1,20 (6H, s), 1,31-1,64 (6H, m), 2,59-2,65 (2H, t), 2,83 (1H, OH, s), 6,34 (1H, s), 6,54 (2H, s), 6,96 (2H, m), 7,04-7,06 (1H, d, J= 7 Hz), 7,20-7,36 (3H, m), 8,64 (2H, OH, s).

### EXEMPLE 30

### 5-[3-(6-hydroxy-6-méthyl-heptyl)-phénoxyméthyl]-benzène-1,3-diol.

### a) 3,5-dihydroxybenzoate de méthyle.

De manière analogue à l'exemple 1(a), par réaction de 44 ml (0,8 mol) d'acide sulfurique concentré avec 61,65 g (0,4 mol) de 3,5-dihydroxybenzoic acid dans 600 ml de méthanol, une poudre blanche (m= 63,86 g ,R= 95%) est obtenue. Tf= 162-3°C.
RMN 1H (CDCl3): 3,85 (3H, s), 6,59-6,61 (1H, t), 7,02-7,03 (2H, d), 8,67 (2H, s).

### b) 3,5-bis-(tert-butyl-diméthyl-silanyloxy)-benzoate de méthyle.

De manière analogue à l'exemple 3(c), par réaction de 126 g (836 mmol) de chlorure de *tert*-butyldiméthylsilane avec 63,86 g (380 mmol) de 3,5-dihydroxybenzoate de méthyle, 2,32 g de diméthylaminopyridine dans 116,5 ml (836 mmol) de triéthylamine et 600 ml de diméthylformamide, après purification sur colonne de silice (dichlorométhane 30 -heptane 90), une huile jaune (m= 113,46 g ; R= 75%) est obtenue.
**RMN 1H** (CDCl3): 0,00 (12H, s), 0,78 (18H, s), 3,68 (3H, s), 6,31-6,32 (1H, t), 6,91-6,92 (2H, d).

### c) [3,5-bis-(tert-butyl-diméthyl-silanyloxy)-phényl]-méthanol.

De manière analogue à l'exemple 1(e) par réaction de 144 ml d'hydrure de diisobutylaluminium 1M/toluène, à -78°C, avec 18,86 g (47 mmol) de 3,5-bis-(*tert*-butyl-diméthyl-silanyloxy)-benzoate de méthyle, une huile incolore (m= 16,97 g ; R= 98%) est obtenue.
**RMN 1H** (CDCl3): 0,00 (12H, s), 0,78 (18H, s), 4,36-4,38 (2H, d), 6,05-6,07 (1H, t), 6,27-6,28 (2H, d).

### d) 1-bromométhyl-3,5-bis-(tert-butyl-diméthyl-silanyloxy)-benzène.

De manière analogue à l'exemple 3(b), par réaction de 45,6 ml (92 mmol) de trioctylphosphine, de 33,56 g (101 mmol) de tétrabromure de carbone avec 16,95 g (46 mmol) de [3,5-bis-(*tert*-butyl-diméthyl-silanyloxy)-phényl]-méthanol dans 300 ml d'éther éthylique, après purification sur colonne de silice (dichlorométhane 10 - Heptane 90), une huile incolore (m= 15,61 g ; R= 79%) est obtenue.
**RMN 1H** (CDCl3): 0,00 (12H, s), 0,78 (18H, s), 4,16 (2H, s), 6,05-6,07 (1H, t), 6,28-6,29 (2H, d).

### e) 3-bromo-[3,5-bis-(tert-butyl-diméthyl-silanyloxy)-benzyloxy]-phényl.

A une solution de 865 mg (5 mmol) de 3-bromo-phenol et 3,24 g (7,5 mmol) de 1-bromométhyl-3,5-bis-(*tert*-butyl-diméthyl-silanyloxy)-benzène dans 80 ml de 2-butanone, on ajoute 1,04 g (7,5 mmol) de carbonate de potassium et 166 mg (1 mmol) de iodure de potassium. Le milieu réactionnel est chauffé 6 heures à reflux, puis agité à température ambiante durant la nuit. Il est ensuite versé dans une solution aqueuse saturée en chlorure d'ammonium et extrait à l'éther. La phase organique est lavée à l'eau, séchée sur sulfate de magnésium et concentrée. Le résidu est purifié sur colonne de silice (dichlorométhane 15 - heptane 85).
Huile jaunâtre. m= 1,09 g. R= 42%.
**RMN 1H** (CDCl3): 0,00 (12H, s), 0,78 (18H, s), 4,76 (2H, s), 6,09-6,11 (1H, t), 6,31-6,32 (2H, d), 6,67-6,71 (1H, m), 6,87-6,98 (3H, m).

### f) 2-(6-{3-[3,5-bis-(tert-butyl-diméthyl-silanyloxy)-benzyloxy]-phényl}-1,1-diméthyl-hexyloxy)-tétrahydro-pyranne.

De manière analogue à l'exemple 8(j), par réaction de 1,06 g (2 mmol) de 3-bromo-[3,5-bis-(*tert*-butyl-diméthyl-silanyloxy)-benzyloxy]-phényl dans 2,5 ml d'éther avec la solution de 135 mg (5,56 mmol) de magnésium, de 1,47 g (5 mmol) de 2-(6-bromo-1,1-diméthyl-hexyloxy)-tétrahydro-pyranne dans 5 ml de THF et catalysée par 45 mg (85 mmol) de [1,2-bis-(diphénylphosphino)éthane]dichloronickel, après purification sur colonne de silice (acétate d'éthyle 3 - heptane 97), une huile jaune (m= 793 mg ; R= 60%) est obtenue.
**RMN 1H** (CDCl3): 0,00 (12H, s), 0,79 (18H, s), 1,00-1,02 (6H, d), 1,08-1,66 (14H, m), 2,35-2,42 (2H, t), 3,23-3,28 (1H, m), 3,74-3,78 (1H, m), 4,51 (1H, m), 4,76 (2H, s), 6,08-6,09 (1H, t), 6,34-6,35 (2H, d), 6,57-6,60 (3H, m), 6,95-7,02 (1H, t).

### g) 5-{3-[6-méthyl-6-(tétrahydro-pyran-2-yloxy)-heptyl]-phénoxyméthyl}-benzène-1,3-diol.

De manière analogue à l'exemple 3(i), par réaction de 777 mg (1,18 mmol) de 2-(6-{3-[3,5-bis-(*tert*-butyl-diméthyl-silanyloxy)-benzyloxy]-phényl}-1,1-diméthyl-hexyloxy)-tétrahydro-pyranne avec 2,4 ml de fluorure de tetrabutylammonium 1M/THF, après purification sur colonne de silice (acétate d'éthyle 30 - heptane 70), une huile jaune (m= 505 mg ; R= 99%) est obtenue.
**RMN 1H** (CDCl3): 1,17-1,19 (6H, d), 1,29-1,84 (14H, m), 2,51-2,57 (2H, t), 3,50 (1H, m), 3,96 (1H, m), 4,73 (1H, m), 4,98 (2H, s), 6,29 (1H, s), 6,45 (2H, s), 6,72-6,75 (3H, m), 7,12-7,18 (1H, t).

### h) 5-[3-(6-hydroxy-6-méthyl-heptyl)-phénoxyméthyl]-benzène-1,3-diol.

De manière analogue à l'exemple 25(d), par réaction de 0,07 ml d'acide sulfurique concentré avec 505 mg (1,18 mmol) de 5-{3-[6-méthyl-6-(tétrahydro-pyran-2-yloxy)-heptyl]-phénoxyméthyl}-benzène-1,3-diol dans 4 ml de THF et 2 ml d'eau, après purification sur colonne de silice (acétate d'éthyle 40 - heptane 60), une pâte jaunâtre (m= 342 mg ; R= 84%) est obtenue.
**RMN 1H** (CDCl3): 1,05 (6H, s), 1,30-1,55 (8H, m), 2,50 (2H, m), 4,07 (1H, OH, s), 4,89 (2H, s), 6,12 (1H, s), 6,26 (2H, s), 6,74-6,78 (3H, m), 7,13-7,16 (1H, t).

### EXEMPLE 31

### 5-{2-[3-(7-hydroxy-7-méthyl-oct-1-enyl)-phényl]-vinyl}-benzene-1,3-diol.

### a) Hept-6-enoate de méthyle.

320 mg (2,6 mmol) de diméthylaminopyridine et 8 ml (197 mmol) de méthanol sont ajoutés à 4 g (31,2 mmol) d'acide heptenoïque dans 100 ml de dichlorométhane. La solution est refroidie à 0°C, et on ajoute 7,2 g (34,9 mmol) de N,N' dicyclohexylcarbodiimide et on laisse remonter à température ambiante. 5h après, le précipité est filtré, le filtrat est évaporé et repris par de l'éther. La phase éthérée est lavée avec de l'acide chlorhydrique 0,5 N puis avec une solution aqueuse saturée en hydrogénocarbonate de sodium. Après décantation, elle est séchée sur sulfate de magnésium et concentrée. Le résidu est purifié sur colonne de silice (dichlorométhane) le produit est très volatile.
Huile incolore. m= 4,1 g. R= 92%.
**RMN 1H** 1,36-1,47 (2H, m), 1,58-1,70 (2H, m), 2,02-2,11 (2H, m), 2,29-2,34 (2H, t), 3,67 (3H, s), 4,93-5,04 (2H, m), 5,71-5,87 (1H, m).

### b) 7-(3-{2-[3,5-bis-(tétrahydro-pyran-2-yloxy)-phényl]-vinyl}-phényl)-hept-6-enoate de méthyle.

De manière analogue à l'exemple 1(h), par réaction de 1,41 g (3,07 mmol) de 3-bromo-{2-[3-(tétrahydro-pyran-2-yl)-5-(tétrahydro-pyran-2-yloxy)-phényl]-vinyl}-phényl dans 15 ml de diméthylformamide avec la solution à 0°C, de 8 ml (4 mmol) de 9-borabicyclo[3,3,1]nonane 0,5 M/THF et 567 mg (4 mmol) de hept-6-enoate de méthyle dans 6 ml de THF, après purification sur colonne de silice (acétate d'éthyle 10 - heptane 90), une huile jaune (m= 971 mg ; R= 61%) est obtenue.
**RMN 1H** (CDCl3): 1,46-1,93 (20 H, m), 3,67 (3H, s), 3,61-3,67 (2H, m), 3,89-3,98 (2H, m), 5,45-5,46 (2H, d), 6,18-6,43 (2H, m), 6,70 (1H, s), 6,87-6,89 (2H, t), 6,97-7,04 (2H, m), 7,20-7,34 (3H, m), 7,45 (1H, s).

### c) 8-(3-{2-[3,5-bis-(tétrahydro-pyran-2-yloxy)-phényl]-vinyl}-phényl)-2-méthyl-oct-7-en-2-ol.

De manière analogue à l'exemple 1(i), par réaction de 2,2 ml (6,6 mmol) de bromure de méthylmagnésium 3M/éther avec 859 mg (1,65 mmol) de 7-(3-{2-[3,5-bis-(tétrahydro-pyran-2-yloxy)-phényl]-vinyl}-phényl)-hept-6-enoate de méthyle dans 10 ml d'éther, après purification sur colonne de silice (acétate d'éthyle 20 - heptane 80), une huile jaune (m= 306 mg ; R= 36 %) est obtenue.
**RMN 1H** (CDCl3):1,22 (6H, s), 1,46-1,87 (20H, m), 3,61-3,67 (2H, m), 3,89-3,98 (2H, m), 5,45-5,46 (2H, m), 6,20-6,43 (2H, m), 6,70 (1H, s), 6,87-6,88 (2H, m), 7,04-7,06 (2H, m), 7,21-7,34 (3H, m), 7,45 (1H, s).

### d) 5-{2-[3-(7-hydroxy-7-méthyl-oct-1-enyl)-phényl]-vinyl}-benzène-1,3-diol.

De manière analogue à l'exemple 25(d), par réaction de 0,037 ml d'acide sulfurique concentré avec 374 mg (0,72 mmol) de 8-(3-{2-[3,5-bis-(tétrahydro-pyran-2-yloxy)-phényl]-vinyl}-phényl)-2-méthyl-oct-7-en-2-ol dans 4 ml de THF et 2 ml d'eau, après purification sur colonne de silice (acétate d'éthyle 40 - heptane 60), une poudre beige (m= 40 mg ; R= 16%) est obtenue. Tf= 152-4°C.
**RMN 1H** (CDCl3): 1,21 (6H, s), 1,48 (6H, m), 2,24-2,26 (2H, m), 2,36 (1H, OH, s), 6,23-6,42 (3H, m), 6,53-6,54 (2H, d), 6,97 (2H, d), 7,23-7,29 (3H, m), 7,42 (1H, s), 8,57 (2H, OH, s).

### EXEMPLE 32

### 5-{2-[3-(7-hydroxy-7-méthyl-octyl)-phényl]-vinyl}-benzène-1,3-diol.

### a) 7-(3-{2-[3,5-bis-(tétrahydro-pyran-2-yloxy)-phényl]-vinyl}-phényl)-heptanoate de méthyle.

De manière analogue à l'exemple 1(h), par réaction de 1,51 g (3,28 mmol) de 3-bromo-{2-[3-(tétrahydro-pyran-2-yl)-5-(tétrahydro-pyran-2-yloxy)-phényl)-vinyl}-phényl dans 15 ml de diméthylformamide avec la solution à 0°C, de 9,85 ml (4,92 mmol) de 9-borabicyclo[3,3,1]nonane 0,5 M/THF et 520 mg (3,61 mmol) de hept-6-enoate de méthyle dans 5 ml de THF, après purification sur colonne de silice (acétate d'éthyle 20 - heptane 80), une huile jaune (m= 1,75 g ; R= 100%) est obtenue.
**RMN 1H** (CDCl3): 1,34-1,92 (22H, m), 2,57-2,64 (2H, t), 3,66 (3H, s), 3,61-3,66 (2H, m), 3,89-3,98 (2H, m), 5,45-5,46 (2H, d), 6,69-6,70 (1H, d), 6,87-6,88 (2H, t), 7,03-7,10 (3H, m), 7,21-7,35 (3H, m).

### b) 8-(3-{2-[3,5-bis-(tétrahydro-pyran-2-yloxy)-phényl]-vinyl}-phényl)-2-méthyl-octan-2-ol.

De manière analogue à l'exemple 1(i), par réaction de 4,45 ml (13,3 mmol) de bromure de méthylmagnésium 3M/éther avec 1,73 g (3,31 mmol) de 7-(3-{2-[3,5-bis-(tétrahydro-pyran-2-yloxy)-phényl]-vinyl}-phényl)-heptanoate de méthyle dans 20 ml d'éther, après purification sur colonne de silice (acétate d'éthyle 20 - heptane 80), une huile jaune (m= 1,2 g ; R= 69%) est obtenue.
**RMN 1H** ( CDCl3 ):1,20 (6H, s), 1,28-1,87 (22H, m), 2,57-2,60 (2H, t), 3,65 (2H, m), 3,93 (2H, m), 5,46 (2H, m), 6,50-6,78 (2H, m), 6,88 (1H, s), 6,98-7,08 (3H, m), 7,25-7,29 (3H, m).

### c) 5-{2-[3-(7-hydroxy-7-méthyl-octyl)-phényl]-vinyl}-benzène-1,3-diol.

De manière analogue à l'exemple 25(d), par réaction de 0,06 ml d'acide sulfurique concentré avec 1,15 g (2,21 mmol) de 8-(3-{2-[3,5-bis-(tétrahydro-pyran-2-yloxy)-phényl]-vinyl}-phényl)-2-méthyl-octan-2-ol dans 20 ml de THF et 10 ml d'eau, après purification sur colonne de silice (acétate d'éthyle 40 - heptane 60), des cristaux rosâtres (m= 331 mg ; R= 42%) sont obtenus. Tf= 127-8°C.
**RMN 1H** (CDCl3): 1,16 (6H, s), 1,40 (8H, m), 1,70 (2H, m), 2,66-2,69 (2H, t), 4,16 (1H, OH, s), 6,27 (1H, s), 6,55 (2H, s), 7,13-7,20 (3H, m), 7,33-7,39 (1H, t), 7,47-7,51 (2H, m), 9,37 (2H, OH, s).

### EXEMPLE 33

### 4-{2-[3-(6-hydroxy-6-méthyl-heptyl)-phényl]-vinyl}-benzène-1,2-diol.

### a) 3,4-bis-(tert-butyl-diméthyl-silanyloxy)-benzaldéhyde.

De manière analogue à l'exemple 3(c), par réaction de 68,36 g (0,44 mol) de chlorure de tributyldiméthylsilane avec 27,62 g (0,2 mol) de 3,4-dihydroxybenzaldéhyde, 1,23 g de diméthylaminopyridine dans 61,5 ml (0,44 mmol) de triéthylamine et 900 ml de diméthylformamide, après purification sur colonne de silice (acétate d'éthyle 5 -heptane 95), une huile orangée (m= 69,82 g ; R= 95%) est obtenue.
**RMN 1H** (CDCl3): 0,01 (6H, s), 0,02 (6H, s), 0,79 (18H, s), 6,72-6,75 (1H, dd, J= 5 Hz, J'= 1,9 Hz), 7,14-7,18 (2H, m), 9,60 (1H, s).

### b) 3-bromo-[2-{3,4-bis-(tert-butyl-diméthyl-silanyloxy)-phényl}-vinyl]-phényl.

De manière analogue à l'exemple 1(g), par réaction de 543 mg (18 mmol) d'hydrure de sodium avec 5,5 g (15 mmol) de 3,4-bis-(*tert*-butyl-diméthyl-silanyloxy)-benzaldéhyde et 5,53 g (18 mmol) de (3-bromo-benzyl)-phosphonate d'éthyle dans 120 ml de THF, après purification sur colonne de silice (dichlorométhane 10 - heptane 90), une huile incolore (m= 5;38 g ; R= 69%) est obtenue.
**RMN 1H** (CDCl3): 0,20 (6H, s), 0,21 (6H, s), 0,99 (9H, s), 1,01 (9H, s), 6,76-6,83 (2H, m), 5,94-7,00 (3H, m), 7,15-7,25 (1H, m), 7,32-7,39 (2H, m), 7,61-7,62 (1H, m).

### c) 2-[6-(3-{2-[3,4-bis-(tert-butyl-diméthyl-silanyloxy)-phényl]-vinyl}-phényl)-1,1-diméthyl-hexyloxy]-tétrahydro-pyranne.

De manière analogue à l'exemple 8(j), par réaction de 1,73 g (3,32 mmol) de 3-bromo-[2-{3,4-bis-(*tert*-butyl-diméthyl-silanyloxy)-phényl}-vinyl]-phényl dans 4 ml d'éther avec la olution de 222 mg (9,13 mmol) de magnésium, de 2,43 g (8,3 mmol) de 2-(6-bromo-l,1-iméthyl-hexyloxy)-tétrahydro-pyranne dans 8 ml de THF et catalysée par 72 mg (0,14 mmol) e [1,2-bis-(diphénylphosphino)éthane]dichloronickel, après purification sur colonne de ilice (acétate d'éthyle 3 - heptane 97), une huile incolore (m= 1,4 g ; R= 65%) est obtenue.
**RMN 1H** (CDCl3): 0,21 (6H, s), 0,23 (6H, s), 0,99 (9H, s), 1,01 (9H, s), 1,18-1,20 (6H, d), ,26-1,83 (14H, m), 2,58-2,64 (2H, t), 3,38-3,47 (1H, m), 3,92-3,98 (1H, m), 4,69-4,71 (1H, m), 6,78-7,06 (6H, m), 7,20-7,32 (3H, m).

### d) 4-{2-[3-(6-hydroxy-6-méthyl-heptyl)-phényl]-vinyl}-benzène-1,2-diol.

e manière analogue à l'exemple 25(d), par réaction de 0,118 ml d'acide sulfurique concentré avec 1,38 g (2,11 mmol) de 2-[6-(3-{2-[3,4-bis-(*tert*-butyl-diméthyl-silanyloxy)-phényl]-nyl}-phényl)-1,1-diméthyl-hexyloxy]-tétrahydro-pyranne dans 20 ml de THF et 10 ml d'eau, après purification sur colonne de silice (acétate d'éthyle 45 - heptane 55), un solide d'aspect cotonneux (m= 522 mg ; R= 77%) est obtenu. Tf= 138-9°C.
**RMN 1H** (CDCl3): 1,20 (6H, s), 1,37-1,44 (6H, m), 1,62-1,68 (2H, m), 2,58-2,64 (2H, t), 6,82-6,95 (4H, m), 7,01-7,08 (2H, m), 7,19-7,30 (3H, m), 7,58 (1H, OH, s), 7,69 (1H, OH, s).

### EXEMPLE 34

### 3-{2-[3-(6-hydroxy-6-méthyl-heptyl)-phényl]-vinyl}-phénol.

### a) 3-(tert-butyl-diméthyl-silanyloxy)-benzaldéhyde.

De manière analogue à l'exemple 3(c), par réaction de 42,72 g (0,275 mol) de chlorure de *tert*-butyldiméthylsilane avec 30,53 g (0,2 mol) de 3-hydroxybenzaldéhyde, 1,52 g de diméthylaminopyridine dans 38,5 ml (0,275 mmol) de triéthylamine et 300 ml de diméthylformamide, après purification sur colonne de silice (dichlorométhane 20 -heptane 80), une huile jaune (m= 55,86 g ; R= 95%) est obtenue.
**RMN 1H** (CDCl3): 0,20 (6H, s), 0,98 (9H, s), 7,06-7,10 (1H, m), 7,24-7,48 (3H, m), 9,93 (1H, s).

### b) {3-[2-(3-bromo-phényl)-vinyl]-phénoxy}-tert-butyl-diméthyl-silane.

De manière analogue à l'exemple 1 (g), par réaction de 543 mg (18 mmol) d'hydrure de sodium avec 3,55 g (15 mmol) de 3-*tert*-butyl-diméthyl-silanyloxy)-benzaldéhyde et 5,53 g (18 mmol) de (3-bromo-benzyl)-phosphonate d'éthyle dans 100 ml de THF, après purification sur colonne de silice (dichlorométhane 10 - heptane 90), une huile incolore (m= 3,73 g ; R= 64%) est obtenue.
**RMN 1H** (CDCl3): 0,22 (6H, s), 1,00 (9H, s), 6,74-6,78 (1H, dd, J= 4,7 Hz, J'= 1,5 Hz), 6,92-7,01 (3H, m), 7,08-7,25 (3H, m), 7,35-7,42 (2H, t), 7,65-7,66 (1H, t).

### c) Tert-butyl-diméthyl-[3-(2-{3-[6-méthyl-6-(tétrahydro-pyran-2-yloxy)-heptyl]-phényl}-vinyl)-phénoxy]-silane.

De manière analogue à l'exemple 8(j), par réaction de 1,30 g (3,32 mmol) de {3-[2-(3-bromo-phényl)-vinyl]-phénoxy}-*tert*-butyl-diméthyl-silane dans 4 ml d'éther avec la solution de 222 mg (9,13 mmol) de magnésium, de 2,43 g (8,3 mmol) de 2-(6-bromo-1,1-diméthyl-hexyloxy)-tétrahydro-pyranne dans 8 ml de THF et catalysée par 72 mg (0,14 mmol) de [1,2-bis-(diphénylphosphino)éthane]dichloronickel, après purification sur colonne de silice (acétate d'éthyle 3 - heptane 97), une huile incolore (m= 1,28 g ; R= 74%) est obtenue.
**RMN 1H** (CDCl3): 0,22 (6H, s), 1,00 (9H, s), 1,18-1,20 (6H, d), 1,25-1,83 (14H, m), 2,59-2,65 (2H, t), 3,38-3,47 (1H, m), 3,92-3,98 (1H, m), 4,69-4,71 (1H, m), 6,71-6,76 (1H, dd), 6,97-6,99 (1H, t), 7,06-7,34 (6H, m).

### d) 3- {2-[3-(6-hydroxy-6-méthyl-heptyl)-phényl]-vinyl}-phénol.

De manière analogue à l'exemple 25(d), par réaction de 0,133 ml d'acide sulfurique concentré avec 1,25 g (2,39 mmol) de *tert*-butyl-diméthyl-[3-(2-{3-[6-méthyl-6-(tétrahydro-pyran-2-yloxy)-heptyl]-phényl}-vinyl)-phénoxy]-silane dans 20 ml de THF et 10 ml d'eau, après cristallisation dans un mélange heptane / acétate d'éthyle, des cristaux blancs (m= 697 mg ; R= 90%) sont obtenus. Tf= 96-7°C.
**RMN 1H** (CDCl3): 1,22 (6H, s), 1,31-1,45 (6H, m), 1,60-1,70 (2H, m), 2,59-2,65 (2H, t), 5,46 (1H, OH, s), 6,71-6,75 (1H, dd, J= 6,2 Hz, J'= 1,7 Hz), 6,99-7,08 (5H, m), 7,18-7,33 (4H, m).

### EXEMPLE 35

### 6-{3-[2-(3,5-bis-hydroxyméthyl-phényl)-vinyl]-phényl}-2-méthyl-hexan-2-ol.

### a) Acide-5-bromo-isophtalique.

33,23 g (0,2 mo1) d'acide isophtalique et 37,42 g (0,12 mol) de sulfate d'argent sont dissous dans 330 ml d'acide sulfurique. On ajoute, ensuite, en 1h, 13,3 ml (0,26 mol) de brome. La solution est chauffée à 55°C pendant 24h. Le milieu est, alors, versé dans de la glace, l'insoluble est filtré et repris par de l'acétate d'éthyle. Le solide restant est repris par de l'eau et basifié avec une solution aqueuse saturée en hydrogénocarbonate de sodium. L'insoluble est filtré, et le filtrat est acidifié puis extrait avec de l'acétate d'éthyle. La phase organique est lavée à l'eau, puis séchée sur sulfate de magnésium et concentrée.
Poudre blanche. m= 42,1 g. R= 86%. Tf= 285-7°C.
**RMN 1H** (DMSO): 8,40 (2H, s), 8,57 (1H, t), 13,76 (2H, COOH, s).

### b) (3-bromo-5-hydroxyméthyl-phényl)-méthanol.

De manière analogue à l'exemple 1(c), par réaction de 690 ml de borane 1M/THF avec 42,06 g (0,172 mol) de l'acide 5-bromo-isophtalique dans 420 ml de THF, après trituration dans l'heptane, une poudre blanche (m= 28,19 g ; R= 76%) est obtenue. Tf= 85-8°C.
**RMN 1H** (DMSO): 4,65 (4H, s), 5,48 (2H, OH, s), 7,41 (1H, s), 7,52 (2H, s).

### c) 1-bromo-3,5-bis-(tert-butyl-diméthyl-silanyloxyméthyl)-benzène.

De manière analogue à l'exemple 3(c), par réaction de 44,43 g (0,286 mol) de chlorure de *tert*-butyldiméthylsilane avec 28,18 g (0,13 mol) de (3-bromo-5-hydroxyméthyl-phényl)-méthanol, 794 mg de diméthylaminopyridine dans 40 ml (0,286 mmol) de triéthylamine et 700 ml dé diméthylforinamide, après purification sur colonne de silice (dichlorométhane 10 - heptane 90), une huile jaunâtre (m= 50,84 g; R= 98%) est obtenue.
**RMN 1H** (CDCl3): 0,00 (12H, s), 0,84 (18H, s), 4,59 (4H, s), 7,10 (1H, s), 7,22 (2H, s),

### d) 3,5-bis-(tert-butyl-diméthyl-silanyloxyméthyl)-benzaldéhyde.

De manière analogue à l'exemple 4(c), par réaction de 13,5 ml (33,75 mmol) de n-Butyllithium 2,5 M/Hexane, à -78°C, avec 13,4 g (30 mmol) de 1-bromo-3,5-bis-(*tert*-butyl-diméthyl-silanyloxyméthyl)-benzène dans 140 ml de THF et 30 minutes après, avec 2,55 ml (33 mmol) de diméthylformamide, après purification sur colonne de silice (dichlorométhane 60 - heptane 40), une huile jaunâtre (m= 9,5 g ; R= 80%) est obtenue.
**RMN 1H** (CDCl3): 0,00 (12H, s), 0,84 (18H, s), 4,68 (4H, s), 7,48 (1H, s), 7,58 (2H, s), 9,89 (1H, s).

### e) 3-bromo-[2-{3,5-bis-(tert-butyl-diméthyl-silanyloxyméthyl)-phényl]-vinyl}-phényl.

De manière analogue à l'exemple 1(g), par réaction de 362 mg (12 mmol) d'hydrure de sodium avec 3,95 g (10 mmol) de 3,5-bis-(*tert*-butyl-diméthyl-silanyloxyméthyl)-benzaldéhyde et 3,61 g (12 mmol) de (3-bromo-benzyl)-phosphonate d'éthyle dans 80 ml de THF, après purification sur colonne de silice (dichlorométhane 10 - heptane 90),une huile jaunâtre (m= 4,93 g ; R= 90%) est obtenue.
**RMN 1H** (CDCl3): 0,00 (12H, s), 0,84 (18H, s), 4,63 (4H, s), 6,84-7,01 (2H, q), 7,05-7,12 (2H, m), 7,21-7,30 (4H, m), 7,52-7,53 (1H, t).

### f) 5-(3-{2-[3,5-bis-(tert-butyl-diméthyl-silanyloxyméthyl)-phényl]-vinyl}-phényl)-pentanoate de méthyle.

De manière analogue à l'exemple 1(h), par réaction de 1,64 g (3 mmol) de 3-bromo-[2-{3,5-bis-(*tert*-butyl-diméthyl-silanyloxyméthyl)-phényl]-vinyl}-phényl dans 20 ml de diméthylformamide avec la solution à 0°C, de 9 ml (4,5 mmol) de 9-borabicyclo[3,3,1]nonane 0,5 M/THF et 397 mg (3,3 mmol) de pent-4-enoate de méthyle dans 5 ml de THF, après purification sur colonne de silice (dichlorométhane 40 - heptane 80), une huile jaunâtre (m= 1,14 g; R= 65%) est obtenue.
**RMN 1H** (CDCl3): 0,00 (12H, s), 0,84 (18H, s), 1,37-1,77 (6H, m), 2,50-2,55 (2H, t), 3,54 (3H, s), 4,63 (4H, s), 6,93-6,96 (3H, m), 7,08-7,22 (6H, m).

### g) 6-(3-{2-[3,5-bis-(tert-butyl-diméthyl-silanyloxyméthyl)-phényl]-vinyl}-phényl)-2-méthyl-hexan-2-ol.

De manière analogue à l'exemple 1(i), par réaction de 2,6 ml (7,73 mmol) de bromure de méthylmagnésium 3M/éther avec 1,13 g (1,93 mmol) de 5-(3-{2-[3,5-bis-(*tert*-butyl-diméthyl-silanyloxyméthyl)-phényl]-vinyl}-phényl)-pentanoate de méthyle dans 10 ml d'éther, après purification sur colonne de silice (acétate d'éthyle 15 - heptane 85), une huile jaune (m= 886 mg ; R= 79%) est obtenue.
**RMN 1H** (CDCl3 ):0,00 (12H, s), 0,84 (18H, s), 1,09 (6H, s), 1,14-1,60 (6H, m), 2,50-2,56 (2H, t), 4,63 (4H, s), 6,94-6,96 (3H, m), 7,08-7,22 (6H, m).

### h) 6-{3-[2-(3,5-bis-hydroxyméthyl-phényl)-vinyl]-phényl}-2-méthyl-hexan-2-ol.

De manière analogue à l'exemple 3(i), par réaction de 858 mg (1,47 mmol) de 6-(3-{2-[3,5-bis-(*tert*-butyl-diméthyl-silanyloxyméthyl)-phényl]-vinyl}-phényl)-2-méthyl-hexan-2-ol avec 3,25 ml de fluorure de tetrabutylammonium 1M/THF, après purification sur colonne de silice (acétate d'éthyle), une pâte blanche (m= 460 mg ; R= 88%) est obtenue.
**RMN 1H** (CDCl3): 1,20 (6H, s), 1,37-1,71 (6H, m), 2,61-2,67 (2H, t), 4,69 (4H, s), 7,03-7,17 (3H, m), 7,23-7,34 (4H, m), 7,42 (2H, s).

### EXEMPLE 36

### 3-{2-[3-(7-hydroxy-7-méthyl-octyl)-phényl]-vinyl}-phénol.

### a) 7-(3-{2-[tert-butyl-diméthyl-silanyloxy)-phényl]-vinyl}-phényl)-heptanoate de méthyle.

De manière analogue à l'exemple 1(h), par réaction de 1,17 g (3 mmol) de {3-[2-(3-bromo-phényl)-vinyl]-phénoxy}-*tert*-butyl-diméthyl-silane dans 12 ml de diméthylformamide avec la solution à 0°C, de 9 ml (4,5 mmol) de 9-borabicyclo[3,3,1]nonane 0,5 M/THF et 470 mg (3,3 mmol) de hex-5-enoate de méthyle dans 5 ml de THF, après purification sur colonne de silice (dichlorométhane 40 - heptane 80), une huile jaunâtre (m= 270 mg ; R= 20%) est obtenue.
**RMN 1H** (CDCl3): 0,22 (6H, s), 1,00 (9H, s), 1,33-1,39 (4H, m), 1,55-1,63 (4H, m), 2,27-2,33 (2H, t), 2,58-2,64 (2H, t), 3,66 (3H, s), 6,72-6,76 (1H, m), 6,97-6,99 (1H, t), 7,08-7,34 (8H, m).

### b) 8-(3-{2-[3-(tert-butyl-diméthyl-silanyloxy)-phényl]-vinyl}-phényl)-2-méthyl-octan-2-ol.

De manière analogue à l'exemple 1(i), par réaction de 0,77 ml (2,29 mmol) de bromure de méthylmagnésium 3M/éther avec 259 mg (0,57 mmol) de 7-(3-{2-[*tert*-butyl-diméthyl-silanyloxy)-phényl]-vinyl}-phényl)-heptanoate de méthyle dans 3 ml d'éther, après purification sur colonne de silice (acétate d'éthyle 20 - heptane 80), une pâte jaunâtre (m= 219 mg ; R= 81%) est obtenue.
**RMN 1H** ( CDCl3 ): 0,21 (6H, s), 1,00 (9H, s), 1,20 (6H, s), 1,28-1,43 (8H, m), 1,61-1,65 (2H, m), 2,59-2,65 (2H, t), 6,72-6,76 (1H, m), 6,97-6,99 (1H, t), 7,06-7,35 (8H, m).

### c) 3-{2-[3-(7-hydroxy-7-méthyl-octyl)-phényl]-vinyl}-phénol.

De manière analogue à l'exemple 3(i), par réaction de 207 mg (0,46 mmol) de 8-(3-{2-[3-(*tert*-butyl-diméthyl-silanyloxy)-phényl]-vinyl}-phényl)-2-méthyl-octan-2-ol avec 0,5 ml de fluorure de tetrabutylammonium 1M/THF, après purification sur colonne de silice (acétate d'éthyle 30 - heptane 70), une poudre blanche (m= 203 mg ; R= 32%) est obtenue. Tf= 142-3°C.
**RMN 1H** (DMSO): 1,05 (6H, s), 1,30 (8H, m), 1,59 (2H, m), 2,55-2,61 (2H, t), 4,03 (1H, OH, s), 6,66-6,69 (1H, dd, J= 6,3 Hz, J'= 1,6 Hz), 6,96-7,28 (7H, m), 7,36-7,39 (2H, d, J= 7,7 Hz).

### EXEMPLE 37

### 7-{3-[2-(3,5-bis-hydroxyméthyl-phényl)-vinyl]-phényl}-2-méthyl-heptan-2-ol.

### a) 2-[6-(3-{2-[3,5-bis-(tert-butyl-diméthyl-silanyloxyméthyl)-phényl]-vinyl}-phényl)-1,1-diméthyl-hexyloxy]-tétrahydro-pyranne.

De manière analogue à l'exemple 8(j), par réaction de 2,40 g (4,38 mmol) de 3-bromo-[2-{3,5-bis-(*tert*-butyl-diméthyl-silanyloxyméthyl)-phényl]-vinyl}-phényl avec la solution de 293 mg (12 mmol) de magnésium, de 3,12 g (10,6 mmol) de 2-(6-bromo-1,1-diméthyl-hexyloxy)-tétrahydro-pyranne dans 10 ml de THF et catalysée par 94 mg (0,178 mmol) de [1,2-bis-(diphénylphosphino)éthane]dichloronickel, après purification sur colonne de silice (acétate d'éthyle 3 - heptane 97), une huile incolore (m= 2,05 g ; R= 69%) est obtenue.
**RMN 1H** (CDCl3): 0,00 (12H, s), 0,84 (18H, s), 1,06-1,08 (6H, d), 1,13-1,71 (14H, m), 2,47-2,53 (2H, t), 3,29-3,33 (1H, m), 3,80-3,84 (1H, m), 4,57-4,59 (1H, m), 4,63 (4H, s), 6,94-6,97 (3H, m), 7,08-7,22 (6H, m).

### b) 7-{3-[2-(3,5-bis-hydroxyméthyl-phényl)-vinyl]-phényl}-2-méthyl-heptan-2-ol.

De manière analogue à l'exemple 25(d), par réaction de 0,5 ml d'acide sulfurique concentré avec 2,04 g (3 mmol) de 2-[6-(3-{2-[3,5-bis-(*tert*-butyl-diméthyl-silanyloxyméthyl)-phényl]-vinyl}-phényl)-1,1-diméthyl-hexyloxy]-tétrahydro-pyranne dans 30 ml de THF et 10 ml d'eau, après purification sur colonne de silice (acétate d'éthyle 80 - heptane 20), des cristaux blancs (m= 646 mg ; R= 58%) sont obtenus. Tf= 108-9°C.
**RMN 1H** (CDCl3): 1,18 (6H, s), 1,37-1,43 (6H, m), 1,63-1,68 (2H, m), 2,59-2,65 (2H, t), 4,29-4,34 (2H, OH, t), 4,64 (2H, s), 4,67 (2H, s), 7,06-7,11 (3H, m), 7,18-7,30 (4H, m), 7,42-7,43 (2H, d).

### EXEMPLE 38

### 7-{3-[2-(3,4-bis-hydroxyméthyl-phényl)-vinyl]-phényl}-2-méthyl-heptan-2-ol.

### a) 2-[6-(3-{2-[3,4-bis-(tert-butyl-diméthyl-silanyloxyméthyl)-phényl]-vinyl}-phényl)-1,1-diméthyl-hexyloxy]-tétrahydro-pyranne.

De manière analogue à l'exemple 8(j), par réaction de 2,40 g (4,38 mmol) de 3-bromo-[2-{3,4-bis-(*tert*-butyl-diméthyl-silanyloxyméthyl)-phényl}-vinyl]-phényl avec la solution de 293 mg (12 mmol) de magnésium, de 3,12 g (10,6 mmol) de 2-(6-bromo-1,1-diméthyl-hexyloxy)-tétrahydro-pyranne dans 10 ml de THF et catalysée par 94 mg (0,178 mmol) de [1,2-bis-(diphénylphosphino)éthane]dichloronickel, après purification sur colonne de silice (acétate d'éthyle 3 - heptane 97), une huile incolore (m= 2,31 g ; R= 77%) est obtenue.
**RMN 1H** (CDCl3): -0,02 (6H, s), 0,00 (6H, s), 0,83 (9H, s), 0,84 (9H, s), 1,06-1,08 (6H, d), 1,13-1,71 (14H, m), 2,47-2,53 (2H, t), 3,28-3,33 (1H, m), 3,80-3,84 (1H, m), 4,57 (1H, m), 4,62 (2H, s), 4,64 (2H, s), 6,93-6,97 (3H, m), 7,10-7,27 (5H, m), 7,46 (1H, s).

### b) 7-{3-[2-(3,4-bis-hydroxyméthyl-phényl)-vinyl]-phényl}-2-méthyl-heptan-2-ol.

De manière analogue à l'exemple 25(d), par réaction de 0,56 ml d'acide sulfurique concentré avec 2,30 g (3,37 mmol) de 2-[6-(3-{2-[3,4-bis-(*tert*-butyl-diméthyl-silanyloxyméthyl)-phényl]-vinyl}-phényl)-1,1-diméthyl-hexyloxy]-tétrahydro-pyranne dans 30 ml de THF et 10 ml d'eau, après purification sur colonne de silice (acétate d'éthyle 80 - heptane 20) des cristaux blancs (m= 475 mg ; R= 38%) sont obtenus. Tf= 93-5°C.
**RMN 1H** (CDCI3): 1,19 (6H, s), 1,28-1,42 (6H, m), 1,62-1,68 (2H, m), 2,59-2,65 (2H, t), 3,50 (2H, OH, s), 4,66 (2H, s), 4,70 (2H, s), 7,00-7,14 (3H, m), 7,22-7,32 (4H, m), 7,39-7,44 (1H, dd, J= 6,2 Hz, J'= 1,55 Hz), 7,47 (1H, s).

### EXEMPLE 39

### 7-{3-[2-(4-hydroxyméthyl-phényl)-vinyl]-phényl}-2-méthyl-heptan-2-ol.

### a) (4-bromo-benzyloxy)-tert-butyl-diméthyl-silane.

De manière analogue à l'exemple 3(c), par réaction de 54,3 g (0,36 mol) de chlorure de *tert*-butyldiméthylsilane avec 56,1 g (0,3 mol) de (4-bromo-phényl)-méthanol, 8,8 g (72 mmol) de diméthylaminopyridine dans 100 ml de triéthylamine et 150 ml de diméthylformamide, après purification sur colonne de silice (heptane), une huile jaune-vert (m= 81 g ; R= 90%) est obtenue.
**RMN 1H** (CDCl3): 0,00 (6H, s), 0,84 (9H, s), 4,58 (2H, s), 7,08-7,11 (2H, d, J= 8,3 Hz), 7,33-7,37 (2H, d, J= 8,4 Hz).

### b) 4-(tert-butyl-diméthyl-silanyloxyméthyl)-benzaldéhyde.

De manière analogue à l'exemple 4(c), par réaction de 13,2 de n-Butyllithium 2,5 M/Hexane, à -78°C, avec 9,04 g (30 mmol) de (4-bromo-benzyloxy)-*tert*-butyl-diméthyl-silane dans 90 ml de THF et 30 minutes après, avec 2,55 ml (33 mmol) de diméthylformamide, après purification sur colonne de silice (acétate d'éthyle 5 - heptane 60), une huile jaunâtre (m= 3,64 g ; R= 48%) est obtenue.
**RMN 1H** (CDCl3): 0,00 (6H, s), 0,83 (9H, s), 4,69 (2H, s), 7,35-7,38 (2H, d, J= 8,07 Hz), 7,71-7,75 (2H, d, J= 8,2 Hz).

### c) {4-[2-(3-bromo-phényl)-vinyl]-benzyloxy}-tert-butyl-diméthyl-silane.

De manière analogue à l'exemple 1(g), par réaction de 513 mg (17 mmol) d'hydrure de sodium avec 3,64 g (14 mmol) de 4-(*tert*-butyl-diméthyl-silanyloxyméthyl)-benzaldéhyde et 5,36 g (17 mmol) de (3-bromo-benzyl)-phosphonate d'éthyle dans 90 ml de THF, après purification sur colonne de silice (dichlorométhane 5 - heptane 95), des cristaux blancs (m= 4,61 g ; R= 82%) sont obtenus. Tf= 65-7°C.
**RMN 1H** (CDCl3): 0,10 (6H, s), 0,93 (9H, s), 4,74 (2H, s), 6,95-7,13 (2H, dd, J= 11,9 Hz, J'= 16,3 Hz), 7,17-7,25 (1H, m), 7,30-7,48 (6H, m), 7,64-7,66 (1H, t).

### d) Tert-butyl-diméthyl-[4-(2-{3-[6-méthyl-6-(tétrahydro-pyran-2-yloxy)-heptyl]-phényl}-vinyl)-benzyloxy]-silane.

De manière analogue à l'exemple 8(j), par réaction de 1,77 g (4,38 mmol) de {4-[2-(3-bromo-phényl)-vinyl]-benzyloxy}-*tert*-butyl-diméthyl-silane avec la solution de 293 mg (12 mmol) de magnésium, de 3,12 g (10,6 mmol) de 2-(6-bromo-1,1-diméthyl-hexyloxy)-tétrahydro-pyranne dans 10 ml de THF et catalysée par 94 mg (0,178 mmol) de [1,2-bis-(diphénylphosphino)éthane]dichloronickel, après purification sur colonne de silice (acétate d'éthyle 3 - heptane 97), une huile incolore (m= 1 g ; R= 43%) est obtenue.
**RMN 1H** (CDCl3): 0,00 (6H, s), 0,84 (9H, s), 1,07-1,09 (6H, d), 1,24-1,53 (14H, m), 2,48-2,54 (2H, t), 3,30-3,34 (1H, m), 3,81-3,85 (1H, m), 4,58 (1H, m), 4,63 (2H, s), 6,94-6,97 (3H, m), 7,11-7,21 (5H, m), 7,35-7,38 (2H, d, J= 8,2 Hz).

### e) 7-{3-[2-(4-hydroxyméthyl-phényl)-vinyl]-phényl}-2-méthyl-heptan-2-ol.

De manière analogue à l'exemple 25(d), par réaction de 0,2 ml d'acide sulfurique concentré avec 1 g (1,86 mmol) de *Tert*-butyl-diméthyl-[4-(2-{3-[6-méthyl-6-(tétrahydro-pyran-2-yloxy)-heptyl]-phényl}-vinyl)-benzyloxy]-silane dans 20 ml de THF et 10 ml d'eau, après purification sur colonne de silice (acétate d'éthyle 50 - heptane 50), des cristaux blancs (m= 350 mg ; R= 56%) sont obtenus. Tf= 105-7°C.
**RMN 1H** (CDCl3): 1,19 (6H, s), 1,28-1,43 (6H, m), 1,63-1,69 (2H, m), 2,59-2,65 (2H, t), 4,67 (2H, s), 7,06-7,09 (3H, m), 7,23-7,36 (5H, m), 7,48-7,51 (2H, d, J= 8,2 Hz).

### EXEMPLE 40

### 4-{2-[3-(7-hydroxy-7-méthyl-oct-1-enyl)-phényl]-vinyl}-benzene-1,2-diol.

### a) 7-(3-{2-[3,4-bis-(tert-butyl-diméthyl-silanyloxy)-phényl]-vinyl}-phényl)-hept-6-enoate de méthyle.

A une solution de 1,33 g (2,56 mmol) de 3-bromo-[2-{3,4-bis-(*tert*-butyl-diméthyl-silanyloxy)-phényl}-vinyl]-phényl et 444 mg (3,1 mmol) de hept-6-enoate de méthyle dans 20 ml de diméthylformamide, on ajoute 0,43 ml de triéthylamine et 72 mg (0,077 mmol) de [1,1'-bis-(diphénylphosphino)ferrocene]dichloropalladium. Le milieu est chauffé à 75°C pendant 3 jours. Il est, alors, versé dans une solution aqueuse saturée en chlorure d'ammonium et extrait à l'éther. La phase organique est lavée avec une solution aqueuse saturée en chlorure de sodium puis à l'eau. Après décantation, elle est séchée sur sulfate de magnésium et concentrée. Le résidu est purifié sur colonne de silice (acétate d'éthyle 5 - heptane 95).
Huile jaune. m= 318 mg. R= 21%.
**RMN 1H** (CDCl3): 0,21 (6H, s), 0,23 (6H, s), 1,00 (9H, s), 1,03 (9H, s), 1,48-1,55 (2H, m), 1,64-1,78 (2H, m), 2,21-2,29 (2H, m), 2,32-2,41 (2H, t), 3,67 (3H, s), 6,18-6,30 (1H, m), 6,37-6,44 (1H, d, J= 15,9 Hz), 6,79-7,02 (6H, m), 7,14-7,43 (2H, m), 7,62 (1H, s).

### b) 8-(3-{2-[3,4-bis-(tert-butyl-diméthyl-silanyloxy)-phényl]-vinyl}-phényl)-2-méthyl-oct-7-en-2-ol.

De manière analogue à l'exemple 1(i), par réaction de 1 ml (3 mmol) de bromure de méthylmagnésium 3M/éther avec 305 mg (0,52 mmol) de 7-(3-{2-[3,4-bis-(*tert*-butyl-diméthyl-silanyloxy)-phényl]-vinyl}-phényl)-hept-6-enoate de méthyle dans 6 ml d'éther, après purification sur colonne de silice (acétate d'éthyle 20 - heptane 80), une huile jaune (m= 190 mg ; R= 63%) est obtenue.
**RMN 1H** ( CDCl3 ): 0,21 (6H, s), 0,23 (6H, s), 0,99 (9H, s), 1,01 (9H, s), 1,23 (6H, s), 1,38-1,62 (6H, m), 2,24-2,26 (2H, m), 6,20-6,32 (1H, m), 6,37-6,44 (1H, d, J= 15,9 Hz), 6,79-7,02 (5H, m), 7,20-7,34 (3H, m), 7,44 (1H, s).

### c) 4-{2-[3-(7-hydroxy-7-méthyl-oct-1-enyl)-phényl]-vinyl}-benzène-1,2-diol.

De manière analogue à l'exemple 3(i), par réaction de 166 mg (0,286 mmol) de 8-(3-{2-[3,4-bis-(*tert*-butyl-diméthyl-silanyloxy)-phényl]-vinyl}-phényl)-2-méthyl-oct-7-en-2-ol avec 0,63 ml de fluorure de tetrabutylammonium 1M/THF, après purification sur colonne de silice (acétate d'éthyle 45 - heptane 55), des cristaux blancs (m= 65 mg ; R= 64%) sont obtenus. Tf= 116-8°C.
**RMN 1H** (CDCl3): 1,21 (6H, s), 1,47-1,54 (6H, m), 2,23-2,25 (2H, m), 6,19-6,31 (1H, m), 6,36-6,42 (1H, d, J= 15,9 Hz), 6,82-7,33 (8H, m), 7,41 (1H, s).

### EXEMPLE 41

### 5-{2-[3-(6-hydroxy-6-méthyl-hept-1-enyl)-phényl]-vinyl}-benzene-1,3-diol.

### a) 3,5-bis-éthoxyméthoxy-benzoate de méthyle.

De manière analogue à l'exemple 1(d), par réaction de 42,04 g (0,25 mol) de 3,5-dihydroxybenzoate de méthyle dans 400 ml de diméthylformamide avec 18,09 g (0,6 mol) d'hydrure de sodium et 51,26 g (0,515 mol) de chlorure de méthoxyméthyle après purification sur colonne de silice (dichlorométhane), une huile jaune (m= 37,72 g ; R= 62%) est obtenue.
**RMN 1H** (CDCl3): 1,19-1,25 (6H, t), 3,b8-3,77 (4H, q), 3,89 (3H, s), 5,23 (4H, s), 6,92-6,94 (1H, t), 7,35-7,36 (2H, d).

### b) (3,5-bis-éthoxyméthoxy-phényl)-méthanol.

De manière analogue à l'exemple 1(e), par réaction de 400 ml d'hydrure de diisobutylaluminium 1M/Toluène, à -78°C, avec 37,7 g (0,133 mol) de 3,5-bis-éthoxyméthoxy-benzoate de méthyle, après purification sur colonne de silice (acétate d'éthyle 20 - Heptane 80), une huile jaune (m= 38,1 g ; R= 86%) est obtenue.
**RMN 1H** (CDCl3): 1,19-1,24 (6H, t), 3,67-3,76 (4H, q),4,61 (2H, s), 5,19 (4H, s), 6,64-6,66 (1H, t), 6,69-6,70 (2H, d).

### c) 3,5-bis-éthoxyméthoxy-benzaldéhyde.

97 g (1,11 mol) de dioxyde de manganese sont ajoutés à une solution de 40,78 g (0,159 mol) de (3,5-bis-éthoxyméthoxy-phényl)-méthanol dans 400 ml de dichlorométhane. Le milieu est agité à température ambiante pendant 5 jours. Il est, alors, filtré sur silice et le solide est lavé au dichlorométhane. Le filtrat est évaporé.
Huile jaune. m= 36,51 g. R= 90%.
**RMN 1H** (CDCl3): 1,20-1,25 (6H, t), 3,69-3,78 (4H, q), 5,25 (4H, s), 6,98-6,99 (1H, t), 7,20-7,21 (2H, d).

### d) 3-bromo-[2-(3,5-bis-éthoxyméthoxy-phényl)-vinyl]-phényl.

De manière analogue à l'exemple 1 (g), par réaction de 2,05 g (68 mmol) d'hydrure de sodium avec 14,5 g (57 mmol) de 3,5-bis-éthoxyméthoxy-benzaldéhyde et 20,88 g (68 mmol) de 3-bromobenzylphosphonate d'éthyle dans 350 ml de THF, après purification sur colonne de silice (dichlorométhane 30 - heptane 70), une huile jaune (m= 22,83 g ; R= 98%) est obtenue.
**RMN 1H** (CDCl3): 1,21-1,27 (6H, t), 3,70-3,79 (4H, q), 5,23 (4H, s), 6,68-6,69 (1H, t), 6,85-6,86 (2H, d), 6,93-7,07 (2H, dd, J= 16,3 Hz, J'= 2,55 Hz), 7,17-7,24 (1H, t), 7,35-7,41 (2H, t), 7,64-7,65 (1H, t).

### e) 6-{3-[2-(3,5-bis-éthoxyméthoxy-phényl)-vinyl]-phényl}-hex-5-enoate de méthyle.

De manière analogue à l'exemple 40(a), par réaction de 4,07 g (0,01 mol) de 3-bromo-[2-(3,5-bis-éthoxyméthoxy-phényl)-vinyl]-phényl dans 50 ml de triéthylamine avec 1,54 g (0,012 mol) de hex-5-enoate de méthyle et comme catalyseur 112 mg (0,5 mmol) d'acétate de Palladium et 262 mg (1 mmol) de triphénylphosphine, après purification sur colonne de silice (acétate d'éthyle 10 - heptane 90), une huile jaune (m= 2 g ; R= 44%) est obtenue.
**RMN 1H** (CDCl3): 1,22-1,27 (6H, t), 1,78-1,89 (2H, m), 2,23-2,41 (4H, m), 3,67 (3H, s), 3,63-3,79 (4H, m), 5,24 (4H, s), 6,17-6,28 (1H, m), 6,38-6,45 (1H, d, J= 15,9 Hz), 6,67 (1H, s), 6,87 (2H, d), 7,05-7,07 (2H, d), 7,22-7,36 (3H, m), 7,47 (1H, s).

### f) 7-{3-[2-(3,5-bis-éthoxyméthoxy-phényl)-vinyl]-phényl}-2-méthyl-hept-6-en-2-ol.

De manière analogue à l'exemple 1(i), par réaction de 6,9 ml (21 mmol) de bromure de méthylmagnésium 3M/éther avec 935 mg (2,06 mmol) de 6-{3-[2-(3,5-bis-éthoxyméthoxy-phényl)-vinyl]-phényl}-hex-5-enoate de méthyle dans 20 ml d'éther, après purification sur colonne de silice (acétate d'éthyle 30 - heptane 70), une huile jaune ( m= 352 mg ; R= 36%) est obtenue.
**RMN 1H** ( CDCl3 ): 1,23 (6H, s), 1,18-1,28 (6H, m), 1,51-1,69 (4H, m), 2,21-2,26 (2H, m), 3,71-3,79 (4H, q), 5,24 (4H, s), 6,21-6,33 (1H, m), 6,38-6,44 (1H, d, J= 15,9 Hz), 6,66-6,67 (1H, t), 6,87-6,88 (2H, d), 7,04-7,05 (2H, d), 7,22-7,36 (3H, m), 7,47 (1H, s).

### g) 5-{2-[3-(6-hydroxy-6-méthyl-hept-1-enyl)-phényl]-vinyl}-benzène-1,3-diol.

De manière analogue à l'exemple 1(j), par réaction de 0,15 ml d'acide sulfurique concentré dans 3 ml de méthanol avec 314 mg (0,67 mmol) de 7-{3-[2-(3,5-bis-éthoxyméthoxy-phényl)-vinyl]-phényl}-2-méthyl-hept-6-en-2-ol dans 3 ml de méthanol et 3 ml de THF, après purification sur colonne de silice (acétate d'éthyle 50 - heptane 50), des cristaux blancs (m= 206 mg ; R= 91%) sont obtenus. Tf= 60-4°C.
**RMN 1H** (CDCl3): 1,24 (6H, s), 1,53-1,61 (4H, m), 2,17-2,22 (2H, m), 6,18-6,30 (1H, m), 6,35-6,36 (2H, d), 6,57 (2H, d), 6,89-7,03 (2H, m), 7,21-7,30 (3H, m), 7,40 (1H, s), 7,96 (2H, OH, s).

### EXEMPLE 42

### 5-{2-[3-(7-éthyl-7-hydroxy-non-1-enyl)-phényl]-vinyl}-benzène-1,3-diol.

### a) 7-{3-[2-(3,5-bis-éthoxyméthoxy-phényl)-vinyl]-phényl}-hept-6-enoate de méthyle.

De manière analogue à l'exemple 40(a), par réaction de 1,03 g (2,5 mmol) de 3-bromo-[2-(3,5-bis-éthoxyméthoxy-phényl)-vinyl]-phényl dans 30 ml de diméthylformamide avec 430 mg (3 mmol) de hept-6-enoate de méthyle et 700 mg (5 mmol) de carbonate de potassium et comme catalyseur 82 mg (0,1 mmol) de [1,1'-bis-(diphénylphosphino)ferrocene]dichloropalladium, après purification sur colonne de silice (acétate d'éthyle 10 - heptane 90), une huile jaunâtre (m= 843 mg ; R= 72%) est obtenue.
**RMN 1H** (CDCl3): 1,22-1,27 (6H, t), 1,47-1,85 (4H, m),. 2,21-2,42 (4H, m), 3,66 (3H, s), 3,67-3,79 (4H, q), 5,24 (4H, s), 6,19-6,30 (1H, m), 6,37-6,44 (1H, d, J= 15,9 Hz), 6,66-6,67 (1H, t), 6,87 (2H, d), 7,05-7,07 (2H, m), 7,21-7,41 (3H, m), 7,47 (1H, s).

### b) 9-{3-[2-(3,5-bis-éthoxyméthoxy-phényl)-vinyl]-phényl}-3-éthyl-non-8-en-3-ol.

De manière analogue à l'exemple 1(i), par réaction de 7 ml de bromure d'éthylmagnésium 1M/THF avec 824 mg (1,76 mmol) de 7-{3-[2-(3,5-bis-éthoxyméthoxy-phényl)-vinyl]-phényl}-hept-6-enoate de méthyle dans 20 ml d'éther, après purification sur colonne de silice (acétate d'éthyle 15 - heptane 85), une huile jaune (m= 728 mg ; R= 83%) est obtenue.
**RMN 1H** ( CDCl3 ): 0,81-0,89 (6H, m), 1,22-1,27 (6H, m), 1,34-1,51 (10H, m), 2,17-2,29 (2H, m), 3,71-3,79 (4H, q), 5,24 (4H, s), 6,21-6,32 (1H, m), 6,37-6,43 (1H, d, J= 15,9 Hz), 6,66-6,67 (1H, t), 6,87 (2H, d), 7,05-7,07 (2H, m), 7,22-7,36 (3H, m), 7,47 (1H, s).

### c) 5-{2-[3-(7-éthyl-7-hydroxy-non-1-enyl)-phényl]-vinyl}-benzène-1,3-diol.

De manière analogue à l'exemple 1 (j), par réaction de 0,3 ml d'acide sulfurique concentré dans 7 ml de méthanol avec 700 mg (1,41 mmol) de 9-{3-[2-(3,5-bis-éthoxyméthoxy-phényl)-vinyl]-phényl}-3-éthyl-non-8-en-3-ol dans 7 ml de méthanol et 7 ml de THF, après purification sur colonne de silice (acétate d'éthyle 40 - heptane 60), des cristaux beiges (m= 477 mg ; R= 77%) sont obtenus. Tf= 98-102°C.
**RMN 1H** (CDCl3): 0,67-0,73 (6H, m), 1,25-1,31 (8H, m), 2,10-2,12 (2H, m), 2,76-2,79 (2H, m, 1H, OH, s), 6,16-6,35 (3H, m), 6,45 (2H, s), 6,96 (2H, s), 7,14-7,15 (2H, m), 7,27 (1H, m), 7,45 (1H, s), 8,16 (1H, s).

### EXEMPLE 43

### 5-{2-[3-(7-hydroxy-1-méthoxy-1,7-diméthyl-octyl)-phényl]-vinyl}-benzène-1,3-diol.

### a) 1,3-bis-éthoxyméthoxy-5-vinyl-benzène.

A 0°C, 18 ml (36 mmol) de phényllithium 2M/cyclohexane-éther sont ajoutés, goutte à goutte, à 14,3 g (0,04 mol) de bromure de méthyltriphénylphosphonium. La solution est agitée à température ambiante pendant 4 h30. Elle est ensuite refroidie à -70°C et on ajoute, goutte à goutte, 5,10 g (0,02 mol) de 3,5-bis-éthoxyméthoxy-benzaldéhyde dans 50 ml de THF. Le milieu est agité 2h à -70°C puis à température ambiante 12h. Il est, alors, versé dans une solution aqueuse saturée en chlorure d'ammonium et extrait à l'éther. La phase organique est lavée à l'eau, séchée sur sulfate de magnésium et concentrée. Le résidu est purifié sur colonne de silice (dichlorométhane 30 - heptane 70).
Huile jaune. m= 4,4 g. R= 87%.
**RMN 1H** (CDCl3): 1,20-1,25 (6H, t), 3,68-3,77 (4H, q), 5,22 (4H, s), 5,69-5,76 (1H, dd, J= 16,9 Hz, J'= 0,55 Hz), 6,58-6,69 (2H, m), 6,75-6,76 (2H, d).

### b) 1-{3-[2-(3,5-bis-éthoxyméthoxy-phényl)-vinyl]-phényl}-éthanone.

De manière analogue à l'exemple 40(a), par réaction de 2,5 g (12,5 mmol) du 3-bromoacétophénone dans 100 ml de diméthylformamide avec 3,47 g (13,7 mmol) de 1,3-bis-éthoxyméthoxy-5-vinyl-benzène et 3,47 g (25 mmol) de carbonate de potassium et comme catalyseur 408 mg (0,5 mmol) de [1,1'-bis-(diphénylphosphino)ferrocene]dichloropalladium, après purification sur colonne de silice (acétate d'éthyle 15 - heptane 85), une huile jaune (m= 3,88 g ; R=84%) est obtenue.
**RMN 1H** (CDCl3): 1,22-1,28 (6H, t), 2,64 (3H, s), 3,71-3,80 (4H, m), 5,25 (4H, s), 6,68-6,70 (1H, t), 6,88-6,89 (2H, d), 7,11 (2H, s), 7,42-7,48 (1H, t), 7,68-7,71 (1H, d, J= 7,8 Hz), 7,82-7,85 (1H, d, J= 7,7 Hz), 8,08 (1H, s).

### c) 2-{3-[2-(3,5-bis-éthoxyméthoxy-phényl)-vinyl]-phényl}-8-méthyl-8-(tétrahydro-pyran-2-yloxy)-nonan-2-ol.

De manière analogue à l'exemple 8(j), par réaction de 1,85 g (5 mmol) de 1-{3-[2-(3,5-bis-éthoxyméthoxy-phényl)-vinyl]-phényl}-éthanone avec la solution de 334 mg (13,7 mmol) de magnésium, de 3,67 g (12,5 mmol) de 2-(6-bromo-1,1-diméthyl-hexyloxy)-tétrahydro-pyranne dans 12 ml de THF et catalysée par 107 mg (0,2 mmol) de [1,2-bis-(diphénylphosphino)éthane]dichloronickel, après purification sur colonne de silice (acétate d'éthyle 15 - heptane 85), une huile jaune (m= 1,16 g ; R= 40%) est obtenue.
**RMN 1H** (CDCl3): 1,14-1,16 (6H, d), 1,22-1,27 (6H, m), 1,58 (3H, s), 1,39-1,81 (16H, m), 3,38-3,43 (1H, m), 3,71-3,79 (4H, m), 4,89-4,94 (1H, m), 4,66 (1H, m), 5,24 (4H, s), 6,65-6,67 (1H, t), 6,87-6,88 (2H, d), 7,01-7,15 (2H, dd, J= 16,3 Hz, J'= 2,4 Hz), 7,30-7,39 (3H, m), 7,58 (1H, s).

### d) 5-{2-[3-(7-hydroxy-1-méthoxy-1,7-diméthyl-octyl)-phényl]-vinyl}-benzène-1,3-diol.

De manière analogue à l'exemple 1 (j), par réaction de 0,2 ml d'acide sulfurique concentré dans 3 ml de méthanol avec 350 mg (0,6 mmol) de 2-{3-[2-(3,5-bis-éthoxyméthoxy-phényl)-vinyl]-phényl}-8-méthyl-8-(tétrahydro-pyran-2-yloxy)-nonan-2-ol dans 3 ml de méthanol et 3 ml de THF, après purification sur colonne de silice (acétate d'éthyle 60 - heptane 40), des cristaux blancs (m= 76 mg ; R= 32%) sont obtenus. Tf= 65-75°C.
RMN 1H (Acétone): 0,91 (6H, s), 1,03-1,16 (8H, m), 1,34 (3H, s), 1,59 (2H, m), 2,70 (2H, OH, s), 2,88 (3H, s), 6,11 (1H, s), 6,40-6,41 (2H, d), 6,93 (2H, s), 7,12-7,18 (2H, m), 7,26-7,29 (1H, d, J= 6,8 Hz), 7,39 (1H, s), 8,11 (1H, s).

### EXEMPLE 44

### 5-{2-[3-(6-hydroxy-1-méthoxy-1,6-diméthyl-heptyl)-phényl]-vinyl}-henzène-1,3-diol.

### a) 2- {3-[2-(3,5-bis-éthoxyméthoxy-phényl)-vinyl]-phényl}-7-méthyl-7-(tétrahydro-pyran-2-yloxy)-octan-2-ol.

De manière analogue à l'exemple 8(j), par réaction de 1,18 g (3,18 mmol) de 1-{3-[2-(3,5-bis-éthoxyméthoxy-phényl)-vinyl]-phényl}-éthanone avec une solution de 213 mg (8,75 mmol) de magnésium, de 2,15 g (7,7 mmol) de 2-(5-bromo-1,1-diméthyl-pentyloxy)-tétrahydro-pyranne dans 8 ml de THF et catalysée par 68 mg (0,13 mmol) de [1,2-bis-(diphénylphosphino)éthane]dichloronickel, après purification sur colonne de silice (acétate d'éthyle 25 - heptane 75), une huile jaune (m= 768 mg ; R= 42%) est obtenue.
**RMN 1H** (CDCl3): 0,94-0,96 (6H, d), 1,02-1,09 (6H, m), 1,38 (3H, s), 1,21-1,64 (14H, m), 3,18-3,25 (1H, m), 3,51-3,60 (4H, m), 3,69-3,74 (1H, m), 4,46-4,48 (1H, m), 5,04 (4H, s), 6,46-6,47 (1H, t), 6,67-6,68 (2H, d), 6,81-6,95 (2H, dd, J= 16,4 Hz, J'= 2,4 Hz), 7,11-7,19 (3H, m), 7,38 (1H, s).

### b) 5-{2-[3-(6-hydroxy-1-méthoxy-1,6-diméthyl-heptyl)-phényl]-vinyl}-benzène-1,3-diol.

De manière analogue à l'exemple 1 (j), par réaction de 0,2 ml d'acide sulfurique concentré dans 3 ml de méthanol avec 340 mg (0,6 mmol) de 2-{3-[2-(3,5-bis-éthoxyméthoxy-phényl)-vinyl]-phényl}-7-méthyl-7-(tétrahydro-pyran-2-yloxy)-octan-2-ol dans 3 ml de méthanol et 3 ml de THF, après purification sur colonne de silice (acétate d'éthyle 60 - heptane 40), des cristaux blancs (m= 72 mg ; R= 32%) sont obtenus. Tf= 65-75°C.
**RMN 1H** (CDCl3): 1,17 (6H, s), 1,26-1,40 (6H, m), 1,55 (3H, s), 1,76-1,82 (2H, t), 3,10 (3H, s), 6,35 (1H, t), 6,60-6,61 (2H, d), 6,93-7,09 (2H, dd, J= 16,3 Hz, J'= 5 Hz), 7,20-7,39 (5H, m), 7,47 (1H, s).

### EXEMPLE 45

### 5-{2-[3-(5-hydroxy-pentyl)-phényl]-vinyl}-benzène-1,3-diol.

### a) 5-{3-[2-(3,5-bis-éthoxyméthoxy-phényl)-vinyl]-phényl}-pentanoate de méthyle.

De manière analogue à l'exemple 1(h), par réaction de 1,54 g (3,77 mmol) de 3-bromo-[2-(3,5-bis-éthoxyméthoxy-phényl)-vinyl]-phényl dans 15 ml de diméthylformamide avec la solution à 0°C, de 1,61 g (6,6 mmol) de 9-borabicyclo[3,3,1]nonane dans 40 ml de THF et 502 mg (4,4 mmol) de pent-4-enoate de méthyle dans 10 ml de THF, après purification sur colonne de silice (acétate d'éthyle 15 - heptane 85), une huile jaune (m= 1,19 g ; R= 71 %) est obtenue.
**RMN 1H** (CDCl3): 1,21-1,27 (6H, t), 1,33-1,87 (4H, m), 2,32-2,38 (2H, t), 2,64 (2H, t), 3,66 (3H, s), 3,70-3,84 (4H, m), 5,24 (4H, s), 6,65-6,67 (1H, t), 6,86-6,87 (2H, d), 7,03-7,09 (3H, m), 7,23-7,34 (3H, m).

### b) 5-{3-[2-(3,5-bis-éthoxyméthoxy-phényl)-vinyl]-phényl}-pentan-1-ol.

De manière analogue à l'exemple 1 (e), par réaction de 5 ml d'hydrure de diisobutylaluminium 1M/toluène, à -78°C, avec 738 mg (1,67 mmol) de 5-{3-[2-(3,5-bis-éthoxyméthoxy-phényl)-vinyl]-phényl}-pentanoate de méthyle, après purification sur colonne de silice (acétate d'éthyle 40 - heptane 60), une huile jaune (m= 259 mg ; R= 37%) est obtenue.
**RMN 1H** (CDCl3): 1,21-1,27 (6H, t), 1,38-1,84 (6H, m), 2,61-2,67 (2H, t), 3,62-3,67 (2H, t), 3,70-3,85 (4H, m), 5,24 (4H, s), 6,65-6,67 (1H, t), 6,86-6,87 (2H, d), 7,03-7,09 (3H, m), 7,22-7,31 (3H, m).

### c) 5-{2-[3-(5-hydroxy-pentyl)-phényl]-vinyl}-benzène-1,3-diol.

De manière analogue à l'exemple 1(j), par réaction de 0,1 ml d'acide sulfurique concentré dans 1 ml de méthanol avec 90 mg (0,2 mmol) de 5-{3-[2-(3,5-bis-éthoxyméthoxy-phényl)-vinyl]-phényl}-pentan-1-ol dans 1 ml de méthanol et 1 ml de THF, après purification sur colonne de silice (acétate d'éthyle 60 - heptane 40), une poudre blanche (m= 33 mg ; R= 55%) est obtenue. Tf= 144-6°C.
**RMN 1H** (CDCl3): 1,46-1,80 (6H, m), 2,68-2,74 (2H, t), 3,58-3,63 (2H, m), 6,38-6,39 (1H, t), 6,66 (2H, d), 7,16 (3H, s), 7,30-7,36 (1H, t), 7,44-7,50 (2H, m), 8,37 (1H, OH, s).

### EXEMPLE 46

### 5-{2-[3-(5-hydroxy-6-méthyl-heptyl)-phényl]-vinyl}-benzène-1,3-diol.

### a) 5-{3-[2-(3,5-bis-éthoxyméthoxy-phényl)-vinyl]-phényl}-pentanal.

586 mg (1,55 mmol) de pyridinium dichromate sont ajoutés à une solution de 162 mg (0,39 mmol) de 5-{3-[2-(3,5-bis-éthoxyméthoxy-phényl)-vinyl]-phényl}-pentan-1-ol dans 2 ml de dichlorométhane. Le milieu réactionnel est agité toute une nuit à température ambiante. Après évaporation, le résidu est purifié sur colonne de silice (dichlorométhane).
Huile jaunâtre. m= 76 mg. R= 47%.
**RMN 1H** (CDCl3): 1,21-1,27 (6H, t), 1,66-1,72 (4H, m), 2,47 (2H, m), 2,65 (2H, m), 3,71-3,79 (4H, m), 5,24 (4H, s), 6,65-6,67 (1H, t), 6,86-6,87 (2H, d), 7,04-7,08 (3H, m), 7,20-7,35 (3H, m), 9,76-9,77 (1H, t).

### b) 7-{3-[2-(3,5-bis-éthoxyméthoxy-phényl)-vinyl]-phényl}-2-méthyl-heptan-3-ol.

0,18 ml (0,38 mmol) de chlorure d'isopropylmagnésium 2M sont ajoutés à 74 mg (0,18 mmol) de 5-{3-[2-(3,5-bis-éthoxyméthoxy-phényl)-vinyl]-phényl}-pentanal dans 1,5 ml d'éther. Le milieu est chauffé à 30-35°C pendant la nuit. Il est, alors, versé dans un mélange éther/eau. Après décantation, la phase organique est lavée avec une solution d'acide chlorhydrique 1N puis avec de l'eau. Elle est ensuite séchée sur sulfate de magnésium et concentrée. Le résidu est purifié sur colonne de silice (acétate d'éthyle 20 - heptane 80).
Huile jaunâtre. m= 62 mg. R= 75%.
**RMN 1H** (CDCl3): 0,89-0,93 (6H, dd), 1,22-1,27 (6H, t), 1,39-1,69 (6H, m), 2,61-2,67 (2H, t), 3,37 (1H, m), 3,71-3,79 (4H, m), 5,24 (4H, s), 6,66-6,67 (1H, t), 6,86-6,87 (2H, d), 6,97-7,09 (3H, m), 7,23-7,31 (3H, m).

### c) 5-{2-[3-(5-hydroxy-6-méthyl-heptyl)-phényl]-vinyl}-benzène-1,3-diol.

De manière analogue à l'exemple 1(j), par réaction de 0,1 ml d'acide sulfurique concentré dans 1 ml de méthanol avec 60 mg (0,13 mmol) de 7- {3-[2-(3,5-bis-éthoxyméthoxy-phényl)-vinyl]-phényl}-2-méthyl-heptan-3-ol dans 1 ml de méthanol et 1 ml de THF, après purification sur colonne de silice (acétate d'éthyle 40 - heptane 60), une huile jaunâtre (m= 7 mg ; R= 16%) est obtenue.
**RMN 1H** (CDCl3): 0,90-0,93 (6H, d), 1,43-1,81 (6H, m), 2,60-2,66 (2H, t), 3,38 (1H, m), 6,32-6,38 (3H, m), 6,58 (2H, s), 6,90-7,05 (2H, dd, J= 16,3 Hz, J'= 4,8 Hz), 7,05-7,08 (1H, d, J= 7,8 Hz), 7,21-7,27 (3H, m).

### EXEMPLE 47

### 5-{2-[3-(6-hydroxy-7-méthyl-octyl)-phényl]-vinyl}-benzène-1,3-diol.

### a) 6-{3-[2-(3,5-bis-éthoxyméthoxy-phényl)-vinyl]-phényl}-hexanoate de méthyle.

De manière analogue à l'exemple 1(h), par réaction de 1,50 g (3,68 mmol) de 3-bromo-[2-(3,5-bis-éthoxyméthoxy-phényl)-vinyl]-phényl dans 30 ml de diméthylformamide avec la solution à 0°C, de 1,61 g (6,6 mmol) de 9-borabicyclo[3,3,1]nonane dans 30 ml de THF et 564 mg (4,4 mmol) de hex-5-enoate de méthyle dans 10 ml de THF, après purification sur colonne de silice (acétate d'éthyle 10 - heptane 90), une huile jaunâtre (m= 430 mg ; R= 26%) est obtenue.
**RMN 1H** (CDCl3): 1,22-1,27 (6H, t), 1,34-1,87 (6H, m), 2,29-2,35 (2H, t), 2,59-2,65 (2H, t), 3,66 (3H, s), 3,71-3,81 (4H, m), 5,24 (4H, s), 6,65-6,67 (1H, t), 6,86-6,87 (2H, d), 6,97-7,11 (3H, m), 7,23-7,34 (3H, m).

### b) 6-{3-[2-(3,5-bis-éthoxyméthoxy-phényl)-vinyl]-phényl}-hexan-1-ol.

De manière analogue à l'exemple 1(e), par réaction de 2,1 ml d'hydrure de diisobutylaluminium 1M/toluène, à -78°C, avec 315 mg (0,69 mmol) de 6-{3-[2-(3,5-bis-éthoxyméthoxy-phényl)-vinyl]-phényl}-hexanoate de méthyle, après purification sur colonne de silice (acétate d'éthyle 40 - heptane 60), une pâte jaune (m= 198 mg ; R= 66%) est obtenue.
**RMN 1H** (CDCl3): 1,21-1,27 (6H, t), 1,38-1,40 (4H, m), 1,52-1,68 (4H, m), 2,59-2,65 (2H, t), 3,61-3,66 (2H, t), 3,70-3,85 (4H, m), 5,24 (4H, s), 6,66-6,67 (1H, t), 6,86-6,87 (2H, d), 6,97-7,11 (3H, m), 7,22-7,33 (3H, m).

### c) 6-{3-[2-(3,5-bis-éthoxyméthoxy-phényl)-vinyl]-phényl}-hexanal.

De manière analogue à l'exemple 46(a), par réaction de 692 mg (1,83 mmol) de pyridinium dichromate avec 197 mg (0,46 mmol) de 6-{3-[2-(3,5-bis-éthoxyméthoxy-phényl)-vinyl]-phényl}-hexan-1-ol dans 3 ml de dichlorométhane, après purification sur colonne de silice (dichlorométhane), une pâte jaune (m= 76 mg ; R= 75%) est obtenue.
**RMN 1H** (CDCl3): 1,22-1,27 (6H, t), 1,39-1,45 (2H, m), 1,62-1,73 (4H, m), 2,40-2,46 (2H, m), 2,60-2,66 (2H, t), 3,71-3,80 (4H, m), 5,24 (4H, s), 6,65-6,67 (1H, t), 6,86-6,87 (2H, d), 6,97-7,12 (3H, m), 7,20-7,34 (3H, m), 9,76 (1H, t).

### d) 8-{3-[2-(3,5-bis-éthoxyméthoxy-phényl)-vinyl]-phényl}-2-méthyl-octan-3-ol.

De manière analogue à l'exemple 46(b), par réaction de 0,35 ml (0,7 mmol) de chlorure d'isopropylmagnésium 2M/éther avec 148 mg (0,35 mmol) de 6-{3-[2-(3,5-bis-éthoxyméthoxy-phényl)-vinyl]-phényl}-hexanal dans 2 ml d'éther, après purification sur colonne de silice (acétate d'éthyle 15 - heptane 85), une huile jaune (m= 130 mg ; R= 79%) est obtenue.
**RMN 1H** (CDCl3): 0,88-0,92 (6H, dd), 1,22-1,27 (6H, t), 1,37-1,68 (8H, m), 2,60-2,66 (2H, t), 3,35 (1H, m), 3,71-3,79 (4H, m), 5,24 (4H, s), 6,66-6,67 (1H, t), 6,86-6,87 (2H, d), 6,97-7,09 (3H, m), 7,23-7,31 (3H, m).

### e) 5-{2-[3-(6-hydroxy-7-méthyl-octyl)-phényl]-vinyl}-benzène-1,3-diol.

De manière analogue à l'exemple 1(j), par réaction de 0,2 ml d'acide sulfurique concentré dans 1 ml de méthanol avec 130 mg (0,27 mmol) de 8-{3-[2-(3,5-bis-éthoxyméthoxy-phényl)-vinyl]-phényl}-2-méthyl-octan-3-ol dans 1 ml de méthanol et 1 ml de THF, après purification sur colonne de silice (acétate d'éthyle 40 - heptane 60), une huile jaunâtre (m= 12 mg ; R= 13%) est obtenue.
**RMN 1H** (CDCl3): 0,90-0,93 (6H, d), 1,36-2,17 (8H, m), 2,57-2,63 (2H, t), 3,38 (1H, m), 6,34-6,38 (1H, t), 6,58-6,59 (2H, d), 6,89-7,04 (2H, dd, J= 16,3 Hz, J'= 3,6 Hz), 7,04-7,06 (1H, d, J= 6,9 Hz), 7,14-7,29 (3H, m).

### EXEMPLE 48

### 5-{2-[3-(5-hydroxy-6-méthyl-hept-1-enyl)-phényl]-vinyl}-benzène-1,3-diol.

### a) 5-{3-[2-(3,5-bis-éthoxyméthoxy-phényl)-vinyl]-phényl}-pent-4-enoate de méthyle.

De manière analogue à l'exemple 40(a), par réaction de 1,5 g (3,68 mmol) de 3-bromo-[2-(3,5-bis-éthoxyméthoxy-phényl)-vinyl]-phényl. dans 30 ml de diméthylformamide avec 502 mg (4,4 mmol) de pent-4-enoate de méthyle et 1,02 g (7,36 mmol) de carbonate de potassium et comme catalyseur 120 mg (0,15 mmol) de [1,1'-bis-(diphénylphosphino)ferrocene]dichloropalladium, après purification sur colonne de silice (acétate d'éthyle 10 - heptane 90),une pâte jaune (m= 883 mg ; R= 54%) est obtenue.
**RMN 1H** (CDCl3): 1,22-1,27 (6H, t), 2,47-2,59 (4H, m), 3,70 (3H, s), 3,67-3,79 (4H, q), 5,24 (4H, s), 6,19-6,30 (1H, m), 6,42-6,48 (1H, d, J= 15,9 Hz), 6,66-6,67 (1H, t), 6,86-6,87 (2H, d, J= 2,1 Hz), 7,04 (2H, s), 7,21-7,37 (3H, m), 7,46 (1H, s).

### b) 5-{3-[2-(3,5-bis-éthoxyméthoxy-phényl)-vinyl]-phényl}-pent-4-en-1-ol.

De manière analogue à l'exemple 1(e), par réaction de 6 ml d'hydrure de diisobutylaluminium 1M/toluène, à -78°C, avec 869 mg (1,97 mmol) de 5-{3-[2-(3,5-bis-éthoxyméthoxy-phényl)-vinyl)-phényl}-pent-4-enoate de méthyle dans 20 ml de toluène, après purification sur colonne de silice (acétate d'éthyle 30 - heptane 70), une pâte jaune (m= 381 mg ; R= 47%) est obtenue.
**RMN 1H** (CDCl3): 1,22-1,27 (6H, t), 1,72-1,83 (2H, m), 2,29-2,38 (2H, m), 3,71-3,79 (6H, m), 5,24 (4H, s), 6,22-6,34 (1H, m), 6,41-6,47 (1H, d, J= 15,9 Hz), 6,66-6,67 (1H, t), 6,86-6,87 (2H, d, J= 1,94 Hz), 7,05 (2H, s), 7,22-7,36 (3H, m), 7,47 (1H, s)

### c) 5-{3-[2-(3,5-bis-éthoxyméthoxy-phényl)-vinyl]-phényl}-pent-4-enal.

De manière analogue à l'exemple 46(a), par réaction de 1,38 g (3,68 mmol) de pyridinium dichromate avec 380 mg (0,92 mmol) de 5-{3-[2-(3,5-bis-éthoxyméthoxy-phényl)-vinyl]-phényl}-pent-4-en-1-ol dans 10 ml de dichlorométhane, après purification sur colonne de silice (dichlorométhane), une pâte jaune (m= 245 mg ; R= 65%) est obtenue.
**RMN 1H** (CDCl3): 1,22-1,27 (6H, t), 2,56-2,66 (4H, m), 3,71-3,79 (4H, q), 5,24 (4H, s), 6,19-6,31 (1H, m), 6,42-6,48 (1H, d, J= 15,9Hz), 6,66-6,68 (1H, t), 6;86-6,87 (2H, d, J= 2,1 Hz), 7,04 (2H, s), 7,20-7,37 (3H, m), 7,46 (1H, s), 9,84 (1H, s).

### d) 7-{3-[2-(3,5-bis-éthoxyméthoxy-phényl)-vinyl]-phényl}-2-méthyl-hept-6-en-3-ol.

De manière analogue à l'exemple 46(b), par réaction de 0,58 ml (1,16 mmol) de chlorure d'isopropylmagnésium 2M/éther avec 239 mg (0,58 mmol) de 5-{3-[2-(3,5-bis-éthoxyméthoxy-phényl)-vinyl]-phényl}-pent-4-enal dans 2,5 ml d'éther, après purification sur colonne de silice (acétate d'éthyle 15 - heptane 85), une huile jaune (m= 137 mg ; R= 52%) est obtenue.
**RMN 1H** (CDCl3): 0,92-0,94 (6H, m), 1,25 (6H, s), 1,62-1,73 (2H, m), 2,26-2,45 (2H, m), 3,44 (1H, m), 3,71-3,79 (4H, q), 5,24 (4H, s), 6,24-6,35 (1H, m), 6,42-6,48 (1H, d, J= 15,9 Hz), 6,66-6,67 (1H, t), 6,86-6,87 (2H, d, J= 2,08 Hz), 7,05 (2H, s), 7,22-7,36 (3H, m), 7,47 (1H, s).

### e) 5-{2-[3-(5-hydroxy-6-méthyl-hept-1-enyl)-phényl]-vinyl}-benzène-1,3-diol.

De manière analogue à l'exemple 1(j), par réaction de 0,3 ml d'acide sulfurique concentré dans 2,8 ml de méthanol avec 140 mg (0,31 mmol) de 7-{3-[2-(3,5-bis-éthoxyméthoxy-phényl)-vinyl]-phényl}-2-méthyl-hept-6-en-3-ol dans 2,8 ml de THF, après purification sur colonne de silice (acétate d'éthyle 40 - heptane 60), une poudre blanche (m= 80 mg ; R= 77%) est obtenue. Tf= 51-60°C.
**RMN 1H** (CDCl3): 0,85-0,88 (6H, d), 1,58-1,63 (2H, m), 2,23-2,37 (2H, m), 3,37-3,46 (1H, m), 6,16-6,37 (3H, m), 6,52 (2H, s), 6,88 (2H, m), 7,13-7,22 (3H, m), 7,31 (2H, s).

### EXEMPLE 49

### 5-{2-[3-(6-hydroxy-7-méthyl-oct-1-enyl)-phényl]-vinyl}-benzène-1,3-diol.

### a) 6-{3-[2-(3,5-bis-éthoxyméthoxy-phényl)-vinyl]-phényl}-hex-5-en-1-ol.

De manière analogue à l'exemple 1(e), par réaction de 3,9 ml d'hydrure de diisobutylaluminium 1M/toluène, à -78°C, avec 581 mg (1,28 mmol) de 6-{3-[2-(3,5-bis-éthoxyméthoxy-phényl)-vinyl]-phényl}-hex-5-enoate de méthyle dans 15 ml de toluène, après purification sur colonne de silice (acétate d'éthyle 30 - heptane 70), une huile jaune (m= 290 mg ; R= 53%) est obtenue.
**RMN 1H** (CDCl3): 1,22-1,27 (6H, t), 1,57-1,68 (4H, m), 2,23-2,31 (2H, m), 3,71-3,79 (6H, m), 5,24 (4H, s), 6,20-6,32 (1H, m), 6,38-6,45 (1H, d, J= 15,9 Hz), 6,66-6,67 (1H, t), 6,86-6,87 (2H, d, J= 2 Hz), 7,05 (2H, s), 7,22-7,36 (3H, m), 7,47 (1H, s).

### b) 6-{3-[2-(3,5-bis-éthoxyméthoxy-phényl)-vinyl]-phényl}-hex-5-enal.

De manière analogue à l'exemple 46(a), par réaction de 1,02 g (2,7 mmol) de pyridinium dichromate avec 290 mg (0,68 mmol) de 6-{3-[2-(3,5-bis-éthoxyméthoxy-phényl)-vinyl]-phényl}-hex-5-en-1-ol dans 10 ml de dichlorométhane, après purification sur colonne de silice (dichlorométhane), une pâte jaunâtre (m= 202 mg ; R= 70%) est obtenue.
**RMN 1H** (CDCl3): 1,22-1,27 (6H, t), 1,78-1,90 (2H, m), 2,24-2,33 (2H, m), 2,49-2,54 (2H, m), 3,71-3,79 (4H, q), 5,24 (4H, s), 6,16-6,28 (1H, m), 6,39-6,45 (1H, d, J= 15,9 Hz), 6,66-6,68 (1H, t), 6,87 (2H, d, J= 2,1 Hz), 7,05 (2H, s), 7,20-7,37 (3H, m), 7,47 (1H, s), 9,80 (1H, s).

### c) 8-{3-[2-(3,5-bis-éthoxyméthaxy-phényl)-vinyl]-phényl}-2-méthyl-oct-7-en-3-ol.

De manière analogue à l'exemple 46(b), par réaction de 0,47 ml (0,94 mmol) de chlorure d'isopropylmagnésium 2M/éther avec 198 mg (0,47 mmol) de 6-{3-[2-(3,5-bis-éthoxyméthoxy-phényl)-vinyl]-phényl}-hex-5-enal dans 2 ml d'éther, après purification sur colonne de silice (acétate d'éthyle 15 - heptane 85), une huile jaune (m= 107 mg ; R= 49%) est obtenue.
**RMN 1H** (CDCl3): 0,90-0,94 (6H, dd), 1,22-1,27 (6H, t), 1,42-1,74 (4H, m), 2,23-2,28 (2H, m), 3,40 (1H, s), 3,71-3,79 (4H, q), 5,24 (4H, s), 6,21-6,33 (1H, m), 6,38-6,44 (1H, d, J= 15,9 Hz), 6,66-6,67 (1H, t), 6,86-6,87 (2H, d, J= 2,1 Hz), 7,05 (2H, s), 7,22-7,36 (3H, m), 7,47 (1H, s).

### d) 5-{2-[3-(6-hydroxy-7-méthyl-oct-1-enyl)-phényl]-vinyl}-benzène-1,3-diol.

De manière analogue à l'exemple 1(j), par réaction de 0,3 ml d'acide sulfurique concentré dans 2,5 ml de méthanol avec 126 mg (0,27 mmol) de 8-{3-[2-(3,5-bis-éthoxyméthoxy-phényl)-vinyl]-phényl}-2-méthyl-oct-7-en-3-ol dans 2,5 ml de THF, après purification sur colonne de silice (acétate d'éthyle 40 - heptane 60), une poudre beige (m= 60 mg ; R= 63%) est obtenue. Tf= 115-20°C.
**RMN 1H** (CDCl3): 0,91-094 (6H, d), 1,50-1,69 (4H, m), 2,24 (2H, m), 3,43-3,54 (1H, m), 6,17-6,40 (3H, m), 6,59 (2H, s), 6,95 (2H, s), 7,20-7,31 (3H, m), 7,38 (1H, s), 7,59 (2H, s).

### EXEMPLE 50

### 5-{2-[3-(1,6-dihydroxy-1,6-diméthyl-heptyl)-phényl]-vinyl}-benzène-1,3-diol.

De manière analogue à l'exemple 1(j), par réaction de 0,6 ml d'acide sulfurique concentré dans 7,5 ml de méthanol avec 369 mg (0,65 mmol) de 2-{3-[2-(3,5-bis-éthoxyméthoxy-phényl)-vinyl]-phényl}-7-méthyl-7-(tétrahydro-pyran-2-yloxy)-octan-2-ol dans 7,4 ml de THF, après purification sur colonne de silice (acétate d'éthyle 70 - heptane 30), une pâte jaunâtre (m= 220 mg ; R= 92%) est obtenue.
**RMN 1H** (CDCl3): 1,16 (6H, s), 1,28-1,39 (6H, m), 1,57 (3H, s), 1,77-1,84 (2H, m), 6,35-6,37 (1H, t), 6,58-6,59 (2H, d), 6,92-7,06 (2H, dd, J= 16,4 Hz), 2,4 Hz), 7,27-7,38 (3H, m), 7,59 (1H, s), 7,83 (2H, OH, s).

### EXEMPLE 51

### 5-{2-[3-(6-hydroxy-1,6-diméthyl-hept-1-enyl)-phényl]-vinyl}-benzène-1,3-diol.

De manière analogue à l'exemple 1(j), par réaction de 0,09 ml d'acide sulfurique concentré dans 3 ml de THF avec 152 mg (0,41 mmol) de 5-{2-[3-(1,6-dihydroxy-1,6-diméthyl-heptyl)-phényl]-vinyl}-benzène-1,3-diol dans 1,5 ml de THF, après purification sur colonne de silice (acétate d'éthyle 50 - heptane 50), une poudre jaune (m= 65 mg ; R= 45%) est obtenue. Tf= 55-60°C.
**RMN 1H** (CDCl3): 1,21 (3H, s), 1,24 (6H, s), 1,45-1,55 (4H, m), 2,23 (1H, m), 2,54 (1H, m), 5,80 (1H, t), 6,34 (1H, s), 6,59 (2H, s), 6,92-7,08 (2H, dd, J= 16,3 Hz, J'= 6 Hz), 7,28-7,33 (3H, m), 7,45 (1H, m).

### EXEMPLE 52

### 6-{[3-(3,4-Bis-hydroxyméthyl-benzyloxy)-phenyl]-méthyl-amino}-2-méthyl-hexan-2-ol

### a) 6- {[3-(p-Toluènesulfonyloxy)-phenyl]-carbométhoxy-amino}-pentanoate d'éthyle

1 g (3.1 mmol) de 3-*p*-toluènesulfonyloxy-N-carbométhoxy-aniline et 1,9 mL (12 mmol) de 5-bromovalérate d'éthyle sont dissous dans 25 mL de DMF anhydre. 140 mg (4,5 mmol) d'hydrure de sodium 75% sont alors ajoutés, et le milieu réactionnel est agité pendant 6 heures. Après traitement par une solution saturée de chlorure d'ammonium et extraction avec du dichlorométhane, puis séchage et évaporation des solvants de la phase organique, le résidu est purifié par chromatographie sur colonne de silice. Un huile incolore est obtenue (m = 1,35 g; R=97%)

### b) 6-{[3-(p-Toluènesulfonyloxy)-phenyl]-carbométhoxy-amino}-2-méthyl-hexan-2-ol

500 mg de 6-{[3-(*p*-toluènesulfonyloxy)-phenyl]-carbométhoxy-amino}-pentanoate d'éthyle (1,2 mmol) sont dissous dans 20 mL de THF. 1 mL d'une solution de bromure de méthylmagnésium 3.0M (3.0 mmol) est alors ajouté, et le milieu réactionnel est agité 1 heure à température ambiante. Après traitement par une solution saturée de chlorure d'ammonium, séchage et évaporation des solvants de la phase organique, puis une purification par chromatographie sur colonne de silice, une huile incolore est obtenue ( m = 480 mg ; R = 92 %).

### c) 6-[(3-Hydroxyphenyl)-méthyl-amino]-2-méthyl-hexan-2-ol

300 mg de 6-{[3-(*p*-toluènesulfonyloxy)-phenyl]-carbométhoxy-amino}-2-méthyl-hexan-2-ol (0,69 mmol) sont dissous dans 10 mL de THF anhydre. 80 mg (2.1 mmol) d'hydrure double de lithium et d'aluminium sont alors ajoutés, puis le milieu réactionnel est chauffé à reflux pendant deux heures. Après refroidissement, 80 □L d'eau puis 80 □L d'une solution de NaOH 15% puis 240 □L d'eau sont séquentiellement ajoutés; après agitation pendant 1 h, le milieu réactionnel est alors filtré puis le solvant est évaporé. Le résidu obtenu est alors dissous dans un mélange composé de 5 mL d'eau, 5 mL d'éthanol et 200 mg de KOH sont ajoutés. Le milieu réactionnel est alors chauffé 12 heures à 70°C, puis refroidi et traité par un mélange de HCl 1N et de dichlorométhane. Après séparation, la phase organique est alors séchée et les solvants sont évaporés. Après purification par chromatographie sur colonne de silice, une huile incolore est obtenue ( m = 125 mg ; R = 79 %).

### d) 6-{[3-(3,4-Bis-carboxyméthyl-benzyloxy)-phenyl]-méthyl-amino}-2-méthyl-hexan-2-ol

90 mg (0.38 mmol) de 6-[(3-hydroxyphenyl)-méthyl-amino]-2-méthyl-hexan-2-ol sont ajoutés à une solution de bromure de 3-(3,4-dicarboxyméthyl)-benzyle (132 mg, 0,46 mmol) dans de la 2-butanone. 64 mg (0.46 mmol) de carbonate de potassium sont alors ajoutés avant de chauffer le milieu réactionnel à reflux pendant 6 heures. Après filtration et évaporation du solvant et purification par chromatographie sur gel de silice (heptane 60 - acétate d'éthyle 40) une huile jaune est obtenue (m = 67 mg ; R = 40 %)

### e) 6-{[3-(3,4-Bis-hydroxyméthyl-benzyloxy)-phenyl]-méthyl-amino}-2-méthyl-hexan-2-ol

30 mg de b-{[3-(3,4-bis-carboxyméthyl-benzyloxy)-phenyl]-méthyl-amino}-2-méthyl-hexan-2-ol (0.07 mmol) sont dissous dans 3 mL de THF anhydre, puis 20 mg (1 mmol) de borohydrure de lithium sont ajoutés avant de porter le milieu réactionnel à reflux pendant 3 heures. Après traitement du milieu réactionnel par une solution saturée de chlorure d'ammonium puis séchage, évaporation et purification par chromatographie sur colonne de silice (heptane 30 - acétate d'éthyle 70) une huile claire est obtenue (m = 19 mg; R = 72 %).
**RMN** ^{**1**}**H** (CDCl₃): 1,19 (s, 6H), 1,26-1,59 (m, 6H), 2,89 (s, 3H), 3,27 (t, 2H, *J* = 7,2 Hz), 4,70 (s, 4H), 5,04 (s, 2H), 6,28-6,34 (m, 3H), 7,11 (t, 1H, *J* = 7,9 Hz), 7,34 (s, 2H), 7,40 (s, 1H).

### EXEMPLE 53

### 5-{[3-(3,4-Bis-hydroxyméthyl-benzyloxy)-phenyl]-méthyl-amino}-2-méthyl-pentan-2-ol

De manière analogue à l'exemple 52(e), par réaction de 80 mg, (0.18 mmol) de 5-{[3-(3,4-bis-carboxyméthyl-benzyloxy)-phenyl]-méthyl-amino}-2-méthyl-pentan-2-ol (préparé de manière analogue aux exemples 52(a-d)) avec 16 mg (0.75 mmol) de borohydrure de lithium et après purification sur colonne de silice, une huile incolore est obtenue (m = 50 mg ; R = 76 %).
**RMN** ^{**1**}**H** (CDCl₃): 1,18 (s, 6H), 1,37-1,43 (m, 2H), 1,54-1,63 (m, 2H), 1,70 (bs, 1H), 2,89 (s, 3H), 3,25 (t, 2H, *J* = 7,4 Hz), 3,38 (bs, 2H), 4,69 (s, 4H), 5,05 (s, 2H), 6,28-6,34 (m, 3H), 7,11 (t, 1H, *J* = 8,0 Hz), 7,35 (s, 2H), 7,39 (s, 1H).

### EXEMPLE 54

### 6-{[3-(3,4-Bis-hydroxyméthyl-benzyloxy)-phenyl]-méthyl-amino}-3-éthyl-hexan-3-ol

De manière analogue à l'exemple 52(e), par réaction de 250 mg (0.45 mmol) de 6-{[3-(3,4-bis-carboxyméthyl-benzyloxy)-phenyl]-méthyl-amino}-3-éthyl-hexan-3-ol (préparé de manière analogue aux exemples 52(a-d)) avec 40 mg (1,8 mmol) de borohydrure de lithium et après purification sur colonne de silice, une huile incolore est obtenue (m = 160 mg ; R = 88 %).
**RMN** ^{**1**}**H** (CDCl₃): 0,82 (t, 6H, *J* = 7,6 Hz), 1,26-1,56 (m, 8H), 2,89 (s, 3H), 3,25 (t, 2H, *J =* 6,9 Hz), 3,46 (bs, 2H), 4,67 (s, 4H), 5,04 (s, 2H), 6,27-6,33 (m, 3H), 7,11 (t, 1H, *J* = 8,1 Hz), 7,33 (m, 2H), 7,38 (s, 1H).

### EXEMPLE 55

### 7-{[3-(3,4-Bis-hydroxyméthyl-benzyloxy)-phenyl]-méthyl-amino}-3-éthyl-heptan-3-ol

De manière analogue à l'exemple 52(e), par réaction de 370 mg (0.8 mmol) de 7-{[3-(3,4-bis-carboxyméthyl-benzyloxy)-phenyl]-méthyl-amino}-3-éthyl-heptan-3-ol (préparé de manière analogue aux exemples 52(a-d)) avec 80 mg (3,5 mmol) de borohydrure de lithium et après purification sur colonne de silice, une huile incolore est obtenue (m = 266 mg ; R = 80 %).
**RMN** ^{**1**}**H** (CDCl₃): 0,84 (t, 6H, *J* = 7,6 Hz), 1,23-1,55 (m, 10H), 1,7 (bs, 1H), 2,89 (s, 3H), 3,26 (t, 2H, *J* = 7,6 Hz), 3,35 (bs, 2H), 4,69 (s, 2H), 4,69 (s, 2H), 5,04 (s, 2H), 6,29-6,34 (m, 3H), 7,11 (t, 1H, *J* = 8,0 Hz), 7,34 (m, 2H), 7,39 (s, 1H).

### EXEMPLE 56

### 5-{[3-(3,4-Bis-hydroxyméthyl-benzyloxy)-phenyl]-éthyl-amino}-2-méthyl-pentan-2-ol

### a) 5-[(3-tert-Butyldiméthylsilyloxyphenyl)-éthyl-amino]-5-oxo-pentan-2-one

1 g (4 mmol) de (3-tert-butyldiméthylsilyloxy)-N-éthyl-aniline sont dissous dans 40 mL de dichlorométhane à 0°C, puis 511 mg (4,4 mmol) d'acide levulinique et 595 mg (4,4 mmol) de 1-hydroxy-benzotriazole sont ajoutés au milieu réactionnel. 908 mg (4,4 mmol) de dicyclohexylcarbodiimide sont alors additionnés par portions. Le milieu réactionnel est réchauffé jusqu'à température ambiante, agité 1 heure puis filtré. Le filtrat est alors agité 12 h, puis traité avec une solution saturée de chlorure d'ammonium, et extrait avec du dichlorométhane. Après séchage et purification sur colonne de silice, une huile incolore est obtenue ( m = 1,27 g ; R = 91%)

### b) 5-[(3-tert-Butyldiméthylsilyloxyphenyl)-éthyl-amino]-5-oxo-2-méthyl-pentan-2-ol

700 mg (2 mmol) de 5-[(3-*tert*-butyldiméthylsilyloxyphenyl)-éthyl-amino]-5-oxo-pentan-2-one sont dissous dans 20 mL de THF anhydre, et le milieu réactionnel est refroidi à 0°C. 0.66 mL (2 mmol) d'une solution de bromure de méthylmagnésium sont alors ajoutés lentement, et le milieu réactionnel est agité pendant 4 heures à cette température. Après traitement par une solution saturée de chlorure d'ammonium, extraction par de l'acétate d'éthyle, séchage puis évaporation, le résidu est purifié par chromatographie sur colonne de silice pour obtenir une huile incolore ( m = 610 mg ; R = 86 %).

### c) 5-{[3-(3,4-Bis-carboxyméthyl-benzyloxy)-phenyl]-éthyl-amino}-5-oxo-2-méthyl-pentan-2-ol

710 mg de 5-[(3-*tert*-butyldiméthylsilyloxyphenyl)-éthyl-amino]-5-oxo-2-méthyl-pentan-2-ol (2 mmol) sont dissous dans 20 mL de THF, puis 2,4 mL d'une solution de fluorure de tetrabutylammonium (1M dans le THF) sont ajoutés goutte à goutte. Après agitation pendant 1 heure à température ambiante, le milieu réactionnel est traité par une solution de chlorure d'ammonium, puis extrait avec de l'acétate d'éthyle. Après concentration sous pression réduite, le résidu obtenu est ajouté à une solution de bromure de 3-(3,4-dicarboxyméthyl)-benzyle (750 mg, 2,6 mmol) dans de la 2-butanone (25 mL). 305 mg (2,2 mmol) de carbonate de potassium sont alors ajoutés avant de chauffer le milieu réactionnel à reflux pendant 6 heures. Après filtration, évaporation du solvant et purification par chromatographie sur gel de silice (heptane 60 - acétate d'éthyle 40), une huile jaune est obtenue (m = 760 mg ; R = 83 %).

### d) 5-{[3-(3,4-Bis-hydroxyméthyl-benzyloxy)-phenyl]-éthyl-amino}-2-méthyl-pentan-2-ol

350 mg (0.76 mmol) de 5-{[3-(3,4-Bis-carboxyméthyl-benzyloxy)-phenyl]-éthyl-amino}-5-oxo-2-méthyl-pentan-2-ol sont dissous dans 10 mL de THF anhydre, puis 120 mg d'hydrure double de lithium et d'aluminium (3.1 mmol) sont ajoutés en deux portions égales. Après 4 heures de chauffage à reflux, le milieu réactionnel est refroidi puis séquentiellement traité par 120 □L d'eau, 120 □L d'une solution de NaOH 15% puis 360 □L d'eau. Après 1 heure d'agitation, le milieu réactionnel est filtré, concentré puis purifié par chromatographie sur gel de silice. Un huile incolore est obtenue ( m = 280 mg ; R = 95 %).
**RMN** ^{**1**}**H** (CDCl₃): 1,12 (t, 3 H, *J* = 7,0 Hz), 1,18 (s, 6H), 1,38-1,45 (m, 2H), 1,56-1,63 (m, 3H), 3,20 (t, 2H, *J* = 7,5 Hz), 3,31 (q, *2H, J* = 7,0 Hz), 4,69 (s, 2H), 4,70 (s, 2H), 5,05 (s, 2H), 6,24-6,32 (m, 3H), 7,10 (t, 1H, *J* = 8,1 Hz), 7,34 (m, 2H), 7,40 (s, 1H).

### EXEMPLE 57

### 6-{[3-(3,4-Bis-hydroxyméthyl-benzyloxy)-phenyl]amino}-3-éthyl-hexan-3-ol

### a) 6-{[3-(3,4-Bis-carboxyméthyl-benzyloxy)-phenyl]-N-benzoyl-amino}-3-éthyl-hexan-3-ol

De manière analogue à l'exemple 52 (d) par réaction de 130 mg (0.38 mmol) de 6-[(3-hydroxyphenyl)-N-benzoyl-amino-3-éthyl-hexan-3-ol (préparé de manière analogue aux exemples 52(a-c)) avec une solution de bromure de 3-(3,4-dicarboxyméthyl)-benzyle (133 mg, 0,46 mmol) dans de la 2-butanone (5 mL) et 60 mg (0,42 mmol) de carbonate de potassium. Après filtration et évaporation du solvant, une purification par chromatographie sur gel de silice (heptane 60 - acétate d'éthyle 40) une huile jaune est obtenue (m = 104 mg ; R = 50 %).

### b) 6-{[3-(3,4-Bis-hydroxyméthyl-benzyloxy)-phenyl]amino}-3-éthyl-hexan-3-ol

De manière analogue à l'exemple 56(d) par traitement de 6-{[3-(3,4-Bis-carboxyméthyl-benzyloxy)-phenyl]-N-benzoyl-amino}-3-éthyl-hexan-3-ol (100 mg, 0.18 mmol) par 40 mg (1 mmol) d'hydrure double de lithium et d'aluminium et après purification sur colonne de silice, une huile incolore est obtenue (m = 75 mg ; R = 87 %).
**RMN** ^{**1**}**H** (CDCl₃): 0,86 (t, 6H, *J* = 7,5 Hz), 1,43-1,70 (m, 9H), 3,1 (bs, 1H), 3,2 (bs, 1H), 3,90 (t, 2H, *J* = 6,3 Hz), 4,31 (s, 2H), 4,70 (s, 4H), 6,14-6,28 (m, 3H), 7,04 (t, 1H, *J* = 8,1 Hz), 7,30 (s, 2H), 7,34 (s, 1H).

### EXEMPLE 58

### (4E,6E)-7-{3-[2-(3,4-Bis-hydroxyméthyl-phenyl)-éthyl]-phenyl}-3-éthyl-octa-4,6-dien-3-ol

### a) 3-(2-Méthyl-[1,3]dioxolan-2-yl)-benzaldehyde

5 g (25 mmol) de 3-bromoacétophenone sont dissous dans 40 mL de toluène, puis 15 mL d'éthylène glycol et 500 mg d'acide *para*-toluènesulfonique sont ajoutés. Le milieu réactionnel est alors porté à reflux équipé d'un montage de distillation de Dean-Stark. Après 12 h, le milieu est refroidi, puis traité par de l'eau et extrait avec du dichlorométhane. Après séchage et concentration, le résidu est purifié sur colonne de silice pour obtenir le 3-(2-Méthyl-[1,3]dioxolan-2-yl)-1-bromobenzene désiré ( R = 85% ). Ce produit (4 g, 16.4 mmol) est alors dissous dans du THF anhydre et le mélange est refroidi à -78°C. 7,2 mL (18 mmol) d'une solution 2,5 M de butyllithium sont alors additionnés goutte à goutte. Après 1 heure d'agitation à la même température, 1,9 mL (18 mmol) de diméthylformamide sont ajoutés. Le mélange est maintenu à -78°C pendant 30 minutes, puis ramené à température ambiante. Le milieu réactionnel est alors traité par une solution saturée de chlorure d'ammonium, et extrait avec du dichlorométhane. Après purification par chromatographie sur colonne de silice (acétate d'éthyle10 / heptane90) une huile incolore est obtenue ( m = 3,09 g ; R = 98 %)

### b) 4-{(E)-2-[3-(2-Méthyl-[1,3]dioxolan-2-yl)-phenyl]-vinyl}-phthalate de diméthyle

4-(Diéthoxy-phosphorylméthyl)-phthalate de diméthyle ( 2 g, 5,8 mmol) est dissous dans 30 mL de THF anhydre, puis refroidi à 0°C. 2,8 mL (5,6 mmol) d'une solution 2 M de diisopropylamidure de lithium sont alors ajoutés goutte à goutte, puis le milieu réactionnel est agité à cette température pendant 30 minutes. Ensuite, une solution de 3-(2-Méthyl-[1,3]dioxolan-2-yl)-benzaldehyde (1,02 g, 5,3 mmol) dans 10 mL de THF est additionnée par l'intermédiaire d'une canule. Le milieu est alors ramené à température ambiante, puis agité 12 heures. Après traitement par une solution saturée de chlorure d'ammonium et extraction avec de l'acétate d'éthyle, la phase organique est séchée puis les solvants sont évaporés. Après purification sur colonne de silice (acétate d'éthyle 30-heptane70), une huile incolore est obtenue ( m = 1,68 g ; R = 83% ).

### c) 4-{2-[3-(2-Méthyl-[1,3]dioxolan-2-yl)-phenyl]-éthyl}-phthalate de diméthyle

1,8 g (4,7 mmol) de 4-{(E)-2-[3-(2-Méthyl-[1,3]dioxolan-2-yl)-phenyl]-vinyl}-phthalate de diméthyle est dissous dans 50 mL d'acétate d'éthyle. La solution est dégasée pendant 30 minutes, puis Pd/C 5% (200 mg) sont ajoutés. Un ballon d'hydrogène (1 bar) est alors fixé au montage et le milieu réactionnel est agité pendant 4 heures. La perte de fluorescence lors d'analyses CCM (□ = 254 nm) indique la fin de la réaction: le milieu réactionnel est alors filtré puis concentré sous pression réduite pour donner une huile incolore (m=1,78g;R=98%).

### d) 1-{3-[2-(3,4-Bis-hydroxyméthyl-phenyl)-éthyl]-phenyl}-éthanone

4-{2-[3-(2-Méthyl-[1,3]dioxolan-2-yl)-phenyl]-éthyl}-phthalate de diméthyle (1,78 g, 4,6 mmol) est dissous dans 20 mL de THF anhydre. 530 mg d'hydrure double de lithium et d'aluminium (14 mmol) sont alors ajoutés en trois fractions égales, puis le milieu réactionnel est porté à reflux pendant 4 heures. Le milieu est alors refroidi, puis séquentiellement traité par 530 □L d'eau, 530 □L de NaOH 15%, puis 1, 6 mL d'eau. Après 1 heure d'agitation, le mélange est filtré puis concentré sous pression réduite. Le résidu obtenu est alors dissous dans 10 mL d'acétone auquels sont ajoutés 5 mL d'eau et 100 mg d'acide *para*-toluènesulfonique. Le milieu réactionnel est agité à 70°C pendant 12 h, puis versé dans 50 mL d'acétate d'éthyle. Après séparation et séchage de la phase organique, les solvants sont évaporés, puis le résidu purifié par chromatographie sur gel de silice (acétate d'éthyle 50-heptane 50) pour obtenir un semi-solide blanc ( m = 1,18 g ; R = 90%).

### e) 1-(3-{2-[3,4-Bis-(tert-butyl-diméthyl-silanyloxyméthyl)-phenyl]-éthyl}-phenyl)-éthanone

1,15 g de 1-{3-[2-(3,4-Bis-hydroxyméthyl-phenyl)-éthyl]-phenyl}-éthanone (4,04 mmol) est dissous dans 20 mL DMF et,20 mL de dichlorométhane. Le mélange est refroidi à 0°C, puis 1,7 mL de triéthylamine (12 mmol) sont ajoutés, suivis de 1,35 g de chlorure de *tert*-butyldiméthylsilane (8,9 mmol). Le milieu est agité à température ambiante pendant 12 h, puis traité par une solution saturée de chlorure d'ammonium et extrait par de l'éther éthylique. La phase organique est lavée à l'eau, puis séchée et concentrée sous pression réduite, pour donner une huile incolore ( m = 2,07 g ; R = 100 %).

### f) (2E,4E)-5-(3-{2-[3,4-Bis-(tert-butyl-diméthyl-silanyloxyméthyl)-phenyl]-éthyl}-phenyl)-hexa-2,4-dienoate d'éthyle

850 mL (3,9 mmol) de 4-diéthylphosphonocrotonate d'éthyle sont dissous dans 5 mL de THF. A ce mélange sont rajoutés 10 mL de DMPU (1,3-dimethyl-3,4,5,6-tetrahydro-2(1H)-pyrimidinone) puis, après refroidissement à 0°C, 1,85 mL (3,7 mmol) d'une solution de diisopropylamidure de lithium 2M. Le mélange est agité pendant 30 minutes à cette même température, puis une solution de 1 g (1,95 mmol) de 1-(3-{2-[3,4-Bis-(*tert*-butyl-diméthyl-silanyloxyméthyl)-phenyl]-éthyl}-phenyl)-éthanone dans 5 mL de THF est additionnée goutte à goutte. Le mélange est alors ramené à température ambiante, puis agité 48 heures. Après traitement par une solution de chlorure d'ammonium et extraction avec de l'acétate d'éthyle, la phase organique est séchée et concentrée sous pression réduite. Après chromatographie sur colonne de silice, une huile jaune clair est obtenue ( m = 1,46 g; R = 81 %).

### g) (4E,6E)-7-(3-{2-[3,4-Bis-(tert-butyl-diméthyl-silanyloxyméthyl)-phenyl]-éthyl}-phenyl)-3-éthyl-octa-4,6-dien-3-ol

500 mg de (2E,4E)-5-(3-{2-[3,4-Bis-(*tert*-butyl-diméthyl-silanyloxyméthyl)-phenyl]-éthyl}-phenyl)-hexa-2,4-dienoate d'éthyle (0,82 mmol) sont dissous dans 10 mL de THF anhydre et le mélange est refroidi à -78°C. 5,4 mL (4 mmol) d'une solution d'éthyllithium fraîchement préparée (0,75 M) sont alors ajoutés goutte à goutte, puis le mélange est ramené à 0°C. Après 1 heure d'agitation, le milieu réactionnel est traité par une solution saturée de chlorure d'ammonium et extrait avec de l'acétate d'éthyle. Après purification par chromatographie sur colonne de silice (acétate d'éthyle 5 - heptane 95), l'isomère tout trans pur est obtenu sous la forme d'une huile incolore (m = 350 mg ; R = 70 %).

### h) (4E,6E)-7-{3-[2-(3,4-Bis-hydroxyméthyl-phenyl)-éthyl]-phenyl}-3-éthyl-octa-4,6-dien-3-ol

340 mg de (4E,6E)-7-(3-{2-[3,4-Bis-(*tert*-butyl-diméthyl-silanyloxyméthyl)-phenyl]-éthyl}-phenyl)-3-éthyl-octa-4,6-dien-3-ol (0,5 mmol) sont dissous dans 10 mL de THF et 1,5 mL (1,5 mmol) d'une solution de fluorure de terabutylammonium (1 M dans le THF) sont ajoutés goutte à goutte. Après 30 minutes, 10 mL de méthanol sont additionnés et le mélange est concentré sous pression réduite. Après purification par chromatographie sur colonne de silice du résidu, une huile incolore est obtenue ( m = 163 mg ; R = 88%).
**RMN** ^{**1**}**H** (CDCl₃): 0,90 (t, 6H, *J* = 7,4 Hz), 1,61 (q, 4H, *J* = 7,5 Hz), 2,17 (s, 3H), 2,92 (bs, 2H), 2,92 (s, 4H), 4,71 (s, 4H), 5,77 (d, 1H, *J* = 15,1 Hz), 6,42 (d, 1H, *J* = 11 Hz), 6,63 (dd, 1H, J1 = 15,1 Hz, J2 = 11 Hz), 7,08-7,29 (m, 7H).

### EXEMPLE 59

### (3E,5E)-6-{3-[2-(3,4-Bis-hydroxyméthyl-phenyl)-éthyl]-phenyl}-2-méthyl-hepta-3,5-dien-2-ol

De manière analogue à l'exemple 58(h), par réaction de 410 mg (0,7 mmol) de (3E,5E)-6-{3-[2-(3,4-Bis-(*tert*-butyl-diméthyl-silanyloxyméthyl)-phenyl)-éthyl]-phenyl}-2-méthyl-hepta-3,5-dien-2-ol (préparé de manière analogue aux exemples 52(a-g)) avec 2 ml (2 mmol) d'une solution de fluorure de tetrabutylammonium (1 M dans le THF), une huile incolore est obtenue ( m = 230 mg ; R = 96 %).
**RMN** ^{**1**}**H** (CDCl₃): 1,39 (s, 6H), 2,15 (s, 3H), 2,91 (s, 4H), 2,92 (bs, 2H), 4,69 (s, 2H), 4,70 (s, 2H), 5,94 (d, 1H, *J* = 15,1 Hz), 6,36 (d, 1H, *J* = 10,9 Hz), 6,62 (dd, 1H, J1 = 15,1 Hz, J2 = 10,9 Hz), 7,06-7,28 (m, 7H).

### EXEMPLE 60

### (4E-6E)-7-{3-[2-(3,4-Bis-hydroxyméthyl-phenyl)-vinyl]-phenyl}-3-éthyl-octa-4,6-dien-3-ol

De manière analogue à l'exemple 58(h), par réaction de 320 mg (0,5 mmol) de (4E,6E)-7-(3-{2-[3,4-Bis-(*tert*-butyl-diméthyl-silanyloxyméthyl)-phenyl]-vinyl}-phenyl)-3-éthyl-octa-4,6-dien-3-ol (préparé de manière analogue aux exemples 52(a-b,d-g)) avec 1,5 ml (1,5 mmol) d'une solution 1 M de fluorure de tetrabutylammonium, une huile incolore est obtenue ( m = 184 mg ; R = 100 %).
**RMN** ^{**1**}**H** (CDCl₃): 0,91 (t, 6H, *J =* 7,4 Hz), 1,62 (q, 4H, *J* = 7,4 Hz), 2,22 (s, 3H), 4,13 (bs, 1H), 4,21 (bs, 1H), 4,72 (s, 2H), 4,75 (s, 2H), 5,82 (d, 1H, *J* = 14,9 Hz), 6,52 (d, 1H, *J* = 11 Hz), 6,65 (dd, 1H, J1 = 14,9 Hz, J2 = 11 Hz), 7,13 (s, 2H), 7,29-7,57 (m, 7H).

### EXEMPLE 61

### (3E,5E)-6-{3-[2-(3,4-Bis-hydroxyméthyl-phenyl)-vinyl]-phenyl}-2-méthyl-hepta-3,5-dien-2-ol

De manière analogue à l'exemple 58(h), par réaction de 450 mg (0,75 mmol) de (3E,SE)-6-{3-[2-(3,4-Bis-(*tert*-butyl-diméthyl-silanyloxyméthyl)-phenyl)-vinyl]-phenyl}-2-méthyl-hepta-3,5-dien-2-ol (préparé de manière analogue aux exemples 58(a-b,d-g)) avec 2 ml (2 mmol) avec une solution de fluorure de tetrabutylammonium (1 M dans le THF), une huile incolore est obtenue ( m = 270 mg ; R = 99 %).
**RMN** ^{**1**}**H** (CDCl₃): 1,40 (s, 6H), 2,21 (s, 3H), 2,35 (bs, 1H), 4,20-4,40 (bs, 2H), 4,71 (s, 2H), 4,74 (s, 2H), 5,98 (d, 1H, *J* = 15 Hz), 6,47 (d, 1H, *J* = 10,9 Hz), 6,67 (dd, 1H, J1 = 15,0 Hz, J2 = 10,9 Hz), 7,13 (s, 2H), 7,31-7,55 (m, 7H).

### EXEMPLE 62

### (4E,6E)-7-[3-(3,4-Bis-hydroxyméthyl-phenyléthynyl)-phenyl]-3-éthyl-octa-4,6-dien-3-ol

### a) 1-(3-Éthynyl-phenyl)-éthanone

4 g de 3-bromoacétophenone (20 mmol) et 4,23 mL (30 mmol) de triméthylsilylacétylène sont dissous dans 50 mL de triéthylamine. CuI (760 mg, 4 mmol) sont ajoutés, et le milieu réactionnel est dégasé pendant 10 minutes par un flux d'argon. *tetrakis*-triphenylphosphine-palladium (1,4 g, 2 mmol) est alors additionné en une fraction, et le milieu réactionnel est agité 12 heures à température ambiante. Le milieu est alors traité par de l'eau, et extrait avec du dichlorométhane. Après séchage et concentration sous pression réduite, le résidu est dissous dans 100 mL de THF, auxquels sont ajoutés 100 mL d'éthanol et 10 mL d'eau. Deux fractions de 1,4 g (24 mmol) de fluorure de potassium sont ajoutées à 9 heures d'intervalle, puis le milieu réactionnel est agité pendant 24 h, avant d'être concentré sous pression réduite. Le résidu est alors repris dans un mélange de dichlorométhane et d'une solution saturée de chlorure d'ammonium. La phase organique est séchée, puis concentrée. Après chromatographie sur colonne de silice (acétate d'éthyle 10 - heptane 90) une huile claire est obtenue (m = 2,68 g ; R = 93 %).

### b) 1-{3-[3,4-Bis-(tert-butyl-diméthyl-silanyloxyméthyl)-phenyléthynyl]-phenyl}-éthanone

A 850 mg (1,7 mmol) de 1,2-Bis-(*tert*-butyl-diméthyl-silanyloxyméthyl)-4-iodo-benzene dans 6 mL de pyrrolidine sont successivement ajoutés 100 mg (0,1 mmol) de tetrakis-triphenylphosphine-palladium, 35 mg (0,2 mmol) de Cul et 250 mg (1,7 mmol) de 1-(3-ethynyl-phenyl)-éthanone. Après 30 minutes à température ambiante, le milieu réactionnel est filtré, puis le filtrat est repris dans du dichlorométhane et lavé à l'eau. Une huile jaune est obtenue ( m = 770 mg ; R = 89%).

### c) (2E,4E)-5-{3-[3,4-Bis-(tert-butyl-diméthyl-silanyloxyméthyl)-phenyléthynyl]-phenyl}-hexa-2,4-dienoate d'éthyle

0,6 mL (2,7 mmol) de 4-diethylphosphonocrotonate d'éthyle sont dissous dans 20 mL de THF anhydre, et le mélange est refroidi à 0°C. 1,27 mL (2,54 mmol) d'une solution 2M de diisopropylamidure de lithium sont ajoutés goutté à goutte. Après 1 heure d'agitation à la même température, une solution de 680 mg (1,34 mmol) de 1-{3-[3,4-bis-(*tert*-butyl-diméthyl-silanyloxyméthyl)-phenyléthynyl]-phenyl}-éthanone dans 5 mL de THF est additionnée lentement par l'intermédiaire d'une canule. Le milieu réactionnel est ramené à température ambiante, puis agité 18 heures. Après traitement par une solution saturée de chlorure d'ammonium et extraction avec de l'acétate d'éthyle, la phase organique est séchée et concentrée sous pression réduite. Le résidu est purifié par chromatographie sur colonne de silice: une huile jaune est obtenue ( m = 480 mg ; R = 60%).

### d) (4E,6E)-7-[3-(3,4-Bis-hydroxyméthyl-phenyléthynyl)-phenyl]-3-éthyl-octa-4,6-dien-3-ol

470 mg de (2E,4E)-5-{3-[3,4-bis-(*tert*-butyl-diméthyl-silanyloxyméthyl)-phenyléthynyl]-phenyl}-hexa-2,4-dienoate d'éthyle (0,77 mmol) sont dissous dans 10 mL de THF anhydre. 0,8 mL (2,4 mmol) d'une solution 3M de bromure d'éthylmagnesium sont additionnés goutte à goutte. Après 30 minutes à température ambiante, le milieu réactionnel est traité par une solution de chlorure d'ammonium, puis extrait avec de l'acétate d'éthyle. Le résidu, obtenu après séchage et concentration de la phase organique, est alors dissous dans 10 mL de THF et 3 mL (3 mmol) d'une solution de fluorure de tetrabutylammonium (1 M dans le THF) sont additionnés. Après 15 minutes le milieu réactionnel est dilué avec 10 mL d'éthanol, puis concentré sous pression réduite. Le résidu est purifié par chromatographie sur gel de silice (acétate d'éthyle 40 - heptane 60). Une huile incolore est obtenue ( m = 55 mg ; R = 18 %).
**RMN** ^{**1**}**H** (CDCl₃): 0,93 (t, 6H, *J* = 6,7 Hz), 1,58 (q, 4H, *J* = 6,7 Hz), 2,07 (s, 3H), 2,92 (bs, 2H), 4,75 (s, 4H), 5,81 (d, 1H, *J* = 14,8 Hz), 6,51 (d, 1H, *J* = 10,9 Hz), 6,65 (dd, 1H, J1 = 14,8 Hz, J2 = 10,9 Hz), 7,26-7,62 (m, 7H).

### EXEMPLE 63 (Comparatif)

### 7-[3-(3,4-Bis-hydroxyméthyl-benzyloxy)phényl]-3-éthyl-3-octanol

### a) 5-[3-(tert-butyl-diméthyl-silanyloxy)-phenyl]-hexa-2,4-dienoate d'éthyle

4,3 g de 4-diéthylphosphonocrotonate d'éthyle sont ajoutés goutte à goutte à une solution de 19 mmol de disopropylamidure de lithium dans 150 mL de THF anhydre refroidie à 0°C. Après 1 heure d'agitation à cette température, 2 g (8 mmol) de 3-(*tert*-Butyl-diméthyl-silanyloxy)-acétophénone dissous dans 20 mL de THF sont additionnés goutte à goutte par l'intermédiaire d'une canule. Le milieu réactionnel est ramené à température ambiante et agité 15 heures. Après traitement par une solution saturée de chlorure d'ammonium et extraction avec du dichlorométhane, la phase organique est séchée et concentrée sous pression réduite. Le résidu est purifié par chromatographie sur colonne de silice. Une huile jaune est obtenue ( m = 1,39 g ; R = 50 %).

### b) 5-[3-(tert-butyl-diméthyl-silanyloxy)-phenyl]-hexanoate d'éthyle

600 mg (1,7 mmol) de 5-[3-(*tert*-butyl-diméthyl-silanyloxy)-phenyl]-hexa-2,4-dienoate d'éthyle sont dissous dans 40 mL d'acétate d'éthyle et la solution est dégasée par un flux d'argon pendant 10 minutes. 100 mg de Pd/C 5% sont alors ajoutés et le milieu réactionnel est équipé d'un ballon d'hydrogène (1 bar). Après 6 heures à température ambiante, le milieu réactionnel est filtré sur un tampon de célite, puis le filtrat est concentré sous pression réduite pour donner une huile incolore ( m = 580 mg ; R = 97% ).

### c) 3-(5-Éthyl-5-hydroxy-1-méthyl-heptyl)-phenol

580 mg (1,66 mmol) de 5-[3-(*tert*-butyl-diméthyl-silanyloxy)-phenyl]-hexanoate d'éthyle sont dissous dans 25 mL d'éther éthylique et le mélange est refroidi à 0°C. 1,9 mL d'une solution de bromure d'éthylmagnesium 3 M (5,8 mmol) sont alors additionnés goutte à goutte et le milieu est agité pendant 3 heures. Après traitement avec une solution saturée de chlorure d'ammonium et extraction avec de l'éther éthylique, la phase organique est séchée puis concentrée sous pression réduite. Le milieu réactionnel est alors dissous dans 20 mL de THF, puis 2,5 mL (2,5 mmol) d'une solution de fluorure de tetrabutylammonium (1 M dans le THF) sont ajoutés en une portion et le milieu réactionnel est agité 1 heures à température ambiante. Le milieu réactionnel est alors concentré sous pression réduite et le résidu est purifié par chromatographie sur colonne de silice. Une huile incolore. est obtenue ( m = 400 mg;R=96%).

### d) 4-[3-(5-éthyl-5-hydroxy-1-méthyl-heptyl)-phenoxyméthyl]-phthalate de diméthyle

450 mg de 3-(5-éthyl-5-hydroxy-1-méthyl-heptyl)-phenol (1,8 mmol) et 620 mg (2,16 mmol) de 4-bromométhyl-phthalate de diméthyle sont dissous dans 20 mL de 2-biatanone. Une quantité catalytique de 18-crown-6 est ajoutée, suivie de 300 mg (2,16 mmol) de carbonate de potassium. Le milieu réactionnel est porté à reflux pendant 15 h, puis refroidi et filtré. Le filtrat est concentré sous pression réduite puis le résidu est purifié par chromatographie sur colonne de silice. Une huile jaune est obtenue ( m = 220 mg ; R = 27 %).

### e) 7-[3-(3,4-Bis-hydroxyrnéthyl-benzyloxy)phényl]-3-éthyl-3-octanol

220 mg (0,48 mmol) de 4-[3-(5-éthyl-5-hydroxy-1-méthyl-heptyl)-phenoxyméthyl]-phthalate de diméthyle sont dissous dans 15 mL de THF anhydre. 42 mg (1,9 mmol) de borohydrure de lithium sont alors ajoutés, et le milieu réactionnel est porté à reflux pendant 12 heures. Après refroidissement, traitement par une solution saturée de chlorure d'ammonium et extraction avec de l'acétate d'éthyle, la phase organique est séchée et concentrée sous pression réduite. Le résidu est alors purifié par chromatographie sur colonne de silice. Une huile incolore est obtenue ( m = 132 mg ; R = 75 % ).
**RMN** ^{**1**}**H** (CDCl₃): 0,79 (t, 6H, *J* = 7,5 Hz), 1,13-1,57 (m, 13H), 1,72 (bs, 1H), 1,65 (m, 1H), 3,44 (bs, 2H), 4,69 (s, 2H), 4,70 (s, 2H), 5,06 (s, 2H), 6,77-6,79 (m, 3H), 7,19 (t, 1H, *J* = 8,5 Hz), 7,31-7,35 (m, 2H), 7,40 (s, 1H).

### EXEMPLE 64

### (4E,6E)-7-[3-(3,4-Bis-hydroxyméthyl-benzyloxy)-phenyl]-3-éthyl-octa-4,6-dien-3-ol

### a) 7-[3-(tert-butyl-dimétltyl-silanyloxy)-phenyl]-3-éthyl-octa-4,6-dien-3-ol

De manière analogue à l'exemple 62(d) par réaction de 700 mg (2 mmol) de 5-[3-(*tert*-butyl-diméthyl-silanyloxy)-phenyl]-hexa-2,4-dienoate d'éthyle (préparé à l'exemple 63(a)) avec 2,7 ml (8 mmol) d'une solution de bromure d'ethylmagnésium (3M), on obtient après purification sur colonne de silice (pentane-acétate d'éthyle 90-10) 121 mg (17%) de (Z)-7-[3-(*tert*-butyl-diméthyl-silanyloxy)-phenyl]-3-éthyl-octa-4,6-dien-3-ol et 146 mg (20%) de (E)- 7-[3-(tert-butyl-diméthyl-silanyloxy)-phenyl]-3-éthyl-octa-4,6-dien-3-ol .

### b) (4E,6E)-7-[3hydroxyphenyl]-3-éthyl-octa-4,6-dien-3-ol

De manière analogue à l'exemple 58(h) à partir de 205 mg (0,57 mmol) de (E)- 7-[3-(*tert*-butyl-diméthyl-silanyloxy)-phenyl]-3-éthyl-octa-4,6-dien-3-ol, on obtient 140 mg (100%) de produit attendu sous forme d'une huile.

### c) 4-[3-((1E,3E)-5-éthyl-5-hydroxy-1-méthyl-hepta-1,3-dienyl)-phenoxyméthyl]-phthalate de diméthyle

De manière analogue à l'exemple 63(d) par réaction de 140 mg (0,57 mmol) du produit précédent avec 196 mg (0,68 mmol) de 4-bromométhyl-phthalate de diméthyle, on obtient 154 mg (60%) de produit attendu sous forme d'une huile.

### d) (4E,6E)-7-[3-(3,4-Bis-hydroxyméthyl-benzyloxy)-phenyl]-3-éthyl-octa-4,6-dien-3-ol

De manière analogue à l'exemple 52(e) par réaction de 150 mg ( 0,34 mmol) de 4-[3-((1E,3E)-5-éthyl-5-hydroxy-1-méthyl-hepta-1,3-dienyl)-phenoxyméthyl]-phthalate de diméthyle avec 30 mg (1,35 mmol) de borohydrure de lithium, une huile incolore est obtenue ( m = 56 mg ; R = 42 %).
**RMN** ^{**1**}**H** (CDCl₃): 0,89 (t, 6H, *J* = 7,5 Hz), 1,59 (q, 4H, *J =* 7,5 Hz), 2,15 (s, 3H), 3,57 (bs, 2H), 4,66 (s, 2H), 4,67 (s, 2H), 5,03 (s, 2H), 5,76 (d, 1 H, *J* = 14,9 Hz), 6,46 (d, 1H, *J* = 10,9 Hz), 6,62 (dd, 1H, *J*_{*1*} = 14,9 Hz, *J*_{*2*} = 10,9 Hz), 6,82 (dd, 1H, *J*_{*1*} = 1,9 Hz, *J*_{*2*} = 7,6 Hz), 7,05 (m, 2H), 7,22 (t, 1H, *J* = 8,2 Hz), 7,30-7,38 (m, 3H).

### EXEMPLE 65

### (4E,6Z)-7-[3-(3,4-Bis-hydroxyméthyl-benzyloxy)-phenyl]-3-éthyl-octa-4,6-dien-3-ol

### a) (4E,6Z)-7-[3hydroxyphenyl]-3-éthyl-octa-4,6-dien-3-ol

De manière analogue à l'exemple 58(h) à partir de 202 mg (0,56 mmol) de (Z)- 7-[3-(*tert*-butyl-diméthyl-silanyloxy)-phenyl]-3-éthyl-octa-4,6-dien-3-ol (obtenu à l'exemple 65(a), on recueille 138 mg (1 00%) de produit attendu sous forme d'une huile.

### b) 4-[3-((1Z,3E)-5-éthyl-5-hydroxy-1-méthyl-hepta-1,3-dienyl)-phenoxyméthyl]-phthalate de diméthyle

De manière analogue à l'exemple 63(d) par réaction de 138 mg (0,56 mmol) du produit précédent avec 196 mg (0,68 mmol) de 4-bromométhyl-phthalate de diméthyle, on obtient 152 mg (60%) de produit attendu sous forme d'une huile.

### c) (4E,6Z)-7-[3-(3,4-Bis-hydroxyméthyl-benzyloxy)-phenyl]-3-éthyl-octa-4,6-dien-3-ol

De manière analogue à l'exemple 52(e) par réaction de 106 mg (0,23 mmol) de 4-[3-((1Z,3E)-5-éthyl-5-hydroxy-1-méthyl-hepta-1,3-dienyl)-phenoxyméthyl]-phthalate de diméthyle avec 20 mg (0,94 mmol) de borohydrure de lithium, une huile incolore est obtenue ( m = 50 mg ; R = 55 %).
**RMN** ^{**1**}**H** (CDCl₃): 0,80 (t, 6H, *J* = 7,5 Hz), 1,49 (q, 4H, *J* = 7,5 Hz), 2,10 (s, 3H), 3,21 (bs, 1H), 4,73 (s, 4H), 5,10 (s, 2H), 5,61 (d, 1H, *J* = 14,9 Hz), 6,12 (d, 1H, *J* = 10,9 Hz), 6,24 (dd, 1H, *J*_{*1*} = 14,9 Hz, *J*_{*2*} = 10,9 Hz), 6,83-6,98 (m, 3H), 7,18-7,42 (m, 4H).

### EXEMPLE 66

### 7-[4-(3,4-Bis-hydroxyméthyl-benzyloxy)phényl]-3-éthyl-3-octanol

De manière analogue à l'exemple 52(e) par réaction de 607 mg (1,33 mmol) de 4-[4-(5-éthyl-5-hydroxy-1-méthyl-heptyl)-phenoxyméthyl]-phthalate de diméthyle (préparé de manière analogue aux exemples 63(a-d)) avec 116 mg (5,32 mmol) de borohydrure de lithium, une huile incolore est obtenue ( m = 421 mg ; R = 79 % ).
**RMN** ^{**1**}**H** (CDCl₃): 0,73 (t, 6H, *J* = 7,5 Hz), 1,09-1,47 (m, 13H), 2,56 (m, 1H), 3,2 (bs, 1H), 4,62 (s, 4H), 4,94 (s, 2H), 6,80 (d, 2H, *J* = 8,6 Hz), 7;01 (d, 2H, *J* = 8,6 Hz), 7,24-7,30 (m, 3H).

### EXEMPLE 67

### (4E,6E)-7-[3-(3,4-Bis-hydroxyméthyl-benzyloxy)-phenyl]-3-éthyl-nona-4,6-dien-3-ol

De manière analogue à l'exemple 52(e) par réaction de 122 mg (0,26 mmol) de 4-[3-((1E,3E)-5-éthyl-5-hydroxy-1-éthyl-hepta-1,3-dienyl)-phenoxyméthyl]-phthalate de diméthyle (préparé de manière analogue aux exemples 64(a-c)) avec 17 mg (0,78 mmol) de borohydrure de lithium, une huile incolore est obtenue ( m = 91 mg ; R = 85 %).
**RMN** ^{**1**}**H** (CDCl₃): 0,81 (t, 6H, *J* = 7,6 Hz), 0,96 (t, 3H, *J =* 7,5 Hz), 1,52 (q, 4H, *J* = 7,6 Hz), 2,56 (q, 2H, *J* = 7,5 Hz), 3,21 (bs, 2H), 4,57 (s, 2H), 4,58 (s, 2H), 4,95 (s, 2H), 5,67 (d, 1H, *J* = 15,1 Hz), 6,25 (d, 1H, *J* = 11 Hz), 6,53 (dd, 1H, *J*_{*1*} = 15,1 Hz, *J*_{*2*} = 11 Hz), 6,75 (dd, 1H, *J*_{*1*} = 1,7 Hz, *J*_{*2*} *=* 8,0 Hz), 6,94-6,96 (m, 2H), 7,14 (t, 1H, *J* = 8,1 Hz), 7,26-7,30 (m, 3H).

### EXEMPLE 68

### (4E,6Z)-7-[3-(3,4-Bis-hydroxyméthyl-benzyloxy)-phenyl]-3-éthyl-nona-4,6-dien-3-ol

De manière analogue à l'exemple 52(e) par réaction de 72 mg (0,15 mmol) de 4-[3-((1Z,3E)-5-éthyl-5-hydroxy-1-éthyl-hepta-1,3-dienyl)-phenoxyméthyl]-phthalate de diméthyle (préparé de manière analogue aux exemples 65(a-b)) avec 10 mg (0,46 mmol) de borohydrure de lithium, une huile incolore est obtenue ( m = 62 mg ; R = 100 % ).
**RMN** ^{**1**}**H** (CDCl₃): 0,80 (t, 6H, *J* = 7,6 Hz), 0,99 (t, 3H, *J* = 7,5 Hz), 1,48 (q, 4H, *J* = 7,6 Hz), 2,41 (q, 2H, *J* = 7,5 Hz), 3,71 (bs, 2H), 4,64 (s, 4H), 5,06 (s, 2H), 5,63 (d, 1H, *J* = 14,7 Hz), 6,08 (d, 1H, *J* = 10,8 Hz), 6,19 (dd, 1H, *J*_{*1*} = 14,7 Hz, *J*_{*2*} = 10,8 Hz), 6,78-6,88 (m, 3H), 7,23 (t, 1 H, *J* = 8,2 Hz), 7,29-7,37 (m, 3H).

### EXEMPLE 69

### (E)-6-[3-(3,4-Bis-hydroxyméthyl-benzyloxy)-phenyl]-2-méthyl-hept-3-en-2-ol

### a) 3-[3-(tert-butyl-diméthyl-silanyloxy)-phenyl]-butyrate d'éthyle.

3 g (12 mmol) de 3-(*tert*-butyl-diméthyl-silanyloxy)-acétophenone dans 20 mL sont additionnés goutte à goutte à une solution à 0°C de 5 g (26,4 mmol) de diéthylphosphonoacétate d'éthyle et de 24 mmol de diisopropylamidure de lithium dans 100 mL de THF. Le milieu réactionnel est agité 15 heures à température ambiante, puis traité par une solution de chlorure d'ammonium et extrait avec de l'acétate d'éthyle. La phase organique est séchée puis concentrée sous pression réduite. Le résidu obtenu est alors dissous dans 100 mL d'acétate d'éthyle. Le mélange est dégasé par un flux d'argon, puis 300 mg de Pd/C 5% sont ajoutés. Le montage est alors équipé d'un ballon d'hydrogène (1 bar), et le milieu réactionnel est agité pendant 4 heures. Après filtration et concentration, le résidu est purifié par chromatographie sur colonne de silice. Une huile incolore est obtenue (m = 3,15 g ; R = 82 %).

### b) (E)-5-[3-(tert-Butyl-diméthyl-silanyloxy)-phenyl]-hex-2-enoate de méthyle

3,15 g (9,8 mmol) d'éthyl 3-[3-(*tert*-butyl-diméthyl-silanyloxy)-phenyl]-butyrate sont dissous dans 150 mL d'éther éthylique anhydre. Le mélange est refroidi à 0°C, puis de l'hydrure double de lithium et d'aluminium (1,12 g, 29,4 mmol) est additionné en 4 portions égales. Le milieu réactionnel est porté à reflux pendant 4 h, puis ramené à température ambiante. Après traitement par 1,12 mL d'eau, 1,12 mL d'une solution de NaOH 15% puis 3,4 mL d'eau, le milieu est agité 1 h, puis filtré. Le filtrat est concentré sous pression réduite. Le résidu obtenu est alors dissous dans 50 mL de dichlorométhane.

Dans- un ballon de 500 mL, 2,28 g de chlorure d'oxalyle (18 mmol) sont dissous dans 100 mL de dichlorométhane et le mélange est refroidi à -78°C. 2,6 mL (36 mmol) de DMSO dans 10 mL de dichlorométhane sont alors ajoutés, puis le mélange est agité pendant 15 minutes à -78°C. La solution de l'alcool obtenu précédemment est alors ajoutée lentement par l'intermédiaire d'une canule, puis le milieu réactionnel est encore agité pendant 30 minutes. 10 mL (72 mmol) de triéthylamine sont alors ajoutés et le milieu réactionnel est ramené à température ambiante. Après 1 heure d'agitation, le milieu est traité par une solution saturée de chlorure d'ammonium, et extrait avec de l'éther éthylique. La phase organique est rincée à l'eau, puis séchée et concentrée sous pression réduite. L'aldéhyde obtenu est immédiatement utilisé tel quel.

Le résidu est dissous dans 120 mL de THF anhydre, puis 6,4 g (19 mmol) de (triphenyl-phosphanylidene)-acétate de méthyle sont ajoutés. Le milieu réactionnel est agité pendant 12 heures à reflux, puis est traité avec une solution saturée de chlorure d'ammonium et extrait avec de l'acétate d'éthyle. Après purification par chromatographie sur colonne de silice, une huile jaune est obtenue ( m = 2,67 g ; R = 82 %).

### c) 3-((E)-5-Hydroxy-1,5-diméthyl-hex-3-enyl)-phenol

800 mg (2,4 mmol) de (E)-5-[3-(tert-butyl-diméthyl-silanyloxy)-phenyl]-hex-2-enoate de méthyle sont dissous dans 50 mL de THF anhydre. Le mélange est refroidi à 0°C, puis 4,5 mL (7,2 mmol) d'une solution 1,6 M de méthyllithium sont additionnés goutte à goutte. Le milieu réactionnel est agité 2 heures à 0°C, puis est traité par une solution saturée de chlorure d'ammonium et extrait avec de l'acétate d'éthyle. Après séchage et concentration sous pression réduite de la phase organique, le résidu est purifié par chromatographie sur colonne de silice. L'alcool obtenu (600 mg, 1,8 mmol) est alors dissous dans 10 mL THF, et 2 mL (2 mmol) d'une solution de fluorure de tetrabutylammonium (1 M dans le THF) sont additionnés. Après 15 min, le milieu réactionnel est concentré sous pression réduite est le résidu est purifié par chromatographie sur colonne de silice. Un huile jaune est obtenue ( m = 358 mg ; R = 68 % ).

### d) (E)-6-[3-(3,4-Bis-hydroxyméthyl-benzyloxy)-phenyl]-2-méthyl-hept-3-en-2-ol

De manière analogue à l'exemple 52(e), par réaction de 517 mg (1,2 mmol) de 4-[3-((E)-5-hydroxy-1,5-diméthyl-hex-3-enyl)-phenoxyméthyl]-phthalate de diméthyle (préparé par réaction du produit précédent avec le 4-bromométhyl-phthalate de diméthyle, de manière analogue à l'exemple 63(d)) avec 80 mg (3,6 mmol) de borohydrure de lithium, une huile incolore est obtenue ( m = 430 mg ; R =, 97% ).
**RMN** ^{**1**}**H** (CDCl₃): 1,18 (s, 6H), 1,22 (d, 3H, *J* = 3,7 Hz), 2,19-2,25 (m, 2H), 2,69 (m, 1H), 4,55 (s, 2H), 4,56 (s,2H), 4,97 (s, 2H), 5,43-5,46 (m, 2H), 6,71-6,77 (m, 3H), 7,14 (t, 1H, *J* = 7,8 Hz), 7,28-7,34 (m, 3H).

### EXEMPLE 70

### (E)-7-[3-(3,4-Bis-hydroxyméthyl-benzyloxy)-phenyl]-3-éthyl-oct-4-en-3-ol

De manière analogue à l'exemple 52(e), par traitement de 63 mg (0,14 mmol) de 4-[3-((E)-5-éthyl-5-hydroxy-1-méthyl-hept-3-enyl)-phenoxyméthyl]-phthalate de diméthyle (préparé de manière analogue à l'exemple 69) avec 10 mg (0,4 mmol) de borohydrure de lithium, une huile incolore est obtenue ( m = 54 mg ; R =, 97% ).
**RMN** ^{**1**}**H** (CDCl₃): 0,70 (t, 3H, *J* = 7,5 Hz), 0,73 (t, 3H, *J* = 7,5 Hz), 1,25 (d, 3H, *J =* 3,7 Hz), 1,42 (m, 4H), 2,29 (t, 2H, *J* = 7 Hz), 2,74 (m, 1H), 3,8 (bs, 2H), 4,65 (s, 2H), 4,66 (s, 2H), 5,03 (s, 2H), 5,23-5,47 (m, 2H), 6,74-6,78 (m, 3H), 7,17 (t, 1H, *J* = 7,8 Hz), 7,30-7,38 (m, 3H).

### EXEMPLE 71

### (E)-7-[3-(3,4-Bis-hydroxyméthyl-benzyloxy)-phenyl]-3-éthyl-oct-6-en-3-ol

### a) 5-(3-hydroxy-phenyl)-hex-4-enoate de méthyle

3,54 g de bromure de (3-carboxypropyl)-triphenylphosphonium (8,25 mmol) sont additionnés à une solution d'hydrure de sodium (400 mg, 16,5 mmol) dans 15 mL de DMSO. Après 20 min, 1 g (5,5 mmol) de 1-(3-Méthoxyméthoxy-phenyl)-éthanone dans 5 mL de DMSO sont additionnés par l'intermédiaire d'une canule, puis le milieu réactionnel est agité 15 heures à température ambiante. Après traitement par 50 mL d'eau et 50 mL de toluène, la phase aqueuse est séparée puis acidifiée avec une solution 2M de HCI, et enfin extraite avec de l'acétate d'éthyle. La phase organique est alors séchée puis concentrée sous pression réduite. Le résidu obtenu est alors dissous dans 50 mL l'éthanol, puis le mélange est porté à reflux après addition de 1 mL d'acide sulfurique. Après 2 heures d'agitation, le milieu réactionnel est versé dans un mélange eau/dichlorométhane, et les phases sont séparées. La phase organique est séchée et concentrée sous pression réduite, et le résidu est purifié par chromatographie sur colonne de silice. Une huile incolore est obtenue ( m = 260 mg ; R = 22%).

### b) 3-éthyl-7-(3-hydroxy-phenyl)-oct-6-en-3-ol

De manière analogue à l'exemple 64(a), à partir de 443 mg (2 mmol) du produit précédent , on obtient 237 mg (48%) de (Z)-3-éthyl-7-(3-hydroxy-phenyl)-oct-6-en-3-ol et 112 mg (23%) de (E)-3-éthyl-7-(3-hydroxy-phenyl)-oct-6-en-3-ol.

### c) 4-[3-((E)-5-éthyl-5-hydroxy-1-méthyl-hept-1-enyl)-phenoxyméthyl]-phthalate de diméthyle

De manière analogue à l'exemple 63(d), par réaction de 112 mg (0,39 mmol) de (E)-3-éthyl-7-(3-hydroxy-phenyl)-oct-6-en-3-ol avec 145 mg (0,51 mmol) de 4-bromométhyl-phthalate de diméthyle, on obtient 131 mg (74%) de produit attendu sous forme d'une huile.

### d) (E)-7-[3-(3,4-Bis-hydroxyméthyl-benzyloxy)-phenyl]-3-éthyl-oct-6-en-3-ol

De manière analogue à l'exemple 52(e), par réaction de 131 mg (0,29 mmol) de 4-[3-((E)-5-éthyl-5-hydroxy-1-méthyl-hept-1-enyl)-phenoxyméthyl]-phthalate de diméthyle avec 25 mg (1,15 mmol) de borohydrure de lithium, une huile incolore est obtenue ( m = 89 mg ; R = 78%).
**RMN** ^{**1**}**H** (CDCl₃): 0,88 (t, 6H, *J* = 7,5 Hz), 1,45-1,57 (m, 4H), 1,98 (s, 3H), 2,03-2,20 (m, 2H), 3,78 (bs, 2H), 4,63 (s, 2H), 4,64 (s, 2H), 5,02 (s, 2H), 5,76 (t, 1H, 7,1 Hz), 6,79 (m, 1H), 6,95-6,98 (m, 2H), 7,15-7,36 (m, 4H).

### EXEMPLE 72

### (Z)-7-[3-(3,4-Bis-hydroxyméthyl-benzyloxy)-phenyl]-3-éthyl-oct-6-en-3-ol

### a) 4-[3-((Z)-5-éthyl-5-hydroxy-1-méthyl-hept-1-enyl)-phenoxyméthyl]-phthalate de diméthyle

De manière analogue à l'exemple 63(d), par réaction de 234 mg (0,95 mmol) de (Z)-3-éthyl-7-(3-hydroxy-phenyl)-oct-6-en-3-ol avec 356 mg (1,24 mmol) de 4-bromométhyl-phthalate de diméthyle, on obtient 398 mg (92%) de produit attendu sous forme d'une huile.

### b) (Z)-7-[3-(3,4-Bis-hydroxyméthyl-benzyloxy)-phenyl]-3-éthyl-oct-6-en-3-ol

De manière analogue à l'exemple 52(e), par réaction de 431 mg (0,95 mmol) de 4-[3-((Z)-5-éthyl-5-hydroxy-1-méthyl-hept-1-enyl)-phenoxyméthyl]-phthalate de diméthyle avec 83 mg (3,8 mmol) de borohydrure de lithium, une huile incolore est obtenue ( m = 286 mg ; R = 76% ).
**RMN** ^{**1**}**H** (CDCl₃): 0,74 (t, 6H, *J* = 7,5 Hz), 1,22-1,42 (m, 6H), 1,84-1,94 (m, 2H), 1,99 (s, 3H), 4 (bs, 2H), 4,59 (s, 2H), 4,60 (s, 2H), 5,01 (s, 2H), 5,41 (t, 1H, 7,1 Hz), 6,75-6,84 (m, 3H), 7,17-7,35 (m, 4H).

### EXEMPLE 73

### (E)-8-[3-(3,4-Bis-hydroxyméthyl-benzyloxy)-phenyl]-2-méthyl-non-7-en-2-ol

De manière analogue à l'exemple 52(e), par réaction de 204 mg (0,46 mmol) de 4-[3-((E)-7-hydroxy-1,7-diméthyl-oct-1-enyl)-phenoxyméthyl]-phthalate de diméthyle (préparé de manière analogue aux exemples 71(a-c)) avec 30 mg (1,4 mmol) de borohydrure de lithium, une huile incolore est obtenue ( m = 146 mg ; R = 82% ).
**RMN** ^{**1**}**H** (CDCl₃): 1,18 (s, 6H), 1,18-1,51 (m, 6H), 1,99 (s, 3H), 2,16-2,22 (m, 2H), 3,7 (bs, 2H), 4,61 (s, 2H), 4,62 (s, 2H), 5,01 (s, 2H), 5,76 (t, 1H), 6,77-6,81 (m, 1H), 6,96-6,99 (m, 2H) 7,15-7,35 (m, 4H).

### EXEMPLE 74

### (Z)-8-[3-(3,4-Bis-hydroxyméthyl-benzyloxy)-phenyl]-2-méthyl-non-7-en-2-ol

De manière analogue à l'exemple 52(e), par réaction de 790 mg (1,8 mmol) de 4-[3-((Z)-7-hydroxy-1,7-diméthyl-oct-1-enyl)-phenoxyméthyl]-phthalate de diméthyle (préparé de manière analogue à l'exemple 72(a)) avec 118 mg (5,4 mmol) de borohydrure de lithium, une huile incolore est obtenue ( m = 621 mg ; R = 86% ).
**RMN** ^{**1**}**H** (CDCl₃): 1,11 (s, 6H), 1,17-1,36 (m, 6H), 1,98 (s, 3H), 1,90-1,99 (m, 2H), 4,56 (s, 2H), 4,57 (s, 2H), 4,98 (s, 2H), 5,41 (t, 1H), 6,75-6,82 (m, 3H), 7,17-7,35 (m, 4H).

### EXEMPLE 75

### (E)-9-[3-(3,4-Bis-hydroxyméthyl-benzyloxy)-phenyl]-3-éthyl-dec-8-en-3-ol

De manière analogue à l'exemple 52(e) par réaction de 397 mg (0,85 mmol) de 4-[3-((E)-7-éthyl-7-hydroxy-1-méthyl-non-1-enyl)-phenoxyméthyl]-phthalate de diméthyle (préparé de manière analogue aux exemples 71(a-c)) avec 55 mg (2,5 mmol) de borohydrure de lithium, une huile incolore est obtenue ( m = 323 mg ; R = 92% ).
**RMN** ^{**1**}**H** (CDCl₃): 0,78 (t, 6H, *J* = 7,5 Hz), 1,20-1,42 (m, 10H), 1,99 (s, 3H), 1,91-1,99 (m, 2H), 4,38 (bs, 2H), 4,57 (s, 2H), 4,58 (s, 2H), 4,99 (s, 2H), 5,42 (t, 1H, *J* = 6,9 Hz), 6,76-6,82 (m, 3H), 7,17-7,35 (m, 4H).

### EXEMPLE 76

### (Z)-9-[3-(3,4-Bis-hydroxyméthyl-benzyloxy)-phenyl]-3-éthyl-dec-8-en-3-ol

De manière analogue à l'exemple 52(e) par réaction de 1,21 g (2,58 mmol) de 4-[3-((Z)-7-éthyl-7-hydroxy-1-méthyl-non-1-enyl)-phenoxyméthyl]-phthalate de diméthyle (préparé de manière analogue aux exemples 72(a)) avec 170 mg (7,76 mmol) de borohydrure de lithium, une huile incolore est obtenue ( m = 865 mg ; R = 81% ).
**RMN** ^{**1**}**H** (CDCl₃) 0,84 (t, 6H, *J* = 7,5 Hz), 1,30-1,48 (m, 10H), 1,99 (s, 3H), 2,16-2,23 (m, 2H), 4,1 (bs, 2H), 4,60 (s, 2H), 4,61 (s, 2H), 5,00 (s, 2H), 5,76 (t, 1H, *J* = 6,2 Hz), 6,76-6,82 (m, 2H), 6,95-6,99 (m, 1H), 7,15-7,35 (m, 4H).

### EXEMPLE 77

### 8-[3-(3,4-Bis-hydroxyméthyl-benzyloxy)phényl]-2-méthyl-2-nonanol

### a) 3-(7-Hydroxy-1,7-diméthyl-octyl)-phenol

125 mg (0,32 mmol)e (Z)-8-[3-(3,4-bis-hydroxyméthyl-benzyloxy)-phenyl]-2-méthyl-non-7-en-2-ol (obtenu à l'exemple 74) sont dissous dans 25 mL de méthanol. NaNO₂ (620 mg, 9 mmol) est alors ajouté à la solution, puis 50 mg de Pd/C 5%. Le montage est alors équipé d'un ballon d'hydrogène (1 bar) et le milieu réactionnel est agité pendant 5 heures. Le milieu réactionnel est alors filtré, puis le filtrat est additionné à 50 mL d'eau puis extrait avec du dichlorométhane. La phase organique est alors séchée et concentrée sous pression réduite. Le résidu est purifié par chromatographie sur colonne de silice (acétate d'éthyle 20- heptane 80) puis acétate d'éthyle 50- heptane 50). Une huile incolore est obtenue ( m = 50 mg ; R = 95 % ).

### b) 8-[3-(3,4-Bis-hydroxyméthyl-benzyloxy)phényl]-2-méthyl-2-nonanol

De manière analogue à l'exemple 52(e), par réaction de 97 mg (0,21 mmol) de 4-[3-(7-hydroxy-1,7-diméthyl-octyl)-phenoxyméthyl]-phthalate de diméthyle (préparé à partir du produit précédent de manière analogue à l'exemple 63(d)) avec 14 mg (0,63 mmol) de borohydrure de lithium, une huile incolore est obtenue ( m = 83 mg ; R = 99 % ).
**RMN** ^{**1**}**H** (CDCl₃): 1,09 (s, 6H), 1,10-1,18 (m, 9H), 1,26-1,31 (m, 2H), 1,40-1,48 (m, 2H), 2,51-2,58 (m, 1H), 3,0 (bs, 2H), 4,60 (s, 2H), 4,61 (s, 2H), 4,96 (s, 2H), 6,68-6,73 (m, 3H), 7,08-7,32 (m, 4H).

### EXEMPLE 78

### 9-[3-(3,4-Bis-hydroxyméthyl-benzyloxy)-phenyl]-3-éthyl-decan-3-ol

De manière analogue à l'exemple 77 à partir de (Z)-9-[3-(3,4-Bis-hydroxyméthyl-benzyloxy)-phenyl]-3-éthyl-dec-8-en-3-ol (préparé à l'exemple 76), on obtient le 9-[3-(3,4-Bis-hydroxyméthyl-benzyloxy)-phenyl]-3-éthyl-decan-3-ol sous forme d'une huile.
**RMN** ^{**1**}**H** (CDCl₃): 0,74 (t, 6H, *J* = 7,6 Hz), 1,12-1,38 (m, 15H), 1,44-1,47 (m, 2H), 2,50-2,60 (m, 1H), 4,70 (bs, 2H), 4,56 (s, 2H), 4,57 (s, 2H), 4,93 (s, 2H), 6,66-6,72 (m, 3H), 7,07-7,29 (m, 4H).

### EXEMPLE 79

### (E)-7-[3-(3,4-Bis-hydroxyméthyl-benzyloxy)-phenyl]-3-éthyl-oct-6-en-4-yn-3-ol

### a) 1-Méthoxyméthoxy-3-((E)-1-méthyl-but-1-en-3-ynyl)-benzene

2,47 g (9,9 mmol) de (3-triméthylsilanyl-prop-2-ynyl)-phosphonate de didiéthyle sont dissous dans 20 mL de THF anhydre, puis la solution est refroidie à -78°C. Une solution de 1,94 g (10,6 mmol) de *bis*-triméthylsilylamidure de lithium dans 10 mL de THF est additionnée goutte à goutte. Après 30 minutes d'agitation à -78°C, 1,2 g (6,6 mmol) de 3-méthoxméthoxyacétophenone dans 10 mL de THF sont additionnés goutte à goutte par l'intermédiaire d'une canule. Le milieu réactionnel est agité pendant 30 minutes à -78°C, puis est ramené à température ambiante et agité 24 heures. Le milieu réactionnel est alors traité avec de l'eau, puis extrait avec de l'acétate d'éthyle. La phase organique est séchée puis concentrée sous pression réduite, puis le résidu est filtré sur un tampon de silice. Le, résidu obtenu est alors dissous dans 25 mL de THF et 8 mL (8 mmol) d'une solution de fluorure de tetrabutylammonium (1 M dans le THF) sont ajoutés. Après 15 minutes à température ambiante, le milieu réactionnel est concentré et le résidu purifié par chromatographie sur colonne de silice (acétate d'éthyle 5 - Heptane 95). Une huile jaune est obtenue ( m = 1,11 g ; R = 83 % ).

### b) (E)-5-(3-Méthoxyméthoxy-phenyl)-hex-4-en-2-ynoate d'éthyle.

764 mg (3,78 mmol) de 1-méthoxyméthoxy-3-((E)-1-méthyl-but-1-en-3-ynyl)-benzene sont dissous dans 20 mL de THF anhydre, et la solution est refroidie à -78°C. 1,8 mL (4,5 mmol) d'une solution 2,5 M de butyllithium sont alors ajoutés goutte à goutte, et le milieu réactionnel est agité pendant 30 minutes à cette température. 0,43 mL (4,5 mmol) de chloroformate d'éthyle sont alors additionnés goutte à goutte, puis le milieu réactionnel est ramené à température ambiante et agité pendant 2 heures. Après traitement par une solution saturée de chlorure d'ammonium et extraction avec de l'acétate d'éthyle, la phase organique est séchée puis concentrée sous pression réduite. Après purification par chromatographie sur colonne de silice (acétate d'éthyle 10 - heptane 90) une huile jaune est obtenue ( m = 836 mg ; R = 81 %).

### c) (E)-5-(3-hydroxy-phenyl)-hex-4-en-2-ynoate d'éthyle

800 mg (2,9 mmol) de (E)-5-(3-méthoxyméthoxy-phenyl)-hex-4-en-2-ynoate d'éthyle sont dissous dans 50 mL d'éthanol, puis 1 mL d'acide sulfurique est ajouté à la solution. Le milieu réactionnel est agité 14 h, traité avec de l'eau et extrait avec du dichlorométhane. La phase organique est séchée, concentrée sous pression réduite et le résidu est purifié par chromatographie (acétate d'éthyle 10 - heptane 90) puis (acétate d'éthyle 20 - heptane 80). Une huile jaune est obtenue ( m = 676 mg ; R = 100% ).

### d) (E)-7-[3-(3,4-Bis-hydroxyméthyl-benzyloxy)-phenyl]-3-éthyl-oct-6-en-4-yn-3-ol

De manière analogue à l'exemple 52(e), par réaction de 390 mg (0,87 mmol) de 4-[3-((E)-5-éthyl-5-hydroxy-1-méthyl-hept-1-en-3-ynyl)-phenoxyméthyl]-phthalate de diméthyle (préparé à partir du produit précédent de manière analogue à l'exemple 63(d)) avec 56 mg (2,6 mmol) de borohydrure de lithium, une huile incolore est obtenue ( m = 248 mg ; R = 72 % ).
**RMN** ^{**1**}**H** (CDCl₃): 1,09 (t, 6H, *J* = 7,6 Hz), 1,70-1,79 (m, 4H), 2,27 (d, 3H, *J* = 1 Hz), 3,14 (bs, 2H), 4,72 (s, 2H), 4,73 (s, 2H), 5,05 (s, 2H), 5,89 (d, 1H, *J* = 1 Hz), 6,86-6,91 (m, 1H), 7,01-7,05 (m, 2H), 7,21-7,41 (m, 4H).

### EXEMPLE 80

### (3E,5E)-7-[3-(3,4-Bis-hydroxyméthyl-benzyloxy)-phenyl]-2,7-diméthyl-octa-3,5-dien-2-ol

### a) 2-[3-(tert-butyl-diméthyl-silanyloxy)-phenyl]-2-méthyl-propionate de méthyle

2 g (7,1 mmol) de [3-(*tert*-butyl-diméthyl-silanyloxy)-phenyl]-acétate de méthyle sont ajoutés à une solution de diisopropylamidure de lithium (20,6 mmol) dans 100 mL de THF à 0°C, puis le milieu est agité pendant 30 minutes. 2,2 mL (35,5 mmol) d'iodure de méthyle sont alors additionnés goutte à goutte et le milieu réactionnel est ramené à température ambiante et agité pendant 18 heures. Le milieu réactionnel est traité avec une solution saturée de chlorure d'ammonium puis extrait avec de l'éther éthylique. Le résidu obtenu est soumis aux mêmes conditions décrites ci-dessus. Après 12 h, le milieu réactionnel est traité avec une solution saturée de chlorure d'ammonium puis extrait avec de l'éther éthylique. La phase organique est séchée puis concentrée sous pression réduite. Le résidu obtenu est alors purifié par chromatographie sur colonne de silice, pour obtenir une huile incolore ( m = 1,526 g ; R = 70 %).

### b) 2-[3-(tert-Butyl-diméthyl-silanyloxy)-phenyl]-2-méthyl-propionaldehyde

1,53 g (4,9 mmol) de 2-[3-(*tert*-butyl-diméthyl-silanyloxy)-phenyl]-2-méthyl-propionate de méthyle sont dissous dans 100 mL d'éther éthylique anhydre. Le mélange est refroidi à 0°C, puis de l'hydrure double de lithium et d'aluminium (760 mg, 20 mmol) est additionné en 2 portions égales. Le milieu réactionnel est porté à reflux pendant 4 h, puis ramené à température ambiante. Après traitement par 0,76 mL d'eau, 0,76 mL d'une solution de NaOH 15% puis 2,3 mL d'eau, le milieu est agité 1 h, puis filtré. Le filtrat est concentré sous pression réduite. Le résidu obtenu est alors dissous dans 20 mL de dichlorométhane.

Dans un ballon de 100 mL, 0,95 g de chlorure d'oxalyle (7,5 mmol) sont dissous dans 20 mL de dichlorométhane et le mélange est refroidi à -78°C. 1,06 mL (15 mmol) de DMSO dans 5 mL de dichlorométhane sont alors ajoutés, puis le mélange est agité pendant 15 minutes à -78°C. La solution de l'alcool obtenu précédemment est alors ajoutée lentement par l'intermédiaire d'une canule, puis le milieu réactionnel est encore agité pendant 30 minutes. 4,2 mL (30 mmol) de triéthylamine sont alors ajoutés et le milieu réactionnel est ramené à température ambiante. Après 1 heure d'agitation, le milieu est traité par une solution saturée de chlorure d'ammonium, et extrait avec de l'éther éthylique. La phase organique est rincée à l'eau, puis séchée et concentrée sous pression réduite. Le résidu est purifié par chromatographie sur colonne de silice ( acétate d'éthyle 5 - heptane 95 ). Une huile incolore est obtenue ( m = 1,08 g ; R = 75 % ).

### c) (2E,4E)-6-[3-(tert-butyl-diméthyl-silanyloxy)-phenyl]-6-méthyl-hepta-2,4-dienoate d'éthyle

1,7 mL (7,5 mmol) de 4-diéthylphosphonocrotonate d'éthyle sont dissous dans 50 mL de THF, puis le mélange est refroidi à 0°C. 3,4 mL (6,8 mmol) d'une solution 2M de diisopropylamidure de lithium sont additionnés goutte à goutte, et le milieu réactionnel est agité pendant 30 minutes Alors 1 g (3,4 mmol) de 2-[3-(*tert*-butyl-diméthyl-silanyloxy)-phenyl]-2-méthyl-propionaldehyde dans 10 mL de THF est additionné lentement par l'intermédiaire d'une canule. Le milieu réactionnel est ramené à température ambiante, puis agité pendant 4 heures. Après traitement par une solution saturée de chlorure d'ammonium, la phase organique est séchée et concentrée sous pression réduite. Le résidu est filtré sur un tampon de silice. Une huile jaune est obtenue ( m = 1,26 g ; R = 99 % ).

### c) (2E,4E)-6-[3-hydroxyphenyl]-6-méthyl-hepta-2,4-dienoate d'éthyle

De manière analogue à l'exemple 58(h) à partir de 568 mg (1,5 mmol) du produit précédent, on obtient 284 mg (75%) de produit attendu sous forme d'une huile.

### d) (3E,5E)-7-[3-(3,4-Bis-hydroxyméthyl-benzyloxy)-phenyl]-2,7-diméthyl-octa-3,5-dien-2-ol

De manière analogue à l'exemple 52(e) par réaction de 360 mg (0,8 mmol) de 4-[3-((2E,4E)-6-hydroxy-1,1,6-triméthyl-hepta-2,4-dienyl)-phenoxyméthyl]-phthalate de diméthyle (préparé à partir du produit précédent de manière analogue à l'exemple 63(d)) avec 70 mg (3,2 mmol) de borohydrure de lithium, une huile incolore est obtenue (m = 220 mg ; R = 70% ).
**RMN** ^{**1**}**H** (CDCl₃): 1,33 (s, 6H), 1,40 (s, 6H), 2,27 (bs, 2H), 4,35 (bs, 1H), 4,71 (s, 4H), 5,04 (s, 2H), 5,75-5,87 (m, 2H), 6,02 (dd, 1H, *J*_{*1*} = 15,3 Hz, *J*_{*2*} = 10 Hz), 6,22 (dd, 1H, *J*_{*1*} = 15,3 Hz, *J*_{*2*} = 10 Hz), 6,78 (dd, 1 H, *J*_{*1*} = 1,6 Hz, *J*_{*2*} = 8,2 Hz), 6,92-6,95 (m, 2H), 7,21 (t, 1H, *J* = 8,2 Hz), 7,37 (s, 2H), 7,44 (s, 1H).

### EXEMPLE 81

### (4E,6E)-7-[3-(3,4-Bis-hydroxyméthyl-phenoxyméthyl)-phenyl]-3-éthyl-octa-4,6-dien-3-ol

### a) (4E,6E)-7-{3-[3,4-Bis-(tert-butyl-dimethyl-silanyloxymethyl)-phenoxymethyl]-phenyl}-3-ethyl-octa-4,6-dien-3-ol

5-{3-[3,4-Bis-(*tert*-butyl-dimethyl-silanyloxymethyl)-phenoxymethyl]-phenyl}-hexa-2,4-dienoate d'éthyle (préparé à l'exemple 6(i)) (800 mg, 1,3 mmol) est dissous dans 10 mL de THF, et refroidi à 0°C. 9 mL (13 mmol) d'une solution 1,5 M d'ethyl lithium sont additionnés lentement, puis l'agitation est poursuivie pendant 3 heures. Après traitement par une solution saturée de chlorure d'ammonium puis extraction avec de l'éther éthylique, les phases organiques sont rassemblées, séchées puis concentrées sous pression réduite. Après purification par chromatographie sur colonne de silice (éluant heptane), le produit attendu est obtenu sous la forme d'une huile incolore ( m = 410 mg, R = 50 %).

### b) (4E,6E)-7-[3-(3,4-Bis-hydroxymethyl-phenoxymethyl)-phenyl]-3-ethyl-octa-4,6-dien-3-ol

De manière analogue à l'exemple 56(c), par réaction de 520 mg (0,83 mmol) de (4E,6E)-7- {3-[3,4-bis-(*tert*-butyl-diméthyl-silanyloxyméthyl)-phenoxyméthyl]-phenyl}-3-éthyl-octa-4,6-dien-3-ol avec 2,5 mL d'une solution de fluorure de tetrabutylammonium (1 N dans le THF), une huile incolore est obtenue ( m = 164 mg ; R = 50% ).
**RMN** ^{**1**}**H** (CDCl₃): 0,90 (t, 6H, *J* = 7,4 Hz), 1,57 (q, 4H, *J* = 7,4 Hz), 2,19 (s, 3H), 2,6 (bs, 2H), 4,69 (s, 2H), 4,71 (s, 2H), 5,08 (s, 2H), 5,79 (d, 1H, *J* = 14,9 Hz), 6,49 (d, 1H, *J* = 10,9 Hz), 6,65 (dd, 1H, *J*_{*1*} = 14,9 Hz, *J*_{*2*} *=* 10,9 Hz), 6,90 (dd, 1H, *J*_{*1*} = 2,6 Hz, *J*_{*2*} = 8,2 Hz), 7,03 (m, 1H), 7,25-7,50 (m, 5H).

### EXEMPLE 82

### (3E,5E)-6-[3-(3,4-Bis-hydroxyméthyl-phenoxyméthyl)-phenyl]-2-méthyl-hepta-3,5-dien-2-ol

### a) (3E,5E)-6-{3-[3,4-Bis-(tert-butyl-dimethyl-silanyloxymethyl)-phenoxymethyl]-phenyl}-2-methyl-hepta-3,5-dien-2-ol

5-{3-[3,4-Bis-(*tert*-butyl-dimethyl-silanyloxymethyl)-phenoxymethyl]-phenyl}-hexa-2,4-dienoate d'éthyle (préparé à l'exemple 6(i)) (417 mg, 0,7 mmol) est dissous dans 6 mL de THF, et refroidi à 0°C. 2 mL (2,8 mmol) d'une solution 1,4 M de méthyl lithium sont additionnés lentement, puis l'agitation est poursuivie pendant 3 heures. Après traitement par une solution saturée de chlorure d'ammonium puis extraction avec de l'éther. éthylique, les phases organiques sont rassemblées, séchées puis concentrées sous pression réduite. Après purification par chromatographie sur colonne de silice (éluant heptane), le produit attendu est obtenu sous la forme d'une huile incolore ( m = 120 mg, R = 30 %).

### b) (3E,5E)-6-[3-(3,4-Bis-hydroxymethyl-phenoxymethyl)-phenyl]-2-methyl-hepta-3,5-dien-2-ol

De manière analogue à l'exemple 56(c), par réaction de 120 mg (0,2 mmol) de (3E,5E)-6-{3-[3,4-bis-(*tert*-butyl-diméthyl-silanyloxyméthyl)-phenoxyméthyl]-phenyl}-2-méthyl-hepta-3,5-dien-2-ol avec 0,6 mL d'une solution de fluorure de tetrabutylammonium (1N dans le THF), une huile incolore est obtenue ( m = 30 mg ; R = 41% ).
**RMN** ^{**1**}**H** (CDCl₃): 1,39 (s, 6H), 2,19 (s, 3H), 2,9 (bs, 2H), 4,67 (s, 2H), 4,69 (s, 2H), 5,07 (s, 2H), 5,96 (d, 1H, *J* = 15 Hz), 6,44 (d, 1H, *J* = 10,9 Hz), 6,64 (dd, 1H, *J*_{*1*} = 15 Hz, *J*_{*2*} = 10,9 Hz), 6,89 (dd, 1H, *J*_{*1*} = 2,7 Hz, *J*_{*2*} = 8,2 Hz), 7,02 (m, 1H), 7,24-7,49 (m, 5H).

### EXEMPLE 83

### (Z)-7-[3-(3,4-Bis-hydroxyméthyl-benzyloxy)-phenyl]-3-éthyl-non-6-en-3-ol

### a) (Z)-5-(3-hydroxyphenyl)-hex-4-enoate d'éthyle

5,3 g (12,4 mmol) de bromure de (3-carboxypropyl)-triphenylphosphonium sont séchés sous vide pendant 1 h par chauffage à 130°C, puis ramenés à température ambiante et dissous dans 100 mL de THF anhydre. 2,75 g (25 mmol) de tert-butylate de potassium dans 50 mL de THF sont alors ajoutés lentement, puis le mélange rouge-orangé est agité pendant 15 minutes. Une solution de 1,2 g (6,2 mmol) de 1-(3-methoxymethoxy-phenyl)-1-propanone dans 50 mL de THF est alors ajoutée goutte à goutte, et le milieu réactionnel est agité pendant 15 heures. Après traitement par une solution saturée de chlorure d'ammonium et extraction avec de l'acétate d'éthyle, puis séchage et évaporation des solvants de la phase organique, le résidu est alors dissous dans 50 mL d'éthanol, puis 1 mL d'acide sulfurique sont ajoutés. Le milieu réactionnel est porté à reflux, et agité pendant 2 heures. Après traitement par de l'eau, le milieu est extrait avec de l'acétate d'éthyle, puis les phases organiques sont réunies, séchées et concentrées sous pression réduite. Le résidu est purifié par chromatographie sur colonne de silice (éluant heptane 95 - acétate d'éthyle 5) pour obtenir une huile jaune (m = 670 mg ; R = 43%).

### b) (Z)-5-{3-[3,4-Bis-(1-phenyl-methanoyloxymethyl)]-phenyl}-hept-4-enoate d'éthyle

670 mg (2.7 mmol) de (Z)-5-(3-hydroxyphenyl)-hex-4-enoate d'éthyle, 1,42 g (3,2 mmol) de bromure de (3,4-bis-benzoyloxymethy)-benzyle et 450 mg (3,2 mmol) de carbonate de potassium sont dissous dans 20 mL de 2-butanone. Le mélange est porté à reflux (80°C) puis agité pendant 4 h. Après refroidissement, le milieu réactionnel est filtré puis concentré sous pression réduite. Le résidu est purifié par chromatographie sur colonne de silice ( m = 1,18 g ; R = 73 %).

### c) (Z)-7-[3-(3,4-Bis-hydroxyméthyl-benzyloxy)-phenyl]-3-éthyl-non-6-en-3-ol

800 mg (1,32 mmol) de (Z)-5-{3-[3,4-bis-(1-phenyl-methanoyloxymethyl)]-phenyl}-hept-4-enoate d'éthyle sont dissous dans 30 mL de THF anhydre, puis le mélange est refroidi à 0°C. 4,4 mL (13 mmol) d'une solution de bromure d'ethylmagnesium 3M sont alors ajoutés, puis le milieu réactionnel est ramené à température ambiante et agité pendant 1 h. Après traitement par une solution saturée de chlorure d'ammonium puis extraction avec de l'acétate d'éthyle, les phases organiques sont réunies, séchées et concentrées sous pression réduite. Après purification par chromatographie sur colonne de silice, une huile incolore est obtenue ( m = 309 mg ; R = 57 %).
**RMN** ^{**1**}**H** (CDCl₃): 0,74 (t, 6H, J = 7,5 Hz), 0,95 (t, 3H, 7,4 Hz), 1,25-1,40 (m, 6H), 1,78-1,90 (m, 2H), 2,29 (q, 2H, J = 7,4 Hz), 3,5 (bs, 2H), 4,68 (s, 4H), 5,07 (s, 2H), 5,38 (t, 1H, J = 7,3 Hz), 6,70-6,85 (m, 2H), 7,18-7,39 (m, 5H).

### EXEMPLE 84

### (Z)-6-[3-(3,4-Bis-hydroxyméthyl-benzyloxy)-phenyl]-2-méthyl-oct-5-en-2-ol

De manière analogue à l'exemple 83(c), par réaction de 380 mg (0,63 mmol) de (Z)-5-{3-[3,4-bis-(1-phenyl-methanoyloxymethyl)]-phenyl}-hept-4-enoate d'éthyle (préparé de manière analogue aux exemples 83(a-b)) avec 2,1 mL (6,3 mmol) d'une solution de bromure de méthylmagnesium 3,0 M, une huile incolore est obtenue ( m = 178 mg ; R = 74% ).
**RMN** ^{**1**}**H** (CDCl₃): 0,87 (t, 3H, 7,4 Hz), 0,98 (s, 6H), 1,32-1,39 (m, 2H), 1,78-1,85 (m, 2H), 2,17-2,24 (m, 2H), 3,7 (bs, 2H), 4,58 (s, 2H), 4,60 (s, 2H), 4,99 (s, 2H), 5,28 (t, 1H), 6,63-6,78 (m, 2H), 7,11-7,30 (m, 5H).

### EXEMPLE 85

### (Z)-7-[3-(3,4-Bis-hydroxyméthyl-benzyloxy)-phenyl]-3-éthyl-dec-6-en-3-ol

De manière analogue à l'exemple 83(c), par réaction de 1 g (1,6 mmol) de (Z)-5-[3-(3,4-bis-hydroxymethyl-benzyloxy)-phenyl]-oct-4-enoate d'éthyle (préparé de manière analogue aux exemples 83(a-b)) avec 5,4 mL (16 mmol) d'une solution de bromure d'éthylmagnesium 3,0 M, une huile incolore est obtenue ( m = 480 mg ; R = 70 %).
**RMN** ^{**1**}**H** (CDCl₃): 0,70 (t, 6H, J = 7,5 Hz), 0,81-0,91 (m, 5H), 1,25-1,37 (m, 6H), 1,45-1,54 (m, 2H), 1,70-1,90 (m, 2H), 2,40-2,45 (m, 2H), 3,5 (bs, 1H), 3,69 (bs, 1H), 4,66 (s, 4H), 5,06 (s, 2H), 5,39 (t, 1H, J = 7,3 Hz), 6,70-6,85 (m, 2H), 7,18-7,39 (m, 5H).

### EXEMPLE 86

### (Z)-6-[3-(3,4-Bis-hydroxyméthyl-benzyloxy)-phenyl]-2-méthyl-non-5-en-2-ol

De manière analogue à l'exemple 83(c), par réaction de 1 g (1,6 mmol) de (Z)-5-[3-(3,4-bis-hydroxymethyl-benzyloxy)-phenyl]-oct-4-enoate d'éthyle (préparé de manière analogue aux exemples 83(a-b)) avec 5,4 mL (16 mmol) d'une solution de bromure de méthylmagnesium 3,0 M. Une huile incolore est obtenue ( m = 516 mg ; R = 81 %).
**RMN** ^{**1**}**H** (CDCl₃): 0,84 (t, 3H, J = 7,4 Hz), 1,06 (s, 6H), 1,28-1,47 (m, 4H),1,84-1,90 (m, 2H), 2,26 (t, 2H, J = 6,7 Hz), 3,5 (bs, 2H), 4,69 (s, 4H), 5,08 (s, 2H), 5,38 (t, 1H), 6,70-6,85 (m, 2H), 7,18-7,40 (m, 5H).

### EXEMPLE 86

### (Z)-8-[3-(3,4-Bis-hydroxyméthyl-benzyloxy)-phenyl]-3-éthyl-non-7-en-3-ol

### a) 6-(3-hydroxyphenyl)-hept-5-enoate d'éthyle

21,6 g (48,7 mmol) de bromure de (4-carboxybutyl)-triphenylphosphonium sont séchés sous vide pendant 1 h par chauffage à 130°C, puis ramenés à température ambiante et dissous dans 300 mL de THF anhydre. 10,9 g (97 mmol) de *tert*-butylate de potassium dans 100 mL de THF sont alors ajoutés lentement, puis le mélange rouge-orangé est agité pendant 15 minutes. Une solution de 6,3 g (32,5 mmol) de 1-(3-methoxymethoxy-phenyl)-1-ethanone dans 50 mL de THF est alors ajoutée goutte à goutte, et le milieu réactionnel est agité pendant 15 heures. Après traitement par une solution saturée de chlorure d'ammonium et extraction avec de l'acétate d'éthyle, puis séchage et évaporation des solvants de la phase organique, le résidu est alors dissous dans 50 mL d'éthanol, puis 1 mL d'acide sulfurique sont ajoutés. Le milieu réactionnel est porté à reflux, et agité pendant 2 heures. Après traitement par de l'eau, le milieu est extrait avec de l'acétate d'éthyle, puis les phases organiques sont réunies, séchées et concentrées sous pression réduite. Le résidu est purifié par chromatographie sur colonne de silice (éluant heptane 80 - acétate d'éthyle 20) pour obtenir 1 g (12%) de (Z)- 6-(3-hydroxyphenyl)-hept-5-enoate d'éthyle et 1,3 g (16%) de (E)- 6-(3-hydroxyphenyl)-hept-5-enoate d'éthyle.

### b) (Z)-6-{3-[3,4-Bis-(1-phenyl-methanoyloxymethyl)]-phenyl}-hept-5-enoate d'éthyle

1 g (4 mmol) de (Z)- 6-(3-hydroxyphenyl)-hept-5-enoate d'éthyle dissous dans 20 ml de DMF et on ajoute 180 mg de NaH (60% dans l'huile). Le milieu réactionnel est agité jusqu'à cessation du dégagement gazeux puis une solution de 1,9 g (4,4 mmol) de bromure de (3,4-bis-benzoyloxymethy)-benzyle dans 100 ml de DMF est ajoutée. Le mélange est agité pendant 14 h, extrait entre eau et acétate d'éthyle, décanté. Les phases organiques sont sèchées sur sulfate de magnésium, évaporées. Le résidu obtenu est purifié par chromatographie sur colonne de silice (heptane 80 - acétate d'éthyle 20)( m = 2,2 g ; R = 90 %).

### c) (Z)-8-[3-(3,4-Bis-hydroxyméthyl-benzyloxy)-phenyl]-3-éthyl-non-7-en-3-ol

De manière analogue à l'exemple 83( c), par réaction de 2,2 g (3,6 mmol) de (Z)-6-{3-[3,4-Bis-(1-phenyl-methanoyloxymethyl)]-phenyl}-hept-5-enoate d'éthyle avec 9,7 mL (29 mmol) d'une solution de bromure d'éthylmagnesium 3M, une huile incolore est obtenue ( m = 1,1 g ; R = 74 %).
**RMN** ^{**1**}**H** (CDCl₃): 0,82 (t, 6H, J = 7,5 Hz), 1,23-1,28 (m, 4H), 1,41 (q, 4H, J = 7,5 Hz), 1,87-1,90 (m, 2H), 2,00 (s, 3H), 3,1 (bs, 2H), 4,75 (s, 2H), 4,76 (s, 2H), 5,09 (s, 2H), 5,42 (t, 1H, J = 5,9 Hz), 6,76-6,88 (m, 3H), 7,22-7,43 (m, 4H).

### EXEMPLE 88

### (E)-8-[3-(3,4-Bis-hydroxyméthyl-benzyloxy)-phenyl]-3-éthyl-non-7-en-3-ol

### a) 4-Hydroxymethyl-phthalate de diméthyle

1,2,4-benzenetricarboxylic anhydride (50g, 260 mmol) est dissous dans 800 mL de dioxanne anhydre, à température ambiante. BH₃.THF (260 mmol, 1 éq.) est alors ajouté goutte à goutte par l'intermédiaire d'une ampoule de coulée, sur une période de 1h30 environ. L'agitation est maintenue pendant 12h, puis le milieu réactionnel est versé dans un mélange contenant 600 mL d'une solution saturée de NH₄Cl et 2 L de dichlorométhane. Après séparation, la phase organique est séchée, les solvants sont évaporés sous pression réduite. Le résidu obtenu est ensuite dissous dans 1 L de methanol, et chauffé à reflux après ajout de 5 mL d'acide sulfurique. Après 18 h de reflux, le milieu réactionnel est refroidi à température ambiante, et directement versé dans un mélange eau/éther éthylique (1 L / 2 L). Après séparation, la phase aqueuse est de nouveau extraite par deux fractions d'éther éthylique (700 mL environ) puis les phases organiques sont réunies et séchées puis concentrées sous pression réduite. Un mélange triester-diester/alcool est obtenu avec un rendement de 80%, contenant 65 % du produit désiré.

### b) 4-(tert-Butyl-dimethyl-silanyloxymethyl)-phthalate de diméthyle

Le mélange obtenu précédemment, contenant environ 135 mmol de produit désiré, est dissous dans 400 mL de DMF anhydre. Le chlorure de *tert*-butyldiméthylsilane (22,5 g, 150 mmol) est alors ajouté en une seule portion. Puis on ajoute en trois portions (légère exothermie) un total de 13,5 g (195 mmol) d'imidazole. Le milieu réactionnel est agité 36 heures, puis concentré sous pression réduite. Le résidu est alors dissous dans 500 ml d'éther éthylique, puis filtré pour éliminer le chlorhydrate d'imidazole formé. Le sel est rinçé avec 2 fractions de 150 mL d'éther éthylique, puis les phases organiques sont séchées et concentrées sous pression réduite. Le résidu est alors purifié par chromatographie: le premier produit collecté (éluant AcOEt 10 - heptane 90) est le 4-(*tert*-Butyl-dimethyl-silanyloxymethyl)-phthalate de diméthyle désiré. Rendement 87%, rendement global depuis l'acide de départ: 45%.

### c) [5-(tert-Butyl-dimethyl-silanyloxymethyl)-2-hydroxymethyl-phenyl]-methanol

Le diester obtenu précédemment (75 g, 220 mmol) est dissous dans 1 L d'éther éthylique, et refroidi à 0°C sous pression positive d'azote. 4 fractions de 5 g de LiAlH₄ (527 mmol) sont ajoutées précautionneusement, puis le mélange est chauffé à 50°C. Après 1h30 d'agitation, le milieu réactionnel est de nouveau refroidi à 0°C, puis traité successivement avec 20 mL d'eau, 20 mL de NaOH 15%, puis 60 mL d'eau. Le milieu réactionnel est agité 30 minutes jusqu'à ce qu'on observe la disparition complète des sels d'aluminium gris et leur précipitation en flocons blancs. Le milieu est alors filtré et après rinçage des sels par trois fractions d'acétate d'éthyle (200 mL), les phases organiques sont rassemblées, séchées et concentrées sous pression réduite. Le produit obtenu représente un rendement de 97%

### d) benzoate de 2-benzoyloxymethyl-4-(tert-butyl-dimethyl-silanyloxymethyl)-benzyle

Le diol brut obtenu ci-dessus (60 g, 212 mmol) est dissous dans 600 mL de THF anhydre et refroidi à 0°C. 74 mL (530 mmol) de triéthylamine sont alors ajoutés, suivis de 52 mL (448 mmol) de chlorure de benzoyle. DMAP (500 mg) est ensuite ajouté en une seule portion et le mélange est agité 30 minutes à 0°C puis 12 heures à température ambiante. Le milieu réactionnel est alors filtré pour éliminer les sels de triéthylammonium précipités, les sels sont rincés avec deux fractions de 200 mL d'acétate d'éthyle, puis le mélange des phases organique est concentré sous pression réduite, repris dans le dichlorométhane, rinçé par une solution saturée de NH₄CL et enfin par une fraction d'eau. Après séchage, la phase organique est concentrée sous pression réduite, donnant un résidu jaune foncé, utilisé tel quel pour l'étape suivante.

### e) benzoate de 2-benzoyloxymethyl-4-hydroxymethyl-benzyl

Le résidu obtenu précédemment est dissous dans 600 mL d'acétate d'éthyle et 220 mL d'une solution de fluorure de tétrabutylammonium (1M dans le THF) sont ajoutés en une seule fraction. Après 30 minutes d'agitation à température ambiante, le milieu réactionnel est versé dans une ampoule à décanter contenant 1 L de NH₄Cl saturé. Après séparation, la phase aqueuse est de nouveau extraite avec 500 mL d'acétate d'éthyle, puis les phases organiques sont rassemblées, séchées et évaporées. Le produit est alors purifié par chromatographie (Acétate d'éthyle 30 / Heptane 70). Un solide blanc est obtenu (pf : 91-93°C).

### f) bromure de (3,4-bis-benzoyloxymethy)-benzyle

L'alcool précédent (65 g, 172 mmol) est dissous dans 350 mL de dichlorométhane et CBr₄ (67.7g, 202 mmol) est ajouté. Le milieu est refoidi à 0°C, puis une solution de triphénylphosphine (53 g, 202 mmol) dans 250 mL de dichlorométhane est ajoutée goutte à goutte. Le milieu réactionnel est alors réchauffé à température ambiante, puis agité 2 heures. Le milieu est alors traité par 500 mL d'eau, puis extrait par le dichlorométhane. Après séchage et concentration des phases organiques, le produit est purifié par chromatographie (éluant CH₂Cl₂/EtOAc), pour obtenir un solide blanc (pf: 83°C) avec un rendement de 93%.

### g) (E)-6-{3-[3,4-Bis-(1-phenyl-methanoyloxymethyl)]-phenyl}-hept-5-enoate d'éthyle

500 mg (2 mmol) de (E)- 6-(3-hydroxyphenyl)-hept-5-enoate d'éthyle (obtenu à l'exemple 87(a)) dissous dans 20 ml de DMF et on ajoute 90 mg de NaH (60% dans l'huile). Le milieu réactionnel est agité jusqu'à cessation du dégagement gazeux puis une solution de 950 mg (2,2 mmol) de bromure de (3,4-bis-benzoyloxymethy)-benzyle (obtenu précédemment) dans 50 ml de DMF est ajoutée. Le mélange est agité pendant 14 h, extrait entre eau et acétate d'éthyle, décanté. Les phases organiques sont sèchées sur sulfate de magnésium, évaporées. Le résidu obtenu est purifié par chromatographie sur colonne de silice (heptane 80 - acétate d'éthyle 20)( m = 1,1 g ; R = 90 %).

### h) (E)-8-[3-(3,4-Bis-hydroxyméthyl-benzyloxy)-phenyl]-3-éthyl-non-7-en-3-ol

De manière analogue à l'exemple 83( c), par réaction de 1,1 g (1,8 mmol) de (E)-6-{3-[3,4-Bis-(1-phenyl-methanoyloxymethyl)]-phenyl}-hept-5-enoate d'éthyle avec 4,8 mL (14,5 mmol) d'une solution de bromure d'éthylmagnesium 3M, une huile incolore est obtenue ( m = 470 mg ; R = 64 % ).
**RMN** ^{**1**}**H** (CDCl₃): 0,86 (t, 6H, J = 7,5 Hz), 1,38-1,57 (m, 8H), 2,00 (s, 3H), 2,16-2,22 (m, 2H), 2,8 (bs, 2H), 4,76 (s, 2H), 4,77 (s, 2H), 5,08 (s, 2H), 5,76 (t, 1H, J = 7,2 Hz), 6,81-6,85 (m, 1H), 6,97-7,00 (m, 2H), 7,22 (t, 1H, J = 8,2 Hz), 7,39 (s, 2H), 7,45 (s, 1H).

### EXEMPLE 89

### 8-[3-(3,4-Bis-hydroxyméthyl-benzyloxy)-phenyl]-3-éthyl-nonan-3-ol

### a) 6-(3-hydroxyphenyl)-heptanoate d'éthyle

De manière analogue à l'exemple 63(b) à partir de 6-(3-hydroxyphenyl)-hept-5-enoate d'éthyle, on obtient le 6-(3-hydroxyphenyl)-heptanoate d'éthyle sous forme d'une huile.

### b) 6-{3-[3,4-Bis-(1-phenyl-methanoyloxymethyl)]-phenyl}-heptanoate d'éthyle

De manière analogue à l'exemple 88(a) par réaction de 6-(3-hydroxyphenyl)-heptanoate d'éthyle avec le bromure de (3,4-bis-benzoyloxymethy)-benzyle, on obtient le 6-{3-[3,4-Bis-(1-phenyl-methanoyloxymethyl)]-phenyl}-heptanoate d'éthyle sous forme d'une huile.

### b) 8-[3-(3,4-Bis-hydroxyméthyl-benzyloxy)-phenyl]-3-éthyl-nonan-3-ol

Le produit est obtenu de manière analogue à l'exemple 83(c) par addition de 4,5 mL d'une solution 3,0 M de bromure d'éthylmagnésium sur 1 g (1,6 mmol) de 6-{3-[3,4-Bis-(1-phenyl-methanoyl oxymethyl)]-phenyl}-heptanoate d'éthyle. Une huile incolore est obtenue ( m = 490 mg ; R = 66 % ).
**RMN** ^{**1**}**H** (CDCl₃): 0,82 (t, 6H, J = 7,5 Hz), 1,14-1,33 (m, 9H), 1,42 (q, 4H, J = 7,5 Hz), 1,46-1,62 (m, 2H), 2,64 (m, 1H), 3,08 (bs, 2H), 4,73 (s, 2H), 4,74 (s, 2H), 5,30 (s, 2H), 6,77-6,80 (m, 3H), 7,20 (t, 1H, J = 8,2 Hz), 7,37 (s, 2H), 7,42 (s, 1H).

### EXEMPLE 90

### (4E,6E)-7-[5-(3,4-Bis-hydroxyméthyl-benzyloxy)-2-methoxy-phenyl]-3-éthyl-nona-4,6-dien-3-ol

### a) 5-méthoxyméthoxy-2-méthoxybenzaldéhyde

A 9,6 g (63 mmol) de 5-hydroxy-2-méthoxybenzaldéhyde dans 150 ml de DMF, on ajoute 3 g (7,6 mmol) de NaH (60% dans l'huile) et agite jusqu'à cessation du dégagement gazeux. On ajoute ensuite 5,3 ml (70 mmol) de chlorure de méthoxyméthyle et agite à température ambiante deux heures. Après traitement usuel et chromatographie sur silice, 12,3 g du produit attendu sont obtenus (100%).

### b) 3-(5-méthoxyméthoxy-2-méthoxyphényl)-3-propanol

12,3 g (63 mmol) du produit précédent dans le THF sont mis en réaction avec 31 ml d'une solution 3M de bromure d'éthylmagnésium (93 mmol). Après une heure d'agitation et traitement usuel, le résidu est purifié par chromatographie sur silice (acétate d'éthyle 20 / heptane 80). On obtient 13,2 g (92%) de 3-(5-méthoxyméthoxy-2-méthoxyphényl)-3-propanol

### c) 3-(5-méthoxyméthoxy-2-méthoxyphényl)-3-propanone

4,04 mL (43,3 mmol) de chlorure d'oxalyle sont dissous dans 150 mL de dichlorométhane puis le mélange est refroidi à -78°C. Une solution de 6,58 mL (92,7 mmol) de DMSO dans 20 mL de dichlororméthane est alors additionnée lentement. Lorsque l'évolution de gaz est achevée (après environ 15 minutes), une solution de 5,3g (23,1 mmol) de de 3-(5-méthoxyméthoxy-2-méthoxyphényl)-3-propanol et de 3,3 mL de triéthylamine (23 mmol) dans 50 mL de dichlorométhane est additionnée goutte à goutte. Après 20 minutes, 22,5 mL (162 mmol) de triéthylamine sont ajoutés puis le milieu réactionnel est ramené à température ambiante et agité pendant 1 heure. Le milieu est alors traité avec une solution saturée de chlorure d'ammonium puis extrait avec de l'éther éthylique. La phase organique est lavée à l'eau, séchée et concentrée sous pression réduite. Le résidu est purifié par chromatographie sur silice (acétate d'éthyle 20 / heptane 80), on recueille 5,2 g (100%) de cétone attendue.

### d) 5-(5-hydroxy-2-méthoxyphényl)hepta-2,4-dienoate d'éthyle

De manière analogue à l'exemple 62 (c) , par réaction de 3,9 g (17 mmol) de 3-(5-méthoxyméthoxy-2-méthoxyphényl)-3-propanone avec 8,7 g (34,8 mmol) de 4-diéthylphosphonocrotonate d'éthyle, on obtient le 5-(5-méthoxyméthoxy-2-méthoxyphényl)hepta-2,4-dienoate d'éthyle que l'on transforme en 5-(5-hydroxy-2-méthoxyphényl)hepta-2,4-dienoate d'éthyle avec de l'acide sulfurique concentré dans l'éthanol 800 mg (17%).

### e) (4E,6E)-7-(5-hydroxy-2-méthoxyphényl)-3-éthyl-nona-4,6-dien-3-ol

De manière analogue à l'exemple 81(a), à partir de 520 mg (1,9 mmol) de 5-(5-hydroxy-2-méthoxyphényl)hepta-2,4-dienoate d'éthyle, on obtient 350 mg (64%) de (4E,6E)-7-(5-hydroxy-2-méthoxyphényl)-3-éthyl-nona-4,6-dien-3-ol sous forme d'une huile.

### f) (4E,6E)-7-[5-(3,4-Bis-benzoyloxyméthyl-benzyloxy)-2-methoxy-phenyl]-3-éthyl-nona-4,6-dien-3-ol

De manière analogue à l'exemple 88 (g), par réaction de 350 mg (1,2 mmol) du produit précédent avec 630 mg (1,44 mmol) de bromure de (3,4-bis-benzoyloxymethy)-benzyle, on obtient 581 mg (75%) de produit attendu.

### g) (4E,6E)-7-[5-(3,4-Bis-hydroxyméthyl-benzyloxy)-2-methoxy-phenyl]-3-éthyl-nona-4,6-dien-3-ol

581 mg de 7-[5-(3,4-Bis-benzoyloxyméthyl-benzyloxy)-2-methoxy-phenyl)-3-éthyl-nona-4,6-dien-3-ol (0,89 mmol) sont dissous dans 20 mL d'une solution de carbonate de potassium 2% dans du methanol, puis le milieu reactionnel est agité pendant 5 heures. Après traitement par une solution saturée de chlorure d'ammonium et extraction avec de l'acétate d'éthyle, les phases organiques sont rassemblées, séchées et concentrées sous pression réduite. Le résidu est purifié par chromatographie sur colonne de silice. Une huile jaune est obtenue ( m 284 mg; R = 72 % ).
**RMN** ^{**1**}**H** (CDCl₃): 0,80 (t, 6H, J = 7,6 Hz), 0,97 (t, 3H, J = 7,4 Hz), 1,47 (q, 4H, J = 7,6 Hz), 2,41 (q, 2H, J = 7,4 Hz), 3,25 (bs, 1H), 3,35 (bs, 1H), 3,73 (s, 3H), 4,71 (s, 4H), 5,05 (s, 2H), 5,60 (d, 1H, J = 15,3 Hz), 5,94 (dd, 1H, J1 = 15,3 Hz, J2 = 10,7 Hz), 6,14 (d, 1H, J = 10,7 Hz), 6,67-6,88 (m, 3H), 7,34-7,56 (m, 3H).

### EXEMPLE 91

### (4E,6E)-7-[5-(3,4-Bis-hydroxyméthyl-benzyloxy)-2-methyl-phenyl]-3-éthyl-nona-4,6-dien-3-ol

De manière analogue à l'exemple 90, à partir de 5-hydroxy-2-méthyl benzaldéhyde, le (4E,6E)-7-[5-(3,4-Bis-hydroxyméthyl-benzyloxy)-2-methyl-phenyl]-3-éthyl-nona-4,6-dien-3-ol est obtenu sous forme d'une huile claire.
**RMN** ^{**1**}**H** (CDCl₃): 0,83-0,97 (m, 9H), 1,60 (q, 4H, J = 7,6 Hz), 2,20 (s, 3H), 2,49 (q, 2H, J = 7,4 Hz), 2,83 (bs, 2H), 3,10 (bs, 1H), 4,75 (s, 4H), 5,03 (s, 2H), 5,65 (d, 1H, J = 15,3 Hz), 5,87 (d, 1H, J = 11 Hz), 6,58 (dd, 1H, J1 = 15,3 Hz, J2 = 11 Hz), 6,74-6,81 (m, 2H), 7,06-7,09 (m, 1H), 7,36-7,43 (m, 3H).

### EXEMPLE 92

### (4E,6E)-7-[3-(3,4-Bis-hydroxyméthyl-benzyloxy)-5-methoxy-phenyl]-3-éthyl-nona-4,6-dien-3-ol

De manière analogue aux exemples 90(c-g), à partir de (3-méthoxy-5-méthoxyméthoxyphényl)éthanone, le (4E,6E)- 7-[3-(3,4-Bis-hydroxyméthyl-benzyloxy)-5-methoxy-phenyl]-3-éthyl-nona-4,6-dien-3-ol est obtenu sous forme d'une huile claire.
**RMN** ^{**1**}**H** (CDCl₃): 0,87 (t, 6H, J = 7,6 Hz), 1,60 (q, 4H, J = 7,6 Hz), 2,16 (m, 3H), 2,88 (bs, 2H), 3,80 (s, 3H), 4,76 (s, 2H), 4,77 (s,2H), 5,05 (s, 2H), 5,78 (d, 1H, J = 15 Hz), 6,44-6,68 (m, 5H), 7,33-7,45 (m, 3H).

### EXEMPLE 93

### (4E,6E)-7-[3-(3,4-Bis-hydroxyméthyl-benzyloxy)-2-methoxy-phenyl]-3-éthyl-nona-4,6-dien-3-ol

De manière analogue à l'exemple 90, à partir de 3-hydroxy-2-méthoxy benzaldéhyde, le (4E,6E)-7-[3-(3,4-Bis-hydroxyméthyl-benzyloxy)-2-methoxy-phenyl]-3-éthyl-nona-4,6-dien-3-ol est obtenu sous forme d'une huile claire.
**RMN** ^{**1**}**H** (CDCl₃): 0,90-1,01 (m, 9H), 1,61 (q, 4H, J = 7,6 Hz), 2,66 (q, 2H, J = 7,6 Hz), 2,75 (bs, 2H), 3,79 (s, 3H), 4,78 (s, 4H), 5,12 (s, 2H), 5,68 (d, 1H, J = 15,3 Hz), 6,09 (d, 1H, J = 11 Hz), 6,62 (dd, 1H, J1 = 15,3 Hz, J2 = 11 Hz), 6,77-6,96 (m, 3H), 7,40-7,46 (m, 3H).

### EXEMPLE 94

### (4E,6E)-7-[3-(3,4-Bis-hydroxyméthyl-benzyloxy)-4-methyl-phenyl]-3-éthyl-nona-4,6-dien-3-ol

De manière analogue aux exemples 90(c-g), à partir de (3-méthoxyméthoxy-4-méthylphényl)éthanone, le (4E,6E)-7-[3-(3,4-Bis-hydroxyméthyl-benzyloxy)-4-methyl-phenyl]-3-éthyl-nona-4,6-dien-3-ol est obtenu sous forme d'une huile claire.
**RMN** ^{**1**}**H** (CDCl₃): 0,90 (t, 6H, J = 7,5 Hz), 1,49 (bs, 1H), 1,60 (q, 4H, J = 7,5 Hz), 2,04 (s, 3H), 2,26 (s, 3H), 3,08 (bs, 2H), 4,73 (s, 2H), 4,74 (s,2H), 5,09 (s, 2H), 5,76 (d, 1H, J = 15 Hz), 6,41 (d, 1H, 10,9 Hz), 6,62 (dd, 1H, J1 = 15 Hz, J2 = 10,9 Hz), 6,96-6,99 (m, 2H), 7,11 (d, 1H, J = 8,0 Hz), 7,35-7,45 (m, 3H).

### EXEMPLE 95

### 1-[3-(3,4)-Bis-hydroxymethyl-benzyloxy)-phenyl]ethanone O -(2-hydroxy-2-methyl-propyl)-oxime

### a) 1-(3-Methoxymethoxy-phenyl)-ethanone oxime

360 mg (2 mmol) de 1-(3-Methoxymethoxy-phenyl)-ethanone sont dissous dans 30 mL d'éthanol anhydre. 417 mg (6 mmol) de chlorhydrate d'hydroxylamine sont additionnés, suivis par 6 mL d'une solution 1N d'hydroxyde de sodium. Le mélange est chauffé à reflux pendant 2 heures, puis le milieu réactionnel est concentré sous pression réduite. Le résidu est repris dans un mélange d'éther éthylique et d'une solution de chlorure d'ammonium. Après extraction à l'éther, les phases organiques sont rassemblées, séchées et concentrées sous pression réduite. Le résidu est purifié par chromatographie sur colonne de silice. Une huile jaune est obtenue ( m = 342 mg ; R = 88 % ).

### b) 1-(3-Methoxymethoxy-phenyl)-ethanone O-(2-hydroxy-2-methyl-propyl)-oxime

330 mg de 1-(3-Methoxymethoxy-phenyl)-ethanone oxime (1,7 mmol) sont dissous dans 10 mL de THF anhydre. 180 mg (1,87 mmol) de tert-butylate de sodium sont alors ajoutés, et le mélange est agité pendant 1 heure. 690 DL (7,6 mmol) d'oxyde d'isobutylène sont alors additionnés, et le mélange est chauffé à reflux pendant 15 heures. Après traitement avec de l'eau, le milieu réactionnel est extrait avec de l'éther éthylique, puis les phases organiques sont rassemblées, séchées et concentrées sous pression réduite. Le résidu est purifié par chromatographie sur colonne de silice. Une huile jaune est obtenue ( m = 215 mg ; R = 48 % )

### c) 1-(3-Hydroxy-phenyl)-ethanone O-(2-hydroxy-2-methyl-propyl)-oxime

210 mg (0,78 mmol) de 1-(3-Methoxymethoxy-phenyl)-ethanone O-(2-hydroxy-2-methyl-propyl)-oxime sont dissous dans 10 mL de méthanol. 200 □L d'acide sulfurique sont ajoutés, et le milieu réactionnel est agité pendant 18 heures à température ambiante. Après traitement à l'eau et extraction avec de l'éther éthylique, les phases organiques sont rassemblées, séchées puis concentrées sous pression réduite. Le résidu est purifié par chromatographie sur colonne de silice. Une huile jaune pâle est obtenue ( m = 175 mg ; R = 100 % ).

### d) 1-[3-(3,4)-Bis-hydroxymethyl-benzyloxy)-phenyl]ethanone O -(2-hydroxy-2-methyl-propyl)-oxime

De manière analogue aux exemples 83 b et 90 b, à partir de 170 mg (0,76 mmol) de 1-(3-Hydroxy-phenyl)-ethanone *O*-(2-hydroxy-2-methyl-propyl)-oxime, on obtient 197 mg (74%) de 1-[3-(3,4)-Bis-hydroxymethyl-benzyloxy)-phenyl]ethanone *O* -(2-hydroxy-2-methyl-propyl)-oxime sous forme d'une huile incolore.
**RMN** ^{**1**}**H** (CDCl₃): 1,22 (s, 6H), 2,23 (s, 3H), 3,44 (bt, 1H), 3,58 (s, 1 H), 4,71 (t, 4H, J = 5,6 Hz), 5,15 (s, 2H), 6,99-7,15 (m, 3H), 7,25-7,39 (m, 4H).

### EXEMPLE 96

### 1-{1-[3-(3,4-Bis-hydroxyméthyl-benzyloxy)-phenyl]-propoxy}-3-éthyl-pentan-3-ol

### a) 1-(3-Methoxymethoxy-phenyl)-propan-1-ol

11,6 g ( 69,8 mmol) de 3-methoxymethoxybenzaldehyde sont dissous dans 40 mL de THF anhydre. Le mélange est refroidi à 0°C, puis est additionné à une solution de bromure d'éthylmagnesium (139 mmol) dans 100 mL d'éther éthylique. Le milieu réactionnel est ramené à température ambiante, puis agité pendant 4 heures. Après traitement par une solution de chlorure d'ammonium et extraction avec de l'éther éthylique, les phases organiques sont rassemblées, séchées et concentrées sous pression réduite. Le résidu est filtré sur une couche de silice puis de nouveau concentré, pour obtenir une huile jaune ( m = 12,9 g ; R = 94 % ).

### b) 3-[1-(3-Methoxymethoxy-phenyl)-propoxy]-propionate d'éthyle

2,9 g (9,8 mmol) de 1-(3-Methoxymethoxy-phenyl)-propan-1-ol et 4,24 mL (39 mmol) d'acrylate d'éthyle sont dissous dans 10 mL de THF anhydre. Cette solution est alors additionnée à une suspension d'hydrure de sodium 60% (390 mg, 9,8 mmol) dans 5 mL de THF maintenue à 0°C. Le milieu est ramené à température ambiante, puis agité pendant 48 heures. Le milieu est alors traité par une solution de chlorure d'ammonium, et extrait avec de l'acétate d'éthyle. Les phases organiques sont rassemblées, séchées puis concentrées sous pression réduite. Le résidu est purifié par chromatographie sur colonne de silice (éluant heptane). Une huile jaune est obtenue ( m = 700 mg ; R = 24 % ).

### c) 3-[1-(3-Hydroxy-phenyl)-propoxy]-propionate d'éthyle

1,09 g (36,8 mmol) de 3-[1-(3-Methoxymethoxy-phenyl}-propoxy]-propionate d'éthyle sont dissous dans 30 mL d'éthanol. 500 □L d'acide sulfurique sont alors ajoutés, et le milieu est agité à température ambiante pendant 15 minutes. Après hydrolyse et extraction par de l'acétate d'éthyle, les phases organiques sont rassemblées, séchées et concentrées sous pression réduite. Le résidu est purifié par chromatographie sur colonne de silice. Une huile jaune est obtenue ( m = 520 mg ; R = 56 % ).

### d) 1-{1-[3-(3,4-Bis-hydroxyméthyl-benzyloxy)-phenyl]-propoxy}-3-éthyl-pentan-3-ol

De manière analogue aux exemples 83 (b, c), 520 mg de 3-[1-(3-Hydroxy-phenyl)-propoxy]-propionate d'éthyle (2,06 mmol) sont converties en 1-{1-[3-(3,4-bis-hydroxyméthyl-benzyloxy)-phenyl]-propoxy}-3-éthyl-pentan-3-ol, sous forme d'une huile jaune pâle ( m = 260mg;R=30%).
**RMN** ^{**1**}**H** (CDCl₃): 0,64-0,74 (m, 9H), 1,24-1,42 (m, 4H), 1,47-1,63 (m, 4H), 3,16 (bs, 3H), 3,32-3,38 (m, 2H), 3,90-4,00 (m, 1H), 4,59 (s, 2H), 4,60 (s, 2H), 4,94 (s, 2H), 6,69-6,78 (m, 3H), 7,08-7,29 (m, 4H).

### EXEMPLE 97

### (E)-7-[3-(3,4-Bis-hydroxyméthyl-phenoxyméthyl)-phenyl]-3-éthyl-non-6-en-3-ol

### a) (E)-5-(3-Bromophenyl)-hept-4-enoate de méthyle

19,3 g (44,9 mmol) de bromure de (3-carboxypropyl)-triphenylphosphonium sont séchés sous vide pendant 1 h par chauffage à 130°C, puis ramenés à température ambiante et dissous dans 200 mL de THF anhydre. 10,1 g (89,8 mmol) de tert-butylate de potassium dans 100 mL de THF sont alors ajoutés lentement, puis le mélange rouge-orangé est agité pendant 15 minutes. Une solution de 6,4 g (29,9 mmol) de 1-(3-bromo-phenyl)-propanone dans 100 mL de THF est alors ajoutée goutte à goutte et le milieu réactionnel est agité pendant 15 heures. Après traitement par une solution saturée de chlorure d'ammonium et extraction avec de l'acétate d'éthyle puis séchage et évaporation des solvants de la phase organique, le résidu est purifié par chromatographie sur colonne de silice. Une huile jaune est obtenue (m = 6,2 g ; R = 74%). Ce produit est alors dissous dans 100 mL de méthanol, puis 2 mL d'acide sulfurique sont ajoutés. Le milieu réactionnel est porté à reflux, et agité pendant 2 heures. Après traitement par de l'eau, le milieu est extrait avec de l'acétate d'éthyle, puis les phases organiques sont réunies, séchées et concentrées sous pression réduite. Le résidu est purifié par chromatographie sur colonne de silice (éluant heptane 95 - acétate d'éthyle 5) pour obtenir l'isomère *trans* pur sous forme d'une huile jaune (m = 6,5 g ; R = 74% total).

### b) (E)-7-(3-Bromophenyl)-3-éthyl-non-6-en-3-ol

6,49 g de (E)-5-(3-bromophenyl)-hept-4-enoate d'éthyle (21,8 mmol) sont dissous. dans 100 mL d'éther éthylique. 29 mL d'une solution 3.0M de bromure d'éthylmagnesium (87 mmol) sont alors ajoutés goutte à goutte, et le milieu réactionnel est agité à température ambiante pendant 1 heure. Après traitement par une solution saturée de chlorure d'ammonium, extraction avec de l'éther éthylique puis séchage et évaporation des solvants de la phase organique, le résidu est purifié par chromatographie sur colonne de silice, une huile incolore est obtenue ( m = 6,79 g ; R = 97 %).

### c) [(E)-5-(3-bromophenyl)-1,1-diéthyl-hept-4-enyloxy]-triéthylsilane

6,79 g (20,9 mmol) de (E)-7-(3-bromophenyl)-3-éthyl-non-6-en-3-ol sont dissous dans 100 mL de dichlorométhane. 75 mg (0,6 mmol) de 4-diméthylaminopyridine et 14,5 mL de triéthylamine (104 mmol) sont ajoutés, et le milieu réactionnel est refroidi à 0°C. 11,8 mL (52 mmol) de triéthylsilyltrifluorométhanesulfonate sont additionnés goutte à goutte. Après l'addition, le milieu réactionnel est ramené à température ambiante, puis traité par de l'eau et extrait avec du dichlorométhane. Après séchage et concentration sous pression réduite des phases organiques, le résidu est purifié par chromatographie sur colonne de silice; un huile jaune est obtenue ( m = 9,1 g ; R = 99 %).

### d) [3-((E)-1,5-diéthyl-5-triéthylsilanyloxy-hept-1-enyl)-phenyl]-méthanol

9,1 g (20,5 mmol) de [(E)-5-(3-bromophenyl)-1,1-diéthyl-hept-4-enyloxy]-triéthyl-silane sont dissous dans 130 mL de THF anhydre, puis le mélange est refroidi à -78°C. 9,18 mL (23 mmol) d'une solution de butyllithium 2,5 M sont alors ajoutés, puis le milieu réactionnel est agité pendant 15 minutes. 1,78 mL de DMF anhydre (23 mmol) sont alors additionnés, puis le milieu réactionnel est ramené à température ambiante et agité pendant 1 h.. Après traitement par une solution saturée de chlorure d'ammonium puis extraction avec de l'acétate d'éthyle, les phases organiques sont réunies, séchées et concentrées sous pression réduite. Le résidu contenant le (E)-5-éthyl-1-éthyl-5-triéthylsilanyloxy-hept-1-enyl)-benzene-2-carbaldehyde désiré est alors dissous dans 100 mL de méthanol anhydre, puis 760 mg (20 mmol) de borohydrure de sodium sont ajoutés en deux portions. Après 10 minutes d'agitation le milieu est traité par une solution de chlorure d'ammonium et extrait par de l'éther éthylique. Les phases organiques sont rassemblées, séchées et concentrées sous pression réduite. Après purification par chromatographie sur colonne de silice, une huile jaune est obtenue ( m = 2 g ; R = 25 % ).

### e) 4-[3-((E)-1,5-diéthyl-triéthylsilanyloxy-hept-1-enyl)-phenylméthoxy]-phthalate de diméthyle

300 mg (0,77 mmol) de [3-((E)-1,5-diéthyl-5-triéthylsilanyloxy-hept-1-enyl)-phenyl]-méthanol sont dissous dans 20 mL de dichlorométhane et refroidis à 0°C. 0,16 mL (1,1 mmol) de triéthylamine sont ajoutés, suivis de 65 □L de chlorure de méthylsulfonate (0,85 mmol). Après 20 minutes d'agitation, le milieu réactionnel est traité par une solution de chlorure d'ammonium et extrait avec du dichlorométhane. Les phases organiques sont rassemblées, séchées et concentrées sous pression reduite. Le résidu obtenu est alors dissous dans 20 mL de 2-butanone, et 244 mg de 4-hydroxyphthalate de diméthyle (1,16 mmol), 160 mg de carbonate de potassium (1,16 mmol) et 10 mg d'iodure de sodium sont ajoutés. Le mélange est chauffé à reflux pendant 6 heures, puis refroidi et filtré. Le filtrat est concentré sous pression réduite, puis purifié par chromatographie sur colonne de silice. Une huile jaune est obtenue (m = 355 mg ; R = 79%).

### f) {4-[3-((E)-1,5-Diéthyl-5-triéthylsilanoxy-hept-1-enyl)-phenoxyméthyl]-2-hydroxymethyl-pheny}méthanol

1,28 g (2,2 mmol) de 4-[3-((E)-1,5-diéthyl-5-hydroxy-hept-1-enyl)-phenylméthoxy]-phthalate de diméthyle sont dissous dans 40 mL d'éther éthylique anhydre. 200 mg (5,3 mmol) d'hydrure double de lithium et d'aluminium sont additionnés, et le milieu réactionnel est agité à température ambiante pendant 30 minutes. 200 □L d'eau, 200 □L de NaOH 15% et 600 □L d'eau sont alors ajoutés lentement et le milieu est filtré. Le filtrat est concentré sous pression réduite, puis le résidu est purifié par chromatographie sur colonne de silice (acétate d'éthyle 70 -heptane 30). Une huile incolore est obtenue (m = 1,03 mg ; R = 89%).

### g) (E)-7-[3-(3,4-Bis-hydroxyméthyl-phenoxyméthyl)-phenyl]-3-éthyl-non-6-en-3-ol

1,03 g (1,96 mmol) de {4-[3-((E)-1,5-Diéthyl-5-triéthylsilanoxy-hept-1-enyl)-phenoxyméthyl]-2-hydroxymethyl-pheny}méthanol sont dissous dans 30 mL de THF. 3,9 mL (3,9 mmol) d'une solution de fluorure de tetrabutylammonium (1 M dans le THF) sont additionnés et le milieu réactionnel est chauffé à 60°C pendant 3 heures. Après traitement par une solution de chlorure d'ammonium et extraction avec de l'acétate d'éthyle, les phases organiques sont rassemblées, séchées et concentrées sous pression réduite. Le résidu est purifié par chromatographie sur colonne de silice. Une huile incolore est obtenue (m = 198 mg ; R = 24% ).
**RMN** ^{**1**}**H** (CDCl₃): 0,69 (t, 6H, J = 7,6 Hz), 0,91 (t, 3H, 7,5 Hz), 1,40-1,50 (m, 6H), 2,14 (q, 2H, J = 7,6 Hz), 2,45 (q, 2H, J = 7,5 Hz), 2,8 (bs, 1H), 3,15 (bs, 1H); 4,59 (s, 2H), 4,61 (s, 2H), 4,99 (s, 2H), 5,57 (t, 1H, J = 7,3 Hz), 6,79-6,95 (m, 2H), 7,10-7,31 (m, 5H).

### EXEMPLE 98

### (E)-7-[3-(3,4-Bis-hydroxyméthyl-benzylsulfanyl)-phenyl]-3-éthyl-oct-6-en-3-ol

### a) Acide (E)-5-(3-dimethylcarbamoylsulfanyl-phenyl)-hex-4-enoique

4 g (17,9 mmol) d'acide diméthyl-thiocarbamique *S*-(3-acetyl-phenyl) ester sont soumis aux mêmes conditions décrites à l'exemple 97 (a) et après chromatographie sur gel de silice, l'acide est obtenu sous forme d'huile épaisse ( m = 3 g ; R = 56 % ).

### b) (E)-5-{3-[3-(4-Methoxycarbonyl-1-methyl-but-1-enyl)-phenyldisulfanyl]-phenyl}-hex-4-enoate de methyle

1,5 g d'acide (E)-5-(3-dimethylcarbamoylsulfanyl-phenyl)-hex-4-enoique sont dissous dans 30 mL d'un mélange 1:1 d'eau et d'éthanol. 400 mg de NaOH sont ajoutés, et le milieu réactionnel est chauffé à reflux pendant 18 heures. Le milieu est alors traité par une solution 1N de chlorure d'hydrogène, puis extrait avec de l'acétate d'éthyle. Les phases organiques sont alors rassemblées, séchées et concentrées sous pression réduite. Le résidu est alors dissous dans 30 mL de méthanol, et 1 mL d'acide sulfurique est ajouté. Le milieu réactionnel est alors agité à reflux pendant 15 heures, puis refroidi et traité avec de l'eau. Après extraction avec de l'acétate d'éthyle, séchage et concentration des phases organiques, le résidu est purifié par chromatographie sur gel de silice. Une huile jaune est obtenue ( m = 360 mg ; R = 30 % ).

### c) (E)-5-[3-(3,4-Bis-hydroxymethyl-benzylsulfanyl)-phenyl]-hex-4-enoate de methyle

200 mg (0,4 mmol) de (E)-5-{3-[3-(4-méthoxycarbonyl-1-méthyl-but-1-enyl)-phenyldisulfanyl]-phenyl}-hex-4-enoate de methyle et 370 mg (0,84 mmol) de 4-bromométhyl-phthalate de diméthyle sont dissous dans 10 mL de dichlorométhane. 140 mg (2,1 mmol) de poudre de zinc sont alors additionnés et 100 □L d'acide acétique sont ajoutés. Le milieu réactionnel est alors agité pendant 24 heures à température ambiante, puis traité avec de l'eau et extrait avec du dichlorométhane. Les phases organiques sont rassemblées, séchées et concentrées sous pression réduite. Après purification par chromatographie sur gel de silice, une huile jaune est obtenue ( m = 100 mg ; R = 40 % ).

### d) (E)-7-[3-(3,4-Bis-hydroxyméthyl-benzylsulfanyl)-phenyl]-3-éthyl-oct-6-en-3-ol

De manière analogue à l'exemple 83 (c), 140 mg (0,24 mmol) de (E)-5-[3-(3,4-bis-hydroxymethyl-benzylsulfanyl)-phenyl]-hex-4-enoate de methyle sont traités avec 1 mL (2 mmol) d'une solution de chlorure d'éthylmagnésium. Après purification sur colonne de silice, une huile incolore est obtenue ( m = 70 mg ; R = 70 % )
**RMN** ^{**1**}**H** (DMSO): 0,84 (t, 6H, J = 7,3 Hz), 1,37-1,48 (m, 6H), 1,98 (s, 3H), 2,14-2,19 (m, 2H), 3,94 (s, 1H), 4,28 (s, 2H), 4,53 (t, 4H, J = 5,2 Hz), 5,08 (t, 1H, J = 5,2 Hz), 5,14 (t, 1H, J = 5,2 Hz), 5,83 (t, 1 H), 7,20-7,48 (m, 7H).

### EXEMPLE 99

### (E)-7-{3-[(3,4-Bis-hydroxyméthyl-benzyl)-methylamino]-phenyl}-3-éthyl-oct-6-en-3-ol

### a) 4-({Methoxycarbonyl-[3-(2-methyl-[1,3]dioxolan-2-yl)-phenyl]-amino}-methyl)-phthalate de diméthyle

4 g (22,3 mmol) de 3-(2-methyl-[1,3]dioxolan-2-yl)-phenylamine sont dissous dans 30 mL de toluène anhydre. 1,85 mL (24 mmol) de chloroformate de méthyle et 1,57 g (24 mmol) de poudre de zinc sont suspendus dans 30 mL de toluène et agités à température ambiante.

La solution précédemment préparée est alors ajoutée goutte à goutte par l'intermédiaire d'une canule, puis le milieu réactionnel est agité pendant 1 heure. Le milieu réactionnel est alors filtré et le résidu solide rinçé avec de l'acétate d'éthyle. Les phases organiques sont lavées avec une solution saturée de bicarbonate de sodium puis séchées et concentrées sous pression réduite. Le résidu est dissous dans 50 mL de DMF anhydre et le mélange est refroidi à 0°C. 1,04 g (24 mmol) d'hydrure de sodium sont alors ajoutés et l'agitation est poursuivie pendant 30 minutes. Une solution de 4-bromométhyl-phthalate de diméthyle (26 mmol) dans 10 mL de DMF est alors ajoutée, le milieu réactionnel est agité à température ambiante pendant 2 heures puis traité par une solution saturée de chlorure d'ammonium et extraite à l'éther. Les phases organiques sont rassemblées, séchées et concentrées sous pression réduite. Le résidu est purifié par chromatographie sur colonne de silice. Une huile jaune est obtenue ( m = 6,5 g ; R = 67 % ).

### b) 1-{3-[(3,4-Bis-hydroxymethyl-benzyl)-methyl-amino]-phenyl}-ethanone

6,5 g de 4-({methoxycarbonyl-[3-(2-methyl-[1,3]dioxolan-2-yl)-phenyl)-amino}-methyl)-phthalate de diméthyle (14,6 mmol) sont dissous dans 200 mL de THF anhydre et le mélange est refroidi à 0°C. 2,8 g (74 mmol) d'hydrure double de lithium et d'aluminium sont ajoutés en trois portions et le mélange est chauffé à reflux pendant 4 heures. Après refroidissement, le milieu réactionnel est traité successivement par 2,8 mL d'eau, 2,8 mL de NaOH 15% et 8,4 mL d'eau. Le milieu est dilué par 200 mL d'éther éthylique, agité pendant 1 heure, puis filtré. Le filtrat est évaporé sous pression réduite et le résidu est dissous dans 100 mL de méthanol. 5 g de gel de silice sont ajoutés, ainsi que 100 DL d'acide sulfurique, et le milieu réactionnel est agité pendant 24 heures puis filtré, rincé avec une solution 0,5 M de NaOH. La phase organique est séchée et concentrée sous pression réduite. Une huile incolore est obtenue ( m = 3,98 mg ; R = 91 % ).

### c) 1-(3-{[3,4-Bis-(tert-butyl-dimethyl-silanyloxymethyl)-benzyl]-methyl-amino}-phenyl)-ethanone

3,89 g (13 mmol) de 1-{3-[(3,4-Bis-hydroxymethyl-benzyl)-methyl-amino]-phenyl}-ethanone sont dissous dans 20 mL de DMF anhydre. Le chlorure de tert-butyldiméthylsilane (4,7 g ; 31 mmol) est additionné puis 2,48 g (36,4 mmol) d'imidazole sont ajoutés. Le milieu est agité à température ambiante pendant 4 heures, puis dilué par 100 mL d'éther éthylique et filtré. Le filtrat est lavé par une solution de chlorure d'ammonium puis par de l'eau, et la phase organique est séchée puis concentrée sous pression réduite. Le résidu est purifié par chromatographie sur colonne de silice, pour obtenir une huile incolore très épaisse ( m = 5,83 g ; R = 85 %).

### d) Acide (E)-5-{3-[(3,4-Bis-(tert-butyl-dimethyl-silanyloxymethyl)-benzyl)-methyl amino]-phenyl}-hex-4-enoique

2,85 g (6,6 mmol) de bromure de (3-carboxypropyl)-triphénylphosphonium sont séchés sous vide à 130°C pendant 1 heure puis dissous dans 50 mL de THF anhydre. 1,48 g (13,3 mmol) de *tert*-butylate de potassium sont additionnés et le mélange rouge-orangé est agité pendant 15 minutes.

1 g (1,9 mmol) de 1-(3-{[3,4-Bis-(*tert*-butyl-dimethyl-silanyloxymethyl)-benzyl]-methyl-amino}-phenyl)-ethanone est dissous dans 10 mL de THF et ajouté goutte à goutte à la suspension précédente. Le milieu est alors agité pendant 5 heures puis traité par une solution 1N d'acide chlorhydrique et extrait avec de l'éther éthylique. Les phases organiques sont séchées et concentrées sous pression réduite. Le résidu est purifié par chromatographie sur colonne de silice. Une huile incolore est obtenue ( m = 570 mg ; R = 49 % ).

### e) (E)-7-{3-[(3,4-Bis-hydroxyrnéthyl-benzyl)-methylamino]-phenyl}-3-éthyl-oct-6-en-3-ol

550 mg (0,9 mmol) d'acide (E)-5-{3-[(3,4-Bis-(*tert*-butyl-dimethyl-silanyloxymethyl)-benzyl)-methyl amino]-phenyl}-hex-4-enoique sont dissous dans 20 mL de méthanol anhydre puis 0,5 mL d'acide sulfurique sont ajoutés et le mélange est chauffé à reflux pendant 24 heures. Le milieu réactionnel est alors traité avec de l'eau et extrait avec du dichlorométhane. Les phases organiques sont séchées puis concentrées sous pression réduite. Le résidu est dissous dans 10 mL de THF et traité par 1,5 mL (4,5 mmol) d'une solution de bromure d'éthylmagnésium. Le milieu réactionnel est agité à température ambiante pendant 1 heure puis traité par une solution de chlorure d'ammonium et extrait avec de l'acétate d'éthyle. Les phases organiques sont séchées et concentrées sous pression réduite et le résidu est purifié par chromatographie sur colonne de silice. Une huile incolore est obtenue ( m = 277 mg; R = 71 %).
**RMN** ^{**1**}**H** (DMSO): 0,66 (t, 6H, J = 7,3 Hz), 1,19-1,28 (m, 6H), 1,76 (s, 3H), 1,90-2,00 (m, 2H), 3,21 (s, 3H), 4,09 (d, 2H, J = 5,7 Hz), 4,37 (t, 4H, J = 5,2 Hz), 4,88 (t, 1H, J = 5,2 Hz), 4,94 (t, 1H, J = 5,2 Hz), 5,60 (t, 1H), 6,05 (t, 1H), 6,30 (d, 1H), 6,48 (d, 1H), 6,55 (s, 1H), 6,81 (t, 1H), 7,08 (d, 1H), 7,16 (d, 1H), 7,27 (s, 1H).

### EXEMPLE 100

### (E)-6-[3-(3,4-Bis-hydroxyméthyl-benzyloxy)-phenyl]-3-ethyl-6-methyl-hept-4-en-3-ol

### a) 2-[3-(tert-Butyl-dimethyl-silanyloxy)-phenyl]-2-methyl-propionate de méthyle

10 g de [3-(*tert*-Butyl-dimethyl-silanyloxy)-phenyl]-acetate de méthyle (35,6 mmol) sont dissous dans 200 mL de THF anhydre. 35,6 mL d'une solution 2,0 M de diisopropylamidure de lithium sont alors additionnés puis le milieu est agité pendant 30 minutes. 8,9 mL (142 mmol) d'iodure de méthyle sont ajoutés et le milieu réactionnel est agité à température ambiante pendant 40 heures. Après traitement par une solution de chlorure d'ammonium puis extraction avec de l'éther éthylique, les phases organiques sont rassemblées, lavées avec une solution saturée de chlorure de sodium, séchées et concentrées sous pression réduite. Une huile incolore est obtenue ( m = 10,8 g ; R = 98 % ).

### b) 2-[3-(tert-Butyl-dimethyl-silanyloxy)-phenyl]-2-methyl-propan-1-ol

10,8 g de 2-[3-(*tert*-Butyl-dimethyl-silanyloxy)-phenyl]-2-methyl-propionate de méthyle (35 mmol) sont dissous dans 150 mL de THF anhydre. 4 portions de 500 mg chacune d'hydrure double de lithium et d'aluminium (52,5 mmol) sont ajoutées et le milieu est agité à température ambiante pendant 3 heures. Après traitement, successivement, par 2 mL d'eau, 2 mL de NaOH 15% puis 6 mL d'eau, le milieu réactionnel est dilué par addition de 200 mL d'éther éthylique puis filtré, et le filtrat concentré sous pression réduite. Une hule incolore est obtenue ( m = 9,7 g ; R = 99 % ).

### c) (E)-4-[3-(tert-Butyl-dimethyl-silanyloxy)-phenyl]-4-methyl-pent-2-enoate d'éthyle

4,04 mL (43,3 mmol) de chlorure d'oxalyle sont dissous dans 150 mL de dichlorométhane puis le mélange est refroidi à -78°C. Une solution de 6,58 mL (92,7 mmol) de DMSO dans 20 mL de dichlororméthane est alors additionnée lentement. Lorsque l'évolution de gaz est achevée (après environ 15 minutes), une solution de 6,5 g (23,1 mmol) de 2-[3-(*tert*-butyl-dimethyl-silanyloxy)-phenyl]-2-methyl-propan-1-ol et de 3,3 mL de triéthylamine (23 mmol) dans 50 mL de dichlorométhane est additionnée goutte à goutte. Après 20 minutes, 22,5 mL (162 mmol) de triéthlamine sont ajoutés puis le milieu réactionnel est ramené à température ambiante et agité pendant 1 heure. Le milieu est alors traité avec une solution saturée de chlorure d'ammonium puis extrait avec de l'éther éthylique. La phase organique est lavée à l'eau, séchée et concentrée sous pression réduite. Le résidu obtenu est ensuite dissous dans 100 mL de THF anhydre.

Dans un autre ballon, 9,2 mL (46,3 mmol) de triéthylphosphonoacétate sont dissous dans 100 mL de THF anhydre, puis le mélange est refroidi à 0°C. 22 mL (44 mmol) d'une solution 2,0 M de diisopropylamidure de lithium est alors ajoutée et le milieu est agité à 0°C pendant 30 minutes. La solution préparée précédemment est alors ajoutée à l'aide d'une canule et le milieu réactionnel est ramené à température ambiante puis agité pendant 15 heures. Après traitement par une solution saturée de chlorure d'ammonium puis extraction avec de l'éther éthylique, les phases organiques sont rassemblées, séchées et concentrées sous pression réduite. Le résidu est purifié par chromatographie sur colonne de silice. Une huile incolore est obtenue ( m = 6,28 g ; R = 78% ).

### d) (E)-4-(3-Hydroxy-phenyl)-4-methyl-pent-2-enoate d'éthyle

400 mg (1,14 mmol) de (E)-4-[3-(*tert*-Butyl-dimethyl-silanyloxy)-phenyl]-4-methyl-pent-2-enoate d'éthyle est dissous dans 20 mL de THF anhydre, puis 1,5 mL d'une solution de fluorure de tétrabutylammonium (1M dans le THF) (1,5 mmol) est additionné. Le milieu réactionnel est immédiatement concentré sous pression reduite et purifié par chromatographie. Une huile incolore est obtenue ( m = 262 mg ; R = 98% ).

### e) (E)-6-[3-(3,4-Bis-hydroxyrnéthyl-benzyloxy)-phenyl]-3-ethyl-6-methyl-hept-4-en-3-ol

De manière analogue à l'exemple 83 (b, c), à partir de 262 mg de (E)-4-(3-Hydroxy-phenyl)-4-methyl-pent-2-enoate d'éthyle (1,12 mmol), on obtient le (E)-6-[3-(3,4-Bis-hydroxyrnéthyl-benzyloxy)-phenyl]-3-ethyl-6-methyl-hept-4-en-3-ol sous la forme d'une huile incolore ( m = 355 mg ; R = 79 % ).
**RMN** ^{**1**}**H** (DMSO): 0,71 (t, 6H, J = 7,3 Hz), 1,25 (s, 6H), 1,36 (q, 4H, J = 7,3 Hz), 4,03 (s, 1H), 4,47 (t, 4H, J = 4,7 Hz), 4,96 (s, 2H), 5,02 (t, 1H, J = 4,7 Hz), 5,06 (t, 1H, J = 4,7 Hz), 5,23 (d, 1H, J = 15,9 Hz), 5,64 (d, 1H, J = 15,9 Hz), 6,72-6,87 (m, 3H), 7,13 (t, 1H, J = 7,8 Hz) 7,22 (d, 1H, 7,7 Hz), 7,31 (d, 1H, J = 7,7 Hz), 7,40 (s, 1H).

### EXEMPLE 101

### 7-[3-(3,4-Bis-hydroxyméthyl-benzyloxy)-phenyl]-3-ethyl-7-methyl-octan-3-ol

### a) 4-[3-(tert-Butyl-dimethyl-silanyloxy)-phenyl]-4-methyl-pentan-1-ol

6,15g (17,6 mmol) de (E)-4-[3-(*tert*-Butyl-dimethyl-silanyloxy)-phenyl]-4-methyl-pent-2-enoate d'éthyle (101 c) sont dissous dans 150 mL de methanol anhydre et le mélange est refroidi à 0°C. 1,28 g (52,8 mmol) de copeaux de magnésium sont ajoutés et le milieu réactionnel est agité pendant 4 heures à 0°C. Après traitement par une solution saturée de chlorure d'ammonium puis extraction avec de l'acétate d'éthyle, les phases organiques sont rassemblées, séchées et concentrées sous pression réduite. Le résidu est alors dissous dans 100 mL de THF anhydre et 1 g (26,4 mmol) d'hydrure double de lithium et d'aluminium est ajouté. Après 1 heure d'agitation à température ambiante, le milieu est traité sucessivement par 1 mL d'eau, 1 mL de NaOH 15%, 3 mL d'eau et dilué par addition de 150 mL d'éther éthylique. Après filtration, le filtrat est concentré sous pression réduite. Une huile incolore est obtenue ( m = 3,8 g ; R = 70 %).

### b) tert-Butyl-[3-(4-[1,3]dithian-2-ylidene-1,1-dimethyl-butyl)-phenoxy]-dimethyl-silane

1,94 mL (22,2 mmol) de chlorure d'oxalyle sont dissous dans 100 mL de dichlorométhane, puis le mélange est refroidi à -78°C. Une solution de 3,15 mL (44,4 mmol) de DMSO dans 10 mL de dichlororméthane est alors additionnée lentement. Lorsque l'évolution de gaz est achevée (après environ 15 minutes), une solution de 3,43 g (11,1 mmol) de 4-[3-(*tert*-Butyl-dimethyl-silanyloxy)-phenyl]-4-methyl-pentan-1-ol et de 1,6 mL de triéthylamine (11,1 mmol) dans 50 mL de dichlorométhane est additionnée goutte à goutte. Après 20 minutes, 10,8 mL (77,7 mmol) de triéthylamine sont ajoutés, puis le milieu réactionnel est ramené à température ambiante et agité pendant 1 heure. Le milieu est alors traité avec une solution saturée de chlorure d'ammonium, puis extrait avec de l'éther éthylique. La phase organique est lavée avec deux fractions de chlorure d'ammonium puis avec de l'eau, séchée et concentrée sous pression réduite. Le résidu obtenu est ensuite dissous dans 100 mL de THF anhydre.

Dans un autre ballon, 2,31 mL (12,2 mmol) de 2-triméthylsilyl-1,3-dithiane est dissous dans 50 mL de THF anhydre et le mélange est refroidi à -78°C. 4,88 mL d'une solution 2,5 M de butyllithium (12,2 mmol) sont additionnés. Le milieu réactionnel est agité pendant 30 minutes à -78°C, puis la solution précédemment préparée est additionnée goutte à goutte. Le milieu est encore agité 4 heures à -78°C, puis ramené à température ambiante et traité par une solution de chlorure d'ammonium. Après extraction par de l'éther éthylique, les phases organiques sont rassemblées, séchées et concentrées sous pression réduite. Le résidu est purifié par chromatographie sur colonne de silice. Une huile jaune est obtenue ( m = 3,54 g ; R = 78 %).

### c) 5-(3-Hydroxy-phenyl)-5-methyl-hexanoate de méthyle

3,4 g (8,4 mmol) de *tert*-butyl-[3-(4-[1,3]dithian-2-ylidene-1,1-dimethyl-butyl)-phenoxy]-dimethyl-silane sont dissous dans 200 mL d'acétonitrile et 50 mL d'eau. 2,1 g (21 mmol) de carbonate de calcium, puis 5 g (18,5 mmol) de dichlorure de mercure sont ajoutés. Après 48 heures d'agitation à température ambiante, le milieu réactionnel est traité par une solution saturée de chlorure d'ammonium, puis extrait avec de l'acétate d'éthyle. Les phases organiques sont rassemblées, puis lavées par une solution d'acide chlorhydrique 1N et par de l'eau, et enfin séchées et concentrées sous pression réduite. Le résidu est dissous dans 150 mL de méthanol, et 2 mL d'acide sulfurique sont ajoutés. Le milieu réactionnel est chauffé à reflux pèndant 15 heures, puis traité avec de l'eau et extrait avec de l'éther éthylique. Les phases organiques sont rassemblées, séchées et concentrées sous pression réduite. Le résidu est purifié par chromatographie sur colonne de silice. Une huile incolore est obtenue ( m = 1,1 g ; R = 56 % ).

### d) 7-[3-(3,4-Bis-hydroxyméthyl-benzyloxy)-phenyl]-3-ethyl-7-methyl-octan-3-ol

De manière analogue à l'exemple 100 (é), 1 g de 5-(3-Hydroxy-phenyl)-5-methyl-hexanoate de méthyle (4,2 mmol) sont convertis en 7-[3-(3,4-Bis-hydroxyméthyl-benzyloxy)-phenyl]-3-ethyl-7-methyl-octan-3-ol, sous la forme d'un solide cristallin blanc (PF= 88-89°C ; m = 700 mg ; R = 40 % ).
**RMN** ^{**1**}**H** (DMSO): 0,50 (t, 6H, J = 7,3 Hz), 0,78-0,84 (m, 2H), 0,96-1,07 (m, 12H), 1,30-1,36 (m, 2H), 3,50 (s, 1 H), 4,35 (t, 4H, J = 4,9 Hz), 4,86 (s, 2H), 4,90 (t, 1 H, J = 4,9 Hz), 4,94 (t, 1H, J = 4,9 Hz), 6,60-6,73 (m, 3H), 7,01 (t, 1H, J = 7,8 Hz) 7,11 (d, 1H, 7,7 Hz), 7,20 (d, 1H, J = 7,7 Hz), 7,29 (s, 1H).

### EXEMPLE 102

### (E)-3-Ethyl-7-[3-(4-hydroxyméthyl-3-méthyl-phenoxyméthyl)-phenyl]-non-6-en-3-ol

### a) 4-Iodo-3-méthyl-phenol

15 g (122 mmol) de 4-hydroxy-2-méthylaniline sont dissous dans 180 mL d'acide sulfurique 20% puis le milieu réactionnel est refroidi à 0°C. Une solution de nitrite de sodium (11,3 g, 163 mmol) dans 60 mL d'eau est alors additionnée goutte à goutte puis le milieu est agité pendant 20 minutes. Cette solution est alors lentement additionnée à une solution à 0°C de Cul (32,5 g, 170 mmol) et KI (31,9 g, 193 mmol) dans 180 mL d'acide sulfurique 20%. Le milieu réactinnel est chauffé à reflux pendant 3 heures, versé dans 1 L d'eau et extrait avec de l'éther éthylique. Les phases organiques sont lavées avec une solution saturée de thiosulfate de sodium, d'eau, séchées sur sulfate de magnésium et concentrées sous pression réduite. Après purification sur colonne de silice (acétate d'éthyle 30 - heptane 70), une huile brune est obtenue ( m = 4,2 g ; R = 15 % ).

### b) 4-hydroxy-2-méthylbenzoate de méthyle.

4,2 g (18 mmol) de 4-Iodo-3-méthylphenol sont dissous dans 90 mL de methanol anhydre puis placé dans un réacteur en acier. 5 mL (35 mmol) de triéthylamine et 400 mg (1,8 mmol) de diacétate de palladium sont additionnés, le milieu réactionnel est soumis à une pression de monoxyde de carbone de 3 bars et chauffé à 80 °C pendant 5 heures. Le milieu est alors ramené à pression et température ambiantes puis dissous dans du dichlorométhane et filtré sur célite. Après évaporation, le résidu est purifié par chromatographie sur colonne de silice. Une poudre orange est obtenue ( m = 1,8 g, R = 61 %).

### c) (E)-3-Ethyl-7-[3-(4-hydroxyméthyl-3-méthyl-phenoxyméthyl)-phenyl]-non-6-en-3-ol

De manière identique aux exemples 97 e, f, g, par réaction de 4-hydroxy-2-méthyl-benzoate de méthyle avec [3-((E)-5-Éthyl-1-méthyl-5-triéthylsilanyloxy-hept-1-enyl)-phenyl]-méthanol (obtenu à l'exemple 97 d) puis traitement successif avec de l'hydrure double de lithium et d'aluminium puis avec du fluorure de tétrabutylammonium, une huile épaisse blanche est obtenue..
**RMN** ^{**1**}**H** (DMSO): 0,64 (t, 6H, J = 7,5 Hz), 0,77 (t, 3H, J = 7,3 Hz), 1,18-1,29 (m, 6H), 1,94-2,03 (m, 2H), 2,07 (s, 3H), 2,28-2,38 (m, 2H), 3,74 (s, 1H), 4,24 (d, 2H, J = 5,2 Hz), 4,73 (t, 1H, J = 5,2 Hz), 4,90 (s, 2H), 5,52 (t, 1H, J = 7,3 Hz), 6,60-6,65 (m, 2H), 7,03-7,17 (m, 4H), 7,26 (s, 1 H).

### EXEMPLE 103(E)-3-Ethyl-7-[3-(3-hydroxyméthyl-4-méthyl-phenoxyméthyl)-phenyl]-non-6-en-3-ol

### a) 2-méthyl-5-nitrobenzoate de méthyle

23,2 g (88 mmol) de 2-Iodo-4-nitrotoluène sont dissous dans 400 mL de methanol anhydre puis placé dans un réacteur en acier. 25 mL (177 mmol) de triéthylamine, 1,97 g (8,8 mmol) de diacétate de palladium et 7,3 g de diphényphosphinopropane sont additionnés et le milieu réactionnel est soumis à une pression de monoxyde de carbone de 4 bars et chauffé à 110 °C pendant 18 heures. Le milieu est alors ramené à pression et température ambiantes, dissous dans du dichlorométhane et filtré sur célite. Après évaporation, le résidu est purifié par chromatographie sur colonne de silice. Une poudre orange est obtenue ( m = 6,3 g, R = 37%).

### b) 3-amino-6-méthylbenzoate de méthyle

6,3 g (32 mmol) de 2-méthyl-5-nitrobenzoate de méthyle sont dissous dans 80 mL de méthanol anhydre puis transféres dans un réacteur en acier. Le mélange est dégasé avec de l'azote puis 630 mg de Pd/C (5%) sont ajoutés. Le milieu réactionnel est alors soumis à une pression d'hydrogène de 4 bars et à une température de 80°C pendant 14 heures puis ramené à pression et température ambiantes et filtré. Le résidu, après évaporation, est purifié par chromatographie sur colonne de silice. Une huile brune est obtenue ( m = 4,6 g, R = 86%).

### c) 3-hydroxy-6-méthyl-benzoate de méthyle.

4,6 g (28 mmol) de 3-amino-6-méthylbenzoate de méthyle sont dissous dans 50 mL de THF et 50 mL d'acide sulfurique 1M puis le milieu réactionnel est refroidi à 0°C. Une solution de nitrite de sodium (2,3 g, 33,6 mmol) dans 10 mL d'eau est alors additionnée goutte à goutte puis le milieu est agité pendant 20 minutes. 15 mL d'acide sulfurique pur sont alors additionnés et le milieu réactionnel est chauffé à reflux pendant 2 heures, versé dans 500 mL d'eau et extrait avec de l'éther éthylique. Les phases organiques sont séchées, concentrées sous pression réduite et le résidu obtenu est dissous dans 100 mL de méthanol. 3 mL d'acide sulfurique sont ajoutés et le milieu réactionnel est porté à reflux pendant 14 heures, refroidi, traité avec de l'eau et de l'éther éthylique. Après extraction avec de l'éther éthylique, les phases organiques sont rassemblées, séchées sur sulfate de magnésium et concentrées sous pression réduite. Après purification sur colonne de silice (acétate d'éthyle 30 - heptane 70), une huile brune est obtenue ( m = 1,2 g ; R = 25 % ).

### c) (E)-3-Ethyl-7-[3-(3-hydroxyméthyl-4-méthyl-phenoxyméthyl)-phenyl]-non-6-en-3-ol

De manière identique aux exemples 97 e, f, g, par réaction de 3-hydroxy-6-méthyl-benzoate de méthyle avec [3-((E)-5-Éthyl-1-méthyl-5-triéthylsilanyloxy-hept-1-enyl)-phenyl]-méthanol (obtenu à l'exemple 97 d) puis traitement successif avec de l'hydrure double de lithium et d'aluminium puis avec du fluorure de tétrabutylammonium, une huile incolore est obtenue.
**RMN** ^{**1**}**H** (DMSO): 0,73 (t, 6H, J = 7,5 Hz), 0,85 (t, 3H, J = 7,3 Hz), 1,26-1,38 (m, 6H), 2,02-2,12 (m, 5H), 2,38-2,46 (m, 2H), 3,82 (s, 1H), 4,36 (d, 2H, J = 5,4 Hz), 4,98-5,03 (m, 3H), 5,61 (t, 1H, J = 7,2 Hz), 6,68-6,72 (m, 1H), 6,93-6,96 (m, 2H), 7,22-7,25 (m, 3H), 7,35 (s, 1H).

### EXEMPLE 104

### (E)-7-[3-(3,4-Bis-hydroxyméthyl)-phenoxyméthyl)-phenyl]-3-ethyl-oct-6-en-3-ol

De manière analogue à l'exemple 97, le (E)-7-[3-(3,4-Bis-hydroxyméthyl)-phenoxyméthyl)-phenyl]- 3-ethyl-oct-6-en-3-ol est obtenu sous forme d'une poudre blanche (pf = 89-91 °C).
**RMN** ^{**1**}**H** (DMSO): 0,67 (t, 6H, J = 7,4 Hz), 1,21-1,33 (m, 6H), 1,86 (d, 3H, J = 2,2 Hz), 1,94-2,06 (m, 2H), 3,78 (s, 1 H), 4,30 (d, 2H, J = 5,3 Hz), 4,40 (d, 2H, J = 5,3 Hz), 4,80 (t, 1H, J = 5,3 Hz), 4,95-5,00 (m, 3H), 5,70 (t, 1H, J = 7,4 Hz), 6,69-6,73 (m, 1H), 6,94-6,95 (m, 1H), 7,09-7,20 (m, 4H), 7,33 (s, 1H).

### EXEMPLE 105

### (E)-3-Ethyl-7-[3-(3-hydroxyméthyl -phenoxyméthyl)-phenyl]-non-6-en-3-ol

De manière identique aux exemples 97 e, f, g, par réaction de 3-hydroxybenzaldehyde avec [3-((E)-5-Éthyl-1-méthyl-5-triéthylsilanyloxy-hept-1-enyl)-phenyl]-méthanol (obtenu à l'exemple 97 d), puis traitement successif avec de l'hydrure double de lithium et d'aluminium puis avec du fluorure de tétrabutylammonium, une huile incolore est obtenue.
**RMN** ^{**1**}**H** (CDCl₃): 0,88 (t, 6H, J = 7,5 Hz), 0,98 (t, 3H, J = 7,3 Hz), 1,26 (s, 1H), 1,48-1,60 (m, 6H), 2,17-2,27 (m, 2H), 2,53 (q, 2H, J = 7,3 Hz), 4,62 (s, 2H), 5,06 (s, 2H), 5,65 (t, 1H, J = 7,2 Hz), 6,95-7,00 (m, 2H), 7,26-7,31 (m, 5H), 7,39(s, 1H).

### EXEMPLE 106

### (E)-3-Ethyl-7-[3-(4-hydroxyméthyl -phenoxyméthyl)-phenyl]-non-6-en-3-ol

De manière identique aux exemples 97 e, f, g, par réaction de 4-hydroxybenzaldehyde avec [3-((E)-5-Éthyl-1-méthyl-5-triéthylsilanyloxy-hept-I-enyl)-phenyl]-méthanol (obtenu à l'exemple 97 d), puis traitement successif avec de l'hydrure double de lithium et d'aluminium puis avec du fluorure de tétrabutylammonium, une poudre blanche (pf = 67-70 °C) est obtenue.
**RMN** ^{**1**}**H** (CDCl₃): 0,86 (t, 6H, J = 7,5 Hz), 0,98 (t, 3H, J = 7,3 Hz), 1,26 (s, 1H), 1,47-1,60 (m, 6H), 2,17-2,27 (m, 2H), 2,53 (q, 2H, J = 7,3 Hz), 4,66 (s, 2H), 5,06 (s, 2H), 5,65 (t, 1H, J = 7,2 Hz), 6,89-7,03 (m, 2H), 7,24-7,30 (m, 5H), 7,40 (s, 1H).

### EXEMPLE 107 : EXEMPLES DE FORMULATION

### 1) VOIE ORALE

### (a) On prépare la composition suivante sous la forme d'un comprimé de 0,2 g

| | |
|---|---|
| Composé de l'exemple 1 | 0,005 g |
| Amidon prégélatinisé | 0,065 g |
| Cellulose microcristalline | 0,075 g |
| Lactose | 0,050 g |
| Stéarate de magnésium | 0,005 g |

Pour le traitement de l'ichtyose, on administre à un individu adulte 1 à 3 comprimés par jour pendant 1 à 12 mois selon la gravité du cas traité.

### (b) On prépare une suspension buvable, destinée à être conditionnée en ampoules de 5 ml

| | |
|---|---|
| Composé de l'exemple 2 | 0,050 mg |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

Pour le traitement de l'acné, on administre à un individu adulte 1 ampoule par jour pendant 1 à 12 mois selon la gravité du cas traité.

### (c) On prépare la formulation suivante destinée à être conditionnée en gélules :

| | |
|---|---|
| Composé de l'exemple 4 | 0,0001 mg |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administre à un individu adulte, 1 gélule par jour pendant 1 à 12 mois.

### (d) On prépare la formulation suivante destinée à être conditionnée en gélules :

| | |
|---|---|
| Composé de l'exemple 5 | 0,02 mg |
| Cyclosporine | 0,050 g |
| Amidon de maïs | 0,060 g |
| Lactose q.s.p | 0,300 g |

Les gélules utilisées sont constituées de gélatine, d'oxyde de titane et d'un conservateur.

Dans le traitement du psoriasis, on administre à un individu adulte, 1 gélule par jour pendant 1 à 12 mois.

### 2) VOIE TOPIQUE

### (a) On prépare la crème Eau-dans l'Huile non ionique suivante :

| | |
|---|---|
| Composé de l'exemple 9 | 0,100 g |
| Mélange d'alcools de lanoline émulsifs, de cires et d'huiles raffinés, vendu par la Société BDF sous la dénomination "Eucérine anhydre" | 39,900, g |
| Parahydroxybenzoate de méthyle | 0,075 g |
| Parahydroxybenzoate de propyle | 0,075 g |
| Eau déminéralisée stérile q.s.p | 100,000 g |

Cette crème est appliquée sur une peau psoriatique 1 à 2 fois par jour pendant 1 à 12 mois.

### (b) On prépare un gel en réalisant la formulation suivante :

| | |
|---|---|
| Composé de l'exemple 18 | 0,001 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.

### (c) On prépare une lotion antiséborrhéique en procédant au mélange des ingrédients suivants :

| | |
|---|---|
| Composé de l'exemple 12 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.

### (d) On prépare une composition cosmétique contre les effets néfastes du soleil en procédant au mélange des ingrédients suivants :

| | |
|---|---|
| Composé de l'exemple 27 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p | 100,000 g |

Cette composition est appliquée quotidiennement, elle permet de lutter contre le vieillissement photoinduit.

### (e) On prépare la crème Huile dans l'Eau non ionique suivante :

| | |
|---|---|
| Composé de l'exemple 38 | 0,500 g |
| Acide rétinoique | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 jours pour traitement d'attaque et indéfiniment pour entretien.

### (f) On prépare un gel topique en procédant au mélange des ingrédients suivants :

| | |
|---|---|
| Composé de l'exemple 48 | 0,050 g |
| Ethanol | 43,000 g |
| α -tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel est appliqué dans le traitement de l'acné 1 à 3 fois par jour pendant 6 à 12 semaines selon la gravité du cas traité.

### (g) On prépare une lotion capillaire anti-chute et pour la repousse des cheveux en procédant au mélange des ingrédients suivants :

| | |
|---|---|
| Composé de l'exemple 33 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 1 à 2 fois par jour pendant 3 mois sur un cuir chevelu ayant subi une chute de cheveu et indéfiniment pour traitement d'entretien.

### (h) On prépare une crème anti-acnéique en procédant au mélange des ingrédients suivants :

| | |
|---|---|
| Composé de l'exemple 50 | 0,050 g |
| Acide rétinoïque | 0,010 g |
| Mélange de stéarates de glycérol et de polyéthylène glycol (75 moles) vendu sous le nom de "Gelot 64" ............par la société "GATTEFOSSE" | 15,000 g |
| Huile de noyau polyoxyéthylénée à 6 moles d'oxyde .....d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.

### (i) On prépare une crème huile dans l'eau en réalisant la formulation suivante :

| | |
|---|---|
| Composé de l'exemple 43 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000 g |

Cette crème est appliquée 2 fois par jour sur une peau atteinte de dermatose inflammatoire pendant 30 jours.

### (j) On prépare la crème de type huile dans l'eau suivante :

| | |
|---|---|
| Acide lactique | 5,000 g |
| Composé de l'exemple 8 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" ........par la société "DYNAMIT NOBEL" | 4,000 g |
| Eau q.s.p | 100,000 g |

Cette crème est appliquée 1 fois par jour, elle aide à lutter contre le vieillissement qu'il soit photoinduit ou chronologique.

### (k) On prépare l'onguent anhydre suivant :

| | |
|---|---|
| Composé de l'exemple 19 | 5,000 g |
| Huile de vaseline | 50,00 g |
| butylhydrotoluène | 0,050 g |
| vaseline blanche | qs 100 g |

Cet onguent est appliqué 2 fois par jour sur une peau atteinte de dermatose squameuse pendant 30 jours.

### 3) VOIE INTRALESIONNELLE

### (a) On prépare la composition suivante :

| | |
|---|---|
| Composé de l'exemple 6 | 0,002 g |
| Oléate d'éthyle | qs 10 g |

Dans le traitement du mélanome malin, on injecte la composition à un individu adulte à une fréquence de 1 à 7 fois par semaine pendant 1 à 12 mois.

### (b) On prépare la composition suivante :

| | |
|---|---|
| Composé de l'exemple 11 | 0,050 g |
| Huile d'olive | qs 2 g |

Dans le traitement du carcinome basocellulaire, on injecte la composition à un individu adulte à une fréquence de 1 à 7 fois par semaine pendant 1 à 12 mois.

### (c) On prépare la composition suivante :

| | |
|---|---|
| Composé de l'exemple 36 | 0,1 mg |
| Huile de sésame | qs 2 g |

Dans le traitement du carcinome spinocellulaire, on injecte la composition à un individu adulte à une fréquence de 1 à 7 fois par semaine pendant 1 à 12 mois.

### (d) On prépare la composition suivante :

| | |
|---|---|
| Composé de l'exemple 24 | 0,001mg |
| Benzoate de méthyle | qs 10 g |

Dans le traitement du carcinome du colon, on injecte la composition à un individu adulte à une fréquence de 1 à 7 fois par semaine pendant 1 à 12 mois.

### 4) VOIE INTRAVEINEUSE

### (a) On prépare l'émulsion lipidique injectable suivante :

| | |
|---|---|
| Composé de l'exemple 46 | 0,001mg |
| Huile de soja | 10,000 g |
| Phospholipide d'oeuf | 1,200 g |
| Glycérine | 2,500 g |
| Eau pour injectable q.s.p | 100,000 g |

Dans le traitement du psoriasis, on injecte la composition à un individu adulte à une fréquence de 1 à 7 fois par semaine pendant 1 à 12 mois.

### (b) On prépare l'émulsion lipidique injectable suivante :

| | |
|---|---|
| Composé de l'exemple 13 | 0,010 g |
| Huile de coton | 10,000 g |
| Lécithine de soja | 0,750 g |
| Sorbitol | 5,000 g |
| DL,α Tocophérol | 0,100 g |
| Eau pour injectable q.s.p | 100,000 g |

Dans le traitement de l'ichtyose, on injecte la composition à un individu adulte à une fréquence de 1 à 7 fois par semaine pendant 1 à 12 mois.

### (c) On prépare l'émulsion lipidique injectable suivante :

| | |
|---|---|
| Composé de l'exemple 29 | 0,001 g |
| Huile de soja | 15,000 g |
| Monoglycérides acétylés | 10,000 g |
| Pluronic F-108 | 1,000 g |
| Glycérol | 2,500 g |
| Eau pour injectable q.s.p | 100,000 g |

Dans le traitement de la leucémie, on injecte la composition à un individu adulte à une fréquence de 1 à 7 fois par semaine pendant 1 à 12 mois.

### (d) On prépare la composition micelle mixte suivante :

| | |
|---|---|
| Composé de l'exemple 22 | 0,001 g |
| Lécithine | 16,930 g |
| Acide glycocholique | 8,850 g |
| Eau pour injectable q.s.p | 100,000 g |

Dans le traitement du mélanome malin, on injecte la composition à un individu adulte à une fréquence de 1 à 7 fois par semaine pendant 1 à 12 mois.

### (e) On prépare la composition de cyclodextrine suivante :

| | |
|---|---|
| Composé de l'exemple 31 | 0,1mg |
| βcyclodextrine | 0,100 g |
| Eau pour injectable q.s.p | 10,000 g |

Dans le traitement du rejet de greffe, on injecte la composition à un individu adulte à une fréquence de 1 à 7 fois par semaine pendant 1 à 12 mois.

### (f) On prépare la composition de cyclodextrine suivante :

| | |
|---|---|
| Composé de l'exemple 4 | 0,010 g |
| 2-hydroxypropylβcyclodextrine | 0,100 g |
| Eau pour injectable q.s.p | 10,000 g |

Dans le traitement du cancer du rein, on injecte la composition à un individu adulte à une fréquence de 1 à 7 fois par semaine pendant 1 à 12 mois.

### EXEMPLE 108 : Exempte de test d'évaluation de l'activité biologique des composés de l'invention

L'activité agoniste aux récepteurs de la vitamine D des composés de l'invention peut être également évaluée "in vivo" par induction de la 24-Hydroxylase chez la souris SKH. (Voorhees and al. 1997.108 : 513-518).

Le protocole de test utilisé est le suivant :

Des souris SKH reçoivent une application topique unique d'un composé selon l'invention en solution dans l'éthanol à des concentrations croissantes. Un volume de 50 µl du produit à tester ou du véhicule seul est appliqué sur le dos des souris à l'aide d'une pipette.

D'autres souris SKH reçoivent une application topique unique de 1,25 (OH)2 vitamine D3 en solution dans l'éthanol à des concentrations croissantes. Un volume de 50 µl du produit à tester ou du véhicule seul est appliqué sur le dos des souris à l'aide d'une pipette.

8 heures après l'application topique, les souris sont euthanasiées, la peau traitée est prélevée et l'épiderme est séparé du derme. La quantification de l'ARNm de la 24-Hydroxylase est réalisée par PCR semi quantitative. Les résultats sont normalisés par rapport à l'expression de l'ARNm de GAPDH et les valeurs pour les différentes concentrations de 1,25 (OH)₂ vitamine D3 testées et pour les différents composés de l"invention testés sont exprimés en facteur d'induction par rapport au véhicule.

Les résultats sont résumés dans le tableau suivant :

| Expression de l'ARNm de la 24-Hydroxylase | | |
|---|---|---|
| **Composé testé** | **concentration % (poids/volume)** | **Facteur d'induction versus éthanol** |
| 1,25 (OH)₂ vitamine D3 | 0,0001 | 6,7 |
| 1,25 (OH)₂ vitamine D3 | 0,001 | 10,3 |
| 1,25 (OH)₂ vitamine D3 | 0,01 | 20,1 |
| 1,25 (OH)₂ vitamine D3 | 0,1 | 26 |
| Exemple 4 | 1 | 11 |
| Exemple 58 | 0,1 | 20,5 |
| Exemple 60 | 1 | 10 |
| Exemple 64 | 1 | 15 |
| Exemple 67 | 0,1 | 25 |
| Exemple 71 | 0,1 | 17 |
| exemple 81 | 0,1 | 21 |

Ces résultats montrent que la 1,25 (OH)₂ vitamine D3 administrée en application topique unique induit chez la souris de façon dose-dépendante l'expression de l'ARNm de la 24 - hydroxylase dans l'épiderme.

L'activité biologique des composés de l'invention est évaluée par comparaison entre les facteurs d'inductions obtenus pour les composés de l'invention et les facteurs d'inductions obtenus pour la 1,25 (OH)₂ vitamine D3.

Ainsi, le composé 6-[3-(3,4-Bis-hydroxymethyl-benzyloxy)-phenyl]-2-ethyl-hepta -3,5-dien-2-ol (exemple 4) présente à la concentration de 1 % une activité comparable à celle de la 1,25 (OH)₂ vitamine D3 à 0.001 %.

## Revendications

1. Composés, **caractérisés par le fait qu'**ils répondent à la formule générale (I) suivante : 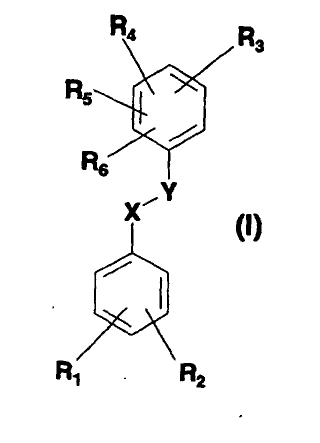 dans laquelle:
- R₁ représente un atome d' hydrogène, un radical méthyl ou un radical -(CH₂)ₙ-OR₇,
- R₂ représente un radical -(CH₂)ₙ-OR₈,
n, R₇ et R₈ ayant les significations données ci-après,
- X-Y représente une liaison choisie parmi les liaisons de formules (a) et (b) suivantes pouvant être lues de gauche à droite ou inversement : 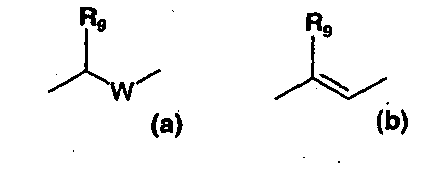 R₉ et W ayant les significations données ci-après,
- R₃ représente la chaîne de la vitamine D₂ ou de la Vitamine D₃, 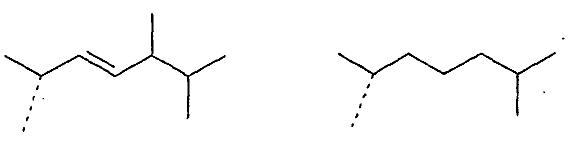 les traits en pointillés représentent la liaison reliant la chaîne au cycle benzénique représenté sur la figure (I),
ou R₃ représente une chaîne ayant de 4 à 8 atomes de carbone substituée par un ou plusieurs groupements hydroxyles, les groupements hydroxyles pouvant être protégés sous forme d'acétoxy, de méthoxy ou d'éthoxy, de triméthylsilyloxy, de tertiobutyldiméthylsilyloxy, de tétrahydropyranyloxy et éventuellement en outre :
- substituée par un ou plusieurs groupements alkyles linéaires ou ramifiés de 1 à 6 atomes de carbone ou cycloalkyles et/ou
- substituée par un ou plusieurs atomes d'halogène et/ou
- substituée par un ou plusieurs groupements CF₃ et/ou
- dans laquelle un ou plusieurs atomes de carbone de la chaîne sont remplacés par un ou plusieurs atomes d'oxygène, de soufre ou d'azote, les atomes d'azote pouvant éventuellement être substitués par des radicaux alkyles linéaires ou ramifiés de 1 à 6 atomes de carbone et/ou
- dans laquelle une ou plusieurs liaisons simples de la chaîne sont remplacées par une ou plusieurs liaisons doubles et/ou triples,
- R₃ étant positionné sur le cycle benzénique en *para* ou *méta* de la liaison X-Y,
- R₄, R₅ et R₆, identiques ou différents, représentent un atome d'hydrogène, un radical alkyle linéaire ou ramifié de 1 à 6 atomes de carbone, un atome d'halogène, un radical -OR₁₀, un radical polyéther de 2 à 5 atomes de carbone interrompus par au moins 2 atomes d'oxygène,
R₁₀ ayant la signification donnée ci-après,
- n étant 0,1 ou 2,
- R₇ et R₈ identiques ou différents, représentent un atome d'hydrogène, un radical acétylé, un radical triméthylsilyle, un radical tertiobutyldiméthylsilyle, un radical tétrahydropyranyle,
- R₉ représente un atome d'hydrogène ou un radical alkyle linéaire ou ramifié de 1 à 6 atomes de carbone,
- W représente un atome d'oxygène, de soufre, un radical -CH₂- ou un radical -NH- pouvant éventuellement être substitué par un radical alkyle linéaire ou ramifié de 1 à 6 atomes de carbone,
- R₁₀ représente un atome d'hydrogène ou un radical alkyle linéaire ou ramifié de 1 à 6 atomes de carbone,
et les isomères optiques et géométriques desdits composés de formule (I) ainsi que leurs sels .

2. Composés selon la revendication 1, **caractérisés par le fait qu'**ils se présentent sous forme de sels d'un acide minéral ou organique, en particulier l'acide chlorhydrique, sulfurique, acétique, fumarique, hémisuccinique, maléique et mandélique.

3. Composés selon l'une des revendications 1 ou 2, **caractérisés par le fait que** les radicaux alkyles linéaires ou ramifiés de 1 à 6 atomes de carbone sont choisis parmi les radicaux méthyle, éthyle, isopropyle, tertiobutyle et hexyle.

4. Composés selon l'une des revendications précédentes, **caractérisés en ce que** le radical cycloalkyle correspond à un radical adamantyle ou un radical 1-méthylcyclohexyle.

5. Composés selon l'une des revendications précédentes, caractérisés en ce l'atome d'halogène correspond à un atome de fluor, de chlore ou de brome.

6. Composés selon l'une quelconque des revendications précédentes, **caractérisés en ce que** le radical polyéther correspond à un radical méthoxyméthoxy, méthoxyéthoxy ou méthoxyéthoxyméthoxy.

7. Composés selon la revendication 1, **caractérisés par le fait qu'**ils sont pris, seuls ou en mélanges, dans le groupe constitué par :
3-hydroxyméthyl-5-{2-[3-(5-hydroxy-5-méthyl-hexyl)-phényl)-vinyl}-phénol,
3-hydroxyrnéthyl-5-{2-[3-(6-hydroxy-6-méthyl-heptyl)-phényl]-vinyl}-phenol,
3-[3-(5-hydroxy-1,5-diméthyl-hexyl)-phenoxyméthyl]-5-hydroxyméthyl-phenol,
6-[3-(3,4-bis-hydroxyméthyl-benzyloxy)-phényl]-2-méthyl-hepta-3,5-dien-2-ol,
6-[3-(3,4-bis-hydroxyméthyl-benzyloxy)-phényl]-2-méthyl-hexan-2-ol,
6-[3-(3,4-bis-hydroxyméthyl-phenoxyméthyl)-phényl]-2-méthyl-heptan-2-ol,
7-[3-(3,4-bis-hydroxyméthyl-phénoxyméthyl)-phényl]-3-éthyl-octan-3-ol,
5-{2-[4-(5-hydroxy-5-méthyl-hexyl)-phényl]-vinyl}-benzène-1,3-diol,
5-{2-[4-(5-hydroxy-5-méthyl-hexyl)-phényl]-éthyl}-benzène-1,3 diol,
5-{2-[4-(6-hydroxy-6-méthyl-heptyl)-phényl]vinyl}-benzene-1,3-diol,
5-{2-[3-(6-hydroxy-6-méthyl-heptyl)-phényl]-vinyl}-benzene-1,3-diol,
5-{2-[4-(6-hydroxy-6-méthyl-heptyl)-phényl]-éthyl}-benzène-1,3-diol,
5-{2-[3-(6-hydroxy-6-méthyl-heptyl)-phényl]-éthyl}-benzène-1,3-diol,
2-hydroxyméthyl-4-{2-[3-(5-hydroxy-5-méthyl-hexyl)-phényl]-vinyl}-phenol,
2-hydroxyméthyl-4-{2-[4-(5-hydroxy-5-méthyl-hexyl)-phényl]-vinyl}-phénol,
2-hydroxyrnéthyl-4-{2-[3-(6-hydroxy-6-méthyl-heptyl)-phényl]-vinyl}-phénol,
2-hydroxyméthyl-4-{2-[4-(6-hydroxy-6-méthyl-heptyl)-phényl]-vinyl}-phénol,
2-hydroxyméthyl-4-{2-[3-(5-hydroxy-5-méthyl-heptyl)-phényl]-éthyl}-phénol,
2-hydroxyméthyl-4-{2-[4-(5-hydroxy-5-méthyl-hexyl)-phényl]-éthyl}-phénol,
2-hydroxyméthyl-4-{2-[3-(6-hydroxy-6-méthyl-heptyl)-phényl]-éthyl}-phénol,
2-hydroxyméthyl-4-{2-[4-(6-hydroxy-6-méthyl-heptyl)-phényl]-éthyl}-phénol,
2-hydroxyméthyl-5-{2-[4-(5-hydroxy-5-méthyl-hexyl)-phényl]-vinyl}-phénol,
6-[3-(3,4-bis-hydroxyméthyl-benzyloxy)-phényl]-2-méthyl-heptan-2-ol,
4-[3-(5-hydroxy-1,5-diméthyl-hexyl)-phenoxyméthyl]-2-hydroxyméthyl-phenol,
6-{3-[2-(3,4-bis-hydroxyméthyl-phényl)-vinyl]-phényl}-2-méthyl-hexan-2-ol,
7-{4-[2-(3,4-bis-hydroxyméthyl-phényl)-vinyl]-phényl}-2-méthyl-heptan-2-ol,
6-{4-[2-(3,4-bis-hydroxyméthyl-phényl)-vinyl]-phényl}-2-méthyl-hexan-2-ol,
5-{2-[3-(6-hydroxy-6-méthyl-heptyl)-phényl]-1-méthyl-vinyl}-benzène-1,3-diol,
5-{2-[3-{5-hydroxy-5-méthyl-hexyl)-phényl]-vinyl}-benzène-1,3-diol,
5-[3-(6-hydroxy-6-méthyl-heptyl)-phénoxyméthyl]-benzène-1,3-diol,
5-{2-[3-(7-hydroxy-7-méthyl-oct-1-enyl)-phényl]-vinyl}-benzene-1,3-diol,
5-{2-[3-(7-hydroxy-7-méthyl-octyl)-phényl]-vinyl}-benzène-1,3-diol,
4-{2-[3-(6-hydroxy-6-méthyl-heptyl)-phényl]-vinyl}-benzène-1,2-diol,
3-{2-[3-(6-hydroxy-6-méthyl-heptyl)-phényl]-vinyl}-phénol,
6-{3-[2-(3,5-bis-hydroxyméthyl-phényl)-vinyl]-phényl}-2-méthyl-hexan-2-ol,
3-{2-[3-(7-hydroxy-7-méthyl-octyl)-phényl]-vinyl}-phénol,
7-{3-[2-(3,5-bis-hydroxyméthyl-phényl)-vinyl]-phényl}-2-méthyl-heptan-2-ol,
7-{3-[2-(3,4-bis-hydroxyméthyl-phényl}-vinyl]-phényl}-2-méthyl-heptan-2-ol,
7-{3-[2-(4-hydroxyméthyl-phényl)-vinyl]-phényl}-2-méthyl-heptan-2-ol,
4-{2-[3-(7-hydroxy-7-méthyl-oct-1-enyl)-phényl]-vinyl}-benzene-1,2-diol,
5-{2-[3-(6-hydroxy-6-méthyl-hept-1-enyl)-phényl]-vinyl}-benzene-1,3-diol,
5-{2-[3-(7-éthyl-7-hydroxy-non-1-enyl)-phényl]-vinyl}-benzène-1,3-diol,
5-{2-[3-(7-hydroxy-1-méthoxy-1,7-diméthyl-octyl)-phényl]-vinyl}-benzène-1,3-diol,
5-{2-[3-(6-hydroxy-1-méthoxy-1,6-diméthyl-heptyl)-phényl]-vinyl}-benzène-1,3-diol,
5-{2-[3-(5-hydroxy-pentyl)-phényl]-vinyl}-benzène-1,3-diol,
5-{2-[3-(5-hydroxy-6-méthyl-heptyl)-phényl]-vinyl}-benzène-1,3-diol,
5-{2-[3-(6-hydroxy-7-méthyl-octyl)-phényl]-vinyl}-benzène-1,3-diol,
5-{2-[3-(5-hydroxy-6-méthyl-hept-1-enyl)-phényl]-vinyl}-benzène-1,3-diol,
5-{2-[3-(6-hydroxy-7-méthyl-oct-1-enyl)-phényl]-vinyl}-benzène-1,3-diol,
5-{2-[3-(1,6-dihydroxy-1,6-diméthyl-heptyl)-phényl]-vinyl}-benzène-1,3-diol,
5-{2-[3-(6-hydroxy-1,6-diméthyl-hept-1-enyl)-phényl]-vinyl}-benzène-1,3-diol,
6-{[3-(3,4-Bis-hydroxyméthyl-benzyloxy)-phenyl]-méthyl-amino}-2-méthyl-hexan-2-ol,
5-{[3-(3,4-Bis-hydroxyméthyl-benzyloxy)-phenyl]-méthyl-amino}-2-méthyl-pentan-2-ol,
6-{[3-(3,4-Bis-hydroxyméthyl-benzyloxy)-phenyl]-méthyl-amino}-3-éthyl-hexan-3-ol,
7-{[3-(3,4-Bis-hydroxyméthyl-benzyloxy)-phenyl]-méthyl-amino}-3-éthyl-heptan-3-ol,
5-{[3-(3,4-Bis-hydroxyméthyl-benzyloxy)-phenyl]-éthyl-amino}-2-méthyl-pentan-2-ol,
6-{[3-(3,4-Bis-hydroxyméthyl-benzyloxy)-phenyl]-benzyl-amino}-3-éthyl-hexan-3-ol,
(4E, 6E)-7-{3-[2-(3,4-Bis-hydroxyméthyl-phenyl)-éthyl]-phenyl}-3-éthyl-octa-4,6-dien-3-ol,
(3E, 5E)-6-{3-[2-(3,4-Bis-hydroxyméthyl-phenyl)-éthyl]-phenyl}-2-méthyl-hepta-3,5-dien-2-ol,
(4E, 6E)-7-{3-[2-(3,4-Bis-hydroxyméthyl-phenyl)-vinyl]-phenyl}-3-éthyl-octa-4,6-dien-3-ol,
(3E, 5E)-6-{3-[2-(3,4-Bis-hydroxyméthyl-phenyl)-vinyl]-phenyl}-2-méthyl-hepta-3,5-dien-2-ol,
7-[3-(3,4-Bis-hydroxyméthyl-benzyloxy)phényl]-3-éthyl-3-octanol,
(4E,6E)-7-[3-(3,4-Bis-hydroxyméthyl-benzyloxy)-phenyl]-3-éthyl-octa-4,6-dien-3-ol,
(4E,6Z)-7-[3-(3,4-Bis-hydroxyméthyl-benzyloxy)-phenyl]-3-éthyl-octa-4,6-dien-3-ol,
7-[4-(3,4-Bis-hydroxyméthyl-benzyloxy)phényl]-3-éthyl-3-octanol,
(4E,6E)-7-[3-(3,4-Bis-hydroxyméthyl-benzyloxy)-phenyl]-3-éthyl-nona-4,6-dien-3-ol,
(4E,6Z)-7-[3-(3,4-Bis-hydroxyméthyl-benzyloxy)-phenyl]-3-éthyl-nona-4,6-dien-3-ol,
(E)-6-[3-(3,4-Bis-hydroxyméthyl-benzyloxy)-phenyl]-2-méthyl-hept-3-en-2-ol,
(E)-7-[3-(3,4-Bis-hydroxyméthyl-benzyloxy)-phenyl]-3-éthyl-oct-4-en-3-ol,
(E)-7-[3-(3,4-Bis-hydroxyméthyl-benzyloxy)-phenyl]-3-éthyl-oct-6-en-3-ol,
(Z)-7-[3-(3,4-Bis-hydroxyméthyl-benzyloxy)-phenyl]-3-éthyl-oct-6-en-3-ol,
(E)-8-[3-(3,4-Bis-hydroxyméthyl-benzyloxy)-phenyl]-2-méthyl-non-7-en-2-ol,
(Z)-8-[3-(3,4-Bis-hydroxyméthyl-benzyloxy)-phenyl]-2-méthyl-non-7-en-2-ol,
(E)-9-[3-(3,4-Bis-hydroxyméthyl-benzyloxy)-phenyl]-3-éthyl-dec-8-en-3-ol,
(Z)-9-[3-(3,4-Bis-hydroxyméthyl-benzyloxy)-phenyl]-3-éthyl-dec-8-en-3-ol,
8-[3-(3,4-Bis-hydroxyméthyl-benzyloxy)phényl]-2-méthyl-2-nonanol,
9-[3-(3,4-Bis-hydroxyméthyl-benzyloxy)-phenyl]-3-éthyl-decan-3-ol,
(E)-7-[3-(3,4-Bis-hydroxyméthyl-benzyloxy)-phenyl]-3-éthyl-oct-6-en-4-yn-3-ol,
(3E,5E)-7-[3-(3,4-Bis-hydroxyméthyl-benzyloxy)-phenyl]-2,7-diméthyl-octa-3,5-dien-2-ol,
(4E,6E)-7-[3-(3,4-Bis-hydroxyméthyl-phenoxyméthyl)-phenyl]-3-éthyl-octa-4,6-dien-3-ol,
(3E,5E)-6-[3-(3,4-Bis-hydroxyméthyl-phenoxyméthyl)-phenyl]-2-méthyl-hepta-3,5-dien-2-ol,
(Z)-7-[3-(3,4-Bis-hydroxyméthyl-benzyloxy)-phenyl]-3-éthyl-non-6-en-3-ol,
(Z)-6-[3-(3,4-Bis-hydroxyméthyl-benzyloxy)-phenyl]-2-méthyl-oct-5-en-2-ol,
(Z)-7-[3-(3,4-Bis-hydroxyméthyl-benzyloxy)-phenyl]-3-éthyl-dec-6-en-3-ol,
(Z)-6-[3-(3,4-Bis-hydroxyméthyl-benzyloxy)-phenyl]-2-méthyl-non-5-en-2-ol,
(Z)-8-[3-(3,4-Bis-hydroxyméthyl-benzyloxy)-phenyl]-3-éthyl-non-7-en-3-ol,
(E)-8-[3-(3,4-Bis-hydroxyméthyl-benzyloxy)-phenyl]-3-éthyl-non-7-en-3-ol,
8-[3-(3,4-Bis-hydroxyméthyl-benzyloxy)-phenyl]-3-éthyl-nonan-3-ol,
(4E, 6E)-7-[5-(3,4-Bis-hydroxyméthyl-benzyloxy)-2-methoxy-phenyl]-3-éthyl-nona-4,6-dien-3-ol,
(4E, 6E)-7-[5-(3,4-Bis-hydroxyméthyl-benzyloxy)-2-methyl-phenyl]-3-éthyl-nona-4,6-dien-3-ol,
(4E, 6E)-7-[3-(3,4-Bis-hydroxyméthyl-benzyloxy)-5-methoxy-phenyl]-3-éthyl-nona-4,6-dien-3-ol,
(4E, 6E)-7-[3-(3,4-Bis-hydroxyméthyl-benzyloxy)-2-methoxy-phenyl]-3-éthyl-nona-4,6-dien-3-ol,
(4E, 6E)-7-[3-(3,4-Bis-hydroxyméthyl-benzyloxy)-4-methyl-phenyl]-3-éthyl-nona-4,6-dien-3-ol,
1-[3-(3,4)-Bis-hydroxymethyl-benzyloxy)-phenyl]ethanone O -(2-hydroxy-2-methyl-propyl)-oxime,
1-{1-[3-(3,4-Bis-hydroxyméthyl-benzyloxy)-phenyl]-propoxy}-3-éthyl-pentan-3-ol,
(E)-7-[3-(3,4-Bis-hydroxyméthyl-phenoxyméthyl)-phenyl]-3-éthyl-non-6-en-3-ol,
(E)-7-[3-(3,4-Bis-hydroxyméthyl-benzylsulfanyl)-phenyl]-3-éthyl-oct-6-en-3-ol,
(E)-7-{3-[(3,4-Bis-hydroxyméthyl-benzyl)-methylamino]-phenyl}-3-éthyl-oct-6-en-3-ol,
(E)-6-[3-(3,4-Bis-hydroxyméthyl-benzyloxy)-phenyl]-3-ethyl-6-methyl-hept-4-en-3-ol,
7-[3-(3,4-Bis-hydroxyméthyl-benzyloxy)-phenyl]-3-ethyl-7-methyl-octan-3-ol,
7-[3-(3,4-Bis-hydroxymethyl-benzyloxy)-phenyl]-3,7-diethyl-nonan-3-ol,
(E)-6-[3-(3,4-Bis-hydroxymethyl-benzyloxy)-phenyl]-1,1,1-trifluoro-2-trifluoromethyl-oct-5-en-2-ol,
2-{4-[3-(3,4-Bis-hydroxymethyl-benzyloxy)-phenyl]-hexyl}-1,1,1,3,3,3-hexafluoro-propan-2-ol,
(E)-7-[3-(3,4-Bis-hydroxymethyl-benzyloxy)-phenyl]-3-ethyl-4,4-difluoro-non-6-en-3-ol,
7-[3-(3,4-Bis-hydroxymethyl-benzyloxy)-phenyl]-3-ethyl-4,4-difluoro-7-methyl-octan-3-ol,
(E)-6-[3-(3,4-Bis-hydroxymethyl-benzyloxy)-phenyl]-2-methyl-oct-5-en-3-ol,
(E)-4-[3-(3,4-Bis-hydroxymethyl-benzyloxy)-phenyll-1-cyclopropyl-hex-3-en-1-ol,
(E)-7-[3-(3,4-Bis-hydroxymethyl-benzyloxy)-phenyl]-3-ethyl-4-methyl-non-6-en-3-ol,
(E)-7-[3-(3,4-Bis-hydroxymethyl-benzyloxy)-phenyl]-3-ethyl-4-methyl-dec-6-en-3-ol,
(4E,6E)-7-[3-(3,4-Bis-hydroxymethyl-phenoxymethyl)-phenyl]-3-ethyl-nona-4,6-dien-3-ol,
(4E,6E)-7-{3-[2-(3,4-Bis-hydroxymethyl-phenyl)-ethyl]-phenyl}-3-ethyl-nona-4,6-dien-3-ol,
(E)-7-{3-[2-(3,4-Bis-hydroxymethyl-phenyl)-ethyl]-phenyl}-3-ethyl-non-6-en-3-ol,
(E)-3-Ethyl-7-[3-(3-hydroxymethyl-phenoxymethyl)-phenyl]-non-6-en-3-ol,
(E)-3-Ethyl-7-[3-(4-hydroxymethyl-phenoxymethyl)-phenyl]-non-6-en-3-ol,
(E)-3-Ethyl-7-[(E)-3-(3-hydroxymethyl-4-methyl-phenoxymethyl)-phenyl]-non-6-en-3-ol,
(E)-3-Ethyl-7-[(E)-3-(4-hydroxymethyl-3-methyl-phenoxymethyl)-phenyl]-non-6-en-3-ol,
2-{4-[3-(3,4-Bis-hydroxymethyl-benzyloxy)-phenyl]-4-methyl-pentyl}-1,1,1,3,3,3-hexafluoro-propan-2-ol,
7-[3-(3,4-Bis-hydroxymethyl-benzyloxy)-phenyl]-3-ethyl-4,4-difluoro-7-methyl-octan-3-ol,
7-{3-[2-(3,4-Bis-hydroxymethyl-phenyl)-ethyl]-phenyl}-3,7-diethyl-nonan-3-ol
7-[3-(3,4-Bis-hydroxymethyl-benzyloxy)-phenyl]-3-ethyl-7-methyl-octane-3,4-diol
7-{3-[2-(3,4-Bis-hydroxymethyl-phenyl)-ethyl]-phenyl}-3,7-diethyl-nonane-3,4-diol
7-{3-[2-(3,4-Bis-hydroxymethyl-phenyl)-ethyl]-phenyl}-3-ethyl-7-methyl-octane-3,4-diol
(E)-4-{3-[2-(3,4-Bis-hydroxymethyl-phenyl)-ethyl]-phenyl}-1-cyclopropyl-hex-3-en-1-ol
(4E,6E)-7-{3-[2-(3,4-Bis-hydroxymethyl-phenyl)-ethyl]-phenyl}-3-ethyl-4-methyl-nona-4,6-dien-3-ol
(4E,6E)-7-[3-(3,4-Bis-hydroxymethyl-benzyloxy)-phenyl]-3-ethyl-4-methyl-nona-4,6-dien-3-ol
(E)-3-Ethyl-7-[3-(4-hydroxymethyl-3-methyl-phenoxymethyl)-phenyl]-non-6-en-3-ol ,
(E)-3-Ethyl-7-[3-(3-hydroxymethyl-4-methyl-phenoxymethyl)-phenyl]-non-6-en-3-ol,
(E)-7-[3-(3,4-Bis-hydroxymethyl-phenoxymethyl)-phenyl]-3-ethyl-oct-6-en-3-ol,
(E)-3-Ethyl-7-[3-(3-hydroxymethyl-phenoxymethyl)-phenyl]-non-6-en-3-ol,
(E)-3-Ethyl-7-[3-(4-hydroxymethyl-phenoxymethyl)-phenyl]-non-6-en-3-ol.

8. Composés selon la revendication 1, **caractérisés par le fait qu'**ils présentent l'une au moins des caractéristiques suivantes :
- R₁ représente le radical -(CH₂)ₙOH,
- R₂ représente le radical -(CH₂)ₙOH,
- X-Y représente une liaison de formule (a) ou (b),
- R₃ représente une chaîne de 4 à 8 atomes de carbone substituée par au moins un radical hydroxyle et/ou un radical alkyle linéaire ou ramifié de 1 à 6 atomes de carbone.

9. Composé selon l'une quelconque des revendications précédentes à titre de médicament.

10. Utilisation d'un composé selon la revendication 9 pour la fabrication d'un médicament destiné au traitement des affections dermatologiques liées à un désordre de la différenciation ou de la prolifération des kératinocytes ou des sébocytes, pour traiter les acnés vulgaires, comédoniennes, polymorphes, rosacées, les acnés nodulokystiques, conglobata, les acnés séniles, les acnés secondaires, l'acné solaire, médicamenteuse ou professionnelle; les ichtyoses, les états ichtyosiformes, la maladie de Darier, les kératodermies palmoplantaires, les leucoplasies et les états leucoplasiformes, le lichen cutané ou muqueux (buccal); des affections dermatologiques liées à un trouble de la kératinisation avec une composante inflammatoire et/ou immuno-allergique, toutes les formes de psoriasis qu'il soit cutané, muqueux ou unguéal, le rhumatisme psoriatique, l'atopie cutanée, l'eczéma, l'atopie respiratoire, l'hypertrophie gingivale; des affections inflammatoires ne présentant pas de trouble de la kératinisation; des proliférations dermiques ou épidermiques qu'elles soient bénignes ou malignes, qu'elles soient ou non d'origine virale, les verrues vulgaires, les verrues planes et l'épidermodysplasie verruciforme, les papillomatoses orales ou florides et les proliférations pouvant être induites par les ultra-violets les épithélioma baso et spinocellulaires; les dermatoses bulleuses et les maladies du collagène ; du vieillissement de la peau, qu'il soit photo-induit ou chronologique, des pigmentations et les kératoses actiniques, toutes pathologies associées au vieillissement chronologique ou actinique; des troubles de la cicatrisation ou des vergetures; des troubles de la fonction sébacée, l'hyperséborrhée de l'acné, la séborrhée simple, l'eczéma séborrhéique; des troubles ophtalmologiques, les coméopathies; des états cancéreux ou précancéreux de cancers présentant ou pouvant être induits par des récepteurs de vitamine D, le cancer du sein, la leucémie, les syndromes myélodysplasiques et les lymphomes, les carcinomes des cellules de l'épithélium malpighien et les cancers gastro-intestinaux, les mélanomes, et l'ostéosarcome; des affections inflammatoires, l'arthrite ou la polyarthrite rhumatoïde; des affections d'origine virale au niveau cutané ou général; de l'alopécie de différentes origines, l'alopécie due à la chimiothérapie ou aux rayonnements; des affections dermatologiques ou générales à composante immunologique; des affections immunitaires, les maladies auto-immunes, le diabète sucré de type 1, la sclérose en plaques, le lupus et les affections de type lupus, l'asthme, ou la glomérulonéphrite; des dysfonctionnements sélectifs du système immunitaire; les affections endocriniennes; des affections **caractérisées par** une gestion anormale du calcium intracellulaire; des carences en vitamine D et des affections de l'homéostasie des minéraux dans le plasma et les os, le rachitisme, l'ostéomalacie, l'ostéoporose, l'ostéoporose des femmes ménopausées, l'ostéodystrophie rénale ou les troubles de la fonction parathyroïdienne.

11. Composition pharmaceutique, **caractérisée par le fait qu'**elle comprend, dans un support pharmaceutiquement acceptable, au moins l'un des composés tels que définis à l'une quelconque des revendications 1 à 8.

12. Composition selon la revendication 11, **caractérisée en ce que** la concentration en composé(s) selon l'une des revendications 1 à 8 est comprise entre 0,0001 % et 5 % en poids par rapport au poids total de la composition.

13. Composition cosmétique, **caractérisée par le fait qu'**elle comprend, dans un support cosmétiquement acceptable, au moins l'un des composés tels que définis à l'une quelconque des revendications 1 à 8.

14. Composition selon la revendication 13, **caractérisée en ce que** la concentration en composé(s) est comprise entre 0,001 % et 3 % en poids par rapport au poids total de la composition.

15. Utilisation d'une composition cosmétique telle que définie à l'une des revendications 13 ou 14 pour l'hygiène corporelle ou capillaire.

## Patentansprüche

1. Verbindungen, die **dadurch gekennzeichnet sind, dass** sie der folgenden allgemeinen Formel (I) entsprechen, 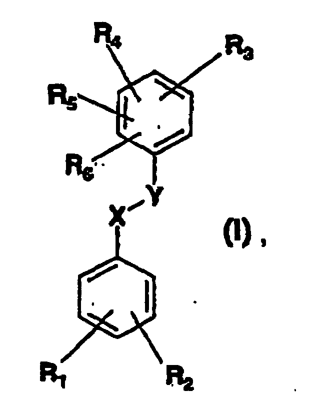 in der bedeuten:
- R₁ ein Wasserstoffatom, eine Methylgruppe oder eine Gruppe -(CH₂)ₙ-OR₇,
- R₂ eine Gruppe -(CH₂)ₙ-OR₈,
wobei n, R₇ und R₈ die nachstehend angegebenen
Bedeutungen besitzen,
- X-Y eine Bindung, die ausgewählt ist unter Bindungen der folgenden Formeln (a) und (b), die von links nach rechts oder umgekehrt gelesen werden können: 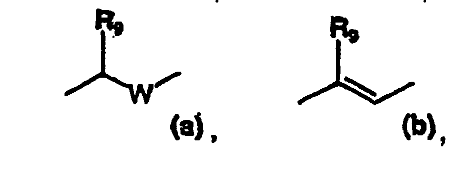 worin R₉ und W die nachstehend angegebenen Bedeutungen besitzen,
- R₃ die Kette von Vitamin D₂ oder Vitamin D₃, 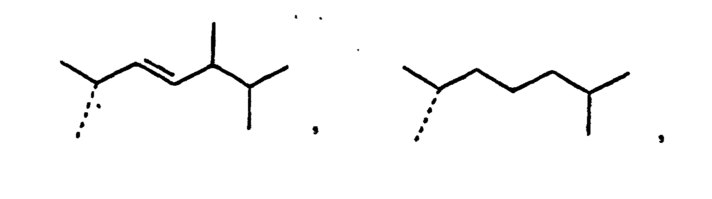 wobei die punktierten Linien die Bindung bedeuten, welche die Kette mit dem in Formel (I) dargestellten Phenylring verbindet,
oder R₃ eine Kette mit 4 bis 8 Kohlenstoffatomen, die mit einer oder mehreren Hydroxygruppen substituiert ist, wobei die Hydroxygruppen in Form von Acetoxy, Methoxy oder Ethoxy, Trimethylsilyloxy, t-Butyldimethylsilyloxy, Tetrahydropyranyloxy geschützt sein können,
wobei gegebenenfalls ferner
- die Kette mit einer oder mehreren geradkettigen oder verzweigten Alkylgruppen mit 1 bis 6 Kohlenstoffatomen oder Cycloalkylgruppen substituiert ist
und/oder
- die Kette mit einem oder mehreren Halogenatomen substituiert ist
und/oder
- die Kette mit einer oder mehreren CF₃-Gruppen substituiert ist
und/oder
- ein oder mehrere Kohlenstoffatome der Kette durch ein oder mehrere Sauerstoffatome, Schwefelatome oder Stickstoffatome ersetzt sind, wobei die Stickstoffatome gegebenenfalls mit geradkettigen oder verzweigten Alkylgruppen mit 1 bis 6 Kohlenstoffatomen substituiert sind,
und/oder
- eine oder mehrere Einfachbindungen der Kette durch eine oder mehrere Doppelbindungen und/oder Dreifachbindungen ersetzt sind,
- wobei sich R₃ in Bezug auf die Bindung X-Y in Para- oder Meta-Stellung am Phenylring befindet,
- R₄, R₅ und R₆, die gleich oder verschieden sind, ein Wasserstoffatom, eine geradkettige oder verzweigte Alkylgruppe mit 1 bis 6 Kohlenstoffatomen, ein Halogenatom, eine Gruppe -OR₁₀, eine Polyethergruppe mit 2 bis 5 Kohlenstoffatomen, die durch mindestens zwei Sauerstoffatome unterbrochen sind, wobei R₁₀ die nachstehend angegebene Bedeutung besitzt,
- n 0,1 oder 2,
- R₇ und R₈, die gleich oder verschieden sind, ein Wasserstoffatom, eine Acetylgruppe, eine Trimethylsilyl-Gruppe, eine Tertiärbutyldimethylsilyl-Gruppe, eine Tetrahydropyranyl-Gruppe,
- R₉ ein Wasserstoffatom oder eine geradkettige oder verzweigte Alkylgruppe mit 1 bis 6 Kohlenstoffatomen,
- W ein Sauerstoffatom, ein Schwefelatom, eine Gruppe -CH₂- oder eine Gruppe -NH-, die gegebenenfalls mit einer geradkettigen oder verzweigten Alkylgruppe mit 1 bis 6 Kohlenstoffatomen substituiert ist,
und
- R₁₀ ein Wasserstoffatom oder eine geradkettige oder verzweigte Alkylgruppe mit 1 bis 6 Kohlenstoffatomen,
und die optischen und geometrischen Isomeren der Verbindungen der Formel (I) sowie ihre Salze.

2. Verbindungen nach Anspruch 1, **dadurch gekennzeichnet, dass** sie in Form von Salzen einer anorganischen oder organischen Säure vorliegen, insbesondere von Chlorwasserstoffsäure, Schwefelsäure, Essigsäure, Fumarsäure, Hemibernsteinsäure, Maleinsäure und Mandelsäure.

3. Verbindungen nach Anspruch 1 oder 2, **dadurch gekennzeichnet, dass** die geradkettigen oder verzweigten Alkylgruppen mit 1 bis 6 Kohlenstoffatomen unter Methyl, Ethyl, Isopropyl, t-Butyl und Hexyl ausgewählt sind.

4. Verbindungen nach einem der vorhergehenden Ansprüche, **dadurch gekennzeichnet, dass** die Cycloalkylgruppe einer Adamantyl-Gruppe oder einer 1-Methylcyclohexyl-Gruppe entspricht.

5. Verbindungen nach einem der vorhergehenden Ansprüche, **dadurch gekennzeichnet, dass** das Halogenatom einem Fluoratom, einem Chloratom oder einem Bromatom entspricht.

6. Verbindungen nach einem der vorhergehenden Ansprüche, **dadurch gekennzeichnet, dass** die Polyethergruppe einer Methoxymethoxy-Gruppe, einer Methoxyethoxy-Gruppe oder einer Methoxyethoxymethoxy-Gruppe entspricht.

7. Verbindungen nach Anspruch 1, **dadurch gekennzeichnet, dass** sie, allein oder in Form von Gemischen, ausgewählt sind aus der Gruppe, die besteht aus:
3-Hydroxymethyl-5-{2-[3-(5-hydroxy-5-methyl-hexyl)-phenyl]-vinyl}phenol,
3-Hydroxymethyl-5-{2-[3-(6-hydroxy-6-methyl-heptyl)-phenyl]-vinyl}-phenol,
3-[3-(5-Hydroxy-1,5-dimethyl-hexyl)-phenoxymethyl]-5-hydroxymethyl-phenol,
6-[3-(3,4-Bis-hydroxymethyl-benzyloxy)-phenyl]-2-methyl-hepta-3,5-dien-2-ol,
6-[3-(3,4-Bis-hydroxymethyl-benzyloxy)-phenyl]-2-methyl-hexan-2-ol,
6-[3-(3,4-Bis-hydroxymethyl-phenoxymethyl)-phenyl]-2-methylheptan-2-ol,
7-[3-(3,4-Bis-hydroxymethyl-phenoxymethyl)-phenyl]-3-ethyloctan-3-ol,
5-{2-[4-(5-Hydroxy-5-methyl-hexyl)-phenyl]-vinyl}-benzol-1,3-diol,
5-{2-[4-(5-Hydroxy-5-methyl-hexyl)-phenyl]-ethyl}-benzol-1,3-diol,
5-{2-[4-(6-Hydroxy-6-methyl-heptyl)-phenyl]-vinyl}-benzol-1,3-diol,
5-{2-[3-(6-Hydroxy-6-methyl-heptyl)-phenyl]-vinyl}-benzol-1,3-diol,
5-{2-[4-(6-Hydroxy-6-methyl-heptyl)-phenyl]-ethyl}-benzol-1,3-diol,
5-{2-[3-(6-Hydroxy-6-methyl-heptyl)-phenyl]-ethyl}-benzol-1,3-diol,
2-Hydroxymethyl-4-{2-[3-(5-hydroxy-5-methyl-hexyl)-phenyl]-vinyl}phenol,
2-Hydroxymethyl-4-{2-[4-(5-hydroxy-5-methyl-hexyl)-phenyl]-vinyl}phenol,
2-Hydroxymethyl-4-{2-[3-(6-hydroxy-6-methyl-heptyl)-phenyl]-vinyl}-phenol,
2-Hydroxymethyl-4-{2-[4-(6-hydroxy-6-methyl-heptyl)-phenyl]-vinyl}-phenol,
2-Hydroxymethyl-4-{2-[3-(5-hydroxy-5-methyl-heptyl)-phenyl]-ethyl}-phenol,
2-Hydroxymethyl-4-{2-[4-(5-hydroxy-5-methyl-hexyl)-phenyl]-ethyl}-phenol,
2-Hydroxymethyl-4-{2-[3-(6-hydroxy-6-methyl-heptyl)-phenyl]-ethyl}-phenol,
2-Hydroxymethyl-4-{2-[4-(6-hydroxy-6-methyl-heptyl)-phenyl]-ethyl}-phenol,
2-Hydroxymethyl-5-{2-[4-(5-hydroxy-5-methyl-hexyl)-phenyl]-vinyl}phenol,
6-[3-(3,4-Bis-hydroxymethyl-benzyloxy)-phenyl]-2-methyl-heptan-2-ol,
4-[3-(5-Hxydroxy-1,5-dimethyl-hexyl)-phenoxymethyl]-2-hydroxymethyl-phenol,
6-{3-[2-(3,4-Bis-hydroxymethyl-phenyl)-vinyl]-phenyl}-2-methylhexan-2-ol,
7-{4-[2-(3,4-Bis-hydroxymethyl-phenyl)-vinyl]-phenyl}-2-methylheptan-2-ol,
6-{4-[2-(3,4-Bis-hydroxymethyl-phenyl)-vinyl]-phenyl}-2-methylhexan-2-ol,
5-{2-[3-(6-Hydroxy-6-methyl-heptyl)-phenyl]-1-methyl-vinyl}benzol-1,3-diol,
5-{2-[3-(5-Hydroxy-5-methyl-hexyl)-phenyl]-vinyl}-benzol-1,3-diol,
5-[3-(6-Hydroxy-6-methyl-heptyl)-phenoxymethyl]-benzol-1,3-diol,
5-{2-[3-(7-Hydroxy-7-methyl-oct-1-enyl)-phenyl]-vinyl}-benzol-1,3-diol,
5-{2-[3-(7-Hydroxy-7-methyl-octyl)-phenyl]-vinyl}-benzol-1,3-diol,
4-{2-[3-(6-Hydroxy-6-methyl-heptyl)-phenyl]-vinyl}-benzol-1,2-diol,
3-{2-[3-(6-Hydroxy-6-methyl-heptyl)-phenyl]-vinyl}-phenol,
6-{3-[2-(3,5-Bis-hydroxymethyl-phenyl)-vinyl]-phenyl}-2-methylhexan-2-ol,
3-{2-[3-(7-Hydroxy-7-methyl-octyl)-phenyl]-vinyl}-phenol,
7-{3-[2-(3,5-Bis-hydroxymethyl-phenyl)-vinyl]-phenyl}-2-methylheptan-2-ol,
7-{3-[2-(3,4-Bis-hydroxymethyl-phenyl)-vinyl]-phenyl}-2-methylheptan-2-ol,
7-{3-[2-(4-Hydroxymethyl-phenyl)-vinyl]-phenyl}-2-methyl-heptan-2-ol,
4-{2-[3-(7-Hydroxy-7-methyl-oct-1-enyl)-phenyl]-vinyl}-benzol-1,2-diol,
5-{2-[3-(6-Hydroxy-6-methyl-hept-1-enyl)-phenyl]-vinyl}-benzol-1,3-diol,
5-{2-[3-(7-Ethyl-7-hydroxy-non-1-enyl)-phenyl]-vinyl}-benzol-1,3-diol,
5-{2-[3-(7-Hydroxy-1-methoxy-1,7-dimethyl-octyl)-phenyl]-vinyl}benzol-1,3-diol,
5-{2-[3-(6-Hydroxy-1-methoxy-1,6-dimethyl-heptyl)-phenyl]-vinyl}benzol-1,3-diol,
5-{2-[3-(5-Hydroxy-pentyl)-phenyl]-vinyl}-benzol-1,3-diol,
5-{2-[3-(5-Hydroxy-6-methyl-heptyl)-phenyl]-vinyl}-benzol-1,3-diol,
5-{2-[3-(6-Hydroxy-7-methyl-octyl)-phenyl]-vinyl}-benzol-1,3-diol,
5-{2-[3-(5-Hydroxy-6-methyl-hept-1-enyl)-phenyl]-vinyl}-benzol-1,3-diol,
5-{2-[3-(6-Hydroxy-7-methyl-oct-1-enyl)-phenyl]-vinyl}-benzol-1,3-diol,
5-{2-[3-(1,6-Dihydroxy-1,6-dimethyl-heptyl)-phenyl]-vinyl}-benzol-1,3-diol,
5-{2-[3-(6-Hydroxy-1,6-dimethyl-hept-1-enyl)-phenyl]-vinyl}-benzol-1,3-diol,
6-{[3-(3,4-Bis-hxdroxymethyl-benzyloxy)-phenyl]-methyl-amino}-2-methyl-hexan-2-ol,
5-{[3-(3,4-Bis-hydroxymethyl-benzyloxy)-phenyl]-methyl-amino}-2-methyl-pentan-2-ol,
6-{[3-(3,4-Bis-hydroxymethyl-benzyloxy)-phenyl]-methyl-amino}-3-ethyl-hexan-3-ol,
7-{[3-(3,4-Bis-hydroxymethyl-benzyloxy)-phenyl]-methyl-amino}-3-ethyl-heptan-3-ol,
5-{[3-(3,4-Bis-hydroxymethyl-benzyloxy)-phenyl]-ethyl-amino}-2-methyl-pentan-2-ol,
6-{[3-(3,4-Bis-hydroxymethyl-benzyloxy)-phenyl]-benzyl-amino}-3-ethyl-hexan-3-ol,
(4E,6E)-7-{3-[2-(3,4-Bis-hydroxymethyl-phenyl)-ethyl]-phenyl}-3-ethyl-octa-4,6-dien-3-ol,
(3E,5E)-6-{3-[2-(3,4-Bis-hydroxymethyl-phenyl)-ethyl]-phenyl}-2-methyl-hepta-3,5-dien-2-ol,
(4E,6E)-7-{3-[2-(3,4-Bis-hydroxymethyl-phenyl)-vinyl]-phenyl}-3-ethyl-octa-4,6-dient-3-ol,
(3E,5E)-6-{3-[2-(3,4-Bis-hydroxymethyl-phenyl)-vinyl]-phenyl}-2-methyl-hepta-3,5-dien-2-ol,
7-[3-(3,4-Bis-hydroxymethyl-benzyloxy)-phenyl]-3-ethyl-3-octanol,
(4E,6E)-7-[3-(3,4-Bis-hydroxymethyl-benzyloxy)-phenyl]-3-ethylocta-4,6-dien-3-ol,
(4E,6Z)-7-[3-(3,4-Bis-hydroxymethyl-benzyloxy)-phenyl]-3-ethylocta-4,6-dien-3-ol,
7-[4-(3,4-Bis-hydroxymethyl-benzyloxy)-phenyl]-3-ethyl-3-octanol,
(4E,6E)-7-[3-(3,4-Bis-hydroxymethyl-benzyloxy)-phenyl]-3-ethylnona-4,6-dien-3-ol,
(4E,6Z)-7-[3-(3,4-Bis-hydroxymethyl-benzyloxy)-phenyl]-3-ethylnona-4,6-dien-3-ol,
(E)-6-[3-(3,4-Bis-hydroxynethyl-benzyloxy)-phenyl]-2-methyl-hept-3-en-2-ol,
(E)-7-[3-(3,4-Bis-hydroxymethyl-benzyloxy)-phenyl]-3-ethyl-oct-4-en-3-ol,
(E)-7-[3-(3,4-Bis-hydroxymethyl-benzyloxy)-phenyl]-3-ethyl-oct-6-en-3-ol,
(Z)-7-[3-(3,4-Bis-hydroxymethyl-benzyloxy)-phenyl]-3-ethyl-oct-6-en-3-ol,
(E)-8-[3-(3,4-Bis-hydroxymethyl-benzyloxy)-phenyl]-2-methyl-non-7-en-2-ol,
(Z)-8-[3-(3,4-Bis-hydroxymethyl-benzyloxy)-phenyl]-2-methyl-non-7-en-2-ol,
(E)-9-[3-(3,4-Bis-hydroxymethyl-benzyloxy)-phenyl]-3-ethyl-dec-8-en-3-ol,
(Z)-9-[3-(3,4-Bis-hydroxymethyl-benzyloxy)-phenyl]-3-ethyl-dec-8-en-3-ol,
8-[3-(3,4-Bis-hydroxymethyl-benzyloxy)-phenyl]-2-methyl-2-nonanol,
9-[3-(3,4-Bis-hydroxymethyl-benzyloxy)-phenyl]-3-ethyl-decan-3-ol,
(E)-7-[3-(3,4-Bis-hydroxymethyl-benzyloxy)-phenyl]-3-ethyl-oct-6-in-3-ol,
(3E,5E)-7-[3-(3,4-Bis-hydroxymethyl-benzyloxy)-phenyl]-2,7-dimethyl-octa-3,5-dien-2-ol,
(4E,6E)-7-[3-(3,4-Bis-hydroxymethyl-phenoxymethyl)-phenyl]-3-ethyl-octa-4,6-dien-3-ol,
(3E,5E)-6-[3-(3,4-Bis-hydroxymethyl-phenoxymethyl)-phenyl]-2-methyl-hepta-3,5-dien-2-ol,
(Z)-7-[3-(3,4-Bis-hydroxymethyl-benzyloxy)-phenyl]-3-ethyl-non-6-en-3-ol,
(Z)-6-[3-(3,4-Bis-hydroxymethyl-benzyloxy)-phenyl]-2-methyl-oct-5-en-2-ol,
(Z)-7-[3-(3,4-Bis-hydroxymethyl-benzyloxy)-phenyl]-3-ethyl-dec-6-en-3-ol,
(Z)-6-[3-(3,4-Bis-hydroxymethyl-benzyloxy)-phenyl]-2-methyl-non-5-en-2-ol,
(Z)-8-[3-(3,4-Bis-hydroxymethyl-benzyloxy)-phenyl]-3-ethyl-non-7-en-3-ol,
(E)-8-[3-(3,4-Bis-hydroxymethyl-benzyloxy)-phenyl]-3-ethyl-non-7-en-3-ol,
8-[3-(3,4-Bis-hydroxymethyl-benzyloxy)-phenyl]-3-ethyl-nonan-3-ol,
(4E,6E)-7-[5-(3,4-Bis-hydroxymethyl-benzyloxy)-2-methoxyphenyl]-3-ethyl-nona-4,6-dien-3-ol,
(4E,6E)-7-[5-(3,4-Bis-hydroxymethyl-benzyloxy)-2-methyl-phenyl]-3-ethyl-nona-4,6-dien-3-ol,
(4E,6E)-7-[3-(3,4-Bis-hydroxymethyl-benzyloxy)-5-methoxyphenyl]-3-ethyl-nona-4,6-dien-3-ol,
(4E,6E)-7-[3-(3,4-Bis-hydroxymethyl-benzyloxy)-2-methoxyphenyl]-3-ethyl-nona-4,6-dien-3-ol,
(4E,6E)-7-[3-(3,4-Bis-hydroxymethyl-benzyloxy)-4-methyl-phenyl]-3-ethyl-nona-4,6-dien-3-ol,
1-[3-(3,4-Bis-hydroxymethyl-benzyloxy)-phenyl]-ethanon-O-(2-hydroxy-2-methyl-propyl)-oxim,
1-{1-[3-(3,4-Bis-hydroxymethyl-benzyloxy)-phenyl]-propoxy}-3-ethyl-pentan-3-ol,
(E)-7-[3-(3,4-Bis-hydroxymethyl-phenoxymethyl)-phenyl]-3-ethylnon-6-en-3-ol,
(E)-7-[3-(3,4-Bis-hydroxymethyl-benzylsulfanyl)-phenyl]-3-ethyloct-6-en-3-ol,
(E)-7-{3-[(3,4-Bis-hydroxymethyl-benzyl)-methylamino]-phenyl}-3-ethyl-oct-6-en-3-ol,
(E)-6-[3-(3,4-Bis-hydroxymethyl-benzyloxy)-phenyl]-3-ethyl-6-methyl-hept-4-en-3-ol,
7-[3-(3,4-Bis-hydroxymethyl-benzyloxy)-phenyl]-3-ethyl-7-methyloctan-3-ol,
7-[3-(3,4-Bis-hydroxymethyl-benzyloxy)-phenyl]-3,7-diethyl-nonan-3-ol,
(E)-6-[3-(3,4-Bis-hydroxymethyl-benzyloxy)-phenyl]-1,1,1-trifluor-2-trifluormethyl-oct-5-en-2-ol,
2-{4-[3-(3,4-Bis-hydroxymethyl-benzyloxy)-phenyl]-hexyl}-1,1,1,3,3,3-hexafluor-propan-2-ol,
(E)-7-[3-(3,4-Bis-hydroxymethyl-benzyloxy)-phenyl]-3-ethyl-4,4-difluor-non-6-en-3-ol,
7-[3-(3,4-Bis-hydroxymethyl-benzyloxy)-phenyl]-3-ethyl-4,4-difluor-7-methyl-octan-3-ol,
(E)-6-[3-(3,4-Bis-hydroxymethyl-benzyloxy)-phenyl]-2-methyl-oct-5-en-3-ol,
(E)-4-[3-(3,4-Bis-hydroxymethyl-benzyloxy)-phenyl]-1-cyclopropylhex-3-en-1-ol,
(E)-7-[3-(3,4-Bis-hydroxymethyl-benzyloxy)-phenyl]-3-ethyl-4-methyl-non-6-en-3-ol,
(E)-7-[3-(3,4-Bis-hydroxymethyl-benzyloxy)-phenyl]-3-ethyl-4-methyl-dec-6-en-3-ol,
(4E,6E)-7-[3-(3,4-Bis-hydroxymethyl-phenoxymethyl)-phenyl]-3-ethyl-nona-4,6-dien-3-ol,
(4E,6E)-7-{3-[2-(3,4-Bis-hydroxymethyl-phenyl)-ethyl]-phenyl}-3-ethyl-nona-4,6-dien-3-ol,
(E)-7-{3-[2-(3,4-Bis-hydroxymethyl-phenyl)-ethyl]-phenyl}-3-ethylnon-6-en-3-ol,
(E)-3-Ethyl-7-[3-(3-hydroxymethyl-phenoxymethyl)-phenyl]-non-6-en-3-ol,
(E)-3-Ethyl-7-[3-(4-hydroxymethyl-phenoxymethyl)-phenyl]-non-6-en-3-ol,
(E)-3-Ethyl-7-[(E)-3-(3-hydroxymethyl-4-methyl-phenoxymethyl)-phenyl]-non-6-en-3-ol,
(E)-3-Ethyl-7-[(E)-3-(4-hydroxymethyl-3-methyl-phenoxymethyl)-phenyl]-non-6-en-3-ol,
2-{4-[3-(3,4-Bis-hydroxymethyl-benzyloxy)-phenyl]-4-methylpentyl}-1,1,1,3,3,3-hexafluor-propan-2-ol,
7-[3-(3,4-Bis-hydroxymethyl-benzyloxy)-phenyl]-3-ethyl-4,4-difluor-nonan-3-ol,
7-[3-(3,4-Bis-hydroxymethyl-benzyloxy)-phenyl]-3-ethyl-7-methyloctan-3,4-diol,
7-{3-[2-(3,4-Bis-hydroxymethyl-phenyl)-ethyl]-phenyl}-3,7-diethylnonan-3,4-diol,
7-{3-[2-(3,4-Bis-hydroxymethyl-phenyl)-ethyl]-phenyl}-3-ethyl-7-methyl-octan-3,4-diol,
(E)-4-{3-[2-(3,4-Bis-hydroxymethyl-phenyl)-ethyl]-phenyl}-1-cyclopropyl-hex-3-en-1-ol,
(4E,6E)-7-{3-[2-(3,4-Bis-hydroxymethyl-phenyl)-ethyl]-phcnyl}-3-ethyl-4-methyl-nona-4,6-dien-3-ol,
(4E,6E)-7-[3-(3,4-Bis-hydroxymethyl-benzyloxy)-phenyl]-3-ethyl-4-methyl-nona-4,6-dien-3-ol,
(E)-3-Ethyl-7-[3-(4-hydroxymethyl-3-methyl-phenoxymethyl)-phenyl]-non-6-en-3-ol,
(E)-3-Ethyl-7-[3-(3-hydroxymethyl-4-methyl-phenoxymethyl)-phenyl]-non-6-en-3-ol,
(E)-7-[3-(3,4-Bis-hydroxymethyl-phenoxymethyl)-phenyl]-3-ethyloct-6-en-3-ol,
(E)-3-Ethyl-7-[3-(3-hydroxymethyl-phenoxymethyl)-phenyl]-non-6-en-3-ol,
(E)-3-Ethyl-7-[3-(4-hydroxymethyl-phenoxymethyl)-phenyl]-non-6-en-3-ol.

8. Verbindungen nach Anspruch 1, **dadurch gekennzeichnet, dass** sie mindestens eines der folgenden Merkmale aufweisen:
- R₁ bedeutet eine Gruppe -(CH₂)ₙOH,
- R₂ bedeutet eine Gruppe -(CH₂)ₙOH,
- X-Y bedeutet eine Bindung der Formel (a) oder (b),
- R₃ bedeutet eine Kette mit 4 bis 8 Kohlenstoffatomen, die mit mindestens einer Hydroxygruppe und/oder einer geradkettigen oder verzweigten Alkylgruppe mit 1 bis 6 Kohlenstoffatomen substituiert ist.

9. Verbindung nach einem der vorhergehenden Ansprüche als Arzneimittel.

10. Verwendung einer Verbindung nach Anspruch 9 zur Herstellung eines Arzneimittels zur Behandlung von dermatologischen Affektionen, die mit einer Störung der Differenzierung oder der Proliferation der Keratinocyten oder der Sebocyten in Zusammenhang stehen, zur Behandlung von Acne vulgaris, Komedonenakne, polymorpher Akne, Rosazea, nodulocystischen Aknen, Acne conglobata, senilen Aknen, sekundären Aknen, Sonnenakne, medikamentöser Akne oder Berufsakne; Ichthyosen, ichthyosiformen Zuständen, Darier-Krankheit, palmoplantaren Keratodermien, Leukoplasien und leukoplasiformen Zuständen, Lichen der Haut oder der Schleimhäute (bukkal); dermatologischen Affektionen, die mit einer Störung der Keratinisierung mit einer entzündlichen und/oder immunoallergischen Komponente in Zusammenhang stehen, allen Formen der Psoriasis, also der Psoriasis der Haut, der Schleimhäute oder der Nägel, psoriatischem Rheuma, Hautatopie, Ekzem, respiratorischer Atopie, Zahnfleischhypertrophie; entzündlichen Affektionen, bei denen keine Störung der Keratinisierung vorliegt; benignen oder malignen Proliferationen der Haut oder der Epidermis nichtviralen oder viralen Ursprungs, gewöhnlichen Warzen, Flachwarzen und warzenähnlichen Epidermodysplasien, oralen und floriden Papillomatosen und Proliferationen, die durch UV-Strahlung hervorgerufen werden können, Epitheliomen der unteren Zellschicht und spinocellulären Epitheliomen; bullösen Dermatosen und Kollagenerkrankungen; lichtinduzierter oder altersbedingter Hautalterung, aktinischen Pigmentationen und Keratosen, allen mit altersbedingter oder aktinischer Alterung verbundenen pathologischen Zuständen; Störungen der Narbenbildung oder Schwangerschaftsstreifen; Störungen der Talgbildungsfunktion, Akne-Hyperseborrhoe, einfacher Seborrhoe, seborrhoischem Ekzem; ophthalmologischen Störungen, Corneopathien; cancerösen oder präcancerösen Zuständen von Krebsformen, bei denen Rezeptoren für Vitamin D vorliegen oder die durch Rezeptoren für Vitamin D induziert werden können, Brustkrebs, Leukämie, myelodysplastischen Syndromen und Lymphomen, Carcinomen von Zellen des Malpighi-Epitels und gastrointestinalen Krebsarten, Melanomen und Osteosarkomen, entzündlichen Affektionen, Arthritis oder rheumatoider Polyarthritis; Affektionen viralen Ursprungs im Hautbereich oder in generalisierter Form; Alopezie unterschiedlicher Genese, Alopezie aufgrund von Chemotherapie oder Bestrahlung; dermatologischen oder allgemeinen Affektionen mit immunologischer Komponente; Immunerkrankungen, Autoimmunerkrankungen, Diabetes Typ 1, Plattensklerose, Lupus und Affektionen vom Lupus-Typ, Asthma oder Glomerulonephritis; selektiven Dysfunktionen des Immunsystems; endokrinen Erkrankungen; Affektionen, die durch einen anomalen interzellulären Calciumhaushalt gekennzeichnet sind; Vitamin-D-Mangel und Störungen der Homöostase von Mineralstoffen im Plasma und in den Knochen, Rachitis, Osteomalazie, Osteoporose, Osteoporose bei Frauen nach der Menopause, renaler Osteodystrophie oder Störungen der Nebenschilddrüsenfunktion.

11. Pharmazeutische Zusammensetzung, **dadurch gekennzeichnet, dass** sie in einem pharmazeutisch akzeptablen Träger mindestens eine der Verbindungen wie in einem der Ansprüche 1 bis 18 definiert enthält.

12. Zusammensetzung nach Anspruch 11, **dadurch gekennzeichnet, dass** die Konzentration an Verbindung(en) nach einem der Ansprüche 1 bis 8 im Bereich von 0,0001 % bis 5 Gew.-%, bezogen auf das Gesamtgewicht der Zusammensetzung, liegt.

13. Kosmetische Zusammensetzung, **dadurch gekennzeichnet, dass** sie in einem kosmetisch akzeptablen Träger mindestens eine Verbindung wie in einem der Ansprüche 1 bis 8 definiert enthält.

14. Zusammensetzung nach Anspruch 13, **dadurch gekennzeichnet, dass** die Konzentration an Verbindung(en) im Bereich von 0,001 bis 3 Gew.-%, bezogen auf das Gesamtgewicht der Zusammensetzung, liegt.

15. Verwendung einer kosmetischen Zusammensetzung wie in Anspruch 13 oder 14 definiert zur Körper- oder Haarhygiene.

## Claims

1. Compounds, **characterized in that** they correspond to the following general formula (I): 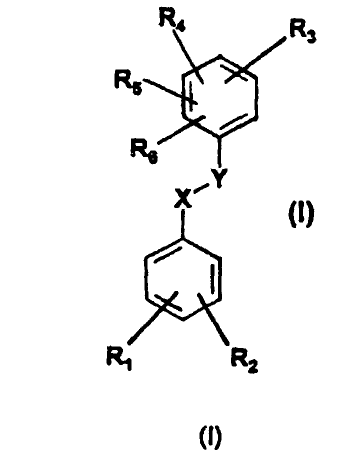 in which:
- R₁ represents a hydrogen atom, a methyl radical or a radical -(CH₂)ₙ-OR₇,
- R₂ represents a radical -(CH₂)ₙ-OR₈,
n, R₇ and R₈ having the meanings given below,
- X-Y represents a bond chosen from the bonds of the following formulae (a) and (b) which may be read from the left to the right or conversely: 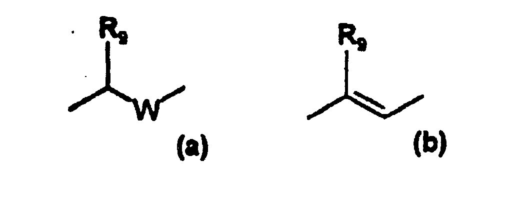 R₉ and W having the meanings given below,
- R₃ represents the chain in vitamin D₂ or in vitamin D₃, 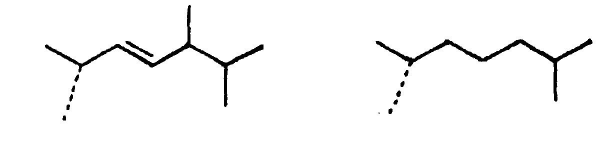 the dotted lines represent the bond linking the chain to the benzene ring represented in Figure (I),
or R₃ represents a chain having from 4 to 8 carbon atoms which is substituted with one or more hydroxyl groups, it being possible for the hydroxyl groups to be protected in the form of acetoxy, methoxy or ethoxy, trimethylsilyloxy, tert-butyldimethylsilyloxy, tetrahydropyranyloxy and optionally in addition:
- which is substituted with one or more linear or branched alkyl groups having from 1 to 6 carbon atoms or cycloalkyl groups and/or
- which is substituted with one or more halogen atoms and/or
- which is substituted with one or more CF₃ groups and/or
- in which one or more carbon atoms of the chain are replaced by one or more oxygen, sulphur or nitrogen atoms, it being possible for the nitrogen atoms to be optionally substituted with linear or branched alkyl radicals having from 1 to 6 carbon atoms and/or
- in which one or more single bonds of the chain are replaced by one or more double and/or triple bonds,
- R₃ being positioned on the benzene ring at the *para* or *meta* position with respect to the X-Y bond,
- R₄, R₅ and R₆, which are identical or different, represent a hydrogen atom, a linear or branched alkyl radical having from 1 to 6 carbon atoms, a halogen atom, a radical -OR₁₀, a polyether radical having from 2 to 5 carbon atoms interrupted by at least 2 oxygen atoms,
R₁₀ having the meaning given below,
- n being 0, 1 or 2,
- R₇ and R₈, which are identical or different, represent a hydrogen atom, an acetyl radical, a trimethylsilyl radical, a tert-butyldimethylsilyl radical, a tetrahydropyranyl radical,
- R₉ represents a hydrogen atom or a linear or branched alkyl radical having from 1 to 6 carbon atoms,
- W represents an oxygen atom, a sulphur atom, a radical -CH₂- or a radical -NH- which may be optionally substituted with a linear or branched alkyl radical having from 1 to 6 carbon atoms,
- R₁₀ represents a hydrogen atom or a linear or branched alkyl radical having from 1 to 6 carbon atoms,
and the optical and geometric isomers of the said compounds of formula (I) as well as their salts.

2. Compounds according to Claim 1, **characterized in that** they are provided in the form of salts of an inorganic or organic acid, in particular hydrochloric, sulphuric, acetic, fumaric, hemisuccinic, maleic and mandelic acid.

3. Compounds according to either of Claims 1 and 2, **characterized in that** the linear or branched alkyl radicals having from 1 to 6 carbon atoms are chosen from methyl, ethyl, isopropyl, tert-butyl and hexyl radicals.

4. Compounds according to one of the preceding claims, **characterized in that** the cycloalkyl radical corresponds to an adamantyl radical or a 1-methylcyclohexyl radical.

5. Compounds according to one of the preceding claims, **characterized in that** the halogen atom corresponds to a fluorine, chlorine or bromine atom.

6. Compounds according to any one of the preceding claims, **characterized in that** the polyether radical corresponds to a methoxymethoxy, methoxyethoxy or methoxyethoxymethoxy radical.

7. Compounds according to Claim 1, **characterized in that** they are chosen, alone or in the form of mixtures, from the group consisting of:
3-Hydroxymethyl-5-{2-[3-(5-hydroxy-5-methylhexyl)phenyl]vinyl}phenol,
3-Hydroxymethyl-5-{2-[3-(6-hydroxy-6-methylheptyl)phenyl]vinyl}phenol,
3-[3-(5-Hydroxy-1,5-dimethylhexyl)phenoxymethyl]-5-hydroxymethylphenol,
6-[3-(3,4-Bis-hydroxymethylbenzyloxy)phenyl]-2-methylhepta-3,5-dien-2-ol,
6-[3-(3,4-Bis-hydroxymethylbenzyloxy)phenyl]-2-methylhexan-2-ol,
6-[3-(3,4-Bis-hydroxymethylphenoxymethyl)phenyl]-2-methylheptan-2-ol,
7-[3-(3,4-Bis-hydroxymethylphenoxymethyl)phenyl]-3-ethyloctan-3-ol,
5-{2-[4-(5-Hydroxy-5-methylhexyl)phenyl]vinyl}benzene-1,3-diol,
5-{2-[4-(5-Hydroxy-5-methylhexyl)phenyl]ethyl}benzene-1,3-diol,
5-{2-[4-(6-Hydroxy-6-methylheptyl)phenyl]vinyl}benene-1,3-diol,
5-{2-[3-(6-Hydroxy-6-methylheptyl)phenyl]vinyl}benene-1,3-diol,
5-{2-[4-(6-Hydroxy-6-methylheptyl)phenyl]ethyl}benzene-1,3-diol,
5-{2-[3-(6-Hydroxy-6-methylheptyl)phenyl]ethyl}benzene-1,3-diol,
2-Hydroxymethyl-4-{2-[3-(5-hydroxy-5-methylhexyl)-phenyl]vinyl}phenol,
2-Hydroxymethyl-4-{2-[4-(5-hydroxy-5-methylhexyl)-phenyl]vinyl}phenol,
2-Hydroxymethyl-4-{2-[3-(6-hydroxy-6-methylheptyl)-phenyl]vinyl}phenol,
2-Hydroxymethyl-4-{2-[4-(6-hydroxy-6-methylheptyl)-phenyl]vinyl}phenol,
2-Hydroxymethyl-4-{2-[3-(5-hydroxy-5-methylheptyl)-phenyl]ethyl}phenol,
2-Hydroxymethyl-4-{2-[4-(5-hydroxy-5-methylhexyl)-phenyl]ethyl}phenol,
2-Hydroxymethyl-4-{2-[3-(6-hydroxy-6-methylheptyl)-phenyl]ethyl}phenol,
2-Hydroxymethyl-4-{2-[4-(6-hydroxy-6-methylheptyl)-phenyl]ethyl}phenol,
2-Hydroxymethyl-5-{2-[4-(5-hydroxy-5-methylhexyl)-phenyl]vinyl}phenol,
6-[3-(3,4-Bis-hydroxymethylbenzyloxy)phenyl]-2-methylheptan-2-ol,
4-[3-(5-Hydroxy-1,5-dimethylhexyl)phenoxymethyl]-2-hydroxymethylphenol,
6-{3-[2-(3,4-Bis-hydroxymethylphenyl)vinyl]phenyl}-2-methylhexan-2-ol,
7-{4-[2-(3,4-Bis-hydroxymethylphenyl)vinyl]phenyl}-2-methylheptan-2-ol,
6-{4-[2-(3,4-Bis-hydroxymethylphenyl)vinyl]phenyl}-2-methylhexan-2-ol,
5-{2-[3-(6-Hydroxy-6-methylheptyl)phenyl]-1-methylvinyl}benzene-1,3-diol,
5-{2-[3-(5-Hydroxy-5-methylhexyl)phenyl]vinyl}benzene-1,3-diol,
5-[3-(6-Hydroxy-6-methylheptyl)phenoxymethyl]benzene-1,3-diol,
5-{2-[3-(7-Hydroxy-7-methyloct-1-enyl)phenyl]vinyl}benzene-1,3-diol,
5-{2-[3-(7-Hydroxy-7-methyloctyl)phenyl]vinyl}benzene-1,3-diol,
4-{2-[3-(6-Hydroxy-6-methylheptyl)phenyl]vinyl}benzene-1,2-diol,
3-{2-[3-(6-Hydroxy-6-methylheptyl)phenyl]vinyl}phenol,
6-{3-[2-(3,5-Bis-hydroxymethylphenyl)vinyl]phenyl}-2-methylhexan-2-ol,
3-{2-[3-(7-Hydroxy-7-methyloctyl)phenyl]vinyl}phenol,
7-{3-[2-(3,5-Bis-hydroxymethylphenyl)vinyl]phenyl}-2-methylheptan-2-ol,
7-{3-[2-(3,4-Bis-hydroxymethylphenyl)vinyl]phenyl}-2-methylheptan-2-ol,
7-{3-[2-(4-Hydroxymethylphenyl)vinyl]phenyl}-2-methylheptan-2-ol,
4-{2-[3-(7-Hydroxy-7-methyloct-1-enyl)phenyl]-vinyl}benzene-1,2-diol,
5-{2-[3-(6-Hydroxy-6-methylhept-1-enyl)phenyl]vinyl}benzene-1,3-diol,
5-{2-[3-(7-Ethyl-7-hydroxynon-1-enyl)phenyl]vinyl}benzene-1,3-diol,
5-{2-[3-(7-Hydroxy-1-methoxy-1,7-dimethyloctyl)phenyl]vinyl}benzene-1,3-diol,
5-{2-[3-(6-Hydroxy-1-methoxy-1,6-dimethylheptyl)-phenyl]vinyl}benzene-1,3-diol,
5-{2-[3-(5-Hydroxypentyl)phenyl]vinyl}benzene-1,3-diol,
5-{2-[3-(5-Hydroxy-6-methylheptyl)phenyl]vinyl}benzene-1,3-diol,
5-{2-[3-(6-Hydroxy-7-methyloctyl)phenyl]vinyl}benzene-1,3-diol,
5-{2-[3-(5-Hydroxy-6-methylhept-1-enyl)phenyl]vinyl}benzene-1,3-diol,
5-{2-[3-(6-Hydroxy-7-methyloct-1-enyl)phenyl]vinyl}benzene-1,3-diol,
5-{2-[3-(1,6-Dihydroxy-1,6-dimethylheptyl)phenyl]-vinyl}benzene-1,3-diol,
5-{2-[3-(6-Hydroxy-1,6-dimethylhept-1-enyl)phenyl]-vinyl}benzene-1,3-diol,
6-{[3-(3,4-Bis-hydroxymethylbenzyloxy)phenyl]-methylamino}-2-methylhexan-2-ol,
5-{[3-(3,4-Bis-hydroxymethylbenzyloxy)phenyl]-methylamino}-2-methylpentan-2-ol,
6-{[3-(3,4-Bis-hydroxymethylbenzyloxy)phenyl]-methylamino}-3-ethylhexan-3-ol,
7-{[3-(3,4-Bis-hydroxymethylbenzyloxy)phenyl]-methylamino}-3-ethylheptan-3-ol,
5-{[3-(3,4-Bis-hydroxymethylbenzyloxy)phenyl]-ethylamino}-2-methylpentan-2-ol,
6-{[3-(3,4-Bis-hydroxymethylbenzyloxy)phenyl]-benzylamino}-3-ethylhexan-3-ol,
(4E,6E)-7-{3-[2-(3,4-Bis-hydroxymethylphenyl)ethyl]-phenyl}-3-ethylocta-4,6-dien-3-ol,
(3E,5E)-6-{3-[2-(3,4-Bis-hydroxymethylphenyl)ethyl]-phenyl}-2-methylhepta-3,5-dien-2-ol,
(4E,6E)-7-{3-[2-(3,4-Bis-hydroxymethylphenyl)vinyl]-phenyl}-3-ethylocta-4,6-dien-3-ol,
(3E,5E)-6-{3-[2-(3,4-Bis-hydroxymethylphenyl)vinyl]-phenyl}-2-methylhepta-3,5-dien-2-ol,
7-[3-(3,4-Bis-hydroxymethylbenzyloxy)phenyl]-3-ethyl-3-octanol,
(4E,6E)-7-[3-(3,4-Bis-hydroxymethylbenzyloxy)-phenyl]-3-ethylocta-4,6-dien-3-ol,
(4E,6Z)-7-[3-(3,4-Bis-hydroxymethylbenzyloxy)phenyl]-3-ethylocta-4,6-dien-3-ol,
7-[4-(3,4-Bis-hydroxymethylbenzyloxy)phenyl]-3-ethyl-3-octanol,
(4E,6E)-7-[3-(3,4-Bis-hydroxymethylbenzyloxy)phenyl]-3-ethylnona-4,6-dien-3-ol,
(4E,6Z)-7-[3-(3,4-Bis-hydroxymethylbenzyloxy)phenyl]-3-ethylnona-4,6-dien-3-ol,
(E)-6-[3-(3,4-Bis-hydroxymethylbenzyloxy)phenyl]-2-methylhept-3-en-2-ol,
(E)-7-[3-(3,4-Bis-hydroxymethylbenzyloxy)phenyl]-3-ethyloct-4-en-3-ol,
(E)-7-[3-(3,4-Bis-hydroxymethylbenzyloxy)phenyl]-3-ethyloct-6-en-3-ol,
(Z)-7-[3-(3,4-Bis-hydroxymethylbenzyloxy)phenyl]-3-ethyloct-6-en-3-ol,
(E)-8-[3-(3,4-Bis-hydroxymethylbenzyloxy)phenyl]-2-methylnon-7-en-2-ol,
(Z)-8-[3-(3,4-Bis-hydroxymethylbenzyloxy)phenyl]-2-methylnon-7-en-2-ol,
(E)-9-[3-(3,4-Bis-hydroxymethylbenzyloxy)phenyl]-3-ethyldec-8-en-3-ol,
(Z)-9-[3-(3,4-Bis-hydroxymethylbenzyloxy)phenyl]-3-ethyldec-8-en-3-ol,
8-[3-(3,4-Bis-hydroxymethylbenzyloxy)phenyl]-2-methyl-2-nonanol,
9-[3-(3,4-Bis-hydroxymethylbenzyloxy)phenyl]-3-ethyl-decan-3-ol,
(E)-7-[3-(3,4-Bis-hydroxymethylbenzyloxy)phenyl]-3-ethyloct-6-en-4-yn-3-ol,
(3E,5E)-7-[3-(3,4-Bis-hydroxymethylbenzyloxy)phenyl]-2,7-dimethylocta-3,5-dien-2-ol,
(4E,6E)-7-[3-(3,4-Bis-hydroxymethylphenoxymethyl)-phenyl]-3-ethylocta-4,6-dien-3-ol,
(3E,5E)-6-[3-(3,4-Bis-hydroxymethylphenoxymethyl)-phenyl]-2-methylhepta-3,5-dien-2-ol,
(Z)-7-[3-(3,4-Bis-hydroxymethylbenzyloxy)phenyl]-3-ethylnon-6-en-3-ol,
(Z)-6-[3-(3,4-Bis-hydroxymethylbenzyloxy)phenyl]-2-methyloct-5-en-2-ol,
(Z)-7-[3-(3,4-Bis-hydroxymethylbenzyloxy)phenyl]-3-ethyldec-6-en-3-ol,
(Z)-6-[3-(3,4-Bis-hydroxymethyl-benzyloxy)-phenyl]-2-methylnon-5-en-2-ol,
(Z)-8-[3-(3,4-Bis-hydroxymethylbenzyloxy)phenyl]-3-ethylnon-7-en-3-ol,
(E)-8-[3-(3,4-Bis-hydroxymethylbenzyloxy)phenyl]-3-ethylnon-7-en-3-ol,
8-[3-(3,4-Bis-hydroxymethylbenzyloxy)phenyl]-3-ethylnonan-3-ol,
(4E,6E)-7-[5-(3,4-Bis-hydroxymethylbenzyloxy)-2-methoxyphenyl]-3-ethylnona-4,6-dien-3-ol,
(4E,6E)-7-[5-(3,4-Bis-hydroxymethylbenzyloxy)-2-methyl-phenyl]-3-ethylnona-4,6-dien-3-ol,
(4E,6E)-7-[3-(3,4-Bis-hydroxymethylbenzyloxy)-5-methoxyphenyl]-3-ethylnona-4,6-dien-3-ol,
(4E,6E)-7-[3-(3,4-Bis-hydroxymethylbenzyloxy)-2-methoxyphenyl]-3-ethylnona-4,6-dien-3-ol,
(4E,6E)-7-[3-(3,4-Bis-hydroxymethylbenzyloxy)-4-methyl-phenyl]-3-ethylnona-4,6-dien-3-ol,
1-[3-(3,4)-Bis-hydroxymethylbenzyloxy)phenyl]ethanone O-(2-hydroxy-2-methylpropyl)oxime,
1-{1-[3-(3,4-Bis-hydroxymethylbenzyloxy)phenyl]-propoxy}-3-ethylpentan-3-ol,
(E)-7-[3-(3,4-Bis-hydroxymethylphenoxymethyl)phenyl]-3-ethylnon-6-en-3-ol,
(E)-7-[3-(3,4-Bis-hydroxymethylbenzylsulfanyl)phenyl]-3-ethyloct-6-en-3-ol,
(E)-7-{3-[(3,4-Bis-hydroxymethylbenzyl)methylamino]-phenyl}-3-ethyloct-6-en-3-ol,
(E)-6-[3-(3,4-Bis-hydroxymethylbenzyloxy)phenyl]-3-ethyl-6-methylhept-4-en-3-ol,
7-[3-(3,4-Bis-hydroxymethylbenzyloxy)phenyl]-3-ethyl-7-methyloctan-3-ol,
7-[3-(3,4-Bis-hydroxymethylbenzyloxy)phenyl]-3,7-diethylnonan-3-ol,
(E)-6-[3-(3,4-Bis-hydroxymethylbenzyloxy)phenyl]-1,1,1-trifluoro-2-trifluoromethyloct-5-en-2-ol,
2-{4-[3-(3,4-Bis-hydroxymethylbenzyloxy)phenyl)hexyl}-1,1,1,3,3,3-hexafluoropropan-2-ol,
(E)-7-[3-(3,4-Bis-hydroxymethylbenzyloxy)phenyl]-3-ethyl-4,4-difluoronon-6-en-3-ol,
7-[3-(3,4-Bis-hydroxymethylbenzyloxy)phenyl]-3-ethyl-4,4-difluoro-7-methyloctan-3-ol,
(E)-6-[3-(3,4-Bis-hydroxymethylbenzyloxy)phenyl]-2-methyloct-5-en-3-ol,
(E)-4-[3-(3,4-Bis-hydroxymethylbenzyloxy)phenyl]-1-cyclopropylhex-3-en-1-ol,
(E)-7-[3-(3,4-Bis-hydroxymethylbenzyloxy)phenyl]-3-ethyl-4-methylnon-6-en-3-ol,
(E)-7-[3-(3,4-Bis-hydroxymethylbenzyloxy)phenyl]-3-ethyl-4-methyldec-6-en-3-ol,
(4E,6E)-7-[3-(3,4-Bis-hydroxymethylphenoxymethyl)-phenyl]-3-ethylnona-4,6-dien-3-ol,
(4E,6E)-7-{3-[2-(3,4-Bis-hydroxymethylphenyl)ethyl]-phenyl}-3-ethylnona-4,6-dien-3-ol,
(E)-7-{3-[2-(3,4-Bis-hydroxymethylphenyl)ethyl]phenyl}-3-ethylnon-6-en-3-ol,
(E)-3-Ethyl-7-[3-(3-hydroxymethylphenoxymethyl)-phenyl]non-6-en-3-ol,
(E)-3-Ethyl-7-[3-(4-hydroxymethylphenoxymethyl)phenyl]-non-6-en-3-ol,
(E)-3-Ethyl-7-[(E)-3-(3-hydroxymethyl-4-methyl-phenoxymethyl)phenyl]non-6-en-3-ol,
(E)-3-Ethyl-7-[(E)-3-(4-hydroxymethyl-3-methyl-phenoxymethyl)phenyl]non-6-en-3-ol,
2-{4-[3-(3,4-Bis-hydroxymethylbenzyloxy)phenyl]-4-methylpentyl}-1,1,1,3,3,3-hexafluoropropan-2-ol,
7-[3-(3,4-Bis-hydroxymethylbenzyloxy)phenyl]-3-ethyl-4,4-difluoro-7-methyl-octan-3-ol,
7-{3-[2-(3,4-Bis-hydroxymethylphenyl)ethyl]phenyl}-3,7-diethylnonan-3-ol
7-[3-(3,4-Bis-hydroxymethylbenzyloxy)phenyl]-3-ethyl-7-methyloctane-3,4-diol
7-{3-[2-(3,4-Bis-hydroxymethylphenyl)ethyl]phenyl}-3,7-diethylnonane-3,4-diol
7-{3-[2-(3,4-Bis-hydroxymethylphenyl)ethyl]phenyl}-3-ethyl-7-methyloctane-3,4-diol
(E)-4-{3-[2-(3,4-Bis-hydroxymethylphenyl)ethyl]phenyl}-1-cyclopropylhex-3-en-1-ol
(4E,6E)-7-{3-[2-(3,4-Bis-hydroxymethylphenyl)ethyl]phenyl}-3-ethyl-4-methylnona-4,6-dien-3-ol
(4E,6E)-7-[3-(3,4-Bis-hydroxymethylbenzyloxy)phenyl]-3-ethyl-4-methylnona-4,6-dien-3-ol
(E)-3-Ethyl-7-[3-(4-hydroxymethyl-3-methylphenoxymethyl)phenyl]non-6-en-3-ol,
(E)-3-Ethyl-7-[3-(3-hydroxymethyl-4-methylphenoxymethyl)phenyl]non-6-en-3-ol,
(E)-7-[3-(3,4-Bis-hydroxymethylphenoxymethyl)phenyl]-3-ethyloct-6-en-3-ol,
(E)-3-Ethyl-7-[3-(3-hydroxymethylphenoxymethyl)phenyl]-non-6-en-3-ol,
(E)-3-Ethyl-7-[3-(4-hydroxymethylphenoxymethyl)phenyl]-non-6-en-3-ol.

8. Compounds according to Claim 1, **characterized in that** they exhibit at least one of the following characteristics:
- R₁ represents the radical -(CH₂)ₙOH,
- R₂ represents the radical -(CH₂)ₙOH,
- X-Y represents a bond of formula (a) or (b),
- R₃ represents a chain of 4 to 8 carbon atoms which is substituted with at least one hydroxyl radical and/or one linear or branched alkyl radical having from 1 to 6 carbon atoms.

9. Compound according to any one of the preceding claims, as a medicament.

10. Use of a compound according to Claim 9, for producing a medicament for treating dermatological conditions linked to a keratinocyte or sebocyte differentiation or proliferation disorder, for treating acne vulgaris, comedo-type acne, polymorphic acne, acne rosacea, nodulocystic acne, acne conglobata, senile acne, secondary acne, solar acne, acne medicamentosa or occupational acne; ichthyosis, ichthyosiform states, Darier's disease, keratosis palmaris et plantaris, leukoplasia, leukoplasiform states, cutaneous or mucosal (buccal) lichen; for treating dermatological conditions linked to a keratinization disorder with an inflammatory and/or immunoallergic component, all the forms of psoriasis, whether cutaneous, mucosal or ungual, psoriatic rheumatism, cutaneous atopy, eczema, respiratory atopy, gingival hypertrophy; for treating inflammatory conditions which do not exhibit keratinization disorders; for treating any dermal or epidermal proliferations whether benign or malignant, whether of viral origin or not, verruca vulgaris, verruca plana and epidermodysplasia verruciformis, oral or florid papillomatoses and proliferations which may be induced by ultraviolet radiation, baso- and spinocellular epithelioma; for treating bullous dermatoses and collagen diseases; for treating skin ageing, whether photoinduced or chronologic, pigmentations and actinic keratoses, any pathologies associated with chronologic or actinic ageing; for treating cicatrization disorders or vibices; for treating disorders of the sebaceous function, hyperseborrhoea of acne, simple seborrhoea, seborrhoeic eczema; for treating ophthalmological disorders, corneopathies; for treating cancerous or precancerous states of cancers exhibiting or capable of being induced by vitamin D receptors, breast cancer, leukaemia, myelodysplasic syndromes and lymphomas, carcinomas of the cells of the Malpighian epithelium and gastrointestinal cancers, melanomas and osteosarcoma; for treating imflammatory conditions, arthritis or rheumatoid arthritis; for treating conditions of viral origin at the cutaneous level or in general; for treating alopecia of various origins, alopecia due to chemotherapy or to radiation; for treating dermatological or general conditions with an immunological component; for treating immunological conditions, autoimmune diseases, type 1 diabetes mellitus, multiple sclerosis, lupus and lupus-type conditions, asthma, glomerulonephritis; for treating selective dysfunctions of the immune system; for treating hormonal conditions; for treating conditions **characterized by** an abnormal management of intracellular calcium; for treating vitamin D deficiencies and conditions of the homeostasis of the minerals in the plasma and the bones, rickets, osteomalacia, osteoporosis, menopausal women osteoporosis, renal osteodystrophy or parathyroid function disorders.

11. Pharmaceutical composition, **characterized in that** it comprises, in a pharmaceutically acceptable carrier, at least one of the compounds as defined in any one of Claims 1 to 8.

12. Composition according to Claim 11, **characterized in that** the concentration of compound(s) according to one of Claims 1 to 8 is between 0.0001% and 5% by weight relative to the total weight of the composition.

13. Cosmetic composition, **characterized in that** it comprises, in a cosmetically acceptable carrier, at least one of the compounds as defined in any one of Claims 1 to 8.

14. Composition according to Claim 13, **characterized in that** the concentration of compound(s) is between 0.001% and 3% by weight relative to the total weight of the composition.

15. Use of a cosmetic composition as defined in either of Claims 13 and 14, for body or hair care.
